# EUROPEAN PATENT SPECIFICATION

(11) **EP 2 986 598 B1**
(45) Date of publication and mention of the grant of the patent: **29.03.2017**
(21) Application number: 14718046.7
(22) Date of filing: 15.04.2014
(51) Int. Cl.: A01N 43/40, A01N 43/54, A01N 43/56, A01N 43/78, A01N 43/80, C07D 213/75, C07D 401/06, C07D 405/06, C07D 413/04, C07D 413/06, C07D 417/06, A01P 7/04

(54) **N-SUBSTITUTED ACYL-IMINO-PYRIDINE COMPOUNDS AND DERIVATIVES FOR COMBATING ANIMAL PESTS**
N-SUSTITUIERTE ACYL-IMINO-PYRIDIN-VERBINDUNGEN UND DEREN DERIVATE ZUR BEKÄMPFUNG VON TIERISCHEN SCHÄDLINGEN
COMPOSÉS ACYL-IMINO-PYRIDINE N-SUBSTITUÉS ET DÉRIVÉS POUR LUTTER CONTRE LES ANIMAUX NUISIBLES

(30) Priority: 19.04.2013 US 201361813690 P
(43) Date of publication of application: 24.02.2016
(73) Proprietor: BASF SE, 67056 Ludwigshafen am Rhein (DE)
(72) Inventor: BANDUR, Nina, Gertrud, 67063 Ludwigshafen (DE); MCLAUGHLIN, Martin, John, 67098 Bad Dürkheim (DE); POHLMAN, Matthias, 67251 Freinsheim (DE)
(86) International application number: PCT/EP2014/057573
(87) International publication number: WO 2014/170300

(56) References cited:
- EP-A1- 0 639 569
- EP-A2- 0 432 600
- CA-A1- 2 808 144

## Description

The present invention relates to N-substituted acyl-imino-pyridine compounds, to the enantiomers, diastereomers, derivatives and salts thereof and to compositions comprising such compounds. The invention also relates to the use of the N-substituted acyl-imino-pyridine compounds, of their salts or of compositions comprising them for combating animal pests. Furthermore the invention relates also to methods of applying such compounds.

Animal pests destroy growing and harvested crops and attack wooden dwelling and commercial structures, causing large economic loss to the food supply and to property. While a large number of pesticidal agents are known, due to the ability of target pests to develop resistance to said agents, there is an ongoing need for new agents for combating animal pests. In particular, animal pests such as insects and acaridae are difficult to be effectively controlled.

It is therefore an object of the present invention to provide compounds having a good pesticidal activity, especially against difficult to control insects and acaridae.

It has been found that these objects are solved by N-substituted acyl-imino-pyridine derivatives of the general formula I: 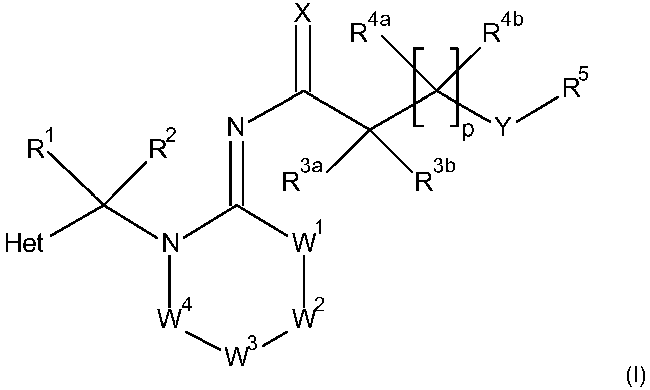 wherein
- p: is an integer selected from 0, 1, 2, 3, 4, 5 or 6;
- X: is O or S;

- Y: is O or S(O)ₘ, wherein m is 0, 1, 2;
- Het: is a 5 or 6 membered carbon-bound or optionally nitrogen-bound heterocyclic or heteroaromatic ring system, each ring members selected from carbon atoms and at least one, up to three heteroatoms independently selected from sulfur, oxygen or nitrogen, wherein the carbon, sulfur and nitrogen ring members can independently be partly or fully oxidized, and wherein each ring is optionally substituted by k substituents selected from R^{6a}, wherein k is an integer selected from 1, 2, 3, 4, or 5, and two or more substituents R^{6a} are selected independently from one another;
- W¹, W², W³ and W⁴: represent a carbon chain group connected to N and C=N, and thus forming a saturated, unsaturated, or partially unsaturated 5 or 6 membered nitrogen containing heterocycle,
wherein W¹, W², W³ and W⁴ each individually represent CR^{w}, wherein each
R^{w} is selected independently from one another from hydrogen, halogen, cyano, azido, nitro, SCN, SF₅, C₁-C₁₀-alkyl, C₃-C₈-cycloalkyl, C₂-C₁₀-alkenyl, C₂-C₁₀-alkinyl, and wherein the carbon atoms of the aforementioned aliphatic and cyclo-aliphatic radicals may be unsubstituted or partly or compeletly halogenated, or may optionally be further substituted independently from one another with one or more R⁷;
OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=S)OR⁸, C(=S)SR⁸, C(=NR^{9a})R⁷, C(=NR^{9a})NR^{9a}R^{9b}, Si(R¹¹)₂R¹²;
phenyl, optionally substituted with with 1, 2, 3, 4 or 5 substituents selected independently from R¹⁰;
a 3-, 4-, 5-, 6- or 7- membered saturated, partly saturated or unsaturated aromatic heterocyclic ring comprising 1, 2, 3 or 4 heteroatoms selected from oxygen, nitrogen and/or sulfur, optionally substituted with 1, 2, 3, 4 or 5 substituents selected independently from R¹⁰, and wherein the nitrogen and/or the sulfur atom(s) of the heterocyclic ring may optionally be oxidized;
or
two of R^{w} present on one ring carbon atom may together form =O, =CR¹³R¹⁴; =S;, =NR^{17a}, =NOR¹⁶;=NNR^{17a};
or two R^{w} of adjacent carbon atoms, may form both together and together with the existing bond a double bond between the adjacent carbon atoms;
and wherein
one of W² or W³ may optionally represent a single or a double bond between the adjacent carbon atoms;
- R¹, R²: are independently from each other selected from the group consisting of hydrogen, halogen, CN, SCN, nitro, C₁-C₆-alkyl, C₃-C₆-cycloalkyl, wherein each of the aforementioned aliphatic carbon chain radicals are unsubstituted, partly or completely halogenated or may carry any combination of one or more radicals R⁷;
Si(R¹¹)₂R¹², OR¹⁶, OSO₂R¹⁶, S(O)ₙR¹⁶, S(O)ₙNR^{17a}R^{17b}, NR^{17a}R^{17b}, C(=O)NR^{17a}R^{17b}, C(=S)NR^{17a}R^{17b}, C(=O)OR¹⁶, C(=O)R¹⁵, C(=S)R¹⁵, phenyl, optionally substituted with one or more, e.g. 1, 2, 3, 4 or 5 substituents R¹⁰, which are independently selected from one another,
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with 1, 2, 3 or 4, substituents R¹⁰, selected independently from one another, and wherein the nitrogen and/or the sulfur atom(s) of the heterocyclic ring may optionally be oxidized,
or
R¹ and R² form, together with the carbon atom, which they attached to, a 3- to 6-membered saturated or partly unsaturated carbocyclic or heterocyclic ring, wherein each of the carbon atoms of said cycle are unsubstituted or may carry any combination of 1 or 2 radicals R⁷,
or
R¹ and R² may together be =O, =CR¹³R¹⁴; =S;, =NR^{17a}, =NOR¹⁶;=NNR^{17a};
- R^{3a}, R^{3b}: are selected each independently from one another from the group consisting of hydrogen, halogen, CN, C₁-C₆-alkyl, C₁-C₆-alkoxy, C₁-C₆-alkylthio, C₁-C₆-alkylsulfinyl, C₁-C₆-alkylsulfonyl and C₃-C₈-cycloalkyl, wherein each of the six last mentioned radicals are unsubstituted, partly or completely halogenated, OR⁸, OSO₂R⁸, S(O)ₙR⁸, S(O)ₙNR^{9a}R^{9b}, NR^{9a}R^{9b}, C(=O)NR^{9a}R^{9b}, C(=S)NR^{9a}R^{9b}, C(=O)R⁷, C(=S)R⁷, phenyl, optionally substituted with one or more, e.g. 1, 2, 3, 4 or 5 substituents R¹⁰, which are independently selected from one another,
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with 1, 2, 3 or 4, substituents R¹⁰, selected independently from one another, and wherein the nitrogen and/or the sulfur atom(s) of the heterocyclic ring may optionally be oxidized,
or
wherein R^{3a} and R^{3b} may form together with the carbon atom they are bound to, a 3, 4 or 5 membered aliphatic ring, wherein each of the carbon atoms of the ring may be unsubstituted or may be partly or fully halogenated,
or
wherein R^{3a} and R^{3b} may together form =O, =CR¹³R¹⁴; =S;, =NR^{17a}, =NOR¹⁶;=NNR^{17a},
and, if p is 1 or more, then one of R^{3a} or R^{3b} may form a double bond with the R^{4a} or R^{4b} of the adjacent carbon atom
- R^{4a}, R^{4b}: are selected each independently from one another and independently from the integer of p from the group consisting of hydrogen, halogen, CN, C₁-C₆-alkyl, C₁-C₆-alkoxy, C₁-C₆-alkylthio, C₁-C₆-alkylsulfinyl, C₁-C₆-alkylsulfonyl and C₃-C₈-cycloalkyl, wherein each of the six last mentioned radicals are unsubstituted, partly or completely halogenated,
or
wherein R^{4a} and R^{4b} may form together with the carbon atom they are bound to, a 3, 4 or 5 membered aliphatic carbocyclic ring, wherein each of the carbon atoms of the ring may be unsubstituted or may be partly or fully halogenated,
or
wherein R^{4a} and R^{4b} may together form =O, =CR¹³R¹⁴; =S;, =NR^{17a}, =NOR¹⁶;=NNR^{17a},
or, if p is 1 or more,
one of R^{4a} or R^{4b} may for a double bond with R^{3a} or R^{3b} or with another R^{4a}, or R^{4b} of the adjacent carbon atom(s).
- R⁵: is selected from the group consisting of hydrogen, cyano, C₁-C₆-alkyl, C₇-C₁₂-alkyl, C₁-C₆-alkylthio, C₁-C₆-alkylsulfinyl, C₁-C₆-alkylsulfonyl, C₃-C₈-cycloalkyl, C₃-C₈-cycloalkylthio, C₃-C₈-cycloalkylsufinyl, C₃-C₈-cycloalkylsulfonyl ,C₂-C₆-alkenyl, C₂-C₆-alkenylthio, C₂-C₆-alkenylsulfinyl, C₂-C₆-alkenylsulfonyl, C₂-C₆-alkinyl, C₂-C₆-alkinylthio, C₂-C₆-alkinylsulfinyl and C₂-C₆-alkinylsulfonyl wherein the carbon chain atoms of the aforementioned aliphatic and cycloaliphatic last radicals are unsubstituted, partly or completely halogenated or may carry any combination of one or more radicals R⁷;
C(=O)NR^{9a}R^{9b}, C(=S)NR^{9a}R^{9b}, C(=O)R⁷, C(=S)R⁷; phenyl or CH₂-phenyl, optionally substituted with one or more, e.g. 1, 2, 3, 4 or 5 substituents R¹⁰, which are independently selected from one another;
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with q substituents R^{y}, selected independently from q and independently from one another, and wherein the nitrogen and/or the sulfur atom(s) of the heterocyclic ring may optionally be oxidized, wherein the heterocyclic ring may be bonded directly or linked via a CH₂ group to the remainder of the molecule, and wherein
q is an integer selected from 1, 2, 3 or 4, and
R^{y} is selected from the group conisisting of hydrogen, halogen, cyano, nitro, , C₁-C₁₀-alkyl, C₂-C₁₀-alkenyl, wherein the carbon atoms of the aforementioned aliphatic radicals may optionally be substituted with one or more R¹⁵, which are selected independently from one another, OR¹⁶, -S(O)ₙR¹⁶, S(O)ₙNR^{17a}R^{17b}, NR^{17a}R^{17b}, C(=O)R¹⁵, C(=O)OR¹⁶,-C(=NR^{17a})R¹⁵, C(=O)NR^{17a}R^{17b}, C(=S)NR^{17a}R^{17b},
or
two R^{y} present together on one atom of a partly saturated heterocyclic may be =O, =CR¹³R¹⁴;, =NR^{17a}, =NOR¹⁶ or =NNR^{17a};
or
two R^{y} on adjacent carbon atoms may be a bridge selected from CH₂CH₂CH₂CH₂, CH=CH-CH=CH, N=CH-CH=CH, CH=N-CH=CH, N=CH-N=CH, CH₂CH₂CH₂, CH=CHCH₂, CH₂CH₂NR^{17a}, CH₂CH=N, CH=CH-NR^{17a}, and form together with the carbon atoms to which the two R^{y} are bonded to a 5-membered or 6-membered fused partly saturated or unsaturated, aromatic carbocyclic or heteocyclic ring, wherein the ring may optionally be substituted with one or two substituents selected from =O, OH, CH₃, OCH₃, halogen, cyano, halomethyl or halomethoxy;
- R^{6a}: is selected from the group consisting of hydrogen, halogen, cyano, azido, nitro, SCN, SF₅, C₁-C₁₀-alkyl, C₃-C₈-cycloalkyl, C₂-C₁₀-alkenyl, C₂-C₁₀-alkinyl, and wherein the carbon atoms of the aforementioned aliphatic and cyclo-aliphatic radicals may optionally be further substituted independently from one another with one or more R⁷,
OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=S)OR⁸, C(=S)SR⁸, C(=NR^{9a})R⁷, C(=NR^{9a})NR^{9a}R^{9b}, Si(R¹¹)₂R¹²;
phenyl, optionally substituted with with 1, 2, 3, 4 or 5 substituents selected independently from R¹⁰;
a 3-, 4-, 5-, 6- or 7- membered saturated, partly saturated or unsaturated aromatic heterocyclic ring comprising 1, 2, 3 or 4 heteroatoms selected from oxygen, nitrogen and/or sulfur, optionally substituted with 1, 2, 3, 4 or 5 substituents selected independently from R¹⁰, and wherein the nitrogen and/or the sulfur atom(s) of the heterocyclic ring may optionally be oxidized;
or two of R^{6a} present on one ring carbon or sulfur atom may together form =O, =CR¹³R¹⁴; =S;, =NR^{17a}, =NOR¹⁶;=NNR^{17a};
or two R^{6a} together form a C₂-C₇ alkylene chain, thus forming, together with the ring atom(s) to which they are bound, a 3-, 4-, 5-, 6-, 7- or 8-membered ring, where the alkylene chain may be interrupted by 1 or 2 O, S and/or NR^{17a} and/or 1 or 2 of the CH₂ groups of the alkylene chain may be replaced by a group C=O, C=S and/or C=NR^{17a}; and/or the alkylene chain may be substituted by one or more radicals selected from the group consisting of halogen, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, phenyl which may be substituted by one or more, e.g. 1, 2, 3, 4 or 5 radicals R¹⁰, and a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, where the heterocyclic ring may be substituted by one or more radicals R¹⁰;
- R⁷: is each independently from one another selected from the group consisting of hydrogen, halogen, cyano, azido, nitro, -SCN, SF₅, C₁-C₆-alkyl, C₁-C₆-haloalkyl, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkinyl, C₂-C₆ haloalkinyl, Si(R¹¹)₂R¹², OR¹⁶, OSO₂R¹⁶, S(O)ₙR¹⁶, S(O)ₙNR^{17a}R^{17b}, NR^{17a}R^{17b}, C(=O)NR^{17a}R^{17b}, C(=S)NR^{17a}R^{17b}, C(=O)OR¹⁶, C(=O)R¹⁵, C(=S)R¹⁵, C(=NR^{17a})R¹⁵,
phenyl, optionally substituted with 1, 2, 3, 4 or 5 substituents R¹⁰, which are independently selected from one another,
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with 1, 2, 3 or 4 substituents R¹⁰, selected independently from one another, and wherein the nitrogen and/or the sulfur atom(s) of the heterocyclic ring may optionally be oxidized,
or
two R⁷ present on one carbon atom may together form =O, =CR¹³R¹⁴; =S;, =NR^{17a}, =NOR¹⁶;=NNR^{17a};
or
two R⁷ may form a 3-, 4-, 5-, 6-, 7- or 8-membered saturated or partly unsaturated carbocyclic or heterocyclic ring together with the carbon atoms to which the two R⁷ are bonded to;
- R⁸: is each independently from one another selected from the group consisting of hydrogen, cyano, C₁-C₆-alkyl, C₁-C₆-haloalkyl, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, C₃-C₈-cycloalkyl, C₄-C₈-alkylcycloalkyl, C₃-C₈-halocycloalkyl, C₂-C₆-alkenyl, C₂-C₆-haloalkenyl, C₂-C₆-alkinyl, C₂-C₆ haloalkinyl, -Si(R¹¹)₂R¹², S(O)ₙR¹⁶, S(O)ₙNR^{17a}R^{17b}, NR^{17a}R^{17b}, -N=CR¹³R¹⁴, -C(=O)R¹⁵, C(=O)NR^{17a}R^{17b}, C(=S)NR^{17a}R^{17b}, C(=O)OR¹⁶, phenyl, optionally substituted with one or more substituents R¹⁰; which are selected independently from one another,
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with 1, 2, 3 or 4 substituents R¹⁰, selected independently from one another, and wherein the nitrogen and/or the sulfur atom(s) of the heterocyclic ring may optionally be oxidized;
- R^{9a}, R^{9b}: are each independently from one another selected from the group consisting of hydrogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkinyl, C₂-C₆ haloalkinyl, S(O)ₙR¹⁶, -S(O)ₙNR^{17a}R^{17b}, C(=O)R¹⁵, C(=O)OR¹⁶, C(=O)NR^{17a}R^{17b}, C(=S)R¹⁵, C(=S)SR¹⁶, C(=S)NR^{17a}R^{17b}, C(=NR^{17a})R¹⁵;
phenyl, optionally substituted with one or more, e.g. 1, 2, 3 or 4, substituents R¹⁰, which are selected independently from one another;
a 3-, 4-, 5-, 6- or 7- membered saturated, partly saturated or unsaturated aromatic heterocyclic ring comprising 1, 2, 3 or 4 heteroatoms selected from oxygen, nitrogen and/or sulfur, optionally substituted with 1, 2, 3 or 4 substituents R¹⁰, selected independently from one another, and wherein the nitrogen and/or the sulfur atom(s) of the heterocyclic ring may optionally be oxidized;
or,
R^{9a} and R^{9b} are together a C₂-C₇ alkylene chain and form a 3-, 4-, 5-, 6-, 7- or 8-membered saturated, partly saturated or unsaturated aromatic ring together with the nitrogen atom they are bonded to, wherein the alkylene chain may contain one or two heteratoms selected from oxygen, sulfur or nitrogen, and may optionally be substituted with halogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkinyl, C₂-C₆ haloalkinyl,
phenyl, optionally substituted with one or more substituents R¹⁰; which are selected independently from one another, a 3-, 4-, 5-, 6,- or 7-membered saturated, partly saturated or unsaturated aromatic heterocyclic ring comprising 1, 2 or 3 heteroatoms selected from oxygen, nitrogen and/or sulfur, optionally substituted with one or more substituents R¹⁰, selected independently from one another, and wherein the nitrogen and/or the sulfur atom(s) of the heterocyclic ring may optionally be oxidized;
or
R^{9a} and R^{9b} together may form a =CR¹³R¹⁴,, =NR¹⁷ or =NOR¹⁶ radical;
- R¹⁰: is each independently from one another selected from the group consisting of hydrogen, halogen, cyano, azido, nitro, SCN, SF₅, C₁-C₁₀-alkyl, C₃-C₈-cycloalkyl, C₂-C₁₀-alkenyl, C₂-C₁₀-alkinyl, wherein the carbon atoms of the aforementioned aliphatic and cyclo-aliphatic radicals may optionally be substituted with one or more R¹⁵, which are selected independently from one another,
Si(R¹¹)₂R¹², OR¹⁶, OS(O)ₙR¹⁶, -S(O)ₙR¹⁶, S(O)ₙNR^{17a}R^{17b}, NR^{17a}R^{17b}, C(=O)R¹⁵, C(=S)R¹⁵, C(=O)OR¹⁶, -C(=NR^{17a})R¹⁵, C(=O)NR^{17a}R^{17b}, C(=S)NR^{17a}R^{17b}, phenyl, optionally substituted with halogen, cyano, nitro, C₁-C₆-alkyl, C₁-C₆-haloalkyl, C₁-C₆-alkoxy or C₁-C₆-haloalkoxy,
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with one or more substituents selected independently from one another from halogen, cyano, NO₂, C₁-C₆-alkyl, C₁-C₆-haloalkyl, C₁-C₆-alkoxy or C₁-C₆-haloalkoxy, and wherein the nitrogen and/or the sulfur atom(s) of the heterocyclic ring may optionally be oxidized;
or
two R¹⁰ present together on one atom of a partly saturated heterocyclic may be =O, =CR¹³R¹⁴;, =NR^{17a}, =NOR¹⁶ or =NNR^{17a};
or,
two R¹⁰ on adjacent carbon atoms may be a bridge selected from CH₂CH₂CH₂CH₂, CH=CH-CH=CH, N=CH-CH=CH, CH=N-CH=CH, N=CH-N=CH, OCH₂CH₂CH₂, OCH=CHCH₂, CH₂OCH₂CH₂, OCH₂CH₂O, OCH₂OCH₂, CH₂CH₂CH₂, CH=CHCH₂, CH₂CH₂O, CH=CHO, CH₂OCH₂, CH₂C(=O)O, C(=O)OCH₂, O(CH₂)O, SCH₂CH₂CH₂, SCH=CHCH₂, CH₂SCH₂CH₂, SCH₂CH₂S, SCH₂SCH₂, CH₂CH₂S, CH=CHS, CH₂SCH₂, CH₂C(=S)S, C(=S)SCH₂, S(CH₂)S, CH₂CH₂NR^{17a}, CH₂CH=N, CH=CH-NR^{17a}, OCH=N, SCH=N and form together with the carbon atoms to which the two R¹⁰ are bonded to a 5-membered or 6-membered partly saturated or unsaturated, aromatic carbocyclic or heteocyclic ring, wherein the ring may optionally be substituted with one or two substituents selected from =O, OH, CH₃, OCH₃, halogen, cyano, halomethyl or halomethoxy;
- R¹¹, R¹²: (are each independently from one another selected from the group consisting of hydrogen, halogen, C₁-C₆ alkyl, C₁-C₆ haloalkyl, C₁-C₆ alkoxy, C₁-C₆ alkoxy-alkyl, C₂-C₆ alkenyl, C₂-C₆ haloalkenyl, C₂-C₆ alkinyl, C₂-C₆ haloalkinyl, C₃-C₈ cycloalkyl, C₃-C₈ halocycloalkyl, C₁-C₆ alkoxyalkyl, C₁-C₆ haloalkoxyalkyl and phenyl, optionally substituted with one or more substituents R¹⁰; which are selected independently from one another;
- R¹³, R¹⁴: are each independently from one another selected from the group consisting of hydrogen, C₁-C₄ alkyl, C₁-C₆ cycloalkyl, C₁-C₄ alkoxyalkyl, phenyl and benzyl;
- R¹⁵: is each independently from one another selected from the group consisting of hydrogen, halogen, cyano, nitro, OH, SH, SCN, SF₅, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, tertbutyldimethylsilyl,
C₁-C₆-alkyl, C₂-C₆-alkenyl, C₂-C₆-alkinyl, C₃-C₈-cycloalkyl, wherein the four last mentioned aliphatic and cyclo-aliphatic radicals may be unsubstituted, partially or fully halogenated and/or oxgenated and/or may carry 1 or 2 radicals selected from C₁-C₄ alkoxy;
phenyl, benzyl, pyridyl, phenoxy, wherein the last four radicals may be unsubstituted, partially or fully halogenated and/or to carry 1, 2 or 3 substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino or di-(C₁-C₆-alkyl)amino,
or
two R¹⁵ present on the same carbon atom may together be =O, =CH(C₁-C₄), =C(C₁-C₄-alkyl)C₁-C₄-alkyl, =N(C₁-C₆-alkyl) or =NO(C₁-C₆-alkyl);
- R¹⁶: is each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl,
C₁-C₆-alkyl, C₂-C₆-alkenyl, C₂-C₆-alkinyl, C₃-C₈-cycloalkyl, wherein the four last mentioned radicals may be unsubstituted, partially or fully halogenated and/or oxygenated and/or may carry 1 or 2 radicals selected from C₁-C₄ alkoxy, phenyl, benzyl, pyridyl, phenoxy, wherein the last four radicals may be unsubstituted, partially or fully halogenated and/or carry 1, 2 or 3 substituents selected from C₁-C₆-alkyl, C₁-C₆-haloalkyl, C₁-C₆-alkoxy, C₁-C₆ haloalkoxy or (C₁-C₆-alkoxy)carbonyl;
- R^{17a}, R^{17b}: are each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkinyl, C₃-C₈-cycloalkyl, wherein the four last mentioned aliphatic and cyclo-aliphatic radicals may be unsubstituted, partially or fully halogenated and/or oxygenated and/or may carry 1 or 2 radicals selected from C₁-C₄-alkoxy,
phenyl, benzyl, pyridyl, phenoxy, wherein the four last mentioned radicals may be unsubstituted, partially or fully halogenated and/or carry 1, 2 or 3 substituents selected from C₁-C₆-alkyl, C₁-C₆-haloalkyl, C₁-C₆-alkoxy, C₁-C₆ haloalkoxy or (C₁-C₆-alkoxy)carbonyl,
or,
R^{17a} and R^{17b} may together be a C₂-C₆ alkylene chain forming a 3- to 7-membered saturated, partly saturated or unsaturated ring together with the nitrogen atom R^{17a} and R^{17b} are bonded to, wherein the alkylene chain may contain 1 or 2 heteroatoms selected from oxygen, sulfur or nitrogen, and may optionally be substituted with halogen, C₁-C₄-haloalkyl, C₁-C₄-alkoxy or C₁-C₄-haloalkoxy, and wherein the nitrogen and/or the sulfur atom(s) of the heterocyclic ring may optionally be oxidized;
- n: is an integer selected independently from one another from 0, 1 or 2;
and/or an enantiomer, diastereomer, E/Z-isomer or agriculturally or veterinarily acceptable salts thereof for controlling and/or combating animal pests.

Substituted nitroimino- und cyanoimino-pyridyls and their use as pesticides have been disclosed in EP 259738. Similar substituted iminopyridines have been described as herbicides in EP 432600.

Trifluoroacetyl heterocyclylimines are disclosed as insecticides in EP 268915. Substituted acylimino pyridyls have been disclosed as insecticides in WO 2012/029672. The production thereof is described in WO 2013/031671. WO2013/129692 relates to a nitrogen-containing heterocyclic derivative having a 2-imino group, and a novel pest control agent using the same. Certain heterocyclylimines and their use as insecticides are described in WO 9215564

The N- substituted acyl-imino-pyridine compounds of the formula I, and their agriculturally or veterinarily acceptable salts are highly active against animal pest, i.e. harmful arthropodes and nematodes, especially against difficult to control insects and acaridae.

Accordingly, the present invention relates to N-substituted acyl-imino-pyridine compounds of the general formula I, to their agriculturally or veterinarily useful salts, their enantiomers or diasteromers.

Moreover, the present invention relates to and includes the following embodiments:
- agricultural and veterinary compositions comprising an amount of at least one compound of the formula I or an enantiomer, diasteromer or salt thereof;
- the use of a compound of formula I or an enantiomer, diasteromer or salt thereof for combating animal pests;
- a method of combating animal pests which comprises contacting the animal pests, their habit, breeding ground, food supply, plant, seed, soil, area, material or environment in which the animal pests are growing or may grow, or the materials, plants, seeds, soils, surfaces or spaces to be protected from animal attack or infestation with a pesticidally effective amount of at least one compound of the formula I or an enantiomer, diasteromer or salt thereof;
- a method for protecting crops from attack or infestation by animal pests, which comprises contacting a crop with a pesticidally effective amount of at least one compound of the formula I or an enantiomer, diasteromer or salt thereof;
- a method for the protection of plant propagation, especially seeds, from soil insects and of the seedlings' roots and shoots from soil and foliar insects comprising contacting the seeds before sowing and/or after pregermination with at least one compound of the formula I, or the enantiomers, diastereomers or salts thereof;
- seeds comprising a compound of the formula I or an enantiomer, diasteromer or salt thereof;
- the use of compounds of formula I or the enantiomers, diastereomers or veterinary acceptable salts thereof for combating parasites in and on animals.
- a method for treating, controlling, preventing or protecting animals against infestation or infection by parasites which comprises orally, topically or parenterally administering or applying to the animals a parasiticidally effective amount of an compound of formula I or the enantiomers, diastereomers and/or veterinary acceptable salt thereof;
- a process for the preparation of a veterinary composition for treating, controlling, preventing or protecting animals against infestation or infection by parasites which comprises adding a parasiticidally effective amount of an compound of formula I or the enantiomers, diastereomers and/or veterinary acceptable salt thereof to a carrier composition suitable for veterinary use;
- the use of a compound of formula I or the enantiomers, diastereomers and/or veterinary acceptable salt thereof for the preparation of a medicament for treating, controlling, preventing or protecting animals against infestation or infection by parasites;

The present invention especially relates to plant propagation materials, in particular as mentioned above to seeds, comprising at least one compound of formula I and/or an agriculturally acceptable salt thereof.

The present invention relates to every possible stereoisomer of the compounds of formula I, i.e. to single enantiomers or diastereomers, as well as to mixtures thereof.

The present invention relates to each isomer alone, or mixtures or combinations of the isomers in any proportion to each other.

The compounds of the present invention may be amorphous or may exist in one ore more different crystalline states (polymorphs) or modifications which may have a different macroscopic properties such as stability or show different biological properties such as activities. The present invention includes both amorphous and crystalline compounds of the formula I, mixtures of different crystalline states or modifications of the respective compound I, as well as amorphous or crystalline salts thereof.

Salts of the compounds of the formula I are preferably agriculturally and/or veterinary acceptable salts. They can be formed in a customary method, e.g. by reacting the compound with an acid of the anion in question if the compound of formula I has a basic functionality or by reacting an acidic compound of formula I with a suitable base.

Suitable agriculturally or veterinary useful salts are especially the salts of those cations or the acid addition salts of those acids whose cations and anions, respectively, do not have any adverse effect on the action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH₄⁺) and substituted ammonium in which one to four of the hydrogen atoms are replaced by C₁-C₄-alkyl, C₁-C₄hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethyl-ammonium, bis(2-hydroxyethyl)ammonium, benzyltrimethylammonium and benzyltriethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting the compounds of the formulae I with an acid of the corresponding anion, preferably of hydrochloric acid, hydrobromic acid, sulfuric acid, phosphoric acid or nitric acid.

The organic moieties mentioned in the above definitions of the variables are - like the term halogen - collective terms for individual listings of the individual group members. The prefix Cₙ-Cₘ indicates in each case the possible number of carbon atoms in the group.

"Halogen" will be taken to mean fluoro, chloro, bromo and iodo.

The term "partially or fully halogenated" will be taken to mean that 1 or more, e.g. 1, 2, 3, 4 or 5 or all of the hydrogen atoms of a given radical have been replaced by a halogen atom, in particular by fluorine or chlorine.

The term "Cₙ-Cₘ-alkyl" as used herein (and also in Cₙ-Cₘ-alkylamino, di-Cₙ-Cₘ-alkylamino, Cₙ-Cₘ-alkylaminocarbonyl, di-(Cₙ-Cₘ-alkylamino)carbonyl, Cₙ-Cₘ-alkylthio, Cₙ-Cₘ-alkylsulfinyl and Cₙ-Cₘ-alkylsulfonyl) refers to a branched or unbranched saturated hydrocarbon group having n to m, e.g. 1 to 10 carbon atoms, preferably 1 to 6 carbon atoms, for example methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, 2-ethylhexyl, nonyl and decyl and their isomers. C₁-C₄-alkyl means for example methyl, ethyl, propyl, 1-methylethyl, butyl, 1-methylpropyl, 2-methylpropyl or 1,1-dimethylethyl.

The term "Cₙ-Cₘ-haloalkyl" as used herein (and also in Cₙ-Cₘ-haloalkylsulfinyl and Cₙ-Cₘ-haloalkylsulfonyl) refers to a straight-chain or branched alkyl group having n to m carbon atoms, e.g. 1 to 10 in particular 1 to 6 carbon atoms (as mentioned above), where some or all of the hydrogen atoms in these groups may be replaced by halogen atoms as mentioned above, for example C₁-C₄-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and the like. The term C₁-C₁₀-haloalkyl in particular comprises C₁-C₂-fluoroalkyl, which is synonym with methyl or ethyl, wherein 1, 2, 3, 4 or 5 hydrogen atoms are substituted by fluorine atoms, such as fluoromethyl, difluoromethyl, trifluoromethyl, 1-fluoroethyl, 2-fluoroethyl, 2,2-difluoroethyl, 2,2,2-trifluoroethyl and pentafluoromethyl.

Similarly, "Cₙ-Cₘ-alkoxy" and "Cₙ-Cₘ-alkylthio" (or Cₙ-Cₘ-alkylsulfenyl, respectively) refer to straight-chain or branched alkyl groups having n to m carbon atoms, e.g. 1 to 10, in particular 1 to 6 or 1 to 4 carbon atoms (as mentioned above) bonded through oxygen or sulfur linkages, respectively, at any bond in the alkyl group. Examples include C₁-C₄-alkoxy such as methoxy, ethoxy, propoxy, isopropoxy, butoxy, sec-butoxy, isobutoxy and tert-butoxy, futher C₁-C₄-alkylthio such as methylthio, ethylthio, propylthio, isopropylthio, and n-butylthio.

Accordingly, the terms "Cₙ-Cₘ-haloalkoxy" and "Cₙ-Cₘ-haloalkylthio" (or Cₙ-Cₘ-haloalkylsulfenyl, respectively) refer to straight-chain or branched alkyl groups having n to m carbon atoms, e.g. 1 to 10, in particular 1 to 6 or 1 to 4 carbon atoms (as mentioned above) bonded through oxygen or sulfur linkages, respectively, at any bond in the alkyl group, where some or all of the hydrogen atoms in these groups may be replaced by halogen atoms as mentioned above, for example C₁-C₂-haloalkoxy, such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy and pentafluoroethoxy, further C₁-C₂-haloalkylthio, such as chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio and pentafluoroethylthio and the like. Similarly the terms C₁-C₂-fluoroalkoxy and C₁-C₂-fluoroalkylthio refer to C₁-C₂-fluoroalkyl which is bound to the remainder of the molecule via an oxygen atom or a sulfur atom, respectively.

The term "C₂-Cₘ-alkenyl" as used herein intends a branched or unbranched unsaturated hydrocarbon group having 2 to m, e.g. 2 to 10 or 2 to 6 carbon atoms and a double bond in any position, such as ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "C₂-Cₘ-alkynyl" as used herein refers to a branched or unbranched unsaturated hydrocarbon group having 2 to m, e.g. 2 to 10 or 2 to 6 carbon atoms and containing at least one triple bond, such as ethynyl, propynyl, 1-butynyl, 2-butynyl, and the like.

The term "C₁-C₄-alkoxy-C₁-C₄-alkyl" as used herein refers to alkyl having 1 to 4 carbon atoms, e.g. like specific examples mentioned above, wherein one hydrogen atom of the alkyl radical is replaced by an C₁-C₄-alkoxy group.

The term "C₃-Cₘ-cycloalkyl" as used herein refers to a monocyclic 3- to m-membered saturated cycloaliphatic radicals, e.g. cyclopropyl, cyclobutyl, cyclopentyl, cyclohexyl, cycloheptyl, cyclooctyl and cyclodecyl.

The term "aryl" as used herein refers to an aromatic hydrocarbon radical such as naphthyl or in particular phenyl.

The term "3- to 6-membered carbocyclic ring" as used herein refers to cyclopropane, cyclobutane, cyclopentane and cyclohexane rings.

The term "3-, 4-, 5-, 6- or 7-membered saturated, partially unsaturated or aromatic heterocyclic ring containing 1, 2 or 3 heteroatoms" or "containing heteroatom groups", wherein those heteroatom(s) (group(s)) are selected from N, O, S, NO, SO and SO₂ and are ring members, as used herein refers to monocyclic radicals, the monocyclic radicals being saturated, partially unsaturated or aromatic. The heterocyclic radical may be attached to the remainder of the molecule via a carbon ring member or via a nitrogen ring member.

Examples of 3-, 4-, 5-, 6- or 7-membered saturated heterocyclyl or heterocyclic rings include: Oxiranyl, aziridinyl, azetidinyl, 2 tetrahydrofuranyl, 3-tetrahydrofuranyl, 2 tetrahydrothienyl, 3 tetrahydrothienyl, 2-pyrrolidinyl, 3-pyrrolidinyl, 3 pyrazolidinyl, 4 pyrazolidinyl, 5-pyrazolidinyl, 2 imidazolidinyl, 4 imidazolidinyl, 2-oxazolidinyl, 4-oxazolidinyl, 5 oxazolidinyl, 3-isoxazolidinyl, 4 isoxazolidinyl, 5 isoxazolidinyl, 2 thiazolidinyl, 4-thiazolidinyl, 5-thiazolidinyl, 3 isothiazolidinyl, 4-isothiazolidinyl, 5 isothiazolidinyl, 1,2,4-oxadiazolidin-3-yl, 1,2,4 oxadiazolidin 5 yl, 1,2,4-thiadiazolidin-3-yl, 1,2,4 thiadiazolidin-5-yl, 1,2,4 triazolidin-3-yl, 1,3,4-oxadiazolidin-2-yl, 1,3,4 thiadiazolidin-2-yl, 1,3,4 triazolidin-2-yl, 2-tetrahydropyranyl, 4 tetrahydropyranyl, 1,3-dioxan-5-yl, 1,4-dioxan-2-yl, 2-piperidinyl, 3-piperidinyl, 4-piperidinyl, 3-hexahydropyridazinyl, 4 hexahydropyridazinyl, 2-hexahydropyrimidinyl, 4-hexahydropyrimidinyl, 5 hexahydropyrimidinyl, 2-piperazinyl, 1,3,5-hexahydrotriazin-2-yl and 1,2,4 hexahydrotriazin-3-yl, 2-morpholinyl, 3-morpholinyl, 2-thiomorpholinyl, 3-thiomorpholinyl, 1-oxothiomorpholin-2-yl, 1-oxothiomorpholin-3-yl, 1,1-dioxothiomorpholin-2-yl, 1,1-dioxothiomorpholin-3-yl, hexahydroazepin-1-, -2-, -3- or -4-yl, hexahydrooxepinyl, hexahydro-1,3-diazepinyl, hexahydro-1,4-diazepinyl, hexahydro-1,3-oxazepinyl, hexahydro-1,4-oxazepinyl, hexahydro-1,3-dioxepinyl, hexahydro-1,4-dioxepinyl and the like.

Examples of 3-, 4-, 5-, 6- or 7-membered partially unsaturated heterocyclyl or heterocyclic rings include: 2,3-dihydrofur-2-yl, 2,3-dihydrofur-3-yl, 2,4-dihydrofur-2-yl, 2,4-dihydrofur-3-yl, 2,3-dihydrothien-2-yl, 2,3 dihydrothien-3-yl, 2,4 dihydrothien-2-yl, 2,4-dihydrothien-3-yl, 2-pyrrolin-2-yl, 2-pyrrolin-3-yl, 3 pyrrolin-2-yl, 3-pyrrolin-3-yl, 2-isoxazolin-3-yl, 3-isoxazolin-3-yl, 4 isoxazolin 3 yl, 2-isoxazolin-4-yl, 3-isoxazolin-4-yl, 4-isoxazolin-4-yl, 2 isoxazolin-5-yl, 3-isoxazolin-5-yl, 4-isoxazolin-5-yl, 2-isothiazolin-3-yl, 3 isothiazolin-3-yl, 4-isothiazolin-3-yl, 2-isothiazolin-4-yl, 3-isothiazolin-4-yl, 4 isothiazolin-4-yl, 2-isothiazolin-5-yl, 3-isothiazolin-5-yl, 4-isothiazolin-5-yl, 2,3 dihydropyrazol-1-yl, 2,3-dihydropyrazol-2-yl, 2,3-dihydropyrazol-3-yl, 2,3 dihydropyrazol-4-yl, 2,3-dihydropyrazol-5-yl, 3,4-dihydropyrazol-1-yl, 3,4 dihydropyrazol-3-yl, 3,4-dihydropyrazol-4-yl, 3,4-dihydropyrazol-5-yl, 4,5 dihydropyrazol-1-yl, 4,5-dihydropyrazol-3-yl, 4,5-dihydropyrazol-4-yl, 4,5 dihydropyrazol-5-yl, 2,3-dihydrooxazol-2-yl, 2,3-dihydrooxazol-3-yl, 2,3 dihydrooxazol-4-yl, 2,3-dihydrooxazol-5-yl, 3,4-dihydrooxazol-2-yl, 3,4 dihydrooxazol-3-yl, 3,4-dihydrooxazol-4-yl, 3,4-dihydrooxazol-5-yl, 3,4 dihydrooxazol-2-yl, 3,4-dihydrooxazol-3-yl, 3,4-dihydrooxazol-4-yl, 2-, 3-, 4-, 5- or 6-di- or tetrahydropyridinyl, 3-di- or tetrahydropyridazinyl, 4 di- or tetrahydro-pyridazinyl, 2-di- or tetrahydropyrimidinyl, 4-di- or tetrahydropyrimidinyl, 5 di- or tetrahydropyrimidinyl, di- or tetrahydropyrazinyl, 1,3,5-di- or tetrahydrotriazin-2-yl, 1,2,4-di- or tetrahydrotriazin-3-yl, 2,3,4,5-tetrahydro[1H]azepin-1-, -2-, -3-, -4-, -5-, -6- or -7-yl, 3,4,5,6-tetrahydro[2H]azepin-2-, -3-, -4-, -5-, -6- or -7-yl, 2,3,4,7 tetrahydro[1H]azepin-1-, -2-, -3-, -4-, -5-, -6- or -7-yl, 2,3,6,7 tetrahydro[1H]azepin-1-, -2-, -3-, -4-, -5-, -6- or -7-yl, tetrahydrooxepinyl, such as 2,3,4,5-tetrahydro[1H]oxepin-2-, -3-, -4-, -5-, -6- or -7-yl, 2,3,4,7 tetrahydro[1H]oxepin-2-, -3-, -4-, -5-, -6- or -7-yl, 2,3,6,7 tetrahydro[1H]oxepin-2-, -3-, -4-, -5-, -6- or -7-yl, tetrahydro-1,3-diazepinyl, tetrahydro-1,4-diazepinyl, tetrahydro-1,3-oxazepinyl, tetrahydro-1,4-oxazepinyl, tetrahydro-1,3-dioxepinyl and tetrahydro-1,4-dioxepinyl.

Examples of 5- or 6-membered aromatic heterocyclyl (hetaryl) or heteroaromatic rings are: 2-furyl, 3-furyl, 2-thienyl, 3-thienyl, 2-pyrrolyl, 3-pyrrolyl, 3-pyrazolyl, 4-pyrazo¬lyl, 5-pyrazolyl, 2-oxazolyl, 4-oxazolyl, 5-oxazolyl, 2-thiazolyl, 4 thiazolyl, 5-thiazo¬lyl, 2-imidazolyl, 4-imidazolyl, 1,3,4-triazol-2-yl, 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

A "C₂-Cₘ-alkylene" is divalent branched or preferably unbranched saturated aliphatic chain having 2 to m, e.g. 2 to 7 carbon atoms, for example CH₂CH₂, -CH(CH₃)-, CH₂CH₂CH₂, CH(CH₃)CH₂, CH₂CH(CH₃), CH₂CH₂CH₂CH₂, CH₂CH₂CH₂CH₂CH₂, CH₂CH₂CH₂CH₂CH₂CH₂, and CH₂CH₂CH₂CH₂CH₂CH₂CH₂.

### Preferences

Embodiments and preferred compounds of the present invention are outlined in the following paragraphs.

The remarks made below concerning preferred embodiments of the variables of the compounds of formula I, especially with respect to their substituents X, Y, W¹, W², W³, W⁴, Het, R¹ ,R², R^{3a}, R^{3b}, R^{4a}, R^{4b} and R⁵ and their variable p are valid both on their own and, in particular, in every possible combination with each other.

When # appears in a formula showing a preferred substructure of a compound of the present invention, it denotes the attachment bond in the remainder molecule.

Preferred are compounds of formula (I), wherein Het is selected from the group consisting of radicals of formulae Het-1 to Het-28: 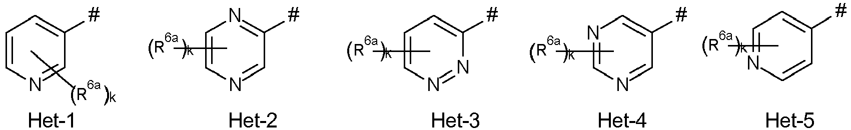 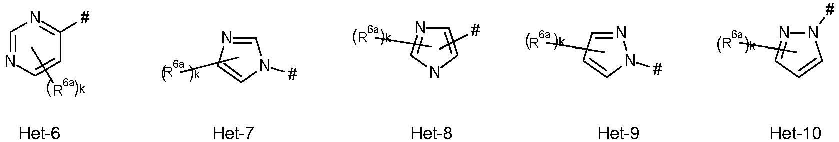 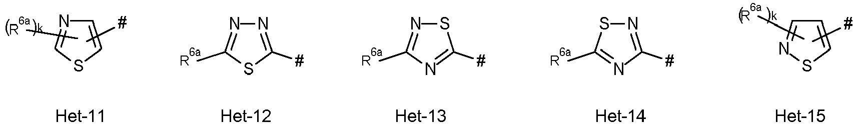 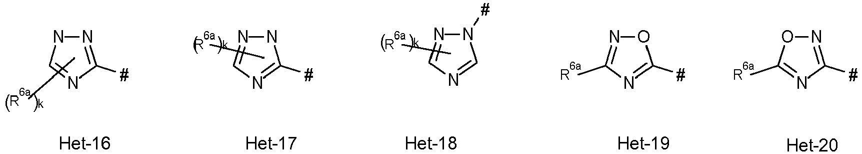 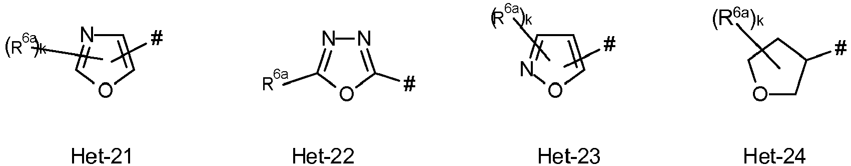 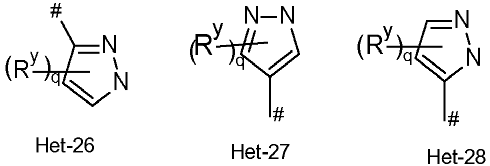 wherein # denotes the bond in formula (I), k is 0, 1 or 2 and R^{6a} has the preferred meaning as defined further below.

Especially preferred are compounds of formula (I), wherein Het is selected from the group consisting of radicals of formulae Het-1, Het-11 a and Het-24: 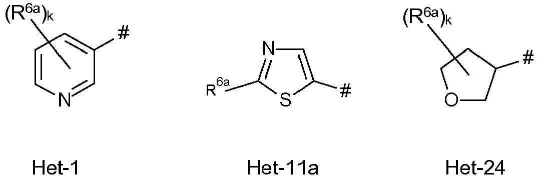 and wherein # denotes the bond in formula (I), k is 0, 1 or 2 and R^{6a} has the preferred meaning as defined further below.

Especially more preferred are compounds of formula (I), wherein Het is Het-1a: 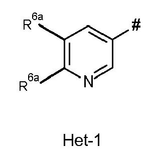 and wherein # denotes the bond in formula (I), and R^{6a} has the preferred meaning as defined further below.

Especially more preferred are compounds of formula (I), wherein Het is Het-11 a: 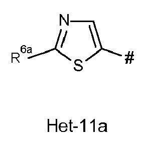 and wherein # denotes the bond in formula (I), and R^{6a} has the preferred meaning as defined further below.

Especially preferred are compounds of formula (I), wherein Het is Het-24: 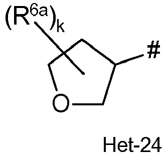 and wherein # denotes the bond in formula (I) and wherein k is 0.

Especially preferred are compounds of formula (I), wherein Het is Het-24 and wherein # denotes the bond in formula (I)and wherein k selected from 1 or 2. and R^{6a} has the preferred meaning as defined further below.

Especially more preferred are compounds of formula (I), wherein Het is Het-23a: 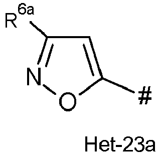 and wherein # denotes the bond in formula (I), and R^{6a} has the preferred meaning as defined further below.

Preferred are compounds of formula (I), wherein on Het, k is selected from 0, 1 or 2.

Especially preferred are compounds of formula (I), wherein on Het, k is 0, 1 or 2 and R^{6a} has the preferred meaning as defined herein below.

Preferred are compounds of formula (I), wherein Het is substituted with R^{6a} selected each independently from one another from the group consisting of hydrogen, halogen, cyano, C₁-C₆-alkyl, C₃-C₈-cycloalkyl, C₂-C₆-alkenyl, C₂-C₆-alkinyl, wherein the carbon atoms of the aforementioned aliphatic and cyclo-aliphatic radicals may optionally be further substituted independently from one another with one or more R⁷, OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=NR^{9a})R⁷, C(=NR^{9a})NR^{9a}R^{9b}.

Especially preferred are compounds of formula (I), wherein Het is substituted with R^{6a} selected each independently from one another from the group consisting of hydrogen, halogen, C₁-C₄-alkoxy or C₁-C₄-alkyl, wherein the carbon atoms of the latter two radicals may be partially of fully halogenated.

Preferred are compounds of formula (I), wherein R¹ and R² are independently from each other selected from the group consisting of hydrogen, halogen, CN, C₁-C₆-alkyl, C₃-C₆-cycloalkyl, C₁-C₆-haloalkyl, C₂-C₆-halocycloalkyl; or
R¹ and R² may together be =O, =CR¹³R¹⁴ or =S; or
R¹ and R² form, together with the carbon atom, which they attached to, a 3- to 5 membered saturated carbocyclic ring;

Especially preferred are compounds of formula (I), wherein R¹ and R² are independently from each other selected from the group consisting of hydrogen, halogen, cyano, C₁-C₃-alkyl or C₁-C₃-haloalkyl.

Especially preferred are compounds of formula (I),wherein R¹ and R² are both hydrogen. Especially preferred are compounds of formula (I),, wherein R¹ is hydrogen, and R² is CH₃.

Preferred are compounds of formula (I), wherein R^{3a} and R^{3b} are selected each independently from one another from the group consisting of hydrogen, halogen, CN, C₁-C₆-alkyl, C₁-C₆-haloalkyl, C₁-C₆-alkoxy, C₁-C₆-haloalkoxy, C₁-C₆-alkylthio, C₁-C₆-haloalkylthio and OR⁸, wherein R⁸ is C(=O)R¹⁵, and R¹⁵ is C₁-C₆-alkyl, wherein the aliphatic radical may be unsubstituted, partially or fully halogenated.

Preferred are compounds of formula (I), wherein R^{3a}, R^{3b} are selected each independently from one another from the group consisting of hydrogen, halogen, CN, C₁-C₆-alkyl and C₁-C₆-haloalkyl.

Especially preferred are compounds of formula (I), wherein R^{3a}, R^{3b} are selected each independently from one another from hydrogen or fluoro.

Preferred are compounds of formula (I), wherein R^{3a}, R^{3b} form together with the carbon atom they are bound to, a cyclopropane ring, wherein each of the carbon atoms of the ring may be unsubstituted or may be partly or fully halogenated.

Preferred are compounds of formula (I), wherein p is 0.

Preferred are compounds of formula (I), wherein p is 1 and R^{4a}, R^{4b} are selected independently from one another from hydrogen or halogen.

Especially preferred are compounds of formula (I), wherein p is 1 and R^{4a}, R^{4b} are selected independently from one another from hydrogen or fluoro.

Especially preferred are compounds of formula (I), wherein p is 1 , and wherein one of R^{4a} and R^{4b} may form a double bond with R^{3a} or R^{3b} of the adjacent carbon atoms.

Preferred are compounds of formula (I), wherein X is S.

Preferred are compounds of formula (I), wherein X is O.

Preferred are compounds of formula (I), wherein Y is selected from O.

Preferred are compounds of formula (I), wherein Y is selected from S, S=O or S(O)₂.

Especially preferred are compounds of formula (I), wherein Y is selected from S.

Especially preferred are compounds of formula (I), wherein Y is selected from S=O.

Especially preferred are compounds of formula (I), wherein Y is selected from S(O)₂.

Preferred are compounds of formula (I), wherein R⁵ is selected from the group consisting of hydrogen, cyano, C₁-C₆-alkyl, C₇-C₁₀-alkyl, C₃-C₁₀-cycloalkyl, C₂-C₆-alkenyl and C₂-C₆-alkinyl, wherein the carbon chain atoms of the four aforementioned aliphatic and cycloaliphatic radicals are unsubstituted, partly or completely halogenated or may carry any combination of one or more radicals R⁷, wherein R⁷ is selected independently from one another from halogen, C₁-C₆-alkyl, C₁-C₆-haloalkyl, C₁-C₆-alkoxy, C₁-C₆-haloalkoxy or C(=O)O-R¹⁶, and wherein R¹⁶ is - independently from one another - C₁-C₆-alkyl, wherein the alkyl radical may be unsubstituted, partially or fully halogenated and/or may carry 1 or 2 radicals selected from C₁-C₄ alkoxy.

Preferred are compounds of formula (I), wherein R⁵ is selected from the group consisting of phenyl or CH₂-phenyl, optionally substituted with 1, 2 or 3 substituents, which are independently selected from one another from hydrogen, halogen, C₁-C₄-alkyl, C₁-C₄-haloalkyl, C₁-C₄-alkoxy, C₁-C₄-haloalkoxy or phenoxy.

Preferred are compounds of formula (I), wherein R⁵ is selected from the group consisting of a 5- or 6- membered saturated, partly saturated or unsaturated aromatic heterocyclic ring selected from pyridine, furan, oxazole, oxadiazole, isoxazole, thiazole, thiadiazole or isothiazole, wherein the heterocyclic ring may be bonded directly or linked via a CH₂ group to the remainder of the molecule, wherein the heterocyclic ring is unsubstituted or optionally substituted with one, two or three substituents R^{y} selected independently from one another, and wherein the R^{y} is selected from the group conisisting of halogen, cyano and C₁-C₄-alkyl, wherein the carbon atoms of the alkyl radical may be unsubstituted or partly or fully halogenated and/or may carry 1,2 or 3 radicals selected from C₁-C₄ alkoxy.

Preferred are compounds of formula (I), wherein R⁵ is selected from the group consisting of a 5- or 6- membered saturated, partly saturated or unsaturated aromatic heterocyclic ring selected from pyridine, furan, oxazole, oxadiazole, isoxazole, thiazole, thiadiazole or isothiazole, wherein the heterocyclic ring is unsubstituted or optionally substituted with one, two or three substituents R^{y} selected independently from one another, and wherein the R^{y} is selected from the group conisisting of halogen, cyano and C₁-C₄-alkyl, wherein the carbon atoms of the alkyl radical may be unsubstituted or partly or fully halogenated and/or may carry 1,2 or 3 radicals selected from C₁-C₄ alkoxy.

Preferred are compounds of formula (I), wherein R⁵ is selected from the group consisting of hydrogen, halogen, cyano, C₁-C₆-alkyl, C₃-C₁₀-cycloalkyl, C₂-C₆-alkenyl or C₂-C₆-alkinyl, wherein each of the four last mentioned aliphatic radicals is unsubstituted, partly or completely halogenated

Preferred are compounds of formula (I), wherein R⁵ is selected from the group consisting of SCN, C₁-C₄ alkylthio, C₃-C₆ cycloalkylthio, C₂-C₆ alkenylthio and C₂-C₆ alkinylthio, wherein each of the four last mentioned aliphatic radicals are unsubstituted, partly or completely halogenated.

Preferred are compounds of formula (I), wherein R⁵ is phenyl or benzyl.

Preferred are compounds of formula (I), wherein R⁵ is phenyl or CH₂-phenyl, wherein the aromatic ring is optionally substituted with 1 or 2 substituents, which are independently selected from one another from the group consisting of hydrogen, halogen, CN, C₁-C₄ alkyl, C₁-C₄ alkoxy, wherein both of the two last mentioned aliphatic radicals may be unsubstituted or partly or completely halogenated.

Preferred are compounds of formula (I), wherein R⁵ is a 5- or 6- membered aromatic heterocyclic ring selected from the group consisting of 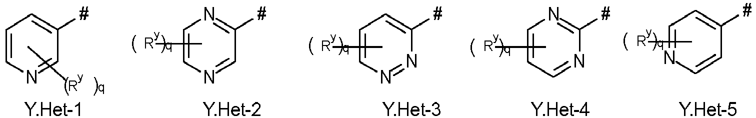 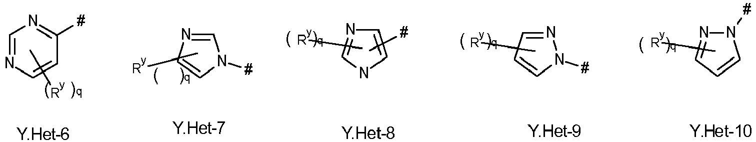 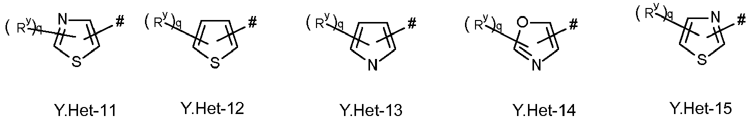 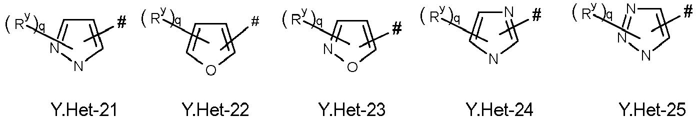 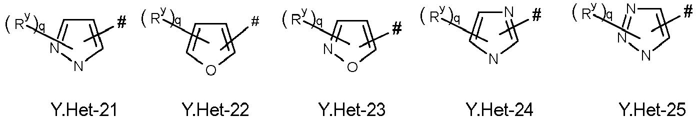 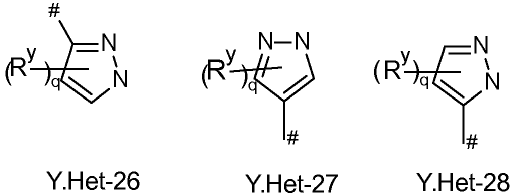 wherein # denotes the bond the remainder of the molecule, and wherein the bond may be a single bond or linked via a CH₂ group to the remainder of the molecule;
wherein
- q: is selected from 0,1 or 2, and wherein
- R^{y}: is selected independently from the value of q and independently from one another, from the group consisting of hydrogen, halogen, CN, - C₁-C₄ alkyl and C₁-C₄ alkoxy, wherein both of the last two mentioned aliphatic radicals may be unsubstituted, partly or completely halogenated
phenyl, optionally substituted with halogen, cyano, nitro, C₁-C₄ alkyl and C₁-C₄ alkoxy, wherein both of the last two mentioned aliphatic radicals may be unsubstituted, partly or completely halogenated,
or
two of R^{y} on adjacent carbon atoms may be a bridge selected from CH=CH-CH=CH or CH=CHCH₂, and thus form together with the carbon atoms to which the two R^{y} are bonded to a fused 5-membered or 6-membered aromatic carbocyclic ring, wherein this ring may optionally be substituted with one or two substituents selected from halogen, C₁-C₄-alkoxy, C₁-C₄-haloalkoxy, C₁-C₄-alkyl or C₁-C₄-haloalkyl.

Preferred are compounds of formula (I), wherein W¹, W², W³ and W⁴ represent a carbon chain group connected to N and C=N, and thus forming a saturated, unsaturated or partially unsaturated 6-membered nitrogen containing heterocycle selected from the following group consisting of W.Het-1, W.Het-2, W.Het-3, W.Het-4, W.Het-5, W.Het-6, W.Het-7, W.Het-8, W.Het-9, W.Het-10, W.Het-11 and W.Het-12: 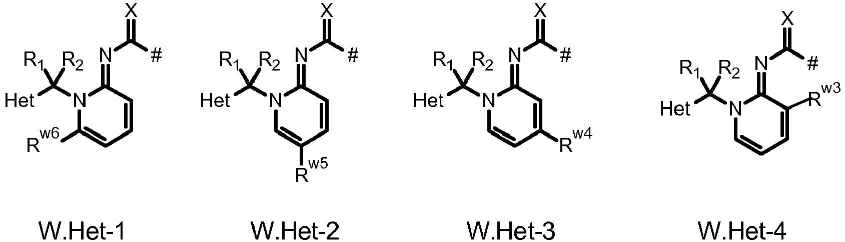 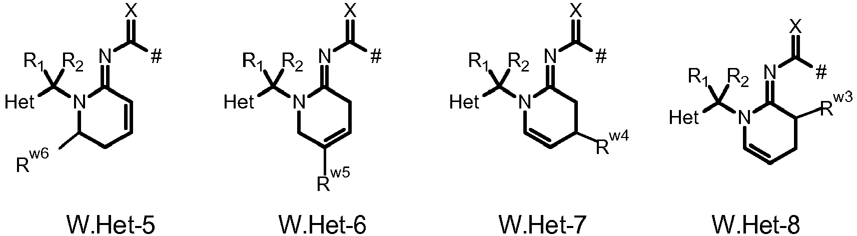 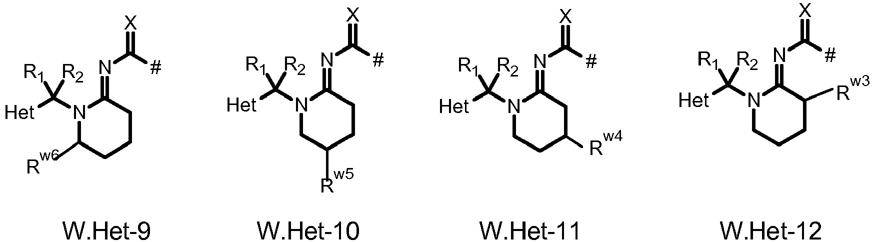 wherein # denotes the bond to the remainder of the molecule; R¹, R² and Het are as defined as above, and wherein R^{w3}, R^{w4},^{,} R^{w5} and R^{w6} are selected from the group consisiting of hydrogen, halogen, C₁-C₄-alkoxy and C₁-C₄-alkyl, wherein both of the last two mentioned aliphatic radicals may be unsubstituted, partly or completely halogenated

Especially preferred are compounds of formula (I), wherein W¹, W², W³ and W⁴ represent a carbon chain group connected to N and C=N, and thus forming a saturated, unsaturated or partly unsaturated 6-membered nitrogen containing heterocycle selected from W.Het-1, W.Het-5 and W.Het-9 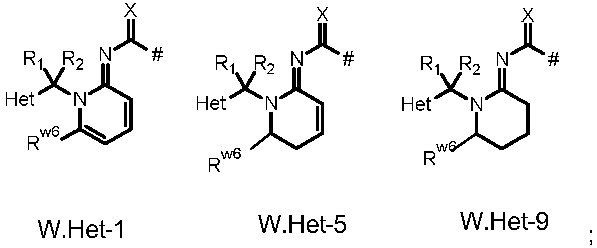 wherein # denotes the bond to the remainder of the molecule, and R^{w6} is selected from hydrogen, halogen, C₁-C₄-alkyl; or C₁-C₄-haloalkyl.

Preferred are compounds of formula (I), wherein each R^{w} is selected independently from one another from hydrogen, halogen, C₁-C₄-alkoxy or C₁-C₄-alkyl, wherein both of the last two mentioned aliphatic radicals may be unsubstituted, partly or completely halogenated.

Preferred compounds of the present invention are compounds of formula (I) 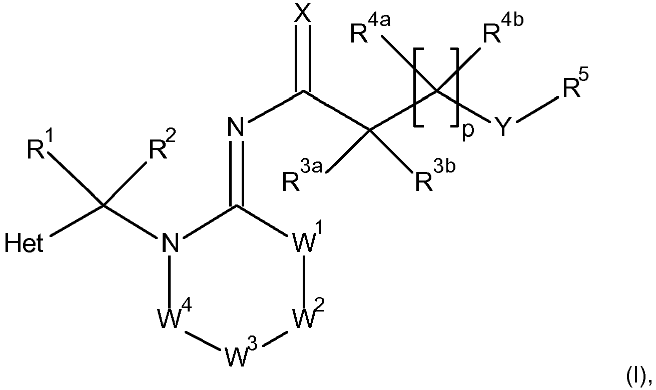 wherein
W¹, W², W³ and W⁴ represent a carbon chain group connected to N and C=N, and thus forming a saturated, unsaturated or partly unsaturated 6-membered nitrogen containing heterocycle selected from W.Het-1, W.Het-5 and W.Het-9 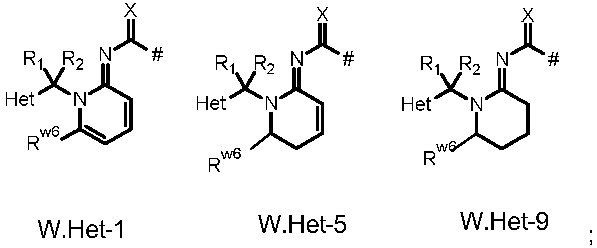 wherein # denotes the bond to the remainder of the molecule, and wherein R^{w6} is selected from hydrogen, halogen, C₁-C₄-alkyl; or C₁-C₄-haloalkyl;
and wherein
Het is selected from the group consisting of radicals of formulae Het-1, Het-11 a and Het-24 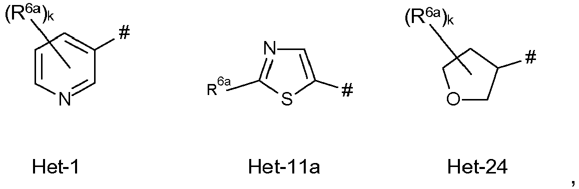
wherein # denotes the bond in formula (I), and wherein R^{6a} is selected from hydrogen, halogen, C₁-C₄-alkoxy or C₁-C₄-alkyl, wherein the carbon atoms of the latter two radicals may be partially of fully halogenated;
k is 0, 1 or 2;
and wherein further
R¹, R² are independently from each other selected from the group consisting of hydrogen, halogen, cyano, C₁-C₃-alkyl, or C₁-C₃-haloalkyl;
X is selected from O or S;
R^{3a}, R^{3b} are selected independently from one another from hydrogen, halogen, CN, C₁-C₆-alkyl, C₁-C₆-haloalkyl, or wherein R^{3a}, R^{3b} form together with the carbon atom they are bound to, a cyclocpropane ring, wherein each of the carbon atoms of the ring may be unsubstituted or may be partly or fully halogenated;
p is 0;
Y is S(O)ₘ, wherein m is 0, 1 or 2;
and
R⁵ is selected from the group consisting of hydrogen, halogen, CN, S-CN, C₁-C₆-alkyl, C₁-C₄ alkylthio, C₃-C₆-cycloalkyl, C₃-C₆ cycloalkylthio, C₂-C₆-alkenyl, C₂-C₆ alkenylthio, C₂-C₆-alkinyl or C₂-C₆ alkinylthio, wherein each of the eight last mentioned aliphatic and cycloaliphatic radicals are unsubstituted, partly or completely halogenated.

Especially preferred are compounds of formula (I), wherein
W¹, W², W³ and W⁴ represent a carbon chain group connected to N and C=N, and thus forming an unsaturated 6-membered nitrogen containing heterocycle W.Het-1 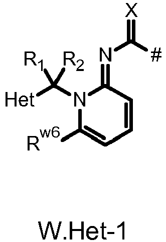 wherein # denotes the bond to the remainder of the molecule, and wherein R^{w6} is selected from hydrogen, halogen, C₁-C₄-alkyl or C₁-C₄-haloalkyl;
and wherein Het is Het-1 or Het-11a 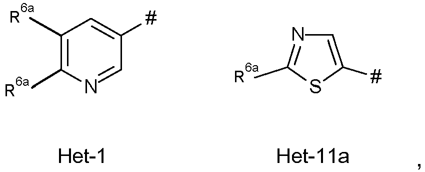 wherein # denotes the bond in formula (I), and
wherein R^{6a} is selected from hydrogen, halogen or C₁-C₄-haloalkyl;
- R¹, R²: are both hydrogen;
- X: is selected from O or S
and wherein further
R^{3a}, R^{3b} are selected independently from one another from hydrogen or fluoro;
p is 0;
Y is S(O)ₘ, wherein m is 0, 1 or 2;
and
R⁵ is C₁-C₃-alkyl, which is unsubstituted or partly or completely halogenated.

Examples of preferred and especially preferred compounds of formula I are given herein below.

Generally, preferred compounds of the present invention are compounds of the formulae I-A.0 to I-P.2 illustrated herein after, wherein Het, R¹, R² R^{3a}, R^{3b} and R⁵ have the meanings as given above.

Especially preferred compounds of formula (I-A.0) to formula (I-P.2) described below are those, wherein Het, R¹, R² R^{3a}, R^{3b} and R⁵ have the meanings given in any of lines 1 to 784 of the following table C.

Examples of such especially preferred compounds are compounds of formula (I-A.0), wherein Het, R¹, R² R^{3a}, R^{3b} and R⁵ have the meanings given in any of lines 1 to 784 of table C.

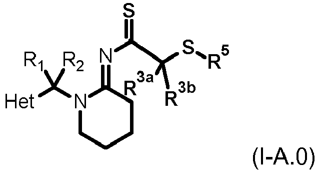

Examples of such especially preferred compounds are compounds of formula (I-A.1), wherein Het, R¹, R² R^{3a}, R^{3b} and R⁵ have the meanings given in any of lines 1 to 784 of table C.

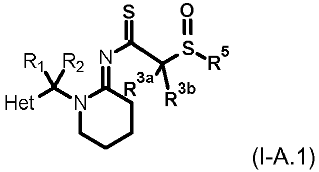

Examples of such especially preferred compounds are compounds of formula (I-A.2), wherein Het, R¹, R² R^{3a}, R^{3b} and R⁵ have the meanings given in any of lines 1 to 784 of table C.

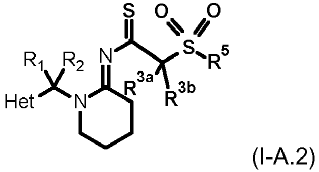

Examples of such especially preferred compounds are compounds of formula (I-B.0), wherein Het, R¹, R² R^{3a}, R^{3b} and R⁵ have the meanings given in any of lines 1 to 784 of table C.

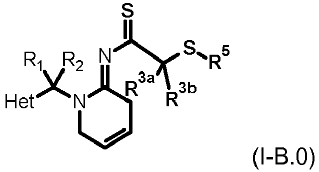

Examples of such especially preferred compounds are compounds of formula (I-B.1), wherein Het, R¹, R² R^{3a}, R^{3b} and R⁵ have the meanings given in any of lines 1 to 784 of table C.

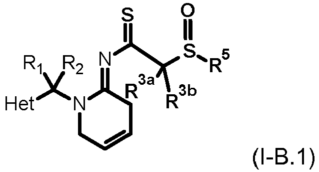

Examples of such especially preferred compounds are compounds of formula (I-B.2), wherein Het, R¹, R² R^{3a}, R^{3b} and R⁵ have the meanings given in any of lines 1 to 784 of table C.

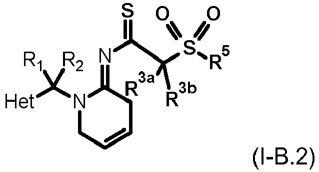

Examples of such especially preferred compounds are compounds of formula (I-C.0), wherein Het, R¹, R² R^{3a}, R^{3b} and R⁵ have the meanings given in any of lines 1 to 784 of table C.

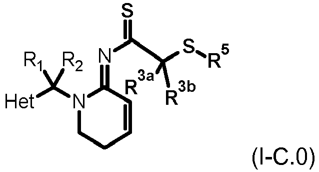

Examples of such especially preferred compounds are compounds of formula (I-C.1), wherein Het, R¹, R² R^{3a}, R^{3b} and R⁵ have the meanings given in any of lines 1 to 784 of table C.

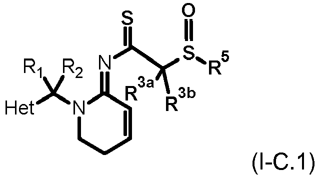

Examples of such especially preferred compounds are compounds of formula (I-C.2), wherein Het, R¹, R² R^{3a}, R^{3b} and R⁵ have the meanings given in any of lines 1 to 784 of table C.

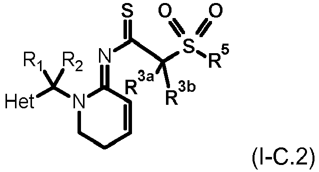

Examples of such especially preferred compounds are compounds of formula (I-D.0), wherein Het, R¹, R² R^{3a}, R^{3b} and R⁵ have the meanings given in any of lines 1 to 784 of table C.

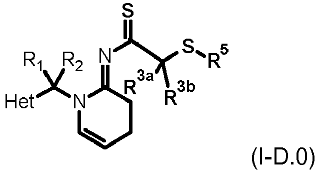

Examples of such especially preferred compounds are compounds of formula (I-D.1), wherein Het, R¹, R² R^{3a}, R^{3b} and R⁵ have the meanings given in any of lines 1 to 784 of table C.

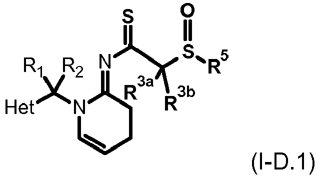

Examples of such especially preferred compounds are compounds of formula (I-D.2), wherein Het, R¹, R² R^{3a}, R^{3b} and R⁵ have the meanings given in any of lines 1 to 784 of table C.

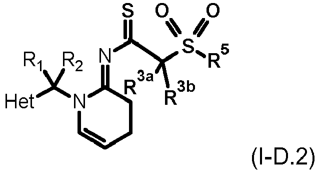

Examples of such especially preferred compounds are compounds of formula (I-E.0), wherein Het, R¹, R² R^{3a}, R^{3b} and R⁵ have the meanings given in any of lines 1 to 784 of table C.

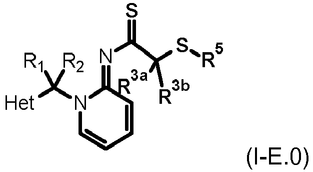

Examples of such especially preferred compounds are compounds of formula (I-E.1), wherein Het, R¹, R² R^{3a}, R^{3b} and R⁵ have the meanings given in any of lines 1 to 784 of table C.

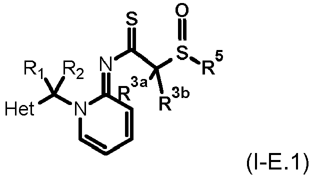

Examples of such especially preferred compounds are compounds of formula (I-E.2), wherein Het, R¹, R² R^{3a}, R^{3b} and R⁵ have the meanings given in any of lines 1 to 784 of table C.

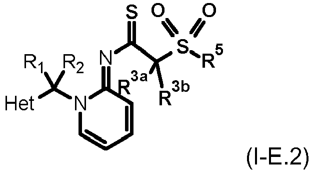

Examples of such especially preferred compounds are compounds of formula (I-F.0), wherein Het, R¹, R² R^{3a}, R^{3b} and R⁵ have the meanings given in any of lines 1 to 784 of table C.

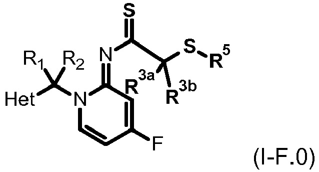

Examples of such especially preferred compounds are compounds of formula (I-F.1), wherein Het, R¹, R² R^{3a}, R^{3b} and R⁵ have the meanings given in any of lines 1 to 784 of table C.

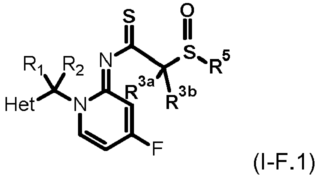

Examples of such especially preferred compounds are compounds of formula (I-F.2), wherein Het, R¹, R² R³a, R^{3b} and R⁵ have the meanings given in any of lines 1 to 784 of table C.

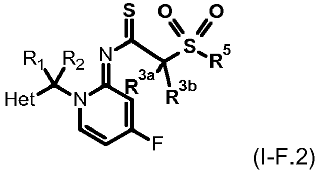

Examples of such especially preferred compounds are compounds of formula (I-G.0), wherein Het, R¹, R² R^{3a}, R^{3b} and R⁵ have the meanings given in any of lines 1 to 784 of table C.

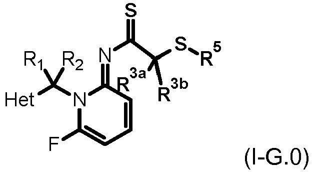

Examples of such especially preferred compounds are compounds of formula (I-G.1), wherein Het, R¹, R² R^{3a}, R^{3b} and R⁵ have the meanings given in any of lines 1 to 784 of table C.

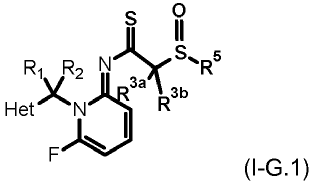

Examples of such especially preferred compounds are compounds of formula (I-G.2), wherein Het, R¹, R² R^{3a}, R^{3b} and R⁵ have the meanings given in any of lines 1 to 784 of table C.

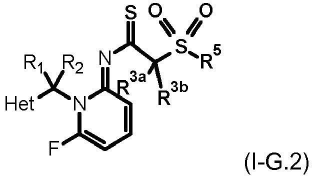

Examples of such especially preferred compounds are compounds of formula (I-H.0), wherein Het, R¹, R² R^{3a}, R^{3b} and R⁵ have the meanings given in any of lines 1 to 784 of table C.

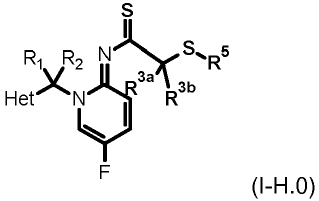

Examples of such especially preferred compounds are compounds of formula (I-H.1), wherein Het, R¹, R² R³a, R^{3b} and R⁵ have the meanings given in any of lines 1 to 784 of table C.

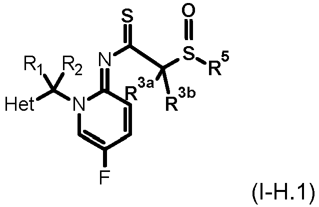

Examples of such especially preferred compounds are compounds of formula (I-H.2), wherein Het, R¹, R² R^{3a}, R^{3b} and R⁵ have the meanings given in any of lines 1 to 784 of table C.

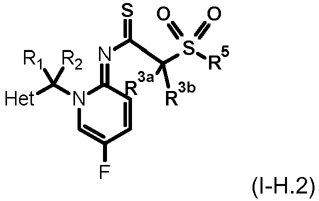

Examples of such especially preferred compounds are compounds of formula (I-I.0), wherein Het, R¹, R² R^{3a}, R^{3b} and R⁵ have the meanings given in any of lines 1 to 784 of table C.

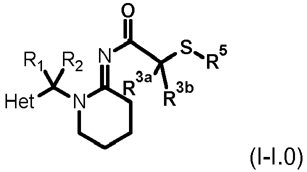

Examples of such especially preferred compounds are compounds of formula (I-I.1), wherein Het, R¹, R² R^{3a}, R^{3b} and R⁵ have the meanings given in any of lines 1 to 784 of table C.

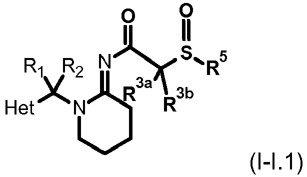

Examples of such especially preferred compounds are compounds of formula (I-I.2), wherein Het, R¹, R² R^{3a}, R^{3b} and R⁵ have the meanings given in any of lines 1 to 784 of table C.

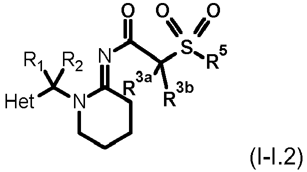

Examples of such especially preferred compounds are compounds of formula (I-J.0), wherein Het, R¹, R² R^{3a}, R^{3b} and R⁵ have the meanings given in any of lines 1 to 784 of table C.

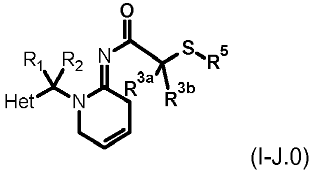

Examples of such especially preferred compounds are compounds of formula (I-J.1), wherein Het, R¹, R² R^{3a}, R^{3b} and R⁵ have the meanings given in any of lines 1 to 784 of table C.

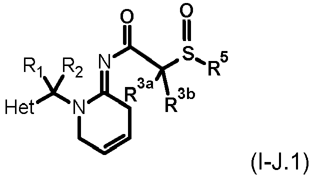

Examples of such especially preferred compounds are compounds of formula (I-J.2), wherein Het, R¹, R² R^{3a}, R^{3b} and R⁵ have the meanings given in any of lines 1 to 784 of table C.

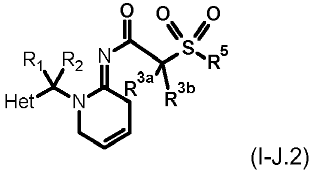

Examples of such especially preferred compounds are compounds of formula (I-K.0), wherein Het, R¹, R² R^{3a}, R^{3b} and R⁵ have the meanings given in any of lines 1 to 784 of table C.

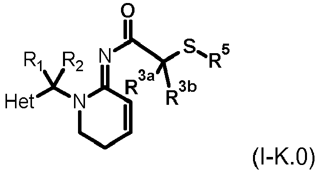

Examples of such especially preferred compounds are compounds of formula (I-K.1), wherein Het, R¹, R² R^{3a}, R^{3b} and R⁵ have the meanings given in any of lines 1 to 784 of table C.

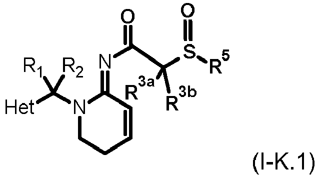

Examples of such especially preferred compounds are compounds of formula (I-K.2), wherein Het, R¹, R² R^{3a}, R^{3b} and R⁵ have the meanings given in any of lines 1 to 784 of table C.

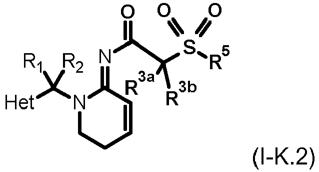

Examples of such especially preferred compounds are compounds of formula (I-L.0), wherein Het, R¹, R² R^{3a}, R^{3b} and R⁵ have the meanings given in any of lines 1 to 784 of table C.

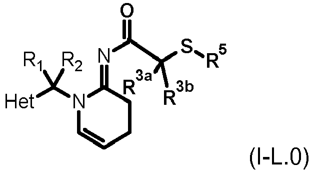

Examples of such especially preferred compounds are compounds of formula (I-L.1), wherein Het, R¹, R² R^{3a}, R^{3b} and R⁵ have the meanings given in any of lines 1 to 784 of table C.

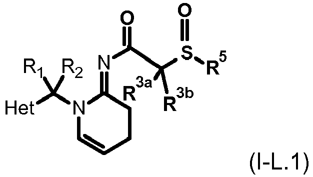

Examples of such especially preferred compounds are compounds of formula (I-L.2), wherein Het, R¹, R² R^{3a}, R^{3b} and R⁵ have the meanings given in any of lines 1 to 784 of table C.

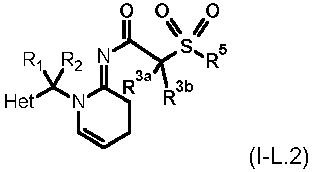

Examples of such especially preferred compounds are compounds of formula (I-M.0), wherein Het, R¹, R² R^{3a}, R^{3b} and R⁵ have the meanings given in any of lines 1 to 784 of table C.

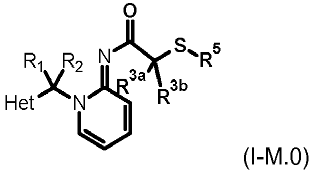

Examples of such especially preferred compounds are compounds of formula (I-M.1), wherein Het, R¹, R² R^{3a}, R^{3b} and R⁵ have the meanings given in any of lines 1 to 784 of table C.

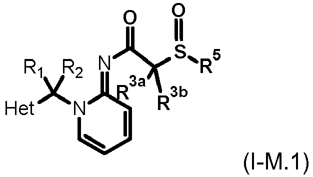

Examples of such especially preferred compounds are compounds of formula (I-M.2), wherein Het, R¹, R² R^{3a}, R^{3b} and R⁵ have the meanings given in any of lines 1 to 784 of table C.

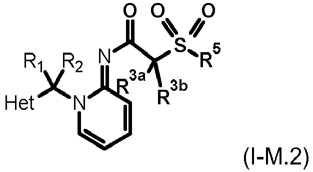

Examples of such especially preferred compounds are compounds of formula (I-N.0), wherein Het, R¹, R² R^{3a}, R^{3b} and R⁵ have the meanings given in any of lines 1 to 784 of table C.

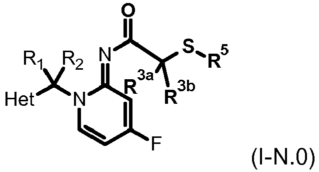

Examples of such especially preferred compounds are compounds of formula (I-N.1), wherein Het, R¹, R² R^{3a}, R^{3b} and R⁵ have the meanings given in any of lines 1 to 784 of table C.

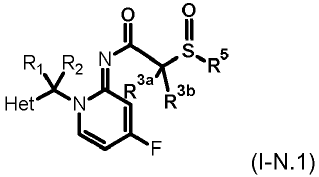

Examples of such especially preferred compounds are compounds of formula (I-N.2), wherein Het, R¹, R² R^{3a}, R^{3b} and R⁵ have the meanings given in any of lines 1 to 784 of table C.

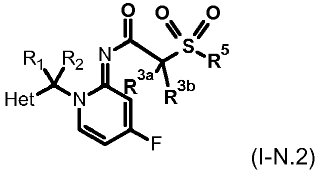

Examples of such especially preferred compounds are compounds of formula (I-O.0), wherein Het, R¹, R² R^{3a}, R^{3b} and R⁵ have the meanings given in any of lines 1 to 784 of table C.

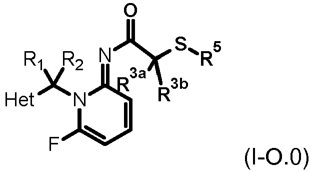

Examples of such especially preferred compounds are compounds of formula (I-O.1), wherein Het, R¹, R² R^{3a}, R^{3b} and R⁵ have the meanings given in any of lines 1 to 784 of table C.

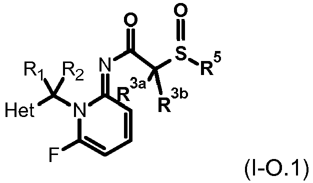

Examples of such especially preferred compounds are compounds of formula (I-0.2), wherein Het, R¹, R² R^{3a}, R^{3b} and R⁵ have the meanings given in any of lines 1 to 784 of table C.

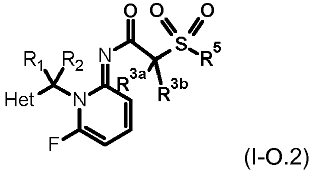

Examples of such especially preferred compounds are compounds of formula (I-P.0), wherein Het, R¹, R² R^{3a}, R^{3b} and R⁵ have the meanings given in any of lines 1 to 784 of table C.

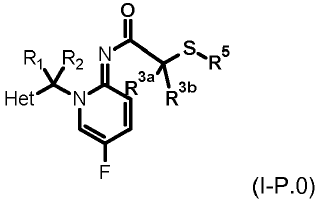

Examples of such especially preferred compounds are compounds of formula (I-P.1), wherein Het, R¹, R² R^{3a}, R^{3b} and R⁵ have the meanings given in any of lines 1 to 784 of table C.

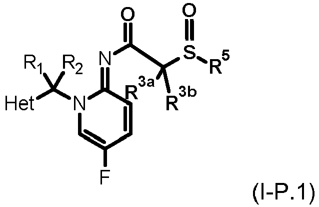

Examples of such especially preferred compounds are compounds of formula (I-P.2), wherein Het, R¹, R² R^{3a}, R^{3b} and R⁵ have the meanings given in any of lines 1 to 784 of table C.

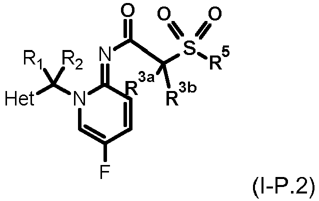

**Table C:**

| **Compound no.** | **Het^{(*)}** | **R¹** | **R²** | **R^{3a}** | **R^{3b}** | **R⁵** |
|---|---|---|---|---|---|---|
| **C.1** | 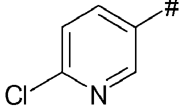 | H | H | H | H | CH₃ |
| **C.2** | 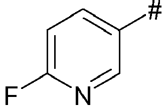 | H | H | H | H | CH₃ |
| **C.3** | 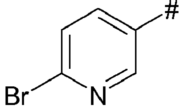 | H | H | H | H | CH₃ |
| **C.4** | 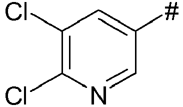 | H | H | H | H | CH₃ |
| **C.5** | 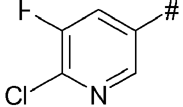 | H | H | H | H | CH₃ |
| **C.6** | 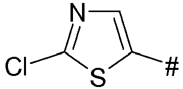 | H | H | H | H | CH₃ |
| **C.7** | 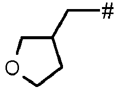 | H | H | H | H | CH₃ |
| **C.8** | 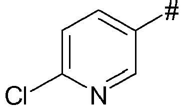 | H | CH₃ | H | H | CH₃ |
| **C.9** | 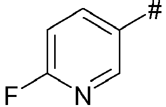 | H | CH₃ | H | H | CH₃ |
| **C.10** | 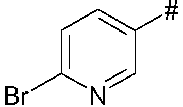 | H | CH₃ | H | H | CH₃ |
| **C.11** | 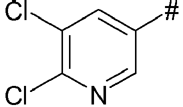 | H | CH₃ | H | H | CH₃ |
| **C.12** | 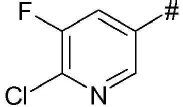 | H | CH₃ | H | H | CH₃ |
| **C.13** | 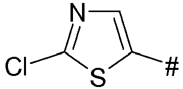 | H | CH₃ | H | H | CH₃ |
| **C.14** | 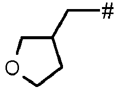 | H | CH₃ | H | H | CH₃ |
| **C.15** | 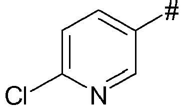 | H | H | H | F | CH₃ |
| **C.16** | 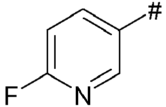 | H | H | H | F | CH₃ |
| **C.17** | 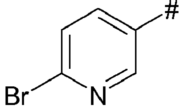 | H | H | H | F | CH₃ |
| **C.18** | 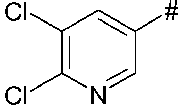 | H | H | H | F | CH₃ |
| **C.19** | 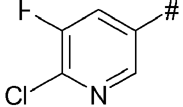 | H | H | H | F | CH₃ |
| **C.20** | 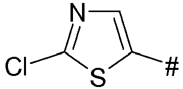 | H | H | H | F | CH₃ |
| **C.21** | 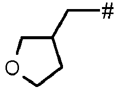 | H | H | H | F | CH₃ |
| **C.22** | 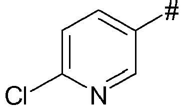 | H | CH₃ | H | F | CH₃ |
| **C.23** | 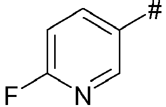 | H | CH₃ | H | F | CH₃ |
| **C.24** | 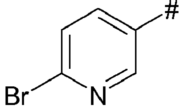 | H | CH₃ | H | F | CH₃ |
| **C.25** | 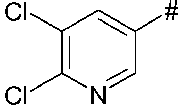 | H | CH₃ | H | F | CH₃ |
| **C.26** | 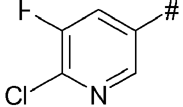 | H | CH₃ | H | F | CH₃ |
| **C.27** | 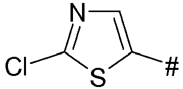 | H | CH₃ | H | F | CH₃ |
| **C.28** | 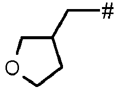 | H | CH₃ | H | F | CH₃ |
| **C.29** | 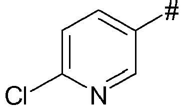 | H | H | F | F | CH₃ |
| **C.30** | 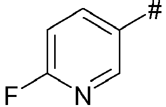 | H | H | F | F | CH₃ |
| **C.31** | 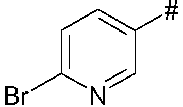 | H | H | F | F | CH₃ |
| **C.32** | 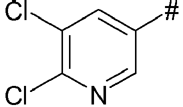 | H | H | F | F | CH₃ |
| **C.33** | 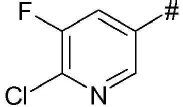 | H | H | F | F | CH₃ |
| **C.34** | 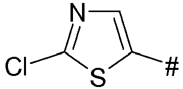 | H | H | F | F | CH₃ |
| **C.35** | 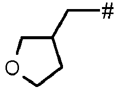 | H | H | F | F | CH₃ |
| **C.36** | 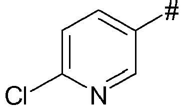 | H | CH₃ | F | F | CH₃ |
| **C.37** | 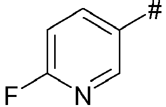 | H | CH₃ | F | F | CH₃ |
| **C.38** | 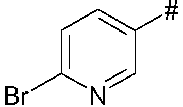 | H | CH₃ | F | F | CH₃ |
| **C.39** | 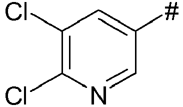 | H | CH₃ | F | F | CH₃ |
| **C.40** | 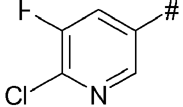 | H | CH₃ | F | F | CH₃ |
| **C.41** | 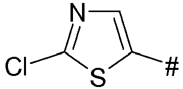 | H | CH₃ | F | F | CH₃ |
| **C.42** | 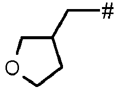 | H | CH₃ | F | F | CH₃ |
| **C.43** | 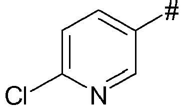 | H | H | H | CH₃ | CH₃ |
| **C.44** | 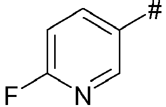 | H | H | H | CH₃ | CH₃ |
| **C.45** | 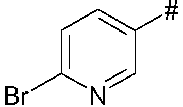 | H | H | H | CH₃ | CH₃ |
| **C.46** | 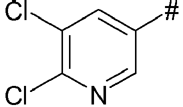 | H | H | H | CH₃ | CH₃ |
| **C.47** | 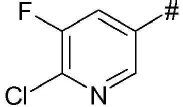 | H | H | H | CH₃ | CH₃ |
| **C.48** | 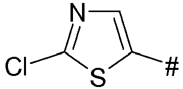 | H | H | H | CH₃ | CH₃ |
| **C.49** | 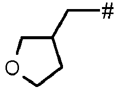 | H | H | H | CH₃ | CH₃ |
| **C.50** | 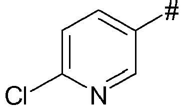 | H | CH₃ | H | CH₃ | CH₃ |
| **C.51** | 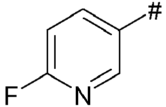 | H | CH₃ | H | CH₃ | CH₃ |
| **C.52** | 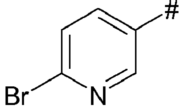 | H | CH₃ | H | CH₃ | CH₃ |
| **C.53** | 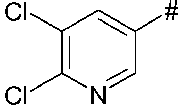 | H | CH₃ | H | CH₃ | CH₃ |
| **C.54** | 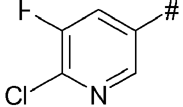 | H | CH₃ | H | CH₃ | CH₃ |
| **C.55** | 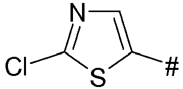 | H | CH₃ | H | CH₃ | CH₃ |
| **C.56** | 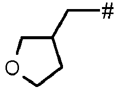 | H | CH₃ | H | CH₃ | CH₃ |
| **C.57** | 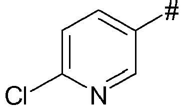 | H | H | H | H | CH₂F |
| **C.58** | 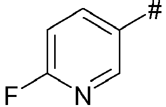 | H | H | H | H | CH₂F |
| **C.59** | 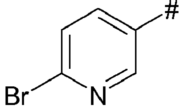 | H | H | H | H | CH₂F |
| **C.60** | 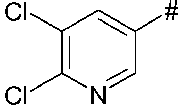 | H | H | H | H | CH₂F |
| **C.61** | 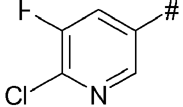 | H | H | H | H | CH₂F |
| **C.62** | 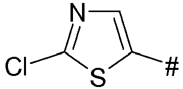 | H | H | H | H | CH₂F |
| **C.63** | 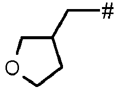 | H | H | H | H | CH₂F |
| **C.64** | 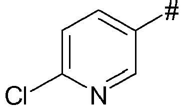 | H | CH₃ | H | H | CH₂F |
| **C.65** | 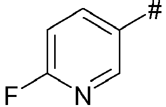 | H | CH₃ | H | H | CH₂F |
| **C.66** | 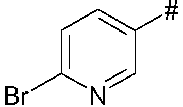 | H | CH₃ | H | H | CH₂F |
| **C.67** | 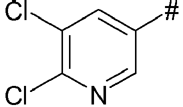 | H | CH₃ | H | H | CH₂F |
| **C.68** | 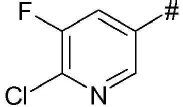 | H | CH₃ | H | H | CH₂F |
| **C.69** | 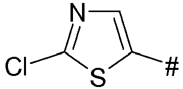 | H | CH₃ | H | H | CH₂F |
| **C.70** | 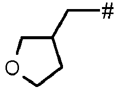 | H | CH₃ | H | H | CH₂F |
| **C.71** | 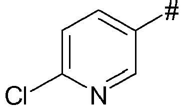 | H | H | H | F | CH₂F |
| **C.72** | 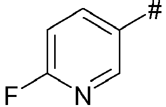 | H | H | H | F | CH₂F |
| **C.73** | 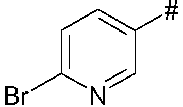 | H | H | H | F | CH₂F |
| **C.74** | 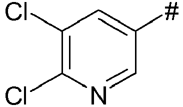 | H | H | H | F | CH₂F |
| **C.75** | 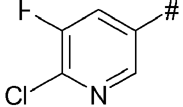 | H | H | H | F | CH₂F |
| **C.76** | 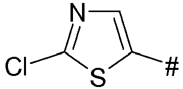 | H | H | H | F | CH₂F |
| **C.77** | 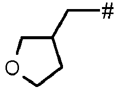 | H | H | H | F | CH₂F |
| **C.78** | 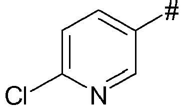 | H | CH₃ | H | F | CH₂F |
| **C.79** | 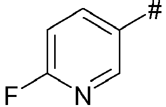 | H | CH₃ | H | F | CH₂F |
| **C.80** | 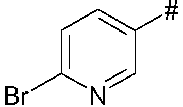 | H | CH₃ | H | F | CH₂F |
| **C.81** | 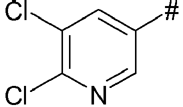 | H | CH₃ | H | F | CH₂F |
| **C.82** | 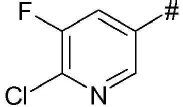 | H | CH₃ | H | F | CH₂F |
| **C.83** | 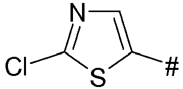 | H | CH₃ | H | F | CH₂F |
| **C.84** | 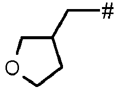 | H | CH₃ | H | F | CH₂F |
| **C.85** | 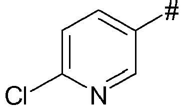 | H | H | F | F | CH₂F |
| **C.86** | 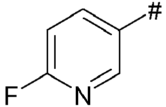 | H | H | F | F | CH₂F |
| **C.87** | 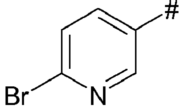 | H | H | F | F | CH₂F |
| **C.88** | 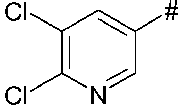 | H | H | F | F | CH₂F |
| **C.89** | 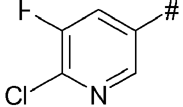 | H | H | F | F | CH₂F |
| **C.90** | 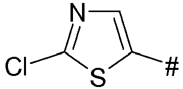 | H | H | F | F | CH₂F |
| **C.91** | 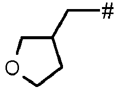 | H | H | F | F | CH₂F |
| **C.92** | 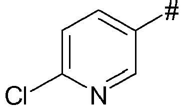 | H | CH₃ | F | F | CH₂F |
| **C.93** | 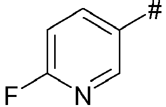 | H | CH₃ | F | F | CH₂F |
| **C.94** | 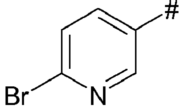 | H | CH₃ | F | F | CH₂F |
| **C.95** | 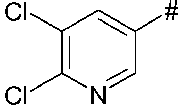 | H | CH₃ | F | F | CH₂F |
| **C.96** | 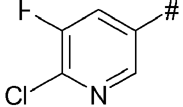 | H | CH₃ | F | F | CH₂F |
| **C.97** | 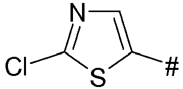 | H | CH₃ | F | F | CH₂F |
| **C.98** | 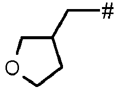 | H | CH₃ | F | F | CH₂F |
| **C.99** | 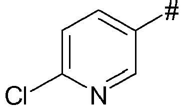 | H | H | H | CH₃ | CH₂F |
| **C.100** | 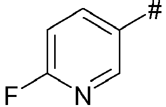 | H | H | H | CH₃ | CH₂F |
| **C.101** | 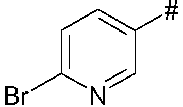 | H | H | H | CH₃ | CH₂F |
| **C.102** | 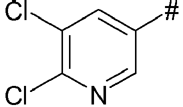 | H | H | H | CH₃ | CH₂F |
| **C.103** | 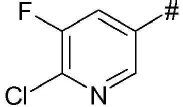 | H | H | H | CH₃ | CH₂F |
| **C.104** | 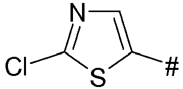 | H | H | H | CH₃ | CH₂F |
| **C.105** | 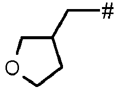 | H | H | H | CH₃ | CH₂F |
| **C.106** | 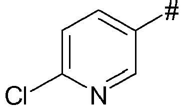 | H | CH₃ | H | CH₃ | CH₂F |
| **C.107** | 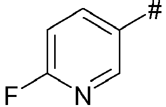 | H | CH₃ | H | CH₃ | CH₂F |
| **C.108** | 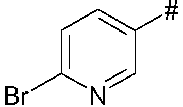 | H | CH₃ | H | CH₃ | CH₂F |
| **C.109** | 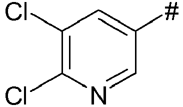 | H | CH₃ | H | CH₃ | CH₂F |
| **C.110** | 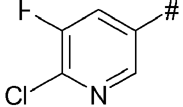 | H | CH₃ | H | CH₃ | CH₂F |
| **C.111** | 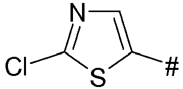 | H | CH₃ | H | CH₃ | CH₂F |
| **C.112** | 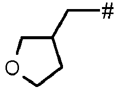 | H | CH₃ | H | CH₃ | CH₂F |
| **C.113** | 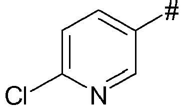 | H | H | H | H | CHF₂ |
| **C.114** | 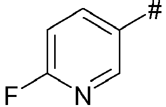 | H | H | H | H | CHF₂ |
| **C.115** | 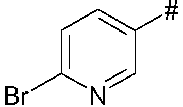 | H | H | H | H | CHF₂ |
| **C.116** | 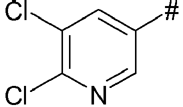 | H | H | H | H | CHF₂ |
| **C.117** | 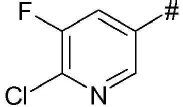 | H | H | H | H | CHF₂ |
| **C.118** | 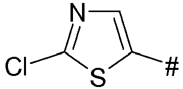 | H | H | H | H | CHF₂ |
| **C.119** | 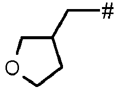 | H | H | H | H | CHF₂ |
| **C.120** | 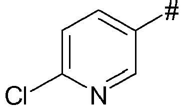 | H | CH₃ | H | H | CHF₂ |
| **C.121** | 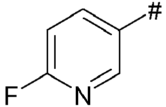 | H | CH₃ | H | H | CHF₂ |
| **C.122** | 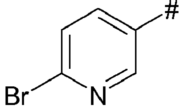 | H | CH₃ | H | H | CHF₂ |
| **C.123** | 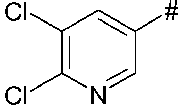 | H | CH₃ | H | H | CHF₂ |
| **C.124** | 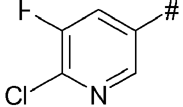 | H | CH₃ | H | H | CHF₂ |
| **C.125** | 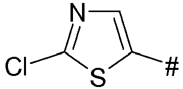 | H | CH₃ | H | H | CHF₂ |
| **C.126** | 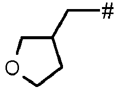 | H | CH₃ | H | H | CHF₂ |
| **C.127** | 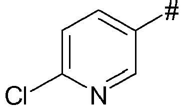 | H | H | H | F | CHF₂ |
| **C.128** | 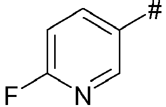 | H | H | H | F | CHF₂ |
| **C.129** | 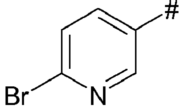 | H | H | H | F | CHF₂ |
| **C.130** | 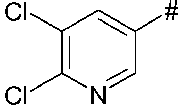 | H | H | H | F | CHF₂ |
| **C.131** | 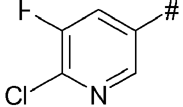 | H | H | H | F | CHF₂ |
| **C.132** | 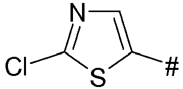 | H | H | H | F | CHF₂ |
| **C.133** | 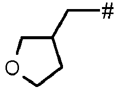 | H | H | H | F | CHF₂ |
| **C.134** | 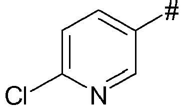 | H | CH₃ | H | F | CHF₂ |
| **C.135** | 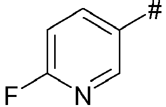 | H | CH₃ | H | F | CHF₂ |
| **C.136** | 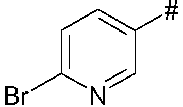 | H | CH₃ | H | F | CHF₂ |
| **C.137** | 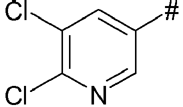 | H | CH₃ | H | F | CHF₂ |
| **C.138** | 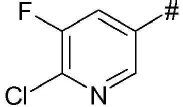 | H | CH₃ | H | F | CHF₂ |
| **C.139** | 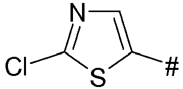 | H | CH₃ | H | F | CHF₂ |
| **C.140** | 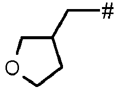 | H | CH₃ | H | F | CHF₂ |
| **C.141** | 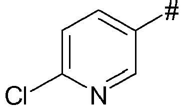 | H | H | F | F | CHF₂ |
| **C.142** | 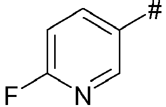 | H | H | F | F | CHF₂ |
| **C.143** | 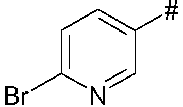 | H | H | F | F | CHF₂ |
| **C.144** | 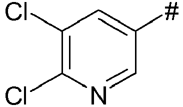 | H | H | F | F | CHF₂ |
| **C.145** | 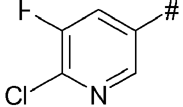 | H | H | F | F | CHF₂ |
| **C.146** | 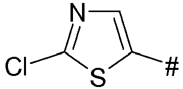 | H | H | F | F | CHF₂ |
| **C.147** | 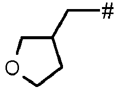 | H | H | F | F | CHF₂ |
| **C.148** | 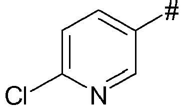 | H | CH₃ | F | F | CHF₂ |
| **C.149** | 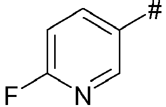 | H | CH₃ | F | F | CHF₂ |
| **C.150** | 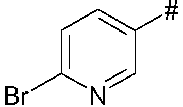 | H | CH₃ | F | F | CHF₂ |
| **C.151** | 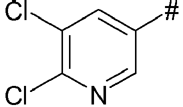 | H | CH₃ | F | F | CHF₂ |
| **C.152** | 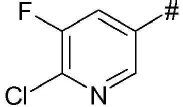 | H | CH₃ | F | F | CHF₂ |
| **C.153** | 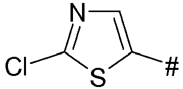 | H | CH₃ | F | F | CHF₂ |
| **C.154** | 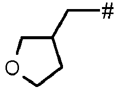 | H | CH₃ | F | F | CHF₂ |
| **C.155** | 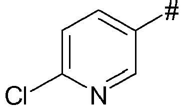 | H | H | H | CH₃ | CHF₂ |
| **C.156** | 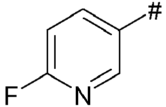 | H | H | H | CH₃ | CHF₂ |
| **C.157** | 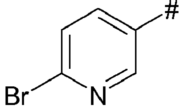 | H | H | H | CH₃ | CHF₂ |
| **C.158** | 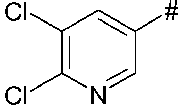 | H | H | H | CH₃ | CHF₂ |
| **C.159** | 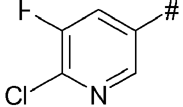 | H | H | H | CH₃ | CHF₂ |
| **C.160** | 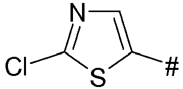 | H | H | H | CH₃ | CHF₂ |
| **C.161** | 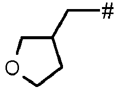 | H | H | H | CH₃ | CHF₂ |
| **C.162** | 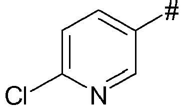 | H | CH₃ | H | CH₃ | CHF₂ |
| **C.163** | 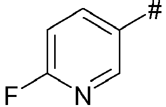 | H | CH₃ | H | CH₃ | CHF₂ |
| **C.164** | 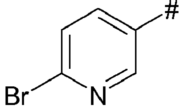 | H | CH₃ | H | CH₃ | CHF₂ |
| **C.165** | 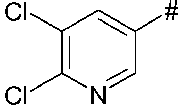 | H | CH₃ | H | CH₃ | CHF₂ |
| **C.166** | 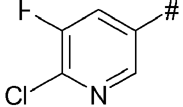 | H | CH₃ | H | CH₃ | CHF₂ |
| **C.167** | 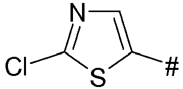 | H | CH₃ | H | CH₃ | CHF₂ |
| **C.168** | 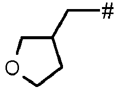 | H | CH₃ | H | CH₃ | CHF₂ |
| **C.169** | 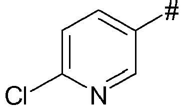 | H | H | H | H | CF₃ |
| **C.170** | 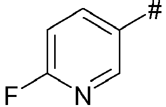 | H | H | H | H | CF₃ |
| **C.171** | 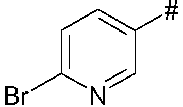 | H | H | H | H | CF₃ |
| **C.172** | 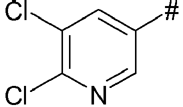 | H | H | H | H | CF₃ |
| **C.173** | 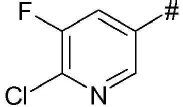 | H | H | H | H | CF₃ |
| **C.174** | 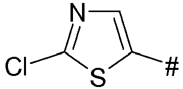 | H | H | H | H | CF₃ |
| **C.175** | 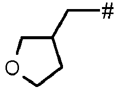 | H | H | H | H | CF₃ |
| **C.176** | 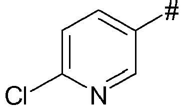 | H | CH₃ | H | H | CF₃ |
| **C.177** | 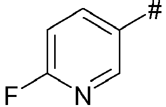 | H | CH₃ | H | H | CF₃ |
| **C.178** | 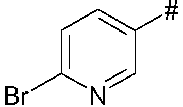 | H | CH₃ | H | H | CF₃ |
| **C.179** | 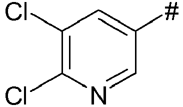 | H | CH₃ | H | H | CF₃ |
| **C.180** | 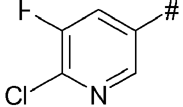 | H | CH₃ | H | H | CF₃ |
| **C.181** | 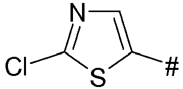 | H | CH₃ | H | H | CF₃ |
| **C.182** | 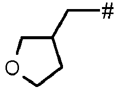 | H | CH₃ | H | H | CF₃ |
| **C.183** | 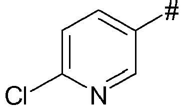 | H | H | H | F | CF₃ |
| **C.184** | 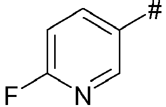 | H | H | H | F | CF₃ |
| **C.185** | 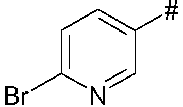 | H | H | H | F | CF₃ |
| **C.186** | 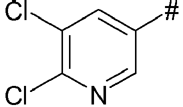 | H | H | H | F | CF₃ |
| **C.187** | 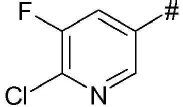 | H | H | H | F | CF₃ |
| **C.188** | 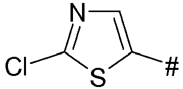 | H | H | H | F | CF₃ |
| **C.189** | 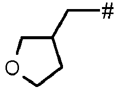 | H | H | H | F | CF₃ |
| **C.190** | 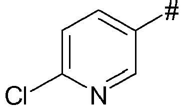 | H | CH₃ | H | F | CF₃ |
| **C.191** | 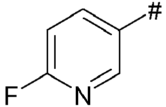 | H | CH₃ | H | F | CF₃ |
| **C.192** | 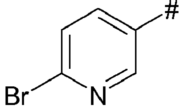 | H | CH₃ | H | F | CF₃ |
| **C.193** | 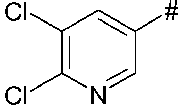 | H | CH₃ | H | F | CF₃ |
| **C.194** | 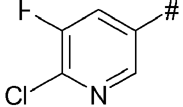 | H | CH₃ | H | F | CF₃ |
| **C.195** | 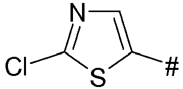 | H | CH₃ | H | F | CF₃ |
| **C.196** | 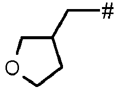 | H | CH₃ | H | F | CF₃ |
| **C.197** | 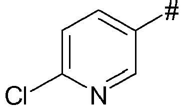 | H | H | F | F | CF₃ |
| **C.198** | 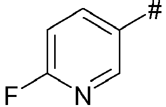 | H | H | F | F | CF₃ |
| **C.199** | 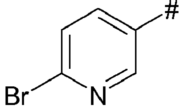 | H | H | F | F | CF₃ |
| **C.200** | 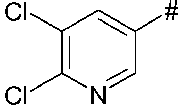 | H | H | F | F | CF₃ |
| **C.201** | 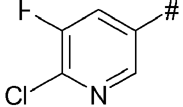 | H | H | F | F | CF₃ |
| **C.202** | 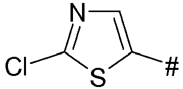 | H | H | F | F | CF₃ |
| **C.203** | 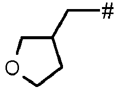 | H | H | F | F | CF₃ |
| **C.204** | 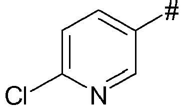 | H | CH₃ | F | F | CF₃ |
| **C.205** | 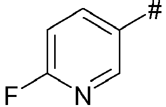 | H | CH₃ | F | F | CF₃ |
| **C.206** | 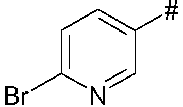 | H | CH₃ | F | F | CF₃ |
| **C.207** | 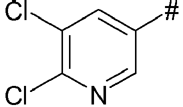 | H | CH₃ | F | F | CF₃ |
| **C.208** | 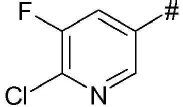 | H | CH₃ | F | F | CF₃ |
| **C.209** | 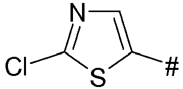 | H | CH₃ | F | F | CF₃ |
| **C.210** | 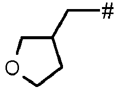 | H | CH₃ | F | F | CF₃ |
| **C.211** | 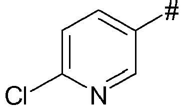 | H | H | H | CH₃ | CF₃ |
| **C.212** | 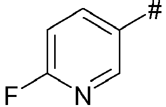 | H | H | H | CH₃ | CF₃ |
| **C.213** | 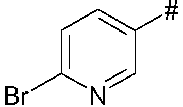 | H | H | H | CH₃ | CF₃ |
| **C.214** | 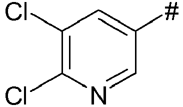 | H | H | H | CH₃ | CF₃ |
| **C.215** | 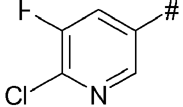 | H | H | H | CH₃ | CF₃ |
| **C.216** | 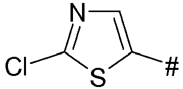 | H | H | H | CH₃ | CF₃ |
| **C.217** | 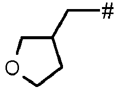 | H | H | H | CH₃ | CF₃ |
| **C.218** | 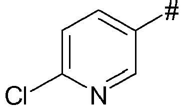 | H | CH₃ | H | CH₃ | CF₃ |
| **C.219** | 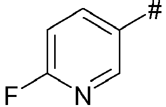 | H | CH₃ | H | CH₃ | CF₃ |
| **C.220** | 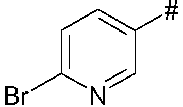 | H | CH₃ | H | CH₃ | CF₃ |
| **C.221** | 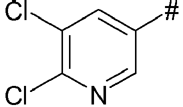 | H | CH₃ | H | CH₃ | CF₃ |
| **C.222** | 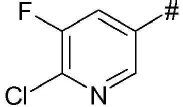 | H | CH₃ | H | CH₃ | CF₃ |
| **C.223** | 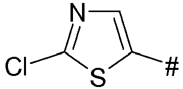 | H | CH₃ | H | CH₃ | CF₃ |
| **C.224** | 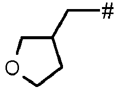 | H | CH₃ | H | CH₃ | CF₃ |
| **C.225** | 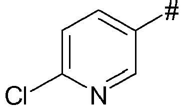 | H | H | H | H | CF₂Cl |
| **C.226** | 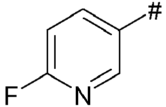 | H | H | H | H | CF₂Cl |
| **C.227** | 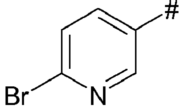 | H | H | H | H | CF₂Cl |
| **C.228** | 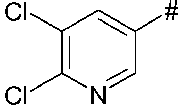 | H | H | H | H | CF₂Cl |
| **C.229** | 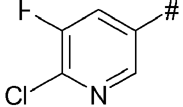 | H | H | H | H | CF₂Cl |
| **C.230** | 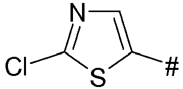 | H | H | H | H | CF₂Cl |
| **C.231** | 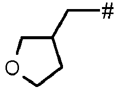 | H | H | H | H | CF₂Cl |
| **C.232** | 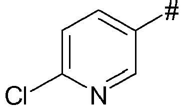 | H | CH₃ | H | H | CF₂Cl |
| **C.233** | 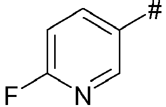 | H | CH₃ | H | H | CF₂Cl |
| **C.234** | 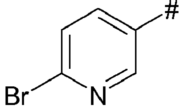 | H | CH₃ | H | H | CF₂Cl |
| **C.235** | 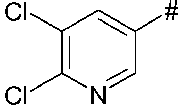 | H | CH₃ | H | H | CF₂Cl |
| **C.236** | 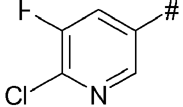 | H | CH₃ | H | H | CF₂Cl |
| **C.237** | 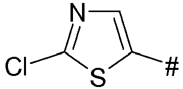 | H | CH₃ | H | H | CF₂Cl |
| **C.238** | 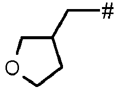 | H | CH₃ | H | H | CF₂Cl |
| **C.239** | 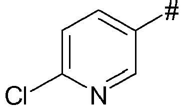 | H | H | H | F | CF₂Cl |
| **C.240** | 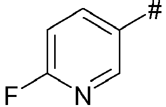 | H | H | H | F | CF₂Cl |
| **C.241** | 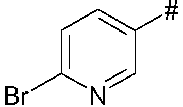 | H | H | H | F | CF₂Cl |
| **C.242** | 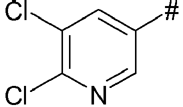 | H | H | H | F | CF₂Cl |
| **C.243** | 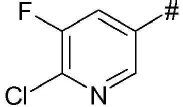 | H | H | H | F | CF₂Cl |
| **C.244** | 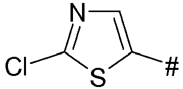 | H | H | H | F | CF₂Cl |
| **C.245** | 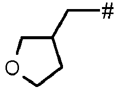 | H | H | H | F | CF₂Cl |
| **C.246** | 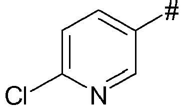 | H | CH₃ | H | F | CF₂Cl |
| **C.247** | 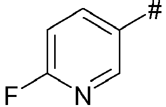 | H | CH₃ | H | F | CF₂Cl |
| **C.248** | 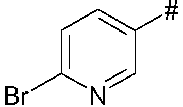 | H | CH₃ | H | F | CF₂Cl |
| **C.249** | 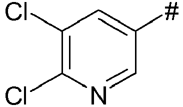 | H | CH₃ | H | F | CF₂Cl |
| **C.250** | 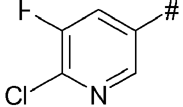 | H | CH₃ | H | F | CF₂Cl |
| **C.251** | 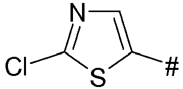 | H | CH₃ | H | F | CF₂Cl |
| **C.252** | 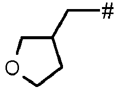 | H | CH₃ | H | F | CF₂Cl |
| **C.253** | 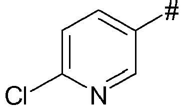 | H | H | F | F | CF₂Cl |
| **C.254** | 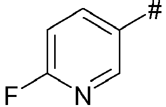 | H | H | F | F | CF₂Cl |
| **C.255** | 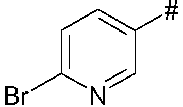 | H | H | F | F | CF₂Cl |
| **C.256** | 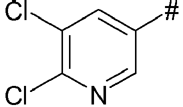 | H | H | F | F | CF₂Cl |
| **C.257** | 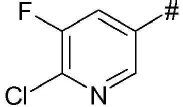 | H | H | F | F | CF₂Cl |
| **C.258** | 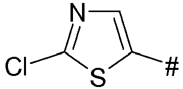 | H | H | F | F | CF₂Cl |
| **C.259** | 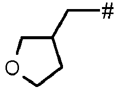 | H | H | F | F | CF₂Cl |
| **C.260** | 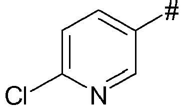 | H | CH₃ | F | F | CF₂Cl |
| **C.261** | 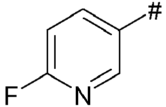 | H | CH₃ | F | F | CF₂Cl |
| **C.262** | 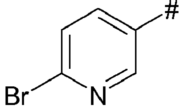 | H | CH₃ | F | F | CF₂Cl |
| **C.263** | 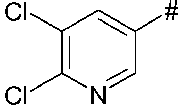 | H | CH₃ | F | F | CF₂Cl |
| **C.264** | 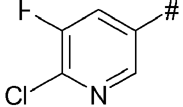 | H | CH₃ | F | F | CF₂Cl |
| **C.265** | 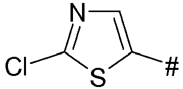 | H | CH₃ | F | F | CF₂Cl |
| **C.266** | 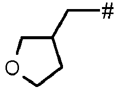 | H | CH₃ | F | F | CF₂Cl |
| **C.267** | 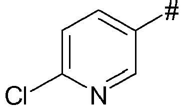 | H | H | H | CH₃ | CF₂Cl |
| **C.268** | 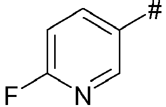 | H | H | H | CH₃ | CF₂Cl |
| **C.269** | 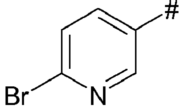 | H | H | H | CH₃ | CF₂Cl |
| **C.270** | 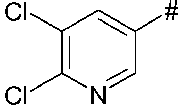 | H | H | H | CH₃ | CF₂Cl |
| **C.271** | 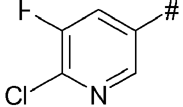 | H | H | H | CH₃ | CF₂Cl |
| **C.272** | 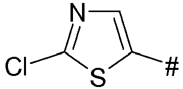 | H | H | H | CH₃ | CF₂Cl |
| **C.273** | 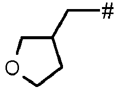 | H | H | H | CH₃ | CF₂Cl |
| **C.274** | 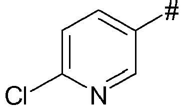 | H | CH₃ | H | CH₃ | CF₂Cl |
| **C.275** | 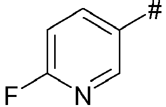 | H | CH₃ | H | CH₃ | CF₂Cl |
| **C.276** | 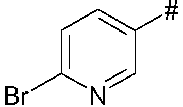 | H | CH₃ | H | CH₃ | CF₂Cl |
| **C.277** | 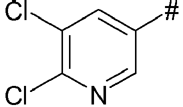 | H | CH₃ | H | CH₃ | CF₂Cl |
| **C.278** | 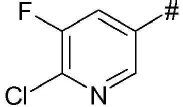 | H | CH₃ | H | CH₃ | CF₂Cl |
| **C.279** | 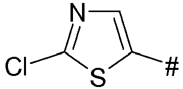 | H | CH₃ | H | CH₃ | CF₂Cl |
| **C.280** | 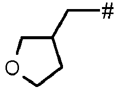 | H | CH₃ | H | CH₃ | CF₂Cl |
| **C.281** | 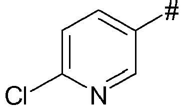 | H | H | H | H | C₂H₅ |
| **C.282** | 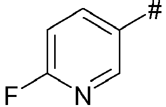 | H | H | H | H | C₂H₅ |
| **C.283** | 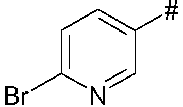 | H | H | H | H | C₂H₅ |
| **C.284** | 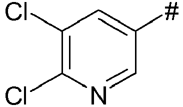 | H | H | H | H | C₂H₅ |
| **C.285** | 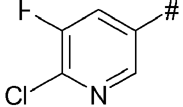 | H | H | H | H | C₂H₅ |
| **C.286** | 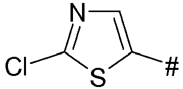 | H | H | H | H | C₂H₅ |
| **C.287** | 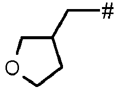 | H | H | H | H | C₂H₅ |
| **C.288** | 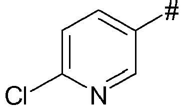 | H | CH₃ | H | H | C₂H₅ |
| **C.289** | 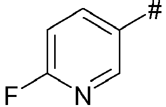 | H | CH₃ | H | H | C₂H₅ |
| **C.290** | 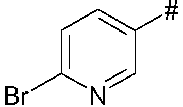 | H | CH₃ | H | H | C₂H₅ |
| **C.291** | 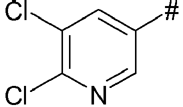 | H | CH₃ | H | H | C₂H₅ |
| **C.292** | 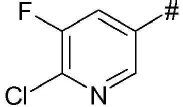 | H | CH₃ | H | H | C₂H₅ |
| **C.293** | 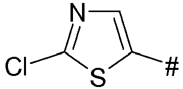 | H | CH₃ | H | H | C₂H₅ |
| **C.294** | 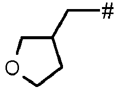 | H | CH₃ | H | H | C₂H₅ |
| **C.295** | 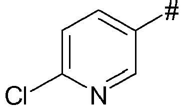 | H | H | H | F | C₂H₅ |
| **C.296** | 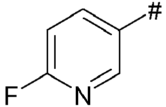 | H | H | H | F | C₂H₅ |
| **C.297** | 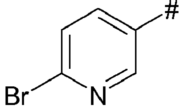 | H | H | H | F | C₂H₅ |
| **C.298** | 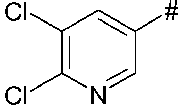 | H | H | H | F | C₂H₅ |
| **C.299** | 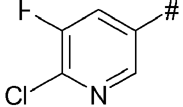 | H | H | H | F | C₂H₅ |
| **C.300** | 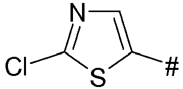 | H | H | H | F | C₂H₅ |
| **C.301** | 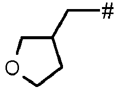 | H | H | H | F | C₂H₅ |
| **C.302** | 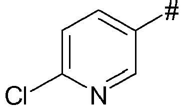 | H | CH₃ | H | F | C₂H₅ |
| **C.303** | 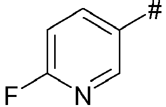 | H | CH₃ | H | F | C₂H₅ |
| **C.304** | 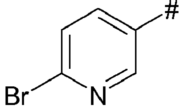 | H | CH₃ | H | F | C₂H₅ |
| **C.305** | 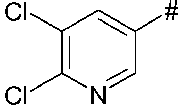 | H | CH₃ | H | F | C₂H₅ |
| **C.306** | 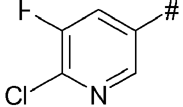 | H | CH₃ | H | F | C₂H₅ |
| **C.307** | 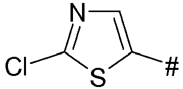 | H | CH₃ | H | F | C₂H₅ |
| **C.308** | 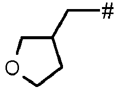 | H | CH₃ | H | F | C₂H₅ |
| **C.309** | 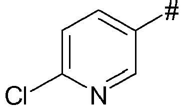 | H | H | F | F | C₂H₅ |
| **C.310** | 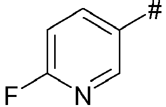 | H | H | F | F | C₂H₅ |
| **C.311** | 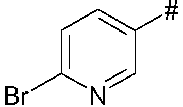 | H | H | F | F | C₂H₅ |
| **C.312** | 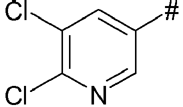 | H | H | F | F | C₂H₅ |
| **C.313** | 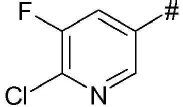 | H | H | F | F | C₂H₅ |
| **C.314** | 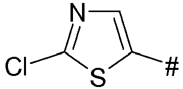 | H | H | F | F | C₂H₅ |
| **C.315** | 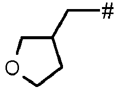 | H | H | F | F | C₂H₅ |
| **C.316** | 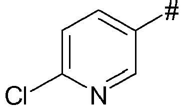 | H | CH₃ | F | F | C₂H₅ |
| **C.317** | 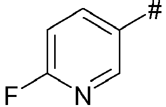 | H | CH₃ | F | F | C₂H₅ |
| **C.318** | 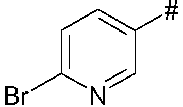 | H | CH₃ | F | F | C₂H₅ |
| **C.319** | 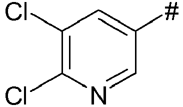 | H | CH₃ | F | F | C₂H₅ |
| **C.320** | 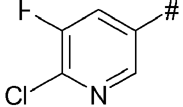 | H | CH₃ | F | F | C₂H₅ |
| **C.321** | 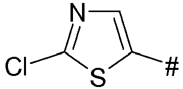 | H | CH₃ | F | F | C₂H₅ |
| **C.322** | 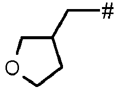 | H | CH₃ | F | F | C₂H₅ |
| **C.323** | 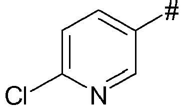 | H | H | H | CH₃ | C₂H₅ |
| **C.324** | 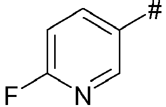 | H | H | H | CH₃ | C₂H₅ |
| **C.325** | 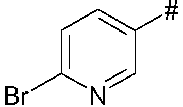 | H | H | H | CH₃ | C₂H₅ |
| **C.326** | 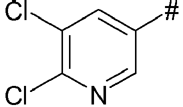 | H | H | H | CH₃ | C₂H₅ |
| **C.327** | 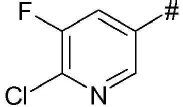 | H | H | H | CH₃ | C₂H₅ |
| **C.328** | 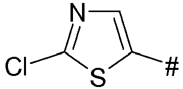 | H | H | H | CH₃ | C₂H₅ |
| **C.329** | 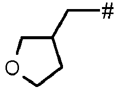 | H | H | H | CH₃ | C₂H₅ |
| **C.330** | 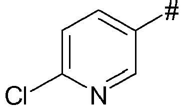 | H | CH₃ | H | CH₃ | C₂H₅ |
| **C.331** | 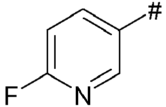 | H | CH₃ | H | CH₃ | C₂H₅ |
| **C.332** | 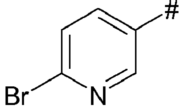 | H | CH₃ | H | CH₃ | C₂H₅ |
| **C.333** | 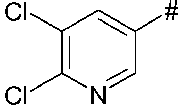 | H | CH₃ | H | CH₃ | C₂H₅ |
| **C.334** | 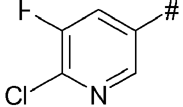 | H | CH₃ | H | CH₃ | C₂H₅ |
| **C.335** | 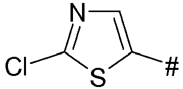 | H | CH₃ | H | CH₃ | C₂H₅ |
| **C.336** | 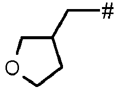 | H | CH₃ | H | CH₃ | C₂H₅ |
| **C.337** | 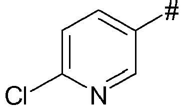 | H | H | H | H | CF₂CF₃ |
| **C.338** | 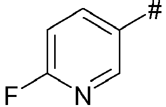 | H | H | H | H | CF₂CF₃ |
| **C.339** | 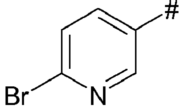 | H | H | H | H | CF₂CF₃ |
| **C.340** | 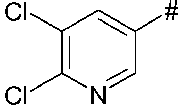 | H | H | H | H | CF₂CF₃ |
| **C.341** | 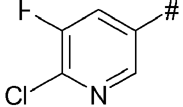 | H | H | H | H | CF₂CF₃ |
| **C.342** | 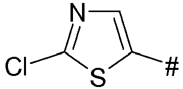 | H | H | H | H | CF₂CF₃ |
| **C.343** | 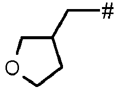 | H | H | H | H | CF₂CF₃ |
| **C.344** | 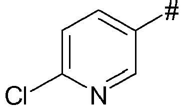 | H | CH₃ | H | H | CF₂CF₃ |
| **C.345** | 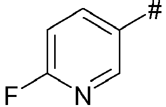 | H | CH₃ | H | H | CF₂CF₃ |
| **C.346** | 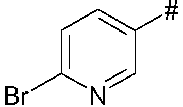 | H | CH₃ | H | H | CF₂CF₃ |
| **C.347** | 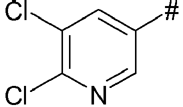 | H | CH₃ | H | H | CF₂CF₃ |
| **C.348** | 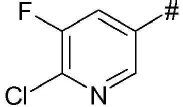 | H | CH₃ | H | H | CF₂CF₃ |
| **C.349** | 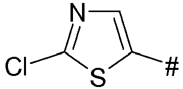 | H | CH₃ | H | H | CF₂CF₃ |
| **C.350** | 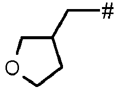 | H | CH₃ | H | H | CF₂CF₃ |
| **C.351** | 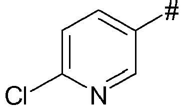 | H | H | H | F | CF₂CF₃ |
| **C.352** | 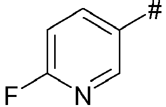 | H | H | H | F | CF₂CF₃ |
| **C.353** | 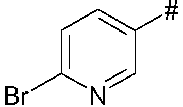 | H | H | H | F | CF₂CF₃ |
| **C.354** | 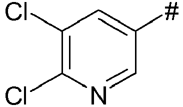 | H | H | H | F | CF₂CF₃ |
| **C.355** | 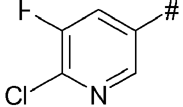 | H | H | H | F | CF₂CF₃ |
| **C.356** | 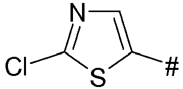 | H | H | H | F | CF₂CF₃ |
| **C.357** | 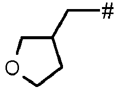 | H | H | H | F | CF₂CF₃ |
| **C.358** | 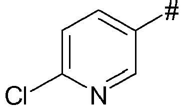 | H | CH₃ | H | F | CF₂CF₃ |
| **C.359** | 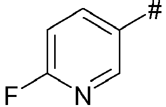 | H | CH₃ | H | F | CF₂CF₃ |
| **C.360** | 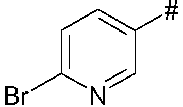 | H | CH₃ | H | F | CF₂CF₃ |
| **C.361** | 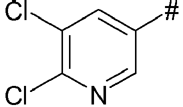 | H | CH₃ | H | F | CF₂CF₃ |
| **C.362** | 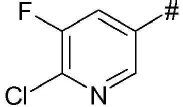 | H | CH₃ | H | F | CF₂CF₃ |
| **C.363** | 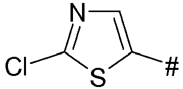 | H | CH₃ | H | F | CF₂CF₃ |
| **C.364** | 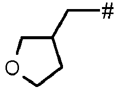 | H | CH₃ | H | F | CF₂CF₃ |
| **C.365** | 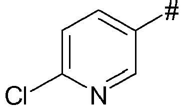 | H | H | F | F | CF₂CF₃ |
| **C.366** | 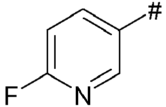 | H | H | F | F | CF₂CF₃ |
| **C.367** | 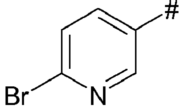 | H | H | F | F | CF₂CF₃ |
| **C.368** | 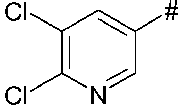 | H | H | F | F | CF₂CF₃ |
| **C.369** | 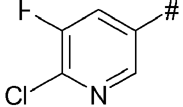 | H | H | F | F | CF₂CF₃ |
| **C.370** | 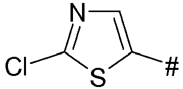 | H | H | F | F | CF₂CF₃ |
| **C.371** | 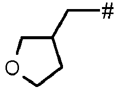 | H | H | F | F | CF₂CF₃ |
| **C.372** | 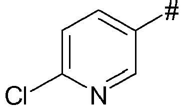 | H | CH₃ | F | F | CF₂CF₃ |
| **C.373** | 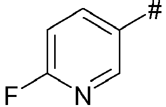 | H | CH₃ | F | F | CF₂CF₃ |
| **C.374** | 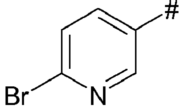 | H | CH₃ | F | F | CF₂CF₃ |
| **C.375** | 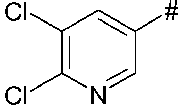 | H | CH₃ | F | F | CF₂CF₃ |
| **C.376** | 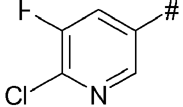 | H | CH₃ | F | F | CF₂CF₃ |
| **C.377** | 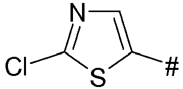 | H | CH₃ | F | F | CF₂CF₃ |
| **C.378** | 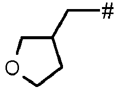 | H | CH₃ | F | F | CF₂CF₃ |
| **C.379** | 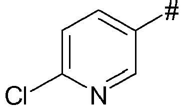 | H | H | H | CH₃ | CF₂CF₃ |
| **C.380** | 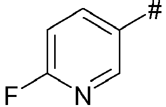 | H | H | H | CH₃ | CF₂CF₃ |
| **C.381** | 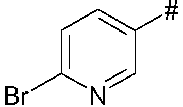 | H | H | H | CH₃ | CF₂CF₃ |
| **C.382** | 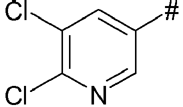 | H | H | H | CH₃ | CF₂CF₃ |
| **C.383** | 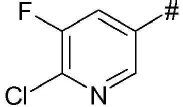 | H | H | H | CH₃ | CF₂CF₃ |
| **C.384** | 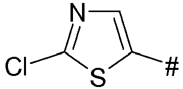 | H | H | H | CH₃ | CF₂CF₃ |
| **C.385** | 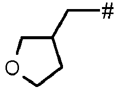 | H | H | H | CH₃ | CF₂CF₃ |
| **C.386** | 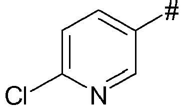 | H | CH₃ | H | CH₃ | CF₂CF₃ |
| **C.387** | 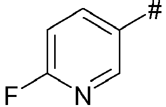 | H | CH₃ | H | CH₃ | CF₂CF₃ |
| **C.388** | 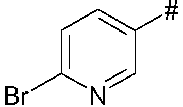 | H | CH₃ | H | CH₃ | CF₂CF₃ |
| **C.389** | 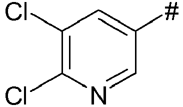 | H | CH₃ | H | CH₃ | CF₂CF₃ |
| **C.390** | 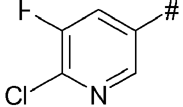 | H | CH₃ | H | CH₃ | CF₂CF₃ |
| **C.391** | 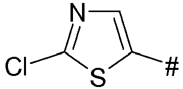 | H | CH₃ | H | CH₃ | CF₂CF₃ |
| **C.392** | 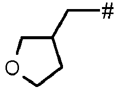 | H | CH₃ | H | CH₃ | CF₂CF₃ |
| **C.393** | 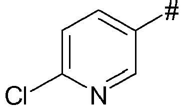 | H | H | H | H | CF₂CF₂ |
| **C.394** | 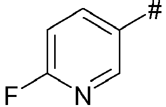 | H | H | H | H | CF₂CF₂ |
| **C.395** | 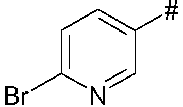 | H | H | H | H | CF₂CF₂ |
| **C.396** | 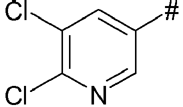 | H | H | H | H | CF₂CF₂ |
| **C.397** | 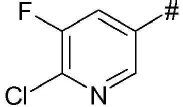 | H | H | H | H | CF₂CF₂ |
| **C.398** | 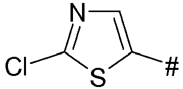 | H | H | H | H | CF₂CF₂ |
| **C.399** | 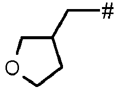 | H | H | H | H | CF₂CF₂ |
| **C.400** | 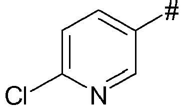 | H | CH₃ | H | H | CF₂CF₂ |
| **C.401** | 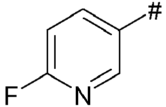 | H | CH₃ | H | H | CF₂CF₂ |
| **C.402** | 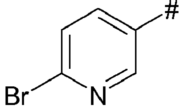 | H | CH₃ | H | H | CF₂CF₂ |
| **C.403** | 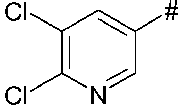 | H | CH₃ | H | H | CF₂CF₂ |
| **C.404** | 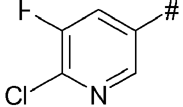 | H | CH₃ | H | H | CF₂CF₂ |
| **C.405** | 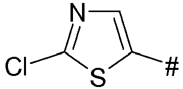 | H | CH₃ | H | H | CF₂CF₂ |
| **C.406** | 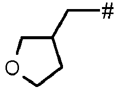 | H | CH₃ | H | H | CF₂CF₂ |
| **C.407** | 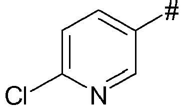 | H | H | H | F | CF₂CF₂ |
| **C.408** | 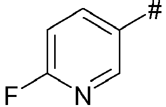 | H | H | H | F | CF₂CF₂ |
| **C.409** | 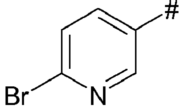 | H | H | H | F | CF₂CF₂ |
| **C.410** | 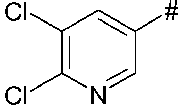 | H | H | H | F | CF₂CF₂ |
| **C.411** | 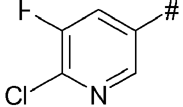 | H | H | H | F | CF₂CF₂ |
| **C.412** | 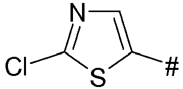 | H | H | H | F | CF₂CF₂ |
| **C.413** | 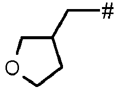 | H | H | H | F | CF₂CF₂ |
| **C.414** | 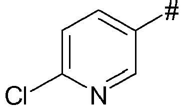 | H | CH₃ | H | F | CF₂CF₂ |
| **C.415** | 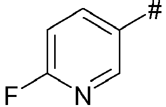 | H | CH₃ | H | F | CF₂CF₂ |
| **C.416** | 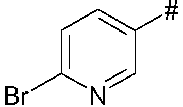 | H | CH₃ | H | F | CF₂CF₂ |
| **C.417** | 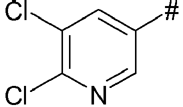 | H | CH₃ | H | F | CF₂CF₂ |
| **C.418** | 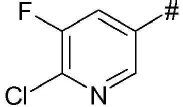 | H | CH₃ | H | F | CF₂CF₂ |
| **C.419** | 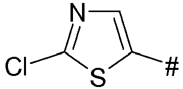 | H | CH₃ | H | F | CF₂CF₂ |
| **C.420** | 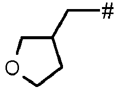 | H | CH₃ | H | F | CF₂CF₂ |
| **C.421** | 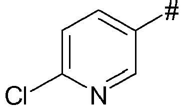 | H | H | F | F | CF₂CF₂ |
| **C.422** | 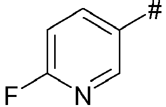 | H | H | F | F | CF₂CF₂ |
| **C.423** | 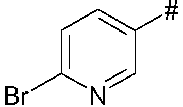 | H | H | F | F | CF₂CF₂ |
| **C.424** | 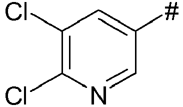 | H | H | F | F | CF₂CF₂ |
| **C.425** | 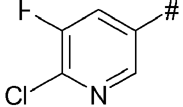 | H | H | F | F | CF₂CF₂ |
| **C.426** | 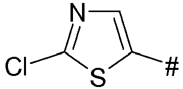 | H | H | F | F | CF₂CF₂ |
| **C.427** | 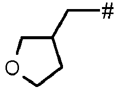 | H | H | F | F | CF₂CF₂ |
| **C.428** | 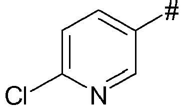 | H | CH₃ | F | F | CF₂CF₂ |
| **C.429** | 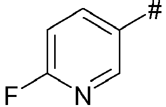 | H | CH₃ | F | F | CF₂CF₂ |
| **C.430** | 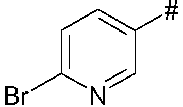 | H | CH₃ | F | F | CF₂CF₂ |
| **C.431** | 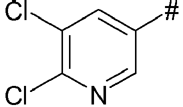 | H | CH₃ | F | F | CF₂CF₂ |
| **C.432** | 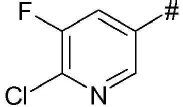 | H | CH₃ | F | F | CF₂CF₂ |
| **C.433** | 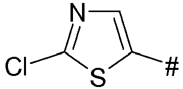 | H | CH₃ | F | F | CF₂CF₂ |
| **C.434** | 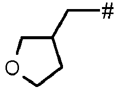 | H | CH₃ | F | F | CF₂CF₂ |
| **C.435** | 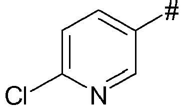 | H | H | H | CH₃ | CF₂CF₂ |
| **C.436** | 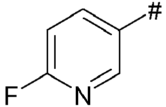 | H | H | H | CH₃ | CF₂CF₂ |
| **C.437** | 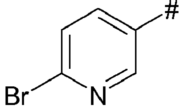 | H | H | H | CH₃ | CF₂CF₂ |
| **C.438** | 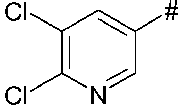 | H | H | H | CH₃ | CF₂CF₂ |
| **C.439** | 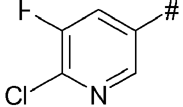 | H | H | H | CH₃ | CF₂CF₂ |
| **C.440** | 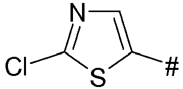 | H | H | H | CH₃ | CF₂CF₂ |
| **C.441** | 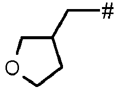 | H | H | H | CH₃ | CF₂CF₂ |
| **C.442** | 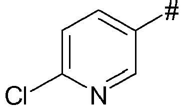 | H | CH₃ | H | CH₃ | CF₂CF₂ |
| **C.443** | 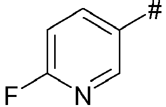 | H | CH₃ | H | CH₃ | CF₂CF₂ |
| **C.444** | 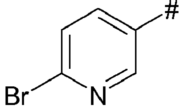 | H | CH₃ | H | CH₃ | CF₂CF₂ |
| **C.445** | 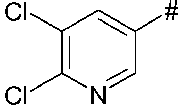 | H | CH₃ | H | CH₃ | CF₂CF₂ |
| **C.446** | 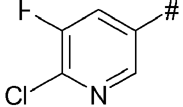 | H | CH₃ | H | CH₃ | CF₂CF₂ |
| **C.447** | 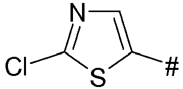 | H | CH₃ | H | CH₃ | CF₂CF₂ |
| **C.448** | 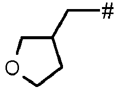 | H | CH₃ | H | CH₃ | CF₂CF₂ |
| **C.449** | 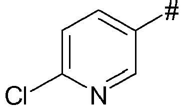 | H | H | H | H | CH₂CF₃ |
| **C.450** | 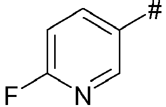 | H | H | H | H | CH₂CF₃ |
| **C.451** | 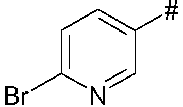 | H | H | H | H | CH₂CF₃ |
| **C.452** | 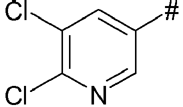 | H | H | H | H | CH₂CF₃ |
| **C.453** | 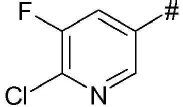 | H | H | H | H | CH₂CF₃ |
| **C.454** | 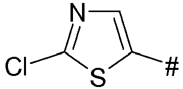 | H | H | H | H | CH₂CF₃ |
| **C.455** | 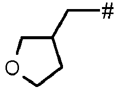 | H | H | H | H | CH₂CF₃ |
| **C.456** | 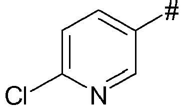 | H | CH₃ | H | H | CH₂CF₃ |
| **C.457** | 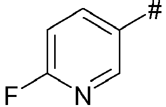 | H | CH₃ | H | H | CH₂CF₃ |
| **C.458** | 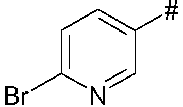 | H | CH₃ | H | H | CH₂CF₃ |
| **C.459** | 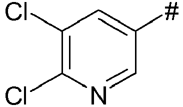 | H | CH₃ | H | H | CH₂CF₃ |
| **C.460** | 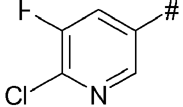 | H | CH₃ | H | H | CH₂CF₃ |
| **C.461** | 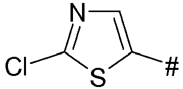 | H | CH₃ | H | H | CH₂CF₃ |
| **C.462** | 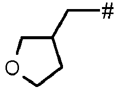 | H | CH₃ | H | H | CH₂CF₃ |
| **C.463** | 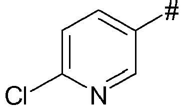 | H | H | H | F | CH₂CF₃ |
| **C.464** | 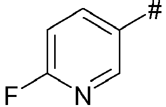 | H | H | H | F | CH₂CF₃ |
| **C.465** | 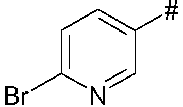 | H | H | H | F | CH₂CF₃ |
| **C.466** | 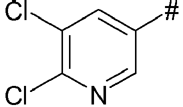 | H | H | H | F | CH₂CF₃ |
| **C.467** | 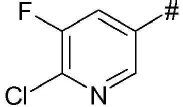 | H | H | H | F | CH₂CF₃ |
| **C.468** | 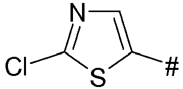 | H | H | H | F | CH₂CF₃ |
| **C.469** | 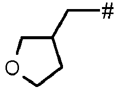 | H | H | H | F | CH₂CF₃ |
| **C.470** | 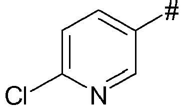 | H | CH₃ | H | F | CH₂CF₃ |
| **C.471** | 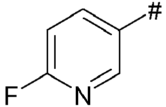 | H | CH₃ | H | F | CH₂CF₃ |
| **C.472** | 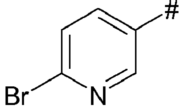 | H | CH₃ | H | F | CH₂CF₃ |
| **C.473** | 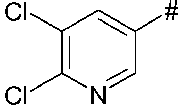 | H | CH₃ | H | F | CH₂CF₃ |
| **C.474** | 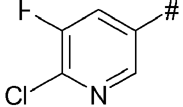 | H | CH₃ | H | F | CH₂CF₃ |
| **C.475** | 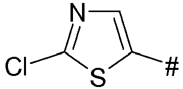 | H | CH₃ | H | F | CH₂CF₃ |
| **C.476** | 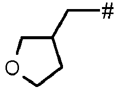 | H | CH₃ | H | F | CH₂CF₃ |
| **C.477** | 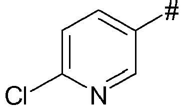 | H | H | F | F | CH₂CF₃ |
| **C.478** | 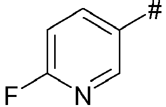 | H | H | F | F | CH₂CF₃ |
| **C.479** | 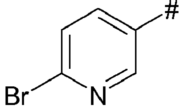 | H | H | F | F | CH₂CF₃ |
| **C.480** | 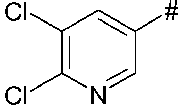 | H | H | F | F | CH₂CF₃ |
| **C.481** | 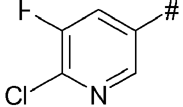 | H | H | F | F | CH₂CF₃ |
| **C.482** | 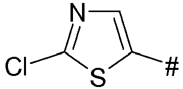 | H | H | F | F | CH₂CF₃ |
| **C.483** | 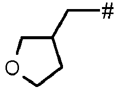 | H | H | F | F | CH₂CF₃ |
| **C.484** | 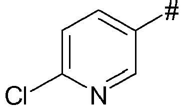 | H | CH₃ | F | F | CH₂CF₃ |
| **C.485** | 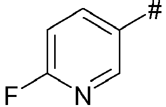 | H | CH₃ | F | F | CH₂CF₃ |
| **C.486** | 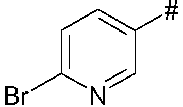 | H | CH₃ | F | F | CH₂CF₃ |
| **C.487** | 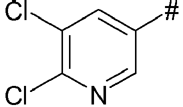 | H | CH₃ | F | F | CH₂CF₃ |
| **C.488** | 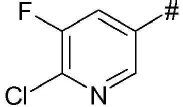 | H | CH₃ | F | F | CH₂CF₃ |
| **C.489** | 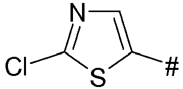 | H | CH₃ | F | F | CH₂CF₃ |
| **C.490** | 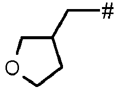 | H | CH₃ | F | F | CH₂CF₃ |
| **C.491** | 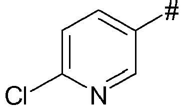 | H | H | H | CH₃ | CH₂CF₃ |
| **C.492** | 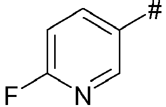 | H | H | H | CH₃ | CH₂CF₃ |
| **C.493** | 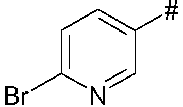 | H | H | H | CH₃ | CH₂CF₃ |
| **C.494** | 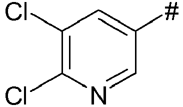 | H | H | H | CH₃ | CH₂CF₃ |
| **C.495** | 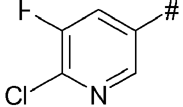 | H | H | H | CH₃ | CH₂CF₃ |
| **C.496** | 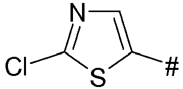 | H | H | H | CH₃ | CH₂CF₃ |
| **C.497** | 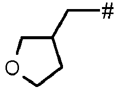 | H | H | H | CH₃ | CH₂CF₃ |
| **C.498** | 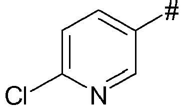 | H | CH₃ | H | CH₃ | CH₂CF₃ |
| **C.499** | 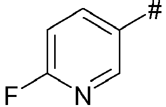 | H | CH₃ | H | CH₃ | CH₂CF₃ |
| **C.500** | 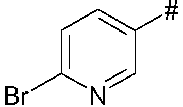 | H | CH₃ | H | CH₃ | CH₂CF₃ |
| **C.501** | 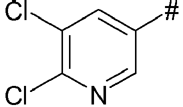 | H | CH₃ | H | CH₃ | CH₂CF₃ |
| **C.502** | 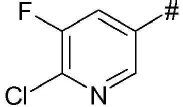 | H | CH₃ | H | CH₃ | CH₂CF₃ |
| **C.503** | 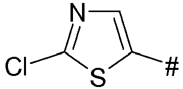 | H | CH₃ | H | CH₃ | CH₂CF₃ |
| **C.504** | 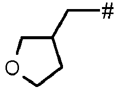 | H | CH₃ | H | CH₃ | CH₂CF₃ |
| **C.505** | 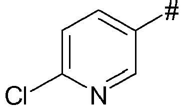 | H | H | H | H | CH₂CCl₃ |
| **C.506** | 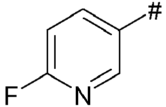 | H | H | H | H | CH₂CCl₃ |
| **C.507** | 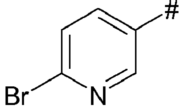 | H | H | H | H | CH₂CCl₃ |
| **C.508** | 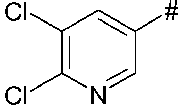 | H | H | H | H | CH₂CCl₃ |
| **C.509** | 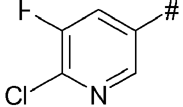 | H | H | H | H | CH₂CCl₃ |
| **C.510** | 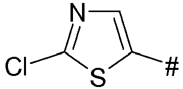 | H | H | H | H | CH₂CCl₃ |
| **C.511** | 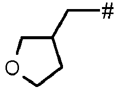 | H | H | H | H | CH₂CCl₃ |
| **C.512** | 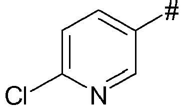 | H | CH₃ | H | H | CH₂CCl₃ |
| **C.513** | 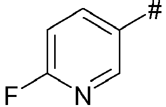 | H | CH₃ | H | H | CH₂CCl₃ |
| **C.514** | 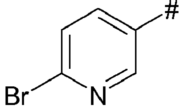 | H | CH₃ | H | H | CH₂CCl₃ |
| **C.515** | 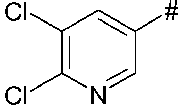 | H | CH₃ | H | H | CH₂CCl₃ |
| **C.516** | 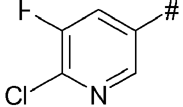 | H | CH₃ | H | H | CH₂CCl₃ |
| **C.517** | 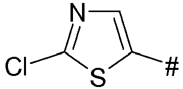 | H | CH₃ | H | H | CH₂CCl₃ |
| **C.518** | 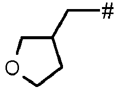 | H | CH₃ | H | H | CH₂CCl₃ |
| **C.519** | 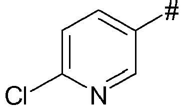 | H | H | H | F | CH₂CCl₃ |
| **C.520** | 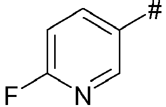 | H | H | H | F | CH₂CCl₃ |
| **C.521** | 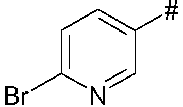 | H | H | H | F | CH₂CCl₃ |
| **C.522** | 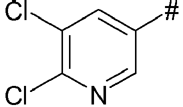 | H | H | H | F | CH₂CCl₃ |
| **C.523** | 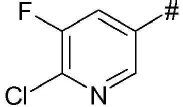 | H | H | H | F | CH₂CCl₃ |
| **C.524** | 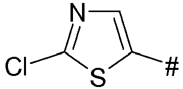 | H | H | H | F | CH₂CCl₃ |
| **C.525** | 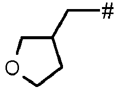 | H | H | H | F | CH₂CCl₃ |
| **C.526** | 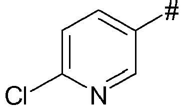 | H | CH₃ | H | F | CH₂CCl₃ |
| **C.527** | 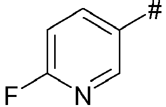 | H | CH₃ | H | F | CH₂CCl₃ |
| **C.528** | 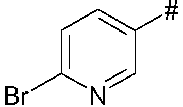 | H | CH₃ | H | F | CH₂CCl₃ |
| **C.529** | 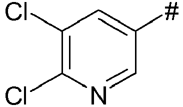 | H | CH₃ | H | F | CH₂CCl₃ |
| **C.530** | 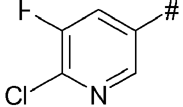 | H | CH₃ | H | F | CH₂CCl₃ |
| **C.531** | 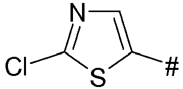 | H | CH₃ | H | F | CH₂CCl₃ |
| **C.532** | 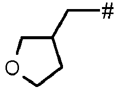 | H | CH₃ | H | F | CH₂CCl₃ |
| **C.533** | 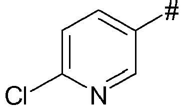 | H | H | F | F | CH₂CCl₃ |
| **C.534** | 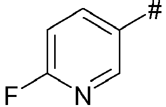 | H | H | F | F | CH₂CCl₃ |
| **C.535** | 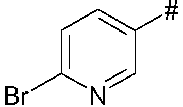 | H | H | F | F | CH₂CCl₃ |
| **C.536** | 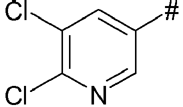 | H | H | F | F | CH₂CCl₃ |
| **C.537** | 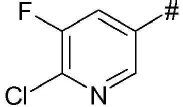 | H | H | F | F | CH₂CCl₃ |
| **C.538** | 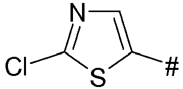 | H | H | F | F | CH₂CCl₃ |
| **C.539** | 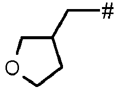 | H | H | F | F | CH₂CCl₃ |
| **C.540** | 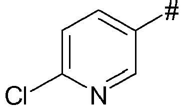 | H | CH₃ | F | F | CH₂CCl₃ |
| **C.541** | 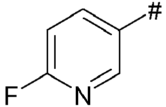 | H | CH₃ | F | F | CH₂CCl₃ |
| **C.542** | 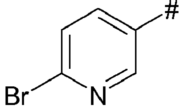 | H | CH₃ | F | F | CH₂CCl₃ |
| **C.543** | 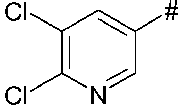 | H | CH₃ | F | F | CH₂CCl₃ |
| **C.544** | 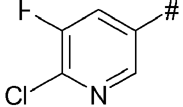 | H | CH₃ | F | F | CH₂CCl₃ |
| **C.545** | 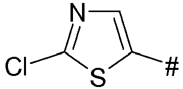 | H | CH₃ | F | F | CH₂CCl₃ |
| **C.546** | 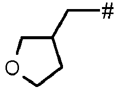 | H | CH₃ | F | F | CH₂CCl₃ |
| **C.547** | 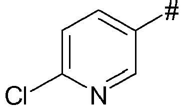 | H | H | H | CH₃ | CH₂CCl₃ |
| **C.548** | 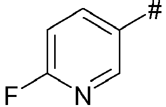 | H | H | H | CH₃ | CH₂CCl₃ |
| **C.549** | 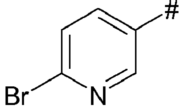 | H | H | H | CH₃ | CH₂CCl₃ |
| **C.550** | 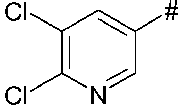 | H | H | H | CH₃ | CH₂CCl₃ |
| **C.551** | 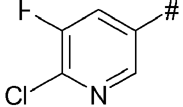 | H | H | H | CH₃ | CH₂CCl₃ |
| **C.552** | 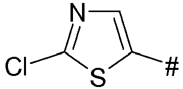 | H | H | H | CH₃ | CH₂CCl₃ |
| **C.553** | 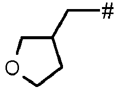 | H | H | H | CH₃ | CH₂CCl₃ |
| **C.554** | 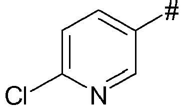 | H | CH₃ | H | CH₃ | CH₂CCl₃ |
| **C.555** | 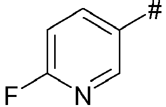 | H | CH₃ | H | CH₃ | CH₂CCl₃ |
| **C.556** | 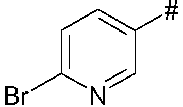 | H | CH₃ | H | CH₃ | CH₂CCl₃ |
| **C.557** | 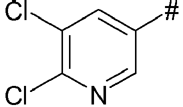 | H | CH₃ | H | CH₃ | CH₂CCl₃ |
| **C.558** | 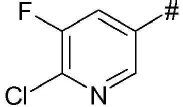 | H | CH₃ | H | CH₃ | CH₂CCl₃ |
| **C.559** | 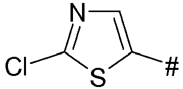 | H | CH₃ | H | CH₃ | CH₂CCl₃ |
| **C.560** | 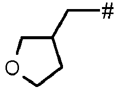 | H | CH₃ | H | CH₃ | CH₂CCl₃ |
| **C.561** | 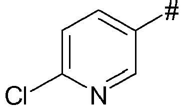 | H | H | H | H | n-C₃H₇ |
| **C.562** | 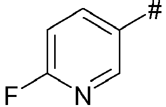 | H | H | H | H | n-C₃H₇ |
| **C.563** | 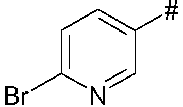 | H | H | H | H | n-C₃H₇ |
| **C.564** | 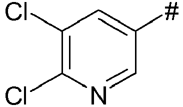 | H | H | H | H | n-C₃H₇ |
| **C.565** | 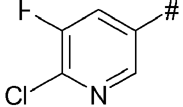 | H | H | H | H | n-C₃H₇ |
| **C.566** | 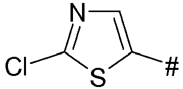 | H | H | H | H | n-C₃H₇ |
| **C.567** | 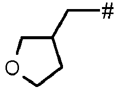 | H | H | H | H | n-C₃H₇ |
| **C.568** | 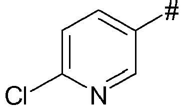 | H | CH₃ | H | H | n-C₃H₇ |
| **C.569** | 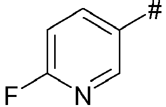 | H | CH₃ | H | H | n-C₃H₇ |
| **C.570** | 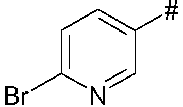 | H | CH₃ | H | H | n-C₃H₇ |
| **C.571** | 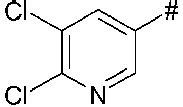 | H | CH₃ | H | H | n-C₃H₇ |
| **C.572** | 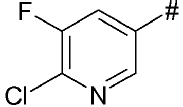 | H | CH₃ | H | H | n-C₃H₇ |
| **C.573** | 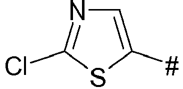 | H | CH₃ | H | H | n-C₃H₇ |
| **C.574** | 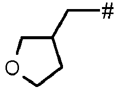 | H | CH₃ | H | H | n-C₃H₇ |
| **C.575** | 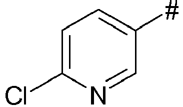 | H | H | H | F | n-C₃H₇ |
| **C.576** | 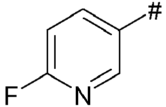 | H | H | H | F | n-C₃H₇ |
| **C.577** | 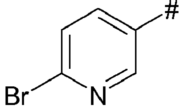 | H | H | H | F | n-C₃H₇ |
| **C.578** | 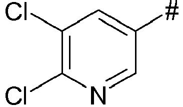 | H | H | H | F | n-C₃H₇ |
| **C.579** | 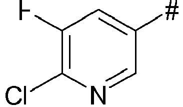 | H | H | H | F | n-C₃H₇ |
| **C.580** | 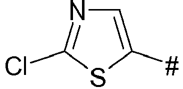 | H | H | H | F | n-C₃H₇ |
| **C.581** | 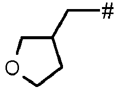 | H | H | H | F | n-C₃H₇ |
| **C.582** | 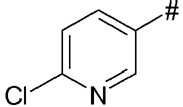 | H | CH₃ | H | F | n-C₃H₇ |
| **C.583** | 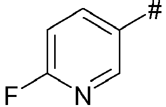 | H | CH₃ | H | F | n-C₃H₇ |
| **C.584** | 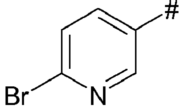 | H | CH₃ | H | F | n-C₃H₇ |
| **C.585** | 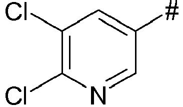 | H | CH₃ | H | F | n-C₃H₇ |
| **C.586** | 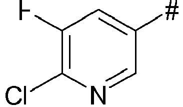 | H | CH₃ | H | F | n-C₃H₇ |
| **C.587** | 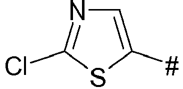 | H | CH₃ | H | F | n-C₃H₇ |
| **C.588** | 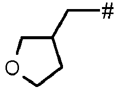 | H | CH₃ | H | F | n-C₃H₇ |
| **C.589** | 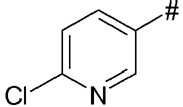 | H | H | F | F | n-C₃H₇ |
| **C.590** | 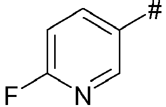 | H | H | F | F | n-C₃H₇ |
| **C.591** | 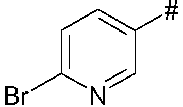 | H | H | F | F | n-C₃H₇ |
| **C.592** | 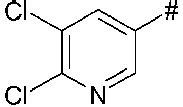 | H | H | F | F | n-C₃H₇ |
| **C.593** | 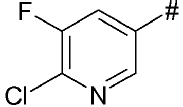 | H | H | F | F | n-C₃H₇ |
| **C.594** | 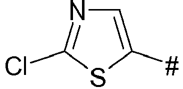 | H | H | F | F | n-C₃H₇ |
| **C.595** | 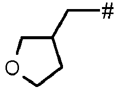 | H | H | F | F | n-C₃H₇ |
| **C.596** | 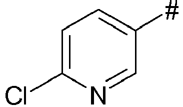 | H | CH₃ | F | F | n-C₃H₇ |
| **C.597** | 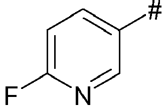 | H | CH₃ | F | F | n-C₃H₇ |
| **C.598** | 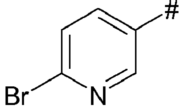 | H | CH₃ | F | F | n-C₃H₇ |
| **C.599** | 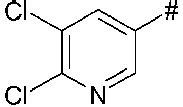 | H | CH₃ | F | F | n-C₃H₇ |
| **C.600** | 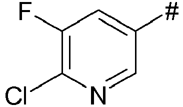 | H | CH₃ | F | F | n-C₃H₇ |
| **C.601** | 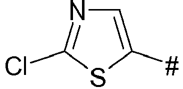 | H | CH₃ | F | F | n-C₃H₇ |
| **C.602** | 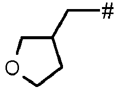 | H | CH₃ | F | F | n-C₃H₇ |
| **C.603** | 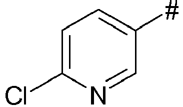 | H | H | H | CH₃ | n-C₃H₇ |
| **C.604** | 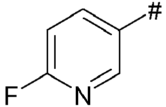 | H | H | H | CH₃ | n-C₃H₇ |
| **C.605** | 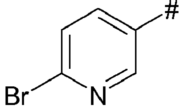 | H | H | H | CH₃ | n-C₃H₇ |
| **C.606** | 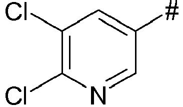 | H | H | H | CH₃ | n-C₃H₇ |
| **C.607** | 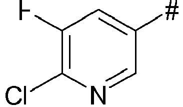 | H | H | H | CH₃ | n-C₃H₇ |
| **C.608** | 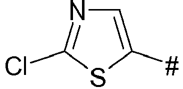 | H | H | H | CH₃ | n-C₃H₇ |
| **C.609** | 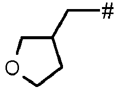 | H | H | H | CH₃ | n-C₃H₇ |
| **C.610** | 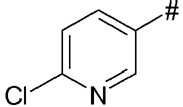 | H | CH₃ | H | CH₃ | n-C₃H₇ |
| **C.611** | 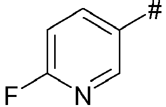 | H | CH₃ | H | CH₃ | n-C₃H₇ |
| **C.612** | 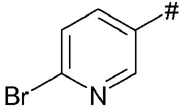 | H | CH₃ | H | CH₃ | n-C₃H₇ |
| **C.613** | 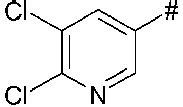 | H | CH₃ | H | CH₃ | n-C₃H₇ |
| **C.614** | 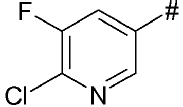 | H | CH₃ | H | CH₃ | n-C₃H₇ |
| **C.615** | 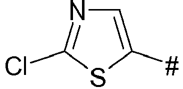 | H | CH₃ | H | CH₃ | n-C₃H₇ |
| **C.616** | 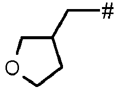 | H | CH₃ | H | CH₃ | n-C₃H₇ |
| **C.617** | 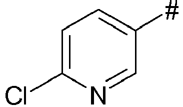 | H | H | H | H | n-C₃F₇ |
| **C.618** | 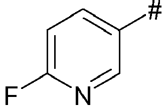 | H | H | H | H | n-C₃F₇ |
| **C.619** | 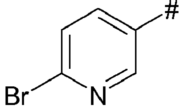 | H | H | H | H | n-C₃F₇ |
| **C.620** | 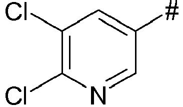 | H | H | H | H | n-C₃F₇ |
| **C.621** | 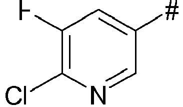 | H | H | H | H | n-C₃F₇ |
| **C.622** | 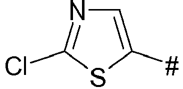 | H | H | H | H | n-C₃F₇ |
| **C.623** | 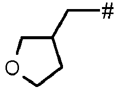 | H | H | H | H | n-C₃F₇ |
| **C.624** | 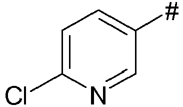 | H | CH₃ | H | H | n-C₃F₇ |
| **C.625** | 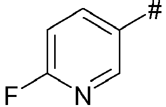 | H | CH₃ | H | H | n-C₃F₇ |
| **C.626** | 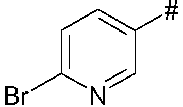 | H | CH₃ | H | H | n-C₃F₇ |
| **C.627** | 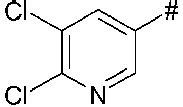 | H | CH₃ | H | H | n-C₃F₇ |
| **C.628** | 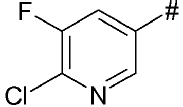 | H | CH₃ | H | H | n-C₃F₇ |
| **C.629** | 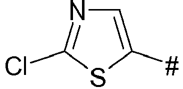 | H | CH₃ | H | H | n-C₃F₇ |
| **C.630** | 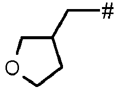 | H | CH₃ | H | H | n-C₃F₇ |
| **C.631** | 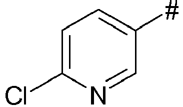 | H | H | H | F | n-C₃F₇ |
| **C.632** | 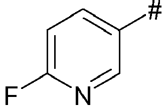 | H | H | H | F | n-C₃F₇ |
| **C.633** | 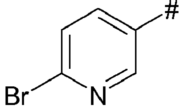 | H | H | H | F | n-C₃F₇ |
| **C.634** | 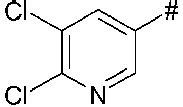 | H | H | H | F | n-C₃F₇ |
| **C.635** | 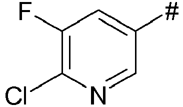 | H | H | H | F | n-C₃F₇ |
| **C.636** | 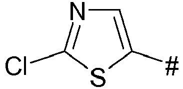 | H | H | H | F | n-C₃F₇ |
| **C.637** | 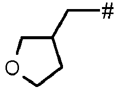 | H | H | H | F | n-C₃F₇ |
| **C.638** | 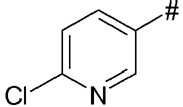 | H | CH₃ | H | F | n-C₃F₇ |
| **C.639** | 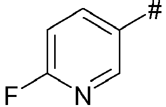 | H | CH₃ | H | F | n-C₃F₇ |
| **C.640** | 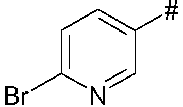 | H | CH₃ | H | F | n-C₃F₇ |
| **C.641** | 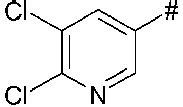 | H | CH₃ | H | F | n-C₃F₇ |
| **C.642** | 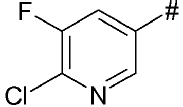 | H | CH₃ | H | F | n-C₃F₇ |
| **C.643** | 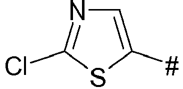 | H | CH₃ | H | F | n-C₃F₇ |
| **C.644** | 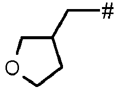 | H | CH₃ | H | F | n-C₃F₇ |
| **C.645** | 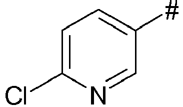 | H | H | F | F | n-C₃F₇ |
| **C.646** | 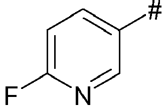 | H | H | F | F | n-C₃F₇ |
| **C.647** | 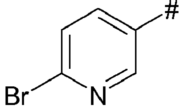 | H | H | F | F | n-C₃F₇ |
| **C.648** | 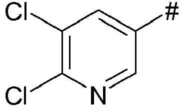 | H | H | F | F | n-C₃F₇ |
| **C.649** | 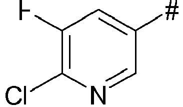 | H | H | F | F | n-C₃F₇ |
| **C.650** | 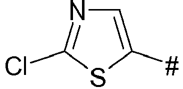 | H | H | F | F | n-C₃F₇ |
| **C.651** | 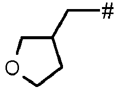 | H | H | F | F | n-C₃F₇ |
| **C.652** | 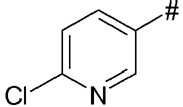 | H | CH₃ | F | F | n-C₃F₇ |
| **C.653** | 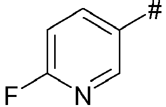 | H | CH₃ | F | F | n-C₃F₇ |
| **C.654** | 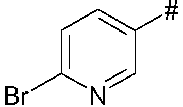 | H | CH₃ | F | F | n-C₃F₇ |
| **C.655** | 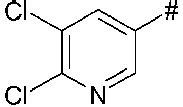 | H | CH₃ | F | F | n-C₃F₇ |
| **C.656** | 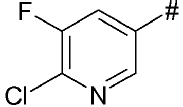 | H | CH₃ | F | F | n-C₃F₇ |
| **C.657** | 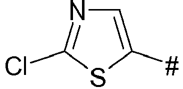 | H | CH₃ | F | F | n-C₃F₇ |
| **C.658** | 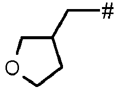 | H | CH₃ | F | F | n-C₃F₇ |
| **C.659** | 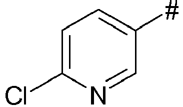 | H | H | H | CH₃ | n-C₃F₇ |
| **C.660** | 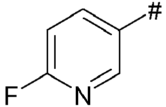 | H | H | H | CH₃ | n-C₃F₇ |
| **C.661** | 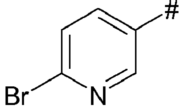 | H | H | H | CH₃ | n-C₃F₇ |
| **C.662** | 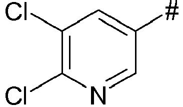 | H | H | H | CH₃ | n-C₃F₇ |
| **C.663** | 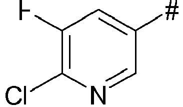 | H | H | H | CH₃ | n-C₃F₇ |
| **C.664** | 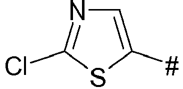 | H | H | H | CH₃ | n-C₃F₇ |
| **C.665** | 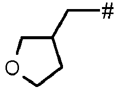 | H | H | H | CH₃ | n-C₃F₇ |
| **C.666** | 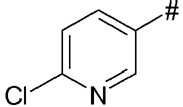 | H | CH₃ | H | CH₃ | n-C₃F₇ |
| **C.667** | 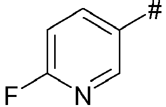 | H | CH₃ | H | CH₃ | n-C₃F₇ |
| **C.668** | 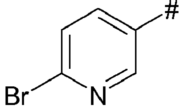 | H | CH₃ | H | CH₃ | n-C₃F₇ |
| **C.669** | 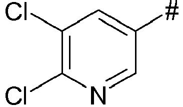 | H | CH₃ | H | CH₃ | n-C₃F₇ |
| **C.670** | 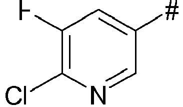 | H | CH₃ | H | CH₃ | n-C₃F₇ |
| **C.671** | 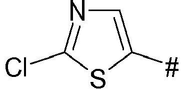 | H | CH₃ | H | CH₃ | n-C₃F₇ |
| **C.672** | 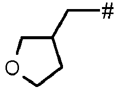 | H | CH₃ | H | CH₃ | n-C₃F₇ |
| **C.673** | 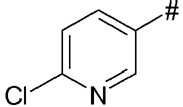 | H | H | H | H | i-C₃H₇ |
| **C.674** | 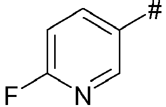 | H | H | H | H | i-C₃H₇ |
| **C.675** | 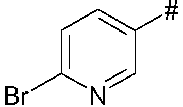 | H | H | H | H | i-C₃H₇ |
| **C.676** | 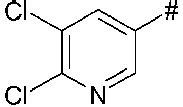 | H | H | H | H | i-C₃H₇ |
| **C.677** | 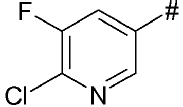 | H | H | H | H | i-C₃H₇ |
| **C.678** | 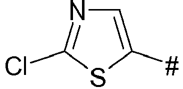 | H | H | H | H | i-C₃H₇ |
| **C.679** | 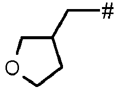 | H | H | H | H | i-C₃H₇ |
| **C.680** | 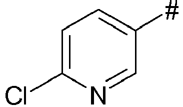 | H | CH₃ | H | H | i-C₃H₇ |
| **C.681** | 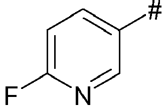 | H | CH₃ | H | H | i-C₃H₇ |
| **C.682** | 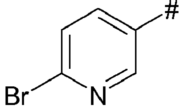 | H | CH₃ | H | H | i-C₃H₇ |
| **C.683** | 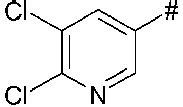 | H | CH₃ | H | H | i-C₃H₇ |
| **C.684** | 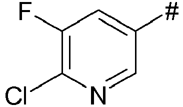 | H | CH₃ | H | H | i-C₃H₇ |
| **C.685** | 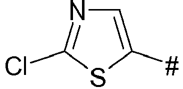 | H | CH₃ | H | H | i-C₃H₇ |
| **C.686** | 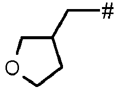 | H | CH₃ | H | H | i-C₃H₇ |
| **C.687** | 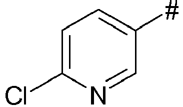 | H | H | H | F | i-C₃H₇ |
| **C.688** | 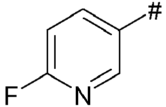 | H | H | H | F | i-C₃H₇ |
| **C.689** | 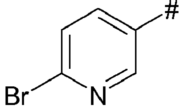 | H | H | H | F | i-C₃H₇ |
| **C.690** | 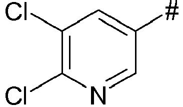 | H | H | H | F | i-C₃H₇ |
| **C.691** | 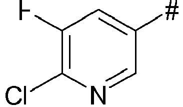 | H | H | H | F | i-C₃H₇ |
| **C.692** | 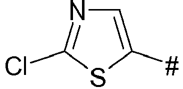 | H | H | H | F | i-C₃H₇ |
| **C.693** | 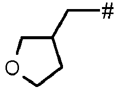 | H | H | H | F | i-C₃H₇ |
| **C.694** | 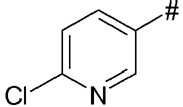 | H | CH₃ | H | F | i-C₃H₇ |
| **C.695** | 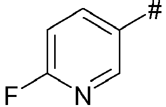 | H | CH₃ | H | F | i-C₃H₇ |
| **C.696** | 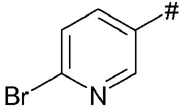 | H | CH₃ | H | F | i-C₃H₇ |
| **C.697** | 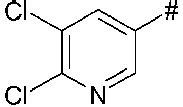 | H | CH₃ | H | F | i-C₃H₇ |
| **C.698** | 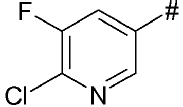 | H | CH₃ | H | F | i-C₃H₇ |
| **C.699** | 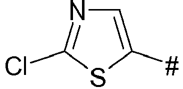 | H | CH₃ | H | F | i-C₃H₇ |
| **C.700** | 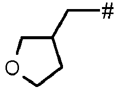 | H | CH₃ | H | F | i-C₃H₇ |
| **C.701** | 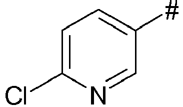 | H | H | F | F | i-C₃H₇ |
| **C.702** | 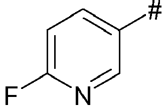 | H | H | F | F | i-C₃H₇ |
| **C.703** | 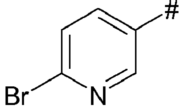 | H | H | F | F | i-C₃H₇ |
| **C.704** | 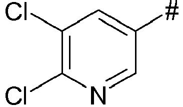 | H | H | F | F | i-C₃H₇ |
| **C.705** | 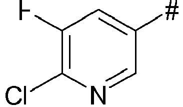 | H | H | F | F | i-C₃H₇ |
| **C.706** | 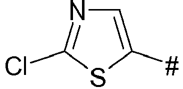 | H | H | F | F | i-C₃H₇ |
| **C.707** | 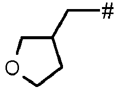 | H | H | F | F | i-C₃H₇ |
| **C.708** | 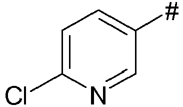 | H | CH₃ | F | F | i-C₃H₇ |
| **C.709** | 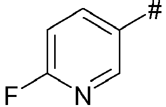 | H | CH₃ | F | F | i-C₃H₇ |
| **C.710** | 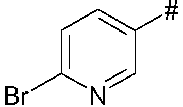 | H | CH₃ | F | F | i-C₃H₇ |
| **C.711** | 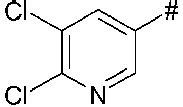 | H | CH₃ | F | F | i-C₃H₇ |
| **C.712** | 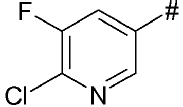 | H | CH₃ | F | F | i-C₃H₇ |
| **C.713** | 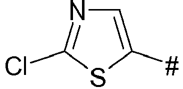 | H | CH₃ | F | F | i-C₃H₇ |
| **C.714** | 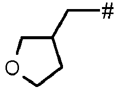 | H | CH₃ | F | F | i-C₃H₇ |
| **C.715** | 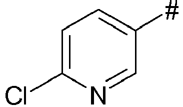 | H | H | H | CH₃ | i-C₃H₇ |
| **C.716** | 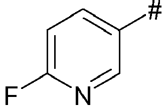 | H | H | H | CH₃ | i-C₃H₇ |
| **C.717** | 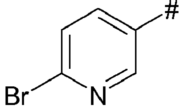 | H | H | H | CH₃ | i-C₃H₇ |
| **C.718** | 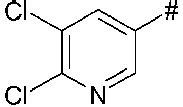 | H | H | H | CH₃ | i-C₃H₇ |
| **C.719** | 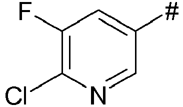 | H | H | H | CH₃ | i-C₃H₇ |
| **C.720** | 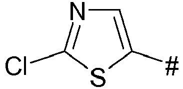 | H | H | H | CH₃ | i-C₃H₇ |
| **C.721** | 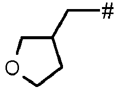 | H | H | H | CH₃ | i-C₃H₇ |
| **C.722** | 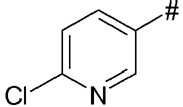 | H | CH₃ | H | CH₃ | i-C₃H₇ |
| **C.723** | 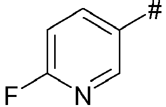 | H | CH₃ | H | CH₃ | i-C₃H₇ |
| **C.724** | 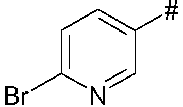 | H | CH₃ | H | CH₃ | i-C₃H₇ |
| **C.725** | 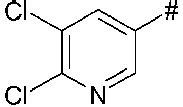 | H | CH₃ | H | CH₃ | i-C₃H₇ |
| **C.726** | 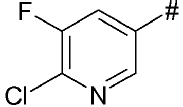 | H | CH₃ | H | CH₃ | i-C₃H₇ |
| **C.727** | 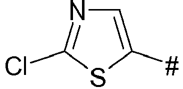 | H | CH₃ | H | CH₃ | i-C₃H₇ |
| **C.728** | 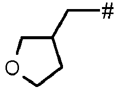 | H | CH₃ | H | CH₃ | i-C₃H₇ |
| **C.729** | 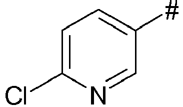 | H | H | H | H | i-C₃F₇ |
| **C.730** | 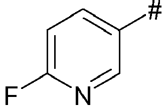 | H | H | H | H | i-C₃F₇ |
| **C.731** | 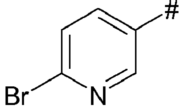 | H | H | H | H | i-C₃F₇ |
| **C.732** | 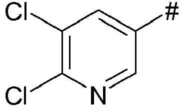 | H | H | H | H | i-C₃F₇ |
| **C.733** | 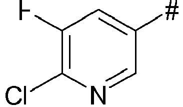 | H | H | H | H | i-C₃F₇ |
| **C.734** | 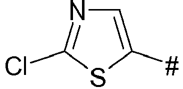 | H | H | H | H | i-C₃F₇ |
| **C.735** | 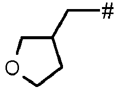 | H | H | H | H | i-C₃F₇ |
| **C.736** | 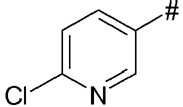 | H | CH₃ | H | H | i-C₃F₇ |
| **C.737** | 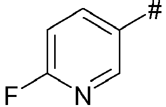 | H | CH₃ | H | H | i-C₃F₇ |
| **C.738** | 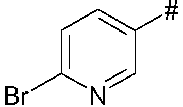 | H | CH₃ | H | H | i-C₃F₇ |
| **C.739** | 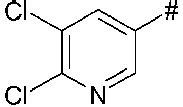 | H | CH₃ | H | H | i-C₃F₇ |
| **C.740** | 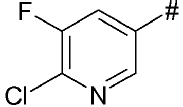 | H | CH₃ | H | H | i-C₃F₇ |
| **C.741** | 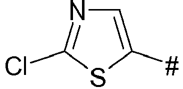 | H | CH₃ | H | H | i-C₃F₇ |
| **C.742** | 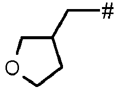 | H | CH₃ | H | H | i-C₃F₇ |
| **C.743** | 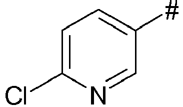 | H | H | H | F | i-C₃F₇ |
| **C.744** | 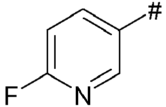 | H | H | H | F | i-C₃F₇ |
| **C.745** | 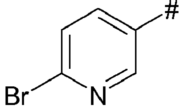 | H | H | H | F | i-C₃F₇ |
| **C.746** | 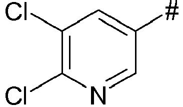 | H | H | H | F | i-C₃F₇ |
| **C.747** | 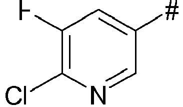 | H | H | H | F | i-C₃F₇ |
| **C.748** | 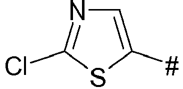 | H | H | H | F | i-C₃F₇ |
| **C.749** | 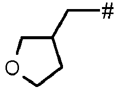 | H | H | H | F | i-C₃F₇ |
| **C.750** | 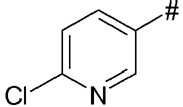 | H | CH₃ | H | F | i-C₃F₇ |
| **C.751** | 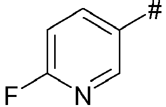 | H | CH₃ | H | F | i-C₃F₇ |
| **C.752** | 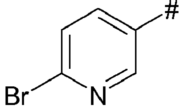 | H | CH₃ | H | F | i-C₃F₇ |
| **C.753** | 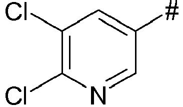 | H | CH₃ | H | F | i-C₃F₇ |
| **C.754** | 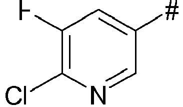 | H | CH₃ | H | F | i-C₃F₇ |
| **C.755** | 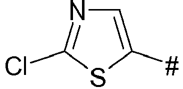 | H | CH₃ | H | F | i-C₃F₇ |
| **C.756** | 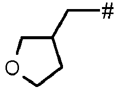 | H | CH₃ | H | F | i-C₃F₇ |
| **C.757** | 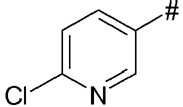 | H | H | F | F | i-C₃F₇ |
| **C.758** | 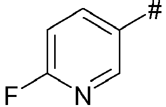 | H | H | F | F | i-C₃F₇ |
| **C.759** | 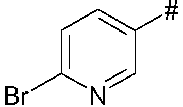 | H | H | F | F | i-C₃F₇ |
| **C.760** | 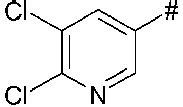 | H | H | F | F | i-C₃F₇ |
| **C.761** | 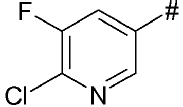 | H | H | F | F | i-C₃F₇ |
| **C.762** | 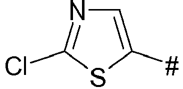 | H | H | F | F | i-C₃F₇ |
| **C.763** | 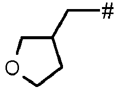 | H | H | F | F | i-C₃F₇ |
| **C.764** | 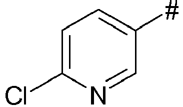 | H | CH₃ | F | F | i-C₃F₇ |
| **C.765** | 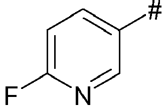 | H | CH₃ | F | F | i-C₃F₇ |
| **C.766** | 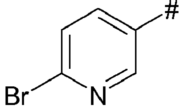 | H | CH₃ | F | F | i-C₃F₇ |
| **C.767** | 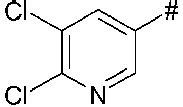 | H | CH₃ | F | F | i-C₃F₇ |
| **C.768** | 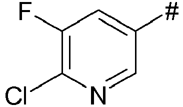 | H | CH₃ | F | F | i-C₃F₇ |
| **C.769** | 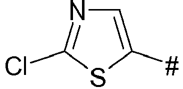 | H | CH₃ | F | F | i-C₃F₇ |
| **C.770** | 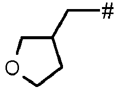 | H | CH₃ | F | F | i-C₃F₇ |
| **C.771** | 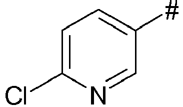 | H | H | H | CH₃ | i-C₃F₇ |
| **C.772** | 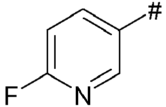 | H | H | H | CH₃ | i-C₃F₇ |
| **C.773** | 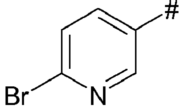 | H | H | H | CH₃ | i-C₃F₇ |
| **C.774** | 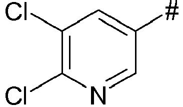 | H | H | H | CH₃ | i-C₃F₇ |
| **C.775** | 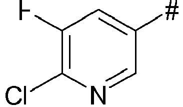 | H | H | H | CH₃ | i-C₃F₇ |
| **C.776** | 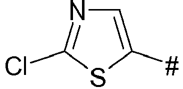 | H | H | H | CH₃ | i-C₃F₇ |
| **C.777** | 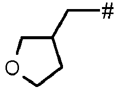 | H | H | H | CH₃ | i-C₃F₇ |
| **C.778** | 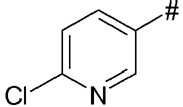 | H | CH₃ | H | CH₃ | i-C₃F₇ |
| **C.779** | 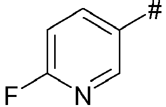 | H | CH₃ | H | CH₃ | i-C₃F₇ |
| **C.780** | 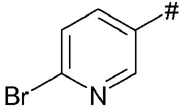 | H | CH₃ | H | CH₃ | i-C₃F₇ |
| **C.781** | 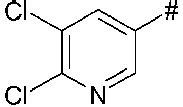 | H | CH₃ | H | CH₃ | i-C₃F₇ |
| **C.782** | 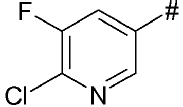 | H | CH₃ | H | CH₃ | i-C₃F₇ |
| **C.783** | 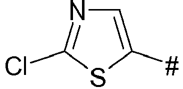 | H | CH₃ | H | CH₃ | i-C₃F₇ |
| **C.784** | 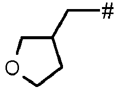 | H | CH₃ | H | CH₃ | i-C₃F₇ |

| | | | | | | |
|---|---|---|---|---|---|---|
| *wherein # denotes the bond to the remainder of the molecule. | | | | | | |

For example, the following especially preferred compound N-[1-[(6-chloro-3-pyridyl)methyl]-2-pyridylidene]-2-methylsulfanyl-acetamide: 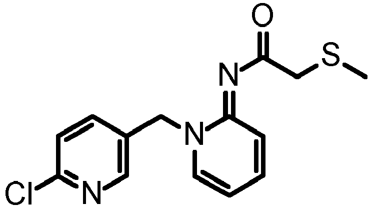 is represented in table C by compound C.1 of formula I-M.0.

Moreover, the meanings mentioned for those individual variables in the tables and further above are per se, independently of the combination in which they are mentioned, a particularly preferred embodiment of the substituents in question.

### Preparation methods

Compound of formula (I) according to the present invention can be prepared e.g. according the preparation methods and preparation schemes as described below.

Compounds of formula (I) according to the present invention can be prepared by standard methods of organic chemistry e.g. by the preparation methods and preparation schemes as described below. The definitions of Het, X, Y, R¹, R², R^{3a}, R^{3b}, R^{4a}, R^{4b} and R⁵ of the molecular structures given in the schemes are as defined above. Room temperature means a temperature range between about 20 and 25 °C.

An examples of a general method for the preparation of compounds of formula (I) is shown below in Scheme A. Thus, construction of pyridine element **3** present in compounds of formula (I) can be achieved, for example, by alkylation of the appropriate 2-amino pyridyl precursor **1** with the appropriate reagent of formula **2.** The transformation is preferably carried out in polar solvents such as acetonitrile, acetone, dichloromethane, tetrahydrofurane, N,N-dimethylformamide, or a C₁-C₆ alcohol ranging between room temperature and the reflux temperature of the solvent. Representative reaction conditions for the alkylation of pyridine precursors analogous to formula **1** are given in Tett. Lett. 2011, 52(23), 3033-3037. The synthesis of precursors of formula **5** can be achieved by acylation of the pyridine nitrogen in compounds of formula **3** using acids **4** which are activated *in situ.* The transformation is preferably carried out in polar solvents such as acetonitrile, acetone, tetrahydrofuran, N,N-dimethylformamide, or in an inert solvent such as dichloromethane, 1,2-dichloroethane, or 1,2-dimethoxyethane at temperatures ranging between room temperature and the reflux temperature of the solvent. A representative procedure conditions for the acylation of 2-substituted pyridines are given in Journal of Medicinal Chemistry, 1988, 31, 4, 807-814. Examples of suitable leaving groups (LG) in formulae **2** and **4** include, but are not limited to, halogen, alkyl sulfonate or haloalkyl sulfonate, alkyl phosphoante, and various activated esters derived from the reaction of the free carbonic acid with a peptide coupling reagent in the presense of an amine base. A reversal of the order of these two steps would also result in an acceptable synthesis of the desired compounds. In the cases where Y is S, the sulfur atom can be oxidized to the sulfoxide or the sulfone by following standard reaction conditions using *m*CPBA, H₂O₂, and NalO₄ as the terminal oxident. A representative procedure for the oxidation can be found in Tetrahedron Letters, 1982 ,23, 22, 2269-2272 or Journal of Heterocyclic Chemistry, 1978 ,15, 1361-1366.

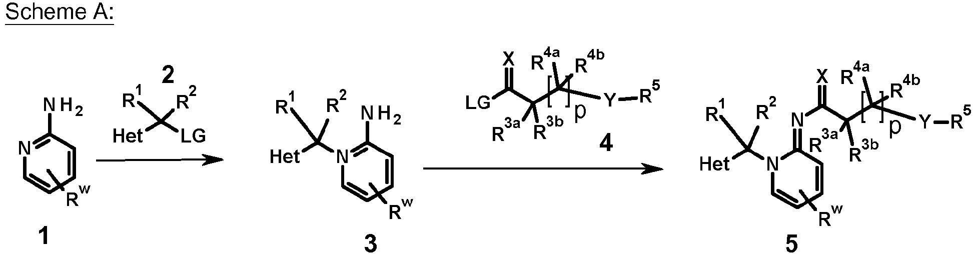

An example of a general method for the preparation of compounds of formula (I) of subclass **9** is presented in Scheme B. Compounds of structure type **9** represent a substructure of compounds of formula (I) with p=0. A solution of compound **3** that is prepared as described above in Scheme A can be acylated with reagent **6** using conditions described for synthesis of **5** to afford a compound of class **7.** Options for LG² include but are not limited to: chloride, bromide, iodide, alkyl sulfonate, and aryl sulfonate. In a subsequent reaction a nucleophile **8** will displace LG² to generate the desired compound **9**. The reaction is ideally performed in a polar solvent such as acetonitrile, acetone, dimethylsulfoxide, N, N-dimethylfornamide, N, N-methylacetamide, N-methylpyrrolidinone, or dimethoxyethane at a temperature ranging from between room temperature and the reflux temperature of the solvent, in the presence of a base such as lithium hydriede, sodium hydride, potassium hydride, potassium carbonate, sodium carbonate, lithium carbonate, sodium bicarbonate, potassium *t*-butoxide, sodium *t*-butoxide, lithium *t*-butoxide. A representative procedure can be found in Journal of Medicinal Chemistry, 2003, 46, 12, 2361-2375

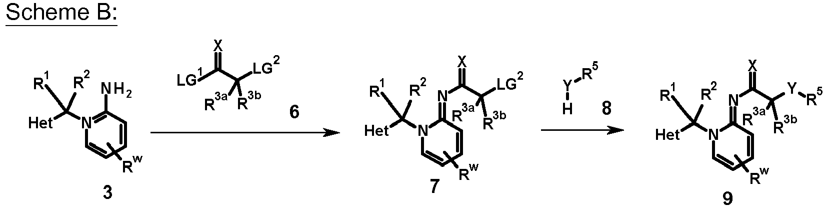

An example of a general method for the preparation of compounds of formula (I) of subclass 12 is presented in Scheme C. Compounds of structure type **12** represent a substructure of compounds of formula (I) with p=1. A solution of compound **3** that is prepared as described above in Scheme A can be acylated with reagent **10** using conditions described for synthesis of **5** to afford a compound of class **11.** Options for LG in compound **10** include but are not limited to: chloride, bromide, iodide, alkyl sulfonate, and aryl sulfonate. In a subsequent reaction a nucleophile **8** will add to the olefin in **11** to generate the desired compound **12.** The reaction is best performed in an inert or polar solvent: dichloromethane, tetrahydrofuran, 1,2-dichloroethane, 1,2-dimethoxyethane, 1,4-dioxane, dimethylsulfoxide, N, N-dimethylfornamide, N, N-methylacetamide, N-methylpyrrolidinone water in the presensce of a base such as: triethylamine, diisopropylethylamine, pyridine, piperadine, trimethylbenzyl hydroxide potassium carbonate, sodium carbonate, lithium carbonate, or sodium bicarbonate. The reaction is performed at a temperature between 0 C and the reflux temperature of the solvent. A representative procedure can be found in Journal of the American Chemical Society, 2008, 130, 16295-16309

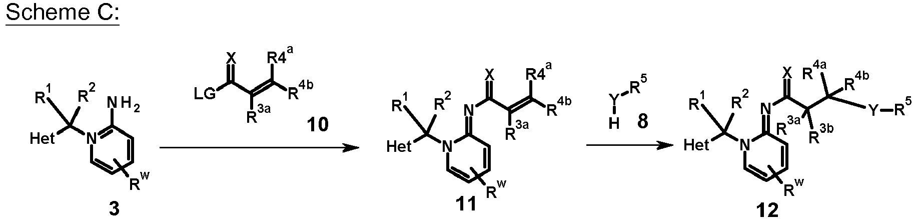

If individual compounds cannot be prepared via the above-described routes, they can be prepared by derivatization of other compounds (I) or by customary modifications of the synthesis routes described.

The reaction mixtures are worked up in the customary manner, for example by mixing with water, separating the phases, and, if appropriate, purifying the crude products by chromatography, for example on alumina or silica gel. Some of the intermediates and end products may be obtained in the form of colorless or pale brown viscous oils, which are freed or purified from volatile components under reduced pressure and at moderately elevated temperature. If the intermediates and end products are obtained as solids, they may be purified by recrystallization or digestion.

### Pests

The compounds of the formula I, and their salts are in particular suitable for efficiently controlling arthropodal pests such as arachnids, myriapedes and insects as well as nematodes.

The compounds of the formula I are especially suitable for efficiently combating the following pests:
insects from the order of the **lepidopterans** (***Lepidoptera*)***,* for example *Acronicta major, Adox-ophyes orana, Aedia leucomelas,* Agrotis spp. such as *Agrotis fucosa, Agrotis segetum, Agrotis ypsilon; Alabama argillacea, Anticarsia gemmatalis, Anticarsia spp., Argyresthia conjugella, Autographa gamma, Barathra brassicae, Bucculatrix thurberiella, Bupalus piniarius, Cacoecia murinana, Cacoecia podana, Capua reticulana, Carpocapsa pomonella, Cheimatobia brumata,* Chilo spp. such as *Chilo suppressalis; Choristoneura fumiferana, Choristoneura occidentalis, Cirphis unipuncta, Clysia ambiguella, Cnaphalocerus spp., Cydia pomonella, Dendrolimus pini, Diaphania nitidalis, Diatraea grandiosella, Earias insulana, Elasmopalpus lignosellus, Ephestia cautella, Ephestia kuehniella, Eupoecilia ambiguella, Euproctis chrysorrhoea, Euxoa spp., Evetria bouliana,* Feltia spp. such as *Feltia subterranean; Galleria mellonella, Grapholitha funebrana, Grapholitha molesta,* Helicoverpa spp. such as *Helicoverpa armigera, Helicoverpa zea;* Heliothis spp. such as *Heliothis armigera, Heliothis virescens, Heliothis zea; Hellula undalis, Hibernia defoliaria, Hofmannophila pseudospretella, Homona magnanima, Hyphantria cunea, Hyponomeuta padella, Hyponomeuta malinellus, Keiferia lycopersicella, Lambdina fiscellaria,* Laphygma spp. such as *Laphygma exigua; Leucoptera coffeella, Leucoptera scitella, Lithocolletis blancardella, Lithophane antennata, Lobesia botrana, Loxagrotis albicosta, Loxostege sticticalis,* Lymantria spp. such as *Lymantria dispar, Lymantria monacha; Lyonetia clerkella, Malacosoma neustria,* Mamestra spp. such as *Mamestra brassicae; Mocis repanda, Mythimna separata, Orgyia pseudotsugata, Oria spp.,* Ostrinia spp. such as *Ostrinia nubilalis; Oulema oryzae, Panolis flammea,* Pectinophora spp. such as *Pectinophora gossypiella; Peridroma saucia, Phalera bucephala,* Phthorimaea spp. such as *Phthorimaea operculella; Phyllocnistis citrella,* Pieris spp. such as *Pieris brassicae, Pieris rapae; Plathypena scabra, Plutella maculipennis, Plutella xylostella, Prodenia spp., Pseudaletia spp., Pseudoplusia includens, Pyrausta nubilalis, Rhyacionia frustrana, Scrobipalpula absoluta, Sitotroga cerealella, Sparganothis pilleriana,* Spodoptera spp. such as *Spodoptera frugiperda, Spodoptera littoralis, Spodoptera litura; Thaumatopoea pityocampa, Thermesia gemmatalis, Tinea pellionella, Tineola bisselliella, Tortrix viridana,* Trichoplusia spp. such as *Trichoplusia ni; Tuta absoluta, and Zeiraphera canadensis,*
**beetles** (***Coleoptera***), for example *Acanthoscehdes obtectus, Adoretus spp., Agelastica alni, Agrilus sinuatus,* Agriotes spp. such as *Agriotes fuscicollis, Agriotes lineatus, Agriotes obscurus; Amphimallus solstitialis, Anisandrus dispar, Anobium punctatum, Anomala rufocuprea,* Anoplophora spp. such as *Anoplophora glabripennis;* Anthonomus spp. such as *Anthonomus grandis, Anthonomus pomorum; Anthrenus spp., Aphthona euphoridae, Apogonia spp., Athous haemorrhoidalis,* Atomaria spp. such as *Atomaria linearis; Attagenus spp., Aulacophora femoralis, Blastophagus piniperda, Blitophaga undata, Bruchidius obtectus,* Bruchus spp. such as *Bruchus lentis, Bruchus pisorum, Bruchus rufimanus; Byctiscus betulae, Callosobruchus chinensis, Cassida nebulosa, Cerotoma trifurcata, Cetonia aurata,* Ceuthorhynchus spp. such as *Ceuthorrhynchus assimilis, Ceuthorrhynchus napi; Chaetocnema tibialis, Cleonus mendicus,* Conoderus spp. such as *Conoderus vespertinus; Cosmopolites spp., Costelytra zealandica, Crioceris asparagi, Cryptorhynchus lapathi,* Ctenicera ssp. such as *Ctenicera destructor; Curculio spp., Dectes texanus, Dermestes spp.,* Diabrotica spp. such as *Diabrotica 12-punctata Diabrotica speciosa, Diabrotica longicornis, Diabrotica semipunctata, Diabrotica virgifera;* Epilachna spp. such as *Epilachna varivestis, Epilachna vigintioctomaculata;* Epitrix spp. such as *Epitrix hirtipennis; Eutinobothrus brasiliensis, Faustinus cubae, Gibbium psylloides, Heteronychus arator, Hylamorpha elegans, Hylobius abietis, Hylotrupes bajulus, Hypera brunneipennis, Hypera postica, Hypothenemus spp., Ips typographus, Lachnosterna consanguinea, Lema bilineata, Lema melanopus,* Leptinotarsa spp. such as *Leptinotarsa decemlineata; Limonius californicus, Lissorhoptrus oryzophilus, Lissorhoptrus oryzophilus, Lixus spp.,* Lyctus spp. such as *Lyctus bruneus; Melanotus communis,* Meligethes spp. such as *Meligethes aeneus; Melolontha hippocastani, Melolontha melolontha, Migdolus spp.,* Monochamus spp. such as *Monochamus alternatus; Naupactus xanthographus, Niptus hololeucus, Oryctes rhinoceros, Oryzae-philus surinamensis, Otiorrhynchus sulcatus, Otiorrhynchus ovatus, Otiorrhynchus sulcatus, Oulema oryzae, Oxycetonia jucunda, Phaedon cochleariae, Phyllobius pyri, Phyllopertha horticola, Phyllophaga spp.,* Phyllotreta spp. such as *Phyllotreta chrysocephala, Phyllotreta nemorum, Phyllotreta striolata; Phyllophaga spp., Phyllopertha horticola, Popillia japonica, Premnotrypes spp., Psylliodes chrysocephala, Ptinus spp., Rhizobius ventralis , Rhizopertha dominica, Sitona lineatus,* Sitophilus spp. such as *Sitophilus granaria, Sitophilus zeamais;* Sphenophorus spp. such as *Sphenophorus levis;* Sternechus spp. such as *Sternechus subsignatus; Symphyletes spp., Tenebrio molitor,* Tribolium spp. such as *Tribolium castaneum; Trogoderma spp., Tychius spp., Xylotrechus spp., and Zabrus spp. such as Zabrus tenebrioides,*
flies, mosquitoes (*Diptera*), e.g. Aedes spp. such as *Aedes aegypti, Aedes albopictus, Aedes vexans; Anastrepha ludens,* Anopheles spp. such as *Anopheles albimanus, Anopheles crucians, Anopheles freeborni, Anopheles gambiae, Anopheles leucosphyrus, Anopheles maculipennis, Anopheles minimus, Anopheles quadrimaculatus, Anopheles sinensis; Bibio hortulanus, Calliphora erythrocephala, Calliphora vicina, Cerafitis capitata, Ceratitis capitata,* Chrysomyia spp. such as *Chrysomya bezziana, Chrysomya hominivorax, Chrysomya macellaria; Chrysops atlanticus, Chrysops discalis, Chrysops silacea,* Cochliomyia spp. such as *Cochliomyia hominivorax;* Contarinia spp. such as *Contarinia sorghicola; Cordylobia anthropophaga,* Culex spp. such as *Culex nigripalpus, Culex pipiens, Culex quinquefasciatus, Culex tarsalis, Culex tri-taeniorhynchus; Culicoides furens, Culiseta inornata, Culiseta melanura, Cuterebra spp., Dacus cucurbitae, Dacus oleae, Dasineura brassicae,* Delia spp. such as *Delia antique, Delia coarctata, Delia platura, Delia radicum; Dermatobia hominis, Drosophila spp.,* Fannia spp. such as *Fannia canicularis;* Gastraphilus spp. such as *Gasterophilus intestinalis; Geomyza Tripunctata, Glossina fuscipes, Glossina morsitans, Glossina palpalis, Glossina tachinoides, Haematobia irritans, Haplodiplosis equestris, Hippelates spp.,* Hylemyia spp. such as *Hylemyia platura;* Hypoderma spp. such as *Hypoderma lineata; Hyppobosca spp., Leptoconops torrens,* Liriomyza spp. such as *Liriomyza sativae, Liriomyza trifolii,* Lucilia spp. such as *Lucilia caprina, Lucilia cuprina, Lucilia sericata; Lycoria pectoralis, Mansonia titillanus,* Mayetiola spp. such as *Mayetiola destructor;* Musca spp. such as *Musca autumnalis, Musca domestica; Muscina stabulans,* Oestrus spp. such as *Oestrus ovis; Opomyza florum,* Oscinella spp. such as *Oscinella frit; Pegomya hysocyami, Phlebotomus argentipes,* Phorbia spp. such as *Phorbia antiqua, Phorbia brassicae, Phorbia coarctata; Prosimulium mixtum, Psila rosae, Psorophora columbiae, Psorophora discolor, Rhagoletis cerasi, Rhagoletis pomonella,* Sarcophaga spp. such as *Sarcophaga haemorrhoidalis; Simulium vittatum,* Stomoxys spp. such as *Stomoxys calcitrans;* Tabanus spp. such as *Tabanus atratus, Tabanus bovinus, Tabanus lineola, Tabanus similis; Tannia spp., Tipula oleracea, Tipula paludosa, and Wohlfahrtia spp.,*
thrips **(*Thysanoptera*)**, e.g. *Baliothrips biformis, Dichromothrips corbetti, Dichromothrips ssp., Enneothrips flavens,* Frankliniella spp. such as *Frankliniella fusca, Frankliniella occidentalis, Frankliniella tritici; Heliothrips spp., Hercinothrips femoralis, Kakothrips spp., Rhipiphorothrips cruentatus,* Scirtothrips spp. such as *Scirtothrips citri; Taeniothrips cardamoni,* Thrips spp. such as *Thrips oryzae, Thrips palmi, Thrips tabaci;*
**termites (Isoptera),** e.g. *Calotermes flavicollis, Coptotermes formosanus, Heterotermes aureus, Heterotermes longiceps, Heterotermes tenuis, Leucotermes flavipes, Odontotermes spp.,* Reticulitermes spp. such as *Reticulitermes speratus, Reticulitermes flavipes, Reticulitermes grassei, Reticulitermes lucifugus, Reticulitermes santonensis, Reticulitermes virginicus; Termes natalensis,*
**cockroaches (Blattaria - Blattodea),** e.g. *Acheta domesticus, Blatta orientalis, Blattella asahinae, Blattella germanica, Gryllotalpa spp., Leucophaea maderae, Locusta spp., Melanoplus spp., Periplaneta americana, Periplaneta australasiae, Periplaneta brunnea, Periplaneta fuligginosa, Periplaneta japonica,*
**bugs, aphids, leafhoppers, whiteflies, scale insects, cicadas (*Hemiptera*),** e.g. Acrosternum spp. such as *Acrosternum hilare;* Acyrthosipon spp. such as *Acyrthosiphon onobrychis, Acyrthosiphon pisum; Adelges laricis, Aeneolamia spp., Agonoscena spp., Aleurodes spp., Aleurolobus barodensis, Aleurothrixus spp., Amrasca spp., Anasa tristis, Antestiopsis spp., Anuraphis cardui, Aonidiella spp., Aphanostigma piri, Aphidula nasturtii,* Aphis spp. such as *Aphis fabae, Aphis forbesi, Aphis gossypii, Aphis grossulariae, Aphis pomi, Aphis sambuci, Aphis schneideri, Aphis spiraecola; Arboridia apicalis, Arilus critatus, Aspidiella spp., Aspidiotus spp., Atanus spp., Aulacorthum solani,* Bemisia spp. such as *Bemisia argentifolii, Bemisia tabaci;* Blissus spp. such as *Blissus leucopterus; Brachycaudus cardui, Brachycaudus helichrysi, Brachycaudus persicae, Brachycaudus prunicola, Brachycolus spp., Brevicoryne brassicae, Calligypona marginata, Calocoris spp., Campylomma livida, Capitophorus horni, Carneocephala fulgida, Cavelerius spp., Ceraplastes spp., Ceratovacuna lanigera, Cercopidae, Cerosipha gossypii, Chaetosiphon fragaefolii, Chionaspis tegalensis, Chlorita onukii, Chromaphis juglandicola, Chrysomphalus ficus, Cicadulina mbila,* Cimex spp. such as *Cimex hemipterus, Cimex lectularius; Coccomytilus halli, Coccus spp., Creontiades dilutus, Cryptomyzus ribis, Cryptomyzus ribis, Cyrtopeltis notatus, Dalbulus spp., Dasynus piperis, Dialeurades spp., Diaphorina spp., Diaspis spp., Dichelops furcatus, Diconocoris hewetti, Doralis spp., Dreyfusia nordmannianae, Dreyfusia piceae, Drosicha spp.,* Dysaphis spp. such as *Dysaphis plantaginea, Dysaphis pyri, Dysaphis radicola; Dysaulacorthum pseudosolani,* Dysdercus spp. such as *Dysdercus cingulatus, Dysdercus intermedius; Dysmicoccus spp.,* Empoasca spp. such as *Empoasca fabae, Empoasca solana; Eriosoma spp., Erythroneura spp.,* Eurygaster spp. such as *Eurygaster integriceps; Euscelis bilobatus, Euschistus spp. such as Euschistuos heros, Euschistus impictiventris, Euschistus servus; Geococcus coffeae,* Halyomorpha spp. such as *Halyomorpha halys; Heliopeltis spp., Homalodisca coagulata, Horcias nobilellus, Hyalopterus pruni, Hyperomyzus lactucae, Icerya spp., Idiocerus spp., Idioscopus spp., Laodelphax striatellus, Lecanium spp., Lepidosaphes spp., Leptocorisa spp., Leptoglossus phyllopus, Lipaphis erysimi,* Lygus spp. such as *Lygus hesperus, Lygus lineolaris, Lygus pratensis; Macropes excavatus,* Macrosiphum spp. such as *Macrosiphum rosae, Macrosiphum avenae, Macrosiphum euphorbiae; Mahanarva fimbriolata, Megacopta cribraria, Megoura viciae, Melanaphis pyrarius, Melanaphis sacchari, Metcafiella spp., Metopolophium dirhodum, Miridae spp., Monellia costalis, Monelliopsis pecanis,* Myzus spp. such as *Myzus ascalonicus, Myzus cerasi, Myzus persicae, Myzus varians; Nasonovia ribis-nigri,* Nephotettix spp. such as *Nephotettix malayanus, Nephotettix nigropictus, Nephotettix parvus, Nephotettix virescens;* Nezara spp. such as *Nezara viridula; Nilaparvata lugens, Oebalus spp., Oncometopia spp., Orthezia praelonga, Parabemisia myricae, Paratrioza spp., Parlatoria spp.,* Pemphigus spp. such as *Pemphigus bursarius; Pentomidae, Peregrinus maidis, Perkinsiella saccharicida, Phenacoccus spp., Phloeomyzus passerinii, Phorodon humuli, Phylloxera spp., Piesma quadrata,* Piezodorus spp. such as *Piezodorus guildinii, Pinnaspis aspidistrae, Planococcus spp., Protopulvinaria pyriformis, Psallus seriatus, Pseudacysta persea, Pseudaulacaspis pentagona,* Pseudococcus spp. such as *Pseudococcus comstocki;* Psylla spp. such as *Psylla mali, Psylla piri; Pteromalus spp., Pyrilla spp., Quadraspidiotus spp., Quesada gigas, Rastrococcus spp., Reduvius senilis, Rhodnius spp., Rhopalomyzus ascalonicus,* Rhopalosiphum spp. such as *Rhopalosiphum pseudobrassicas, Rhopalosiphum insertum, Rhopalosiphum maidis, Rhopalosiphum padi; Sagatodes spp., Sahlbergella singularis, Saissetia spp., Sappaphis mala, Sappaphis mali, Scaphoides titanus, Schizaphis graminum, Schizoneura lanuginosa, Scotinophora spp., Selenaspidus articulatus, Sitobion avenae, Sogata spp., Sogatella furcifera, Solubea insularis , Stephanitis nashi, Stictocephala festina, Tenalaphara malayensis,* Thyanta spp. such as *Thyanta perditor; Tibraca spp., Tinocallis caryaefoliae, Tomaspis spp.,* Toxoptera spp. such as *Toxoptera aurantii;* Trialeurodes spp. such as *Trialeurodes vaporariorum; Triatoma spp., Trioza spp., Typhlocyba spp.,* Unaspis spp. such as *Unaspis yanonensis; and Viteus vitifolii,*
**ants, bees, wasps, sawflies (Hymenoptera),** e.g. *Athalia rosae, Atta capiguara, Atta cephalotes, Atta cephalotes, Atta laevigata, Atta robusta, Atta sexdens, Atta texana, Bombus spp., Camponotus floridanus, Crematogaster spp., Dasymutilla occidentalis, Diprion spp., Dolichovespula maculata,* Hoplocampa spp. such as *Hoplocampa minuta, Hoplocampa testudinea;* Lasius spp. such as *Lasius niger, Linepithema humile, Monomorium pharaonis, Paravespula germanica, Paravespula pennsylvanica, Paravespula vulgaris, Pheidole megacephala, Pogonomyrmex barbatus, Pogonomyrmex californicus, Polistes rubiginosa, Solenopsis geminata, Solenopsis invicta, Solenopsis richteri, Solenopsis xyloni,* Vespa spp. such as *Vespa crabro, and Vespula squamosa,*
**crickets, grasshoppers, locusts (Orthoptera),** e.g. *Acheta domestica, Calliptamus italicus, Chortoicetes terminifera, Dociostaurus maroccanus, Gryllotalpa africana, Gryllotalpa gryllotalpa, Hieroglyphus daganensis, Kraussaria angulifera, Locusta migratoria, Locustana pardalina, Melanoplus bivittatus, Melanoplus femurrubrum, Melanoplus mexicanus, Melanoplus sanguinipes, Melanoplus spretus, Nomadacris septemfasciata, Oedaleus senegalensis, Schistocerca americana, Schistocerca gregaria, Tachycines asynamorus, and Zonozerus variegatus,*
**arachnids (*Arachnida*),** such as acari,e.g. of the families Argasidae, Ixodidae and Sarcoptidae, such as Amblyomma spp. (e.g. *Amblyomma americanum, Amblyomma variegatum, Amblyomma maculatum),* Argas spp. (e.g. *Argas persicus),* Boophilus spp. (e.g. *Boophilus annulatus, Boophilus decoloratus, Boophilus microplus), Dermacentor silvarum, Dermacentor andersoni, Dermacentor variabilis,* Hyalomma spp. (e.g. *Hyalomma truncatum*), Ixodes spp. (e.g. *Ixodes ricinus, Ixodes rubicundus, Ixodes scapularis, Ixodes holocyclus, Ixodes pacificus),* Ornithodorus spp. (e.g. *Ornithodorus moubata, Ornithodorus hermsi, Ornithodorus turicata), Ornithonyssus bacoti, Otobius megnini, Dermanyssus gallinae,* Psoroptes spp. (e.g. *Psoroptes ovis*), Rhipicephalus spp. (e.g. *Rhipicephalus sanguineus, Rhipicephalus appendiculatus, Rhipicephalus evertsi*), *Rhizoglyphus spp.,* Sarcoptes spp. (e.g. *Sarcoptes scabiei*)*,* and Eriophyidae spp. such as *Acaria sheldoni,* Aculops spp. (e.g. *Aculops pelekassi*) Aculus spp. (e.g. *Aculus schlechtendali*)*, Epitrimerus pyri, Phyllocoptruta oleivora* and Eriophyes spp. (e.g. *Eriophyes sheldoni*); Tarsonemidae spp. such as *Hemitarsonemus spp*., *Phytonemus pallidus and Polyphagotarsonemus latus, Stenotarsonemus spp.;* Tenuipalpidae spp. such as Brevipalpus spp. (e.g. *Brevipalpus phoenicis*); Tetranychidae spp. such as *Eotetranychus spp*., *Eutetranychus spp*., *Oligonychus spp., Tetranychus cinnabarinus, Tetranychus kanzawai, Tetranychus pacificus, Tetranychus telarius* and *Tetranychus* urticae; *Bryobia praetiosa*, Panonychus spp. (e.g. *Panonychus ulmi*, *Panonychus citri*), *Metatetranychus spp.* and Oligonychus spp. (e.g. *Oligonychus pratensis*), *Vasates lycopersici*; Araneida, e.g. *Latrodectus mactans*, and *Loxosceles reclusa.* And *Acarus siro, Chorioptes spp*., *Scorpio maurus*
**fleas** (***Siphonaptera***)*,* e.g. *Ceratophyllus spp., Ctenocephalides felis, Ctenocephalides canis, Xenopsylla cheopis*, *Pulex irritans*, *Tunga penetrans*, and *Nosopsyllus fasciatus,*
**silverfish, firebrat** (***Thysanura***), e.g. *Lepisma saccharina* and *Thermobia domestica,*
**centipedes** (***Chilopoda***)*,* e.g. *Geophilus spp.,* Scutigera spp. such as *Scutigera coleoptrata*;
**millipedes** (***Diplopoda***)*,* e.g. *Blaniulus guttulatus*, *Narceus spp*.,
**Earwigs (*Dermaptera*),** e.g. *forficula auricularia,*
**lice (*Phthiraptera*),** e.g. *Damalinia spp.,* Pediculus spp. such as *Pediculus humanus capitis, Pediculus humanus corporis; Pthirus pubis,* Haematopinus spp. such as *Haematopinus eurysternus*, *Haematopinus suis*; Linognathus spp. such as *Linognathus vituli; Bovicola bovis, Menopon gallinae, Menacanthus stramineus* and *Solenopotes capillatus, Trichodectes spp.,*
**springtails (*Collembola*),** *e.g.* Onychiurus ssp. such as *Onychiurus armatus,*

They are also suitable for controlling **nematodes:** plant parasitic nematodes such as root knot nematodes, *Meloidogyne hapla, Meloidogyne incognita, Meloidogyne javanica,* and other Meloidogyne species; cyst-forming nematodes, *Globodera rostochiensis* and other Globodera species; *Heterodera avenae, Heterodera glycines, Heterodera schachtii, Heterodera trifolii,* and other Heterodera species; Seed gall nematodes, Anguina species; Stem and foliar nematodes, Aphelenchoides species such as *Aphelenchoides besseyi* ; Sting nematodes, *Belonolaimus longicaudatus* and other Belonolaimus species; Pine nematodes, *Bursaphelenchus lignicolus Mamiya et Kiyohara, Bursaphelenchus xylophilus* and other Bursaphelenchus species; Ring nematodes, Criconema species, Criconemella species, Criconemoides species, Mesocriconema species; Stem and bulb nematodes, *Ditylenchus destructor, Ditylenchus dipsaci and* other Ditylenchus species; Awl nematodes, Dolichodorus species; Spiral nematodes, *Heliocotylenchus multicinctus* and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, *Longidorus elongatus* and other Longidorus species; Lesion nematodes, *Pratylenchus brachyurus, Pratylenchus neglectus, Pratylenchus penetrans, Pratylenchus curvitatus, Pratylenchus goodeyi and* other Pratylenchus species; Burrowing nematodes, *Radopholus similis* and other Radopholus species; Reniform nematodes, *Rotylenchus robustus, Rotylenchus reniformis* and other Rotylenchus species; Scutellonema species; Stubby root nematodes, *Trichodorus primitivus* and other Trichodorus species, Paratrichodorus species; Stunt nematodes, *Tylenchorhynchus claytoni*, *Tylenchorhynchus dubius* and other Tylenchorhynchus species; Citrus nematodes, Tylenchulus species such as *Tylenchulus semipenetrans*; Dagger nematodes, Xiphinema species; and other plant parasitic nematode species.

Examples of further pest species which may be controlled by compounds of fomula (I) include: from the class of the *Bivalva*, for example, *Dreissena spp*.; from the class of the *Gastropoda,* for example, *Arion spp., Biomphalaria spp., Bulinus spp., Deroceras spp., Galba spp., Lymnaea spp., Oncomelania spp., Succinea spp.;* from the class of the *helminths,* for example, *Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp.,* Haemonchus spp. such as *Haemonchus contortus; Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercora lis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti;* from the order of the *Isopoda,* for example, *Armadillidium vulgare, Oniscus asellus, Porcellio scaber;* from the order of the *Symphyla,* for example, *Scutigerella immaculata.*

Further examples of pest species which may be controlled by compounds of formula (I) include:
*Anisoplia austriaca, Apamea spp., Austroasca viridigrisea, Baliothrips biformis, Caenorhabditis elegans, Cephus spp., Ceutorhynchus napi, Chaetocnema aridula, Chilo auricilius, Chilo indicus , Chilo polychrysus, Chortiocetes terminifera, Cnaphalocroci medinalis, Cnaphalocrosis spp., Colias eurytheme, Collops spp., Cornitermes cumulans, Creontiades spp., Cyclocephala spp., Dalbulus maidis, Deraceras reticulatum , Diatrea saccharalis, Dichelops furcatus, Dicladispa armigera,* Diloboderus spp. such as *Diloboderus abderus; Edessa spp., Epinotia spp., Formicidae, Geocoris spp., Globitermes sulfureus, Gryllotalpidae, Halotydeus destructor, Hipnodes bicolor, Hydrellia philippina, Julus spp., Laodelphax spp., Leptocorsia acuta, Leptocorsia oratorius , Liogenys fuscus, Lucillia spp., Lyogenys fuscus, Mahanarva spp., Maladera matrida, Marasmia spp., Mastotermes spp., Mealybugs, Megascelis ssp, Metamasius hemipterus, Microtheca spp., Mocis latipes, Murgantia spp., Mythemina separata , Neocapritermes opacus, Neocapritermes parvus, Neomegalotomus spp., Neotermes spp., Nymphula depunctalis, Oebalus pugnax,* Orseolia spp. such as *Orseolia oryzae; Oxycaraenus hyalinipennis, Plusia spp., Pomacea canaliculata, Procornitermes ssp, Procornitermes triacifer , Psylloides spp., Rachiplusia spp., Rhodopholus spp., Scaptocoris castanea, Scaptocoris spp.,* Scirpophaga spp. such as *Scirpophaga incertulas, Scirpophaga innotata;* Scotinophara spp. such as *Scotinophara coarctata;* Sesamia spp. such as *Sesamia inferens, Sogaella frucifera, Solenapsis geminata, Spissistilus spp., Stalk borer, Stenchaetothrips biformis, Steneotarsonemus spinki, Sylepta derogata, Telehin licus, Trichostrongylus spp..*

### Formulations

The invention also relates to agrochemical compositions comprising an auxiliary and at least one compound I according to the invention.

An agrochemical composition comprises a pesticidally effective amount of a compound I. The term "effective amount" denotes an amount of the composition or of the compounds I, which is sufficient for controlling harmful pests on cultivated plants, plant propagation material or crops or in the protection of materials and which does not result in a substantial damage to the treated plants. Such an amount can vary in a broad range and is dependent on various factors, such as the pest species to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the " Catalogue of pesticide formulation types and international coding system" , Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Examples for suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharide powders, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon' s, Vol.1: Emulsifiers & Detergents, McCutcheon' s Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:

### i) Water-soluble concentrates (SL, LS)

10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.

### ii) Dispersible concentrates (DC)

5-25 wt% of a compound I according to the invention and 1-10 wt% dispersant (e. g. polyvinylpyrrolidone) are dissolved in up to 100 wt% organic solvent (e.g. cyclohexanone). Dilution with water gives a dispersion.

### iii) Emulsifiable concentrates (EC)

15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.

### iv) Emulsions (EW, EO, ES)

5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.

### v) Suspensions (SC, OD, FS)

In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active substance. For FS type composition up to 40 wt% binder (e.g. polyvinylalcohol) is added.

### vi) Water-dispersible granules and water-soluble granules (WG, SG)

50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e. g. extrusion, spray tower, fluidized bed). Dilution with water gives a stable dispersion or solution of the active substance.

### vii) Water-dispersible powders and water-soluble powders (WP, SP, WS)

50-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dispersion or solution of the active substance.

### viii) Gel (GW, GF)

In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active substance. Dilution with water gives a stable suspension of the active substance.

### ix) Microemulsion (ME)

5-20 wt% of a compound I according to the invention are added to 5-30 wt% organic solvent blend (e.g. fatty acid dimethylamide and cyclohexanone), 10-25 wt% surfactant blend (e.g. alkohol ethoxylate and arylphenol ethoxylate), and water up to 100 %. This mixture is stirred for 1 h to produce spontaneously a thermodynamically stable microemulsion.

### x) Microcapsules (CS)

An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylmethene-4,4' -diisocyanatae) are dispersed into an aqueous solution of a protective colloid (e.g. polyvinyl alcohol). The addition of a polyamine (e.g. hexamethylenediamine) results in the formation of a polyurea microcapsules. The monomers amount to 1-10 wt%. The wt% relate to the total CS composition.

### xi) Dustable powders (DP, DS)

1-10 wt% of a compound I according to the invention are ground finely and mixed intimately with up to 100 wt% solid carrier, e.g. finely divided kaolin.

### xii) Granules (GR, FG)

0.5-30 wt% of a compound I according to the invention is ground finely and associated with up to 100 wt% solid carrier (e.g. silicate). Granulation is achieved by extrusion, spray-drying or the fluidized bed.

### xiii) Ultra-low volume liquids (UL)

1-50 wt% of a compound I according to the invention are dissolved in up to 100 wt% organic solvent, e.g. aromatic hydrocarbon.

The compositions types i) to xiii) may optionally comprise further auxiliaries, such as 0,1-1 wt% bactericides, 5-15 wt% anti-freezing agents, 0,1-1 wt% anti-foaming agents, and 0,1-1 wt% colorants.

The agrochemical compositions generally comprise between 0.01 and 95%, preferably between 0.1 and 90%, and most preferably between 0.5 and 75%, by weight of active substance. The active substances are employed in a purity of from 90% to 100%, preferably from 95% to 100% (according to NMR spectrum).

Water-soluble concentrates (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The compositions in question give, after two-to-tenfold dilution, active substance concentrations of from 0.01 to 60% by weight, preferably from 0.1 to 40% by weight, in the ready-to-use preparations. Application can be carried out before or during sowing. Methods for applying or treating compound I and compositions thereof, respectively, on to plant propagation material, especially seeds include dressing, coating, pelleting, dusting, soaking and in-furrow application methods of the propagation material. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, in particular from 0.1 to 0.75 kg per ha.
In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seed) are generally required.
When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and other pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank and further auxiliaries may be added, if appropriate.

In a further embodiment, either individual components of the composition according to the invention or partially premixed components, e. g. components comprising compounds I and/or active substances from the groups described herein further below, may be mixed by the user in a spray tank and further auxiliaries and additives may be added, if appropriate.

In a further embodiment, either individual components of the composition according to the invention or partially premixed components, e. g. components comprising compounds I and/or active substances from the groups described herein further below, can be applied jointly (e.g. after tank mix) or consecutively.

As mentioned above, in the method of this invention compounds I may be applied with other active ingredients, for example as mixtures with other pesticides, insecticides, herbicides, fertilizers such as ammonium nitrate, urea, potash, and superphosphate, phytotoxicants and plant growth regulators, safeners and nematicides.

These additional ingredients may be used sequentially or in combination with the above-described compositions, if appropriate also added only immediately prior to use (tank mix). For example, the plant(s) may be sprayed with a composition of this invention either before or after being treated with other active ingredients.

### Mixtures

The following list M of pesticides, grouped and numbered according the Mode of Action Classification of the Insecticide Resistance Action Committee (IRAC), together with which the compounds according to the invention can be used and with which potential synergistic effects might be produced, is intended to illustrate the possible combinations, but not to impose any limitation:
M.1 Acetylcholine esterase (AChE) inhibitors from the class of
M.1A carbamates, for example aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodi-carb, thiofanox, trimethacarb, XMC, xylylcarb and triazamate; or from the class of
M.1 B organophosphates, for example acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O- (methoxyaminothiophosphoryl) salicylate, isoxathion, malathion, mecarbam, methamidophos, methidathion, mevinphos, monocrotophos, naled, omethoate, oxydemeton-methyl, parathion, parathionmethyl, phenthoate, phorate, phosalone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon and vamidothion;
M.2. GABA-gated chloride channel antagonists such as:
   M.2A cyclodiene organochlorine compounds, as for example endosulfan or chlordane; or
   M.2B fiproles (phenylpyrazoles), as for example ethiprole, fipronil, flufiprole, pyrafluprole and pyriprole;
M.3 Sodium channel modulators from the class of
M.3A pyrethroids, for example acrinathrin, allethrin, d-cis-trans allethrin, d-trans allethrin, bifenthrin, bioallethrin, bioallethrin S-cylclopentenyl, bioresmethrin, cycloprothrin, cyfluthrin, betacyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, del-tamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin,metofluthrin, momfluorothrin, permethrin, phenothrin, prallethrin, profluthrin, pyrethrin (pyrethrum), resmethrin, silafluofen, tefluthrin, tetramethylfluthrin, tetramethrin, tralomethrin and transfluthrin;
   or
M.3B sodium channel modulators such as DDT or methoxychlor;
M.4 Nicotinic acetylcholine receptor agonists (nAChR) from the class of
M.4A neonicotinoids, for example acteamiprid, chlothianidin, cycloxaprid, dinotefuran, imidaclo-prid, nitenpyram, thiacloprid and thiamethoxam; or the compounds
M.4A.2: (2E-)-1-[(6-Chloropyridin-3-yl)methyl]-N'-nitro-2-pentylidenehydrazinecarboximidamide; or
M4.A.3: 1-[(6-Chloropyridin-3-yl)methyl]-7-methyl-8-nitro-5-propoxy-1,2,3,5,6,7-hexahydroimidazo[1,2-a]pyridine;
   or from the class M.4B nicotine;
M.5 Nicotinic acetylcholine receptor allosteric activators from the class of spinosyns,
   for example spinosad or spinetoram;
M.6 Chloride channel activators from the class of avermectins and milbemycins, for example abamectin, emamectin benzoate, ivermectin, lepimectin or milbemectin;
M.7 Juvenile hormone mimics, such as
M.7Ajuvenile hormone analogues as hydroprene, kinoprene and methoprene; or others as
M.7B fenoxycarb or M.7C pyriproxyfen;
M.8 miscellaneous non-specific (multi-site) inhibitors, for example
M.8A alkyl halides as methyl bromide and other alkyl halides, or
M.8B chloropicrin, or M.8C sulfuryl fluoride, or M.8D borax, or M.8E tartar emetic;
M.9 Selective homopteran feeding blockers, for example
M.9B pymetrozine, or M.9C flonicamid;
M.10 Mite growth inhibitors, for example
M.10A clofentezine, hexythiazox and diflovidazin, or M.10B etoxazole;
M.11 Microbial disruptors of insect midgut membranes, for example *bacillus thuringiensis* or *bacillus sphaericus* and the insecticdal proteins they produce such as *bacillus thuringiensis subsp. israelensis, bacillus sphaericus, bacillus thuringiensis subsp. aizawai, bacillus thurin-giensis subsp. kurstaki* and *bacillus thuringiensis subsp. tenebrionis,* or the Bt crop proteins: Cry1Ab, Cry1Ac, Cry1Fa, Cry2Ab, mCry3A, Cry3Ab, Cry3Bb and Cry34/35Ab1;
M.12 Inhibitors of mitochondrial ATP synthase, for example
M.12A diafenthiuron, or
M.12B organotin miticides such as azocyclotin, cyhexatin or fenbutatin oxide, or M.12C pro-pargite, or M.12D tetradifon;
M.13 Uncouplers of oxidative phosphorylation via disruption of the proton gradient, for example chlorfenapyr, DNOC or sulfluramid;
M.14 Nicotinic acetylcholine receptor (nAChR) channel blockers, for example nereistoxin analogues as bensultap, cartap hydrochloride, thiocyclam or thiosultap sodium;
M.15 Inhibitors of the chitin biosynthesis type 0, such as benzoylureas as for example bistriflu-ron, chlorfluazuron, diflubenzuron, flucycloxuron, flufenoxuron, hexaflumuron, lufenuron, novalu-ron, noviflumuron, teflubenzuron or triflumuron;
M.16 Inhibitors of the chitin biosynthesis type 1, as for example buprofezin;
M.17 Moulting disruptors, Dipteran, as for example cyromazine;
M.18 Ecdyson receptor agonists such as diacylhydrazines, for example methoxyfenozide, tebufenozide, halofenozide, fufenozide or chromafenozide;
M.19 Octopamin receptor agonists, as for example amitraz;
M.20 Mitochondrial complex III electron transport inhibitors, for example
M.20A hydramethylnon, or M.20B acequinocyl, or M.20C fluacrypyrim;
M.21 Mitochondrial complex I electron transport inhibitors, for example
M.21A METI acaricides and insecticides such as fenazaquin, fenpyroximate, pyrimidifen, pyridaben, tebufenpyrad or tolfenpyrad, or M.21 B rotenone;
M.22 Voltage-dependent sodium channel blockers, for example
M.22A indoxacarb, or M.22B metaflumizone, or M.22B.1: 2-[2-(4-Cyanophenyl)-1-[3-(trifluoromethyl)phenyl]ethylidene]-N-[4-(difluoromethoxy)phenyl]-hydrazinecarboxamide or
M.22B.2: N-(3-Chloro-2-methylphenyl)-2-[(4-chlorophenyl)[4-[methyl(methylsulfonyl)amino]phenyl]methylene]-hydrazinecarboxamide;
M.23 Inhibitors of the of acetyl CoA carboxylase, such as Tetronic and Tetramic acid derivatives, for example spirodiclofen, spiromesifen or spirotetramat;
M.24 Mitochondrial complex IV electron transport inhibitors, for example M.24A phosphine such as aluminium phosphide, calcium phosphide, phosphine or zinc phosphide, or M.24B cyanide;
M.25 Mitochondrial complex II electron transport inhibitors, such as beta-ketonitrile derivatives, for example cyenopyrafen or cyflumetofen;
M.28 Ryanodine receptor-modulators from the class of diamides, as for example flubendiamide, chlorantraniliprole (rynaxypyr®), cyantraniliprole (cyazypyr®), or the phthalamide compounds
M.28.1: (R)-3-Chlor-N1-{2-methyl-4-[1,2,2,2- tetrafluor-1-(trifluormethyl)ethyl]phenyl}-N2-(1-methyl-2-methylsulfonylethyl)phthalamid and
M.28.2: (S)-3-Chlor-N1-{2-methyl-4-[1,2,2,2 - tetrafluor-1-(trifluormethyl)ethyl]phenyl}-N2-(1-methyl-2-methylsulfonylethyl)phthalamid, or the compound
M.28.3: 3-bromo-N-{2-bromo-4-chloro-6-[(1-cyclopropylethyl)carbamoyl]phenyl}-1-(3-chlorpyridin-2-yl)-1 H-pyrazole-5-carboxamide (proposed ISO name: cyclaniliprole), or the compound
M.28.4: methyl-2-[3,5-dibromo-2-({[3-bromo-1-(3-chlorpyridin-2-yl)-1H-pyrazol-5-yl]carbonyl}amino)benzoyl]-1,2-dimethylhydrazinecarboxylate; or a compound selected from M.28.5a) to M.28.5l):
M.28.5a) N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide;
M.28.5b) N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide;
M.28.5c) N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide;
M.28.5d) N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide;
M.28.5e) N-[4,6-dichloro-2-[(diethyl-lambda-4-sulfanylidene)carbamoyl]-phenyl]-2-(3-chloro-2-pyridyl)-5-(difluoromethyl)pyrazole-3-carboxamide;
M.28.5f) N-[4,6-dibromo-2-[(di-2-propyl-lambda-4-sulfanylidene)carbamoyl]-phenyl]-2-(3-chloro-2-pyridyl)-5-(trifluoromethyl)pyrazole-3-carboxamide;
M.28.5g) N-[4-chloro-2-[(di-2-propyl-lambda-4-sulfanylidene)carbamoyl]-6-cyano-phenyl]-2-(3-chloro-2-pyridyl)-5-(trifluoromethyl)pyrazole-3-carboxamide;
M.28.5h) N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide;
M.28.5i) N-[2-(5-Amino-1,3,4-thiadiazol-2-yl)-4-chloro-6-methylphenyl]-3-bromo-1-(3-chloro-2-pyridinyl)-1 H-pyrazole-5-carboxamide;
M.28.5j) 3-Chloro-1-(3-chloro-2-pyridinyl)-N-[2,4-dichloro-6-[[(1-cyano-1-methylethyl)amino]carbonyl]phenyl]-1 H-pyrazole-5-carboxamide;
M.28.5k) 3-Bromo-N-[2,4-dichloro-6-(methylcarbamoyl)phenyl]-1-(3,5-dichloro-2-pyridyl)-1H-pyrazole-5-carboxamide;
M.28.5l) N-[4-Chloro-2-[[(1,1-dimethylethyl)amino]carbonyl]-6-methylphenyl]-1-(3-chloro-2-pyridinyl)-3-(fluoromethoxy)-1 H-pyrazole-5-carboxamide;
   or a compound selected from
M.28.6: N-(2-cyanopropan-2-yl)-N-(2,4-dimethylphenyl)-3-iodobenzene-1,2-dicarboxamide; or
M.28.7: 3-Chloro-N-(2-cyanopropan-2-yl)-N-(2,4-dimethylphenyl)-benzene-1,2-dicarboxamide;
M.28.8a) 1-(3-Chloro-2-pyridinyl)-N-[4-cyano-2-methyl-6-[(methylamino)carbonyl]phenyl]-3-[[5-(trifluoromethyl)-2H-tetrazol-2-yl]methyl]-1 H-pyrazole-5-carboxamide; or
M.28.8b) 1-(3-Chloro-2-pyridinyl)-N-[4-cyano-2-methyl-6-[(methylamino)carbonyl]phenyl]-3-[[5-(trifluoromethyl)-1H-tetrazol-1-yl]methyl]-1H-pyrazole-5-carboxamide;
M.UN. insecticidal active compounds of unknown or uncertain mode of action, as for example afidopyropen, afoxolaner, azadirachtin, amidoflumet, benzoximate, bifenazate, bromopropylate, chinomethionat, cryolite, dicofol, flufenerim, flometoquin, fluensulfone, fluopyram, flupyradifurone, fluralaner, metoxadiazone, piperonyl butoxide, pyflubumide, pyridalyl, pyrifluquinazon, sulfoxaflor, tioxazafen, triflumezopyrim, or the compounds
M.UN.3: 11-(4-chloro-2,6-dimethylphenyl)-12-hydroxy-1,4-dioxa-9-azadispiro[4.2.4.2]-tetradec-11-en-10-one, or the compound
M.UN.4: 3-(4' -fluoro-2,4-dimethylbiphenyl-3-yl)-4-hydroxy-8-oxa-1-azaspiro[4.5]dec-3-en-2-one, or the compound
M.UN.5: 1-[2-fluoro-4-methyl-5-[(2,2,2-trifluoroethyl)sulfinyl]phenyl]-3-(trifluoromethyl)-1H-1,2,4-triazole-5-amine, or actives on basis of *bacillus firmus* (Votivo, I-1582); or a compound selected from the group of M.UN.6, wherein the compound is selected from M.UN.6a) to M.UN.6k):
   M.UN.6a) (E/Z)-N-[1-[(6-chloro-3-pyridyl)methyl]-2-pyridylidene]-2,2,2-trifluoro-acetamide;
   M.UN.6b) (E/Z)-N-[1-[(6-chloro-5-fluoro-3-pyridyl)methyl]-2-pyridylidene]-2,2,2-trifluoro-acetamide;
   M.UN.6c) (E/Z)-2,2,2-trifluoro-N-[1-[(6-fluoro-3-pyridyl)methyl]-2-pyridylidene]acetamide;
   M.UN.6d) (E/Z)-N-[1-[(6-bromo-3-pyridyl)methyl]-2-pyridylidene]-2,2,2-trifluoro-acetamide;
   M.UN.6e) (E/Z)-N-[1-[1-(6-chloro-3-pyridyl)ethyl]-2-pyridylidene]-2,2,2-trifluoro-acetamide;
   M.UN.6f) (E/Z)-N-[1-[(6-chloro-3-pyridyl)methyl]-2-pyridylidene]-2,2-difluoro-acetamide;
   M.UN.6g) (E/Z)-2-chloro-N-[1-[(6-chloro-3-pyridyl)methyl]-2-pyridylidene]-2,2-difluoro-acetamide;
   M.UN.6h) (E/Z)-N-[1-[(2-chloropyrimidin-5-yl)methyl]-2-pyridylidene]-2,2,2-trifluoro-acetamide;
   M.UN.6i) (E/Z)-N-[1-[(6-chloro-3-pyridyl)methyl]-2-pyridylidene]-2,2,3,3,3-pentafluoro-propanamide.);
   M.UN.6j) N-[1-[(6-chloro-3-pyridyl)methyl]-2-pyridylidene]-2,2,2-trifluoro-thioacetamide or of the compound
   M.UN.6k) N-[1-[(6-chloro-3-pyridyl)methyl]-2-pyridylidene]-2,2,2-trifluoro-N'-isopropyl-acetamidine
      or the compounds
   M.UN.8: 8-chloro-N-[2-chloro-5-methoxyphenyl)sulfonyl]-6-trifluoromethyl)-imidazo[1,2-a]pyridine-2-carboxamide; or
   M.UN.9: 4-[5-(3,5-dichlorophenyl)-5-(trifluoromethyl)-4H-isoxazol-3-yl]-2-methyl-N-(1-oxothietan-3-yl)benzamide; or
   M.UN.10: 5-[3-[2,6-dichloro-4-(3,3-dichloroallyloxy)phenoxy]propoxy]-1H-pyrazole; or a compound selected from the group of M.UN.11, wherein the compound is selected from M.UN.11a) to M.UN.11p):
      M.UN.11.a) 3-[benzoyl(methyl)amino]-N-[2-bromo-4-[1,2,2,2-tetrafluoro-1-(trifluoromethyl)ethyl]-6-(trifluoromethyl)phenyl]-2-fluoro-benzamide;
      M.UN.11.b) 3-(benzoylmethylamino)-N-[2-bromo-4-[1,2,2,3,3,3-hexafluoro-1-(trifluoromethyl)propyl]-6-(trifluoromethyl)phenyl]-2-fluoro-benzamide;
      M.UN.11.c) 3-(benzoylmethylamino)-2-fluoro-N-[2-iodo-4-[1,2,2,2-tetrafluoro-1-(trifluoromethyl)ethyl]-6-(trifluoromethyl)phenyl]-benzamide;
      M.UN.11.d) N-[3-[[[2-iodo-4-[1,2,2,2-tetrafluoro-1-(trifluoromethyl)ethyl]-6-(trifluoromethyl)phenyl]amino]carbonyl]phenyl]-N-methyl-benzamide;
      M.UN.11.e) N-[3-[[[2-bromo-4-[1,2,2,2-tetrafluoro-1-(trifluoromethyl)ethyl]-6-(trifluoromethyl)phenyl]amino]carbonyl]-2-fluorophenyl]-4-fluoro-N-methyl-benzamide;
      M.UN.11.f) 4-fluoro-N-[2-fluoro-3-[[[2-iodo-4-[1,2,2,2-tetrafluoro-1-(trifluoromethyl)ethyl]-6-(trifluoromethyl)phenyl]amino]carbonyl]phenyl]-N-methyl-benzamide;
      M.UN.11.g) 3-fluoro-N-[2-fluoro-3-[[[2-iodo-4-[1,2,2,2-tetrafluoro-1-(trifluoromethyl)ethyl]-6-(trifluoromethyl)phenyl]amino]carbonyl]phenyl]-N-methyl-benzamide;
      M.UN.11.h) 2-chloro-N-[3-[[[2-iodo-4-[1,2,2,2-tetrafluoro-1-(trifluoromethyl)ethyl]-6-(trifluoromethyl)phenyl]amino]carbonyl]phenyl]- 3-pyridinecarboxamide;
      M.UN.11.i) 4-cyano-N-[2-cyano-5-[[2,6-dibromo-4-[1,2,2,3,3,3-hexafluoro-1-(trifluoromethyl)propyl]phenyl]carbamoyl]phenyl]-2-methyl-benzamide;
      M.UN.11.j) 4-cyano-3-[(4-cyano-2-methyl-benzoyl)amino]-N-[2,6-dichloro-4-[1,2,2,3,3,3-hexafluoro-1-(trifluoromethyl)propyl]phenyl]-2-fluoro-benzamide;
      M.UN.11.k) N-[5-[[2-chloro-6-cyano-4-[1,2,2,3,3,3-hexafluoro-1-(trifluoromethyl)propyl]phenyl]carbamoyl]-2-cyano-phenyl]-4-cyano-2-methyl-benzamide;
      M.UN.11.l) N-[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide;
      M.UN.11.m) N-[5-[[2-bromo-6-chloro-4-[1,2,2,3,3,3-hexafluoro-1-(trifluoromethyl)propyl]phenyl]carbamoyl]-2-cyano-phenyl]-4-cyano-2-methyl-benzamide;
      M.UN.11.n) 4-cyano-N-[2-cyano-5-[[2,6-dichloro-4-[1,2,2,3,3,3-hexafluoro-1-(trifluoromethyl)propyl]phenyl]carbamoyl]phenyl]-2-methyl-benzamide;
      M.UN.11.o) 4-cyano-N-[2-cyano-5-[[2,6-dichloro-4-[1,2,2,2-tetrafluoro-1-(trifluoromethyl)ethyl]phenyl]carbamoyl]phenyl]-2-methyl-benzamide;
      M.UN.11.p) N-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide;
   or a compound selected from the group of M.UN.12, wherein the compound is selected from M.UN.12a) to M.UN.12m):
      M.UN.12.a) 2-(1,3-Dioxan-2-yl)-6-[2-(3-pyridinyl)-5-thiazolyl]-pyridine;
      M.UN.12.b) 2-[6-[2-(5-Fluoro-3-pyridinyl)-5-thiazolyl]-2-pyridinyl]-pyrimidine;
      M.UN.12.c) 2-[6-[2-(3-Pyridinyl)-5-thiazolyl]-2-pyridinyl]-pyrimidine;
      M.UN.12.d) N-Methylsulfonyl-6-[2-(3-pyridyl)thiazol-5-yl]pyridine-2-carboxamide
      M.UN.12.e) N-Methylsulfonyl-6-[2-(3-pyridyl)thiazol-5-yl]pyridine-2-carboxamide
      M.UN.12.f) N-Ethyl-N-[4-methyl-2-(3-pyridyl)thiazol-5-yl]-3-methylthio-propanamide
      M.UN.12.g) N-Methyl-N-[4-methyl-2-(3-pyridyl)thiazol-5-yl]-3-methylthio-propanamide
      M.UN.12.h) N,2-Dimethyl-N-[4-methyl-2-(3-pyridyl)thiazol-5-yl]-3-methylthio-propanamide
      M.UN.12.i) N-Ethyl-2-methyl-N-[4-methyl-2-(3-pyridyl)thiazol-5-yl]-3-methylthio-propanamide
      M.UN.12.j) N-[4-Chloro-2-(3-pyridyl)thiazol-5-yl]-N-ethyl-2-methyl-3-methylthio-propanamide
      M.UN.12.k) N-[4-Chloro-2-(3-pyridyl)thiazol-5-yl]-N,2-dimethyl-3-methylthio-propanamide
      M.UN.12.l) N-[4-Chloro-2-(3-pyridyl)thiazol-5-yl]-N-methyl-3-methylthio-propanamide
      M.UN.12.m) N-[4-Chloro-2-(3-pyridyl)thiazol-5-yl]-N-ethyl-3-methylthio-propanamide; or the compound
   M.UN.13: 2-(4-methoxyiminocyclohexyl)-2-(3,3,3-trifluoropropylsulfonyl)acetonitrile;
      or the compounds
   M.UN.14a) 1-[(6-Chloro-3-pyridinyl)methyl]-1,2,3,5,6,7-hexahydro-5-methoxy-7-methyl-8-nitro-imidazo[1,2-a]pyridine; or
   M.UN.14b) 1-[(6-Chloropyridin-3-yl)methyl]-7-methyl-8-nitro-1,2,3,5,6,7-hexahydroimidazo[1,2-a]pyridin-5-ol; or the compound
   M.UN.15: 1-[(2-Chloro-1,3-thiazol-5-yl)methyl]-3-(3,5-dichlorophenyl)-9-methyl-4-oxo-4H-pyrido[1,2-a]pyrimidin-1-ium-2-olate.

The commercially available compounds of the group M listed above may be found in The Pesticide Manual, 15th Edition, C. D. S. Tomlin, British Crop Protection Council (2011) among other publications.

The neonicotinoid cycloxaprid is known from WO20120/069266 and WO2011/06946, and the neonicotinoid compound M.4A.2, sometimes also to be named as Guadipyr, is known from WO2013/003977, and the neonicotinoid compound M.4A.3. (approved as paichongding in China) is known from WO2010/069266. The Metaflumizone analogue M.22B.1 is described in CN 10171577 and the analogue M.22B.2 in CN102126994. The phthalamides M.28.1 and M.28.2 are both known from WO 2007/101540. The anthranilamide M.28.3 has been described in WO2005/077934. The hydrazide compound M.28.4 has been described in WO 2007/043677. The anthranilamides M.28.5a) to M.28.5h) can be prepared as described in WO 2007/006670, WO2013/024009 and WO2013/024010, the anthranilamide compound M.28.5i) is described in WO2011/085575, the compound M.28.5j) in WO2008/134969, the compound M.28.5k) in US2011/046186 and the compound M.28.5l) in WO2012/034403. The diamide compounds M.28.6 and M.28.7 can be found in CN102613183. The anthranilamide compounds M.28.8a) and M.28.8b) are known from WO2010/069502.

The quinoline derivative flometoquin is shown in WO2006/013896. The aminofuranone compounds flupyradifurone is known from WO 2007/115644. The sulfoximine compound sulfoxaflor is known from WO2007/149134. From the pyrethroids group momfluorothrin is known from US6908945 and heptafluthrin from WO10133098. The oxadiazolone compound metoxadiazone can be found in JP13/166707. The pyrazole acaricide pyflubumide is known from WO2007/020986. The isoxazoline compounds have been described in following publications: fluralaner in WO2005/085216, afoxolaner in WO2009/002809 and in WO2011/149749 and the isoxazoline compound M.UN.9 in WO2013/050317. The pyripyropene derivative afidopyropen has been described in WO 2006/129714. The nematicide tioxazafen has been disclosed in WO09023721 and nematicide fluopyram in WO2008126922, nematicidal mixtures comprising flupyram in WO2010108616. The triflumezopyrim compound was described in WO2012/092115.

The spiroketal-substituted cyclic ketoenol derivative M.UN.3 is known from WO2006/089633 and the biphenyl-substituted spirocyclic ketoenol derivative M.UN.4 from WO2008/067911. The triazoylphenylsulfide M.UN.5 has been described in WO2006/043635, and biological control agents on basis of *bacillus firmus* in WO2009/124707.

The compounds M.UN.6a) to M.UN.6i) listed under M.UN.6 have been described in WO2012/029672 and compounds M.UN.6j) and M.UN.6k) in WO2013129688. The nematicide compound M.UN.8 in WO2013/055584 and the Pyridalyl-type analogue M.UN.10 in WO2010/060379. The carboxamide compounds M.UN.11.a) to M.UN.11.h) can be prepared as described in WO 2010/018714 and the carboxamide M.UN.11i) to M.UN.11.p) are described WO2010/127926. The pyridylthiazoles M.UN.12.a) to M.UN.12.c) are known from WO2010/006713, M.UN.12.c) and M.UN.12.d) WO2012000896 and M.UN.12.f) to M.UN.12.m) in WO2010129497. The malononitrile compound M.UN.13 was described in WO2009/005110. The compounds M.UN.14a) and M.UN.14b) are known from WO2007/101369. The compound M.UN.15 can be found in WO13192035.

In another embodiment of the invention, the compounds of formula (I), or their stereoisomers, salts, tautomers and N-oxides, may also be applied with fungicides as compound II.

The following list F of active substances, in conjunction with which the compounds according to the invention can be used, is intended to illustrate the possible combinations but does not limit them:
F.I) Respiration Inhibitors
   F.I-1) Inhibitors of complex III at Qo site: strobilurins: azoxystrobin, coumethoxystrobin, coumoxystrobin, dimoxystrobin, enestroburin, fluoxastrobin, kresoxim-methyl, metominostrobin, orysastrobin, picoxystrobin, pyraclostrobin, pyrametostrobin, pyraoxystrobin, pyribencarb, triclopyricarb/chlorodincarb, trifloxystrobin, 2-[2-(2,5-dimethyl-phenoxymethyl)-phenyl]-3-methoxy-acrylic acid methyl ester and 2 (2-(3-(2,6-dichlorophenyl)-1-methyl-allylideneaminooxymethyl)-phenyl)-2-methoxyimino-N methylacetamide;
      oxazolidinediones and imidazolinones: famoxadone, fenamidone;
   F.I-2) Inhibitors of complex II (e.g. carboxamides):
      carboxanilides: benodanil, benzovindiflupyr, bixafen, boscalid, carboxin, fenfuram, fenhexamid, fluopyram, flutolanil, furametpyr, isopyrazam, isotianil, mepronil, oxycarboxin, penflufen, penthi-opyrad, sedaxane, tecloftalam, thifluzamide, tiadinil, 2-amino-4 methyl-thiazole-5-carboxanilide, N-(3',4',5' trifluorobiphenyl-2 yl)-3-difluoromethyl-1-methyl-1 H-pyrazole-4 carboxamide (fluxapy-roxad), N-(4'-trifluoromethylthiobiphenyl-2-yl)-3 difluoromethyl-1-methyl-1 H pyrazole-4-carboxamide, N-(2-(1,3,3-trimethyl-butyl)-phenyl)-1,3-dimethyl-5 fluoro-1 H-pyrazole-4 carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 3-(difluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 3-(difluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide;
   F.I-3) Inhibitors of complex III at Qi site: cyazofamid, amisulbrom, [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate, [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate, 3*S*,6*S*,7*R*,8*R*)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate;
   F.I-4) Other respiration inhibitors (complex I, uncouplers) diflumetorim; (5,8-difluoroquinazolin-4-yl)-{2-[2-fluoro-4-(4-trifluoromethylpyridin-2-yloxy)-phenyl]-ethyl}-amine; tecnazen;ametoctradin; silthiofam; nitrophenyl derivates: binapacryl, dinobuton, dinocap, fluazinam, ferimzone, nitrthal-isopropyl,
   and including organometal compounds: fentin salts, such as fentin-acetate, fentin chloride or fentin hydroxide;
F.II) Sterol biosynthesis inhibitors (SBI fungicides)
   F.II-1) C14 demethylase inhibitors (DMI fungicides, e.g. triazoles, imidazoles) triazoles: azaconazole, bitertanol, bromuconazole, cyproconazole, difenoconazole, diniconazole, diniconazole-M, epoxiconazole, fenbuconazole, fluquinconazole, flusilazole, flutriafol, hex-aconazole, imibenconazole, ipconazole, metconazole, myclobutanil, paclobutrazole, pen-conazole, propiconazole, prothioconazole, simeconazole, tebuconazole, tetraconazole, triad-imefon, triadimenol, triticonazole, uniconazole, 1-[*rel*-(2*S*;3*R*)-3-(2-chlorophenyl)-2-(2,4-difluorophenyl)-oxiranylmethyl]-5-thiocyanato-1H-[1,2,4]triazole, 2-[*rel*-(2*S*;3*R*)-3-(2-chlorophenyl)-2-(2,4-difluorophenyl)-oxiranylmethyl]-2H-[1,2,4]triazole-3-thiol; imidazoles: imazalil, pefurazoate, oxpoconazole, prochloraz, triflumizole; pyrimidines, pyridines and piperazines: fenarimol, nuarimol, pyrifenox, triforine, 1-[rel-(2S;3R)-3-(2-chlorophenyl)-2-(2,4-difluorophenyl)-oxiranylmethyl]-5-thiocyanato-1 H-[1,2,4]triazole, 2-[rel-(2S;3R)-3-(2-chlorophenyl)-2-(2,4-difluorophenyl)-oxiranylmethyl]-2H-[1,2,4]triazole-3-thiol;
   F.II-2) Delta14-reductase inhitors (Amines, e.g. morpholines, piperidines) morpholines: aldimorph, dodemorph, dodemorph-acetate, fenpropimorph, tridemorph; piperidines: fenpropidin, piperalin; spiroketalamines: spiroxamine;
   F.II-3) Inhibitors of 3-keto reductase: hydroxyanilides: fenhexamid;
F.III) Nucleic acid synthesis inhibitors
   F.III-1) RNA, DNA synthesis
      phenylamides or acyl amino acid fungicides: benalaxyl, benalaxyl-M, kiralaxyl, metalaxyl, metalaxyl-M (mefenoxam), ofurace, oxadixyl;
      isoxazoles and iosothiazolones: hymexazole, octhilinone;
   F.III-2) DNA topisomerase inhibitors: oxolinic acid;
   F.III-3) Nucleotide metabolism (e.g. adenosin-deaminase), hydroxy (2-amino)-pyrimidines: bupi-rimate;
F.IV) Inhibitors of cell division and or cytoskeleton
   F.IV-1) Tubulin inhibitors: benzimidazoles and thiophanates: benomyl, carbendazim, fuber-idazole, thiabendazole, thiophanate-methyl;
      triazolopyrimidines: 5-chloro-7 (4-methylpiperidin-1-yl)-6-(2,4,6-trifluorophenyl)-[1,2,4]triazolo[1,5 a]pyrimidine;
   F.IV-2) Other cell division inhibitors
      benzamides and phenyl acetamides: diethofencarb, ethaboxam, pencycuron, fluopicolide, zox-amide;
   F.IV-3) Actin inhibitors: benzophenones: metrafenone; pyriofenone;
F.V) Inhibitors of amino acid and protein synthesis
   F.V-1) Methionine synthesis inhibitors (anilino-pyrimidines)
      anilino-pyrimidines: cyprodinil, mepanipyrim, nitrapyrin, pyrimethanil;
   F.V-2) Protein synthesis inhibitors (anilino-pyrimidines)
      antibiotics: blasticidin-S, kasugamycin, kasugamycin hydrochloride-hydrate, mildiomycin, streptomycin, oxytetracyclin, polyoxine, validamycin A;
F.VI) Signal transduction inhibitors
   F.VI-1) MAP / Histidine kinase inhibitors (e.g. anilino-pyrimidines)
      dicarboximides: fluoroimid, iprodione, procymidone, vinclozolin; phenylpyrroles: fenpiclonil, fludioxonil;
   F.VI-2) G protein inhibitors: quinolines: quinoxyfen;
F.VII) Lipid and membrane synthesis inhibitors
   F.VII-1) Phospholipid biosynthesis inhibitors
      organophosphorus compounds: edifenphos, iprobenfos, pyrazophos; dithiolanes: isoprothiolane;
   F.VII-2) Lipid peroxidation: aromatic hydrocarbons: dicloran, quintozene, tecnazene, tolclofos-methyl, biphenyl, chloroneb, etridiazole;
   F.VII-3) Carboxyl acid amides (CAA fungicides)
      cinnamic or mandelic acid amides: dimethomorph, flumorph, mandiproamid, pyrimorph; valinamide carbamates: benthiavalicarb, iprovalicarb, pyribencarb, valifenalate and N-(1-(1-(4-cyano-phenyl)ethanesulfonyl)-but-2-yl) carbamic acid-(4-fluorophenyl) ester;
   F.VII-4) Compounds affecting cell membrane permeability and fatty acids: 1-[4-[4-[5-(2,6-difluorophenyl)-4,5-dihydro-3-isoxazolyl]-2-thiazolyl]-1-piperidinyl]-2-[5-methyl-3-(trifluoromethyl)-1H-pyrazol-1-yl]ethanone, carbamates: propamocarb, propamocarb-hydrochlorid,
   F.VII-5) fatty acid amide hydrolase inhibitors: 1-[4-[4-[5-(2,6-difluorophenyl)-4,5-dihydro-3-isoxazolyl]-2-thiazolyl]-1-piperidinyl]-2-[5-methyl-3-(trifluoromethyl)-1 H-pyrazol-1-yl]ethanone;
F.VIII) Inhibitors with Multi Site Action
   F.VIII-1) Inorganic active substances: Bordeaux mixture, copper acetate, copper hydroxide, copper oxychloride, basic copper sulfate, sulfur;
   F.VIII-2) Thio- and dithiocarbamates: ferbam, mancozeb, maneb, metam, methasulphocarb, metiram, propineb, thiram, zineb, ziram;
   F.VIII-3) Organochlorine compounds (e.g. phthalimides, sulfamides, chloronitriles):
      anilazine, chlorothalonil, captafol, captan, folpet, dichlofluanid, dichlorophen, flusulfamide, hexachlorobenzene, pentachlorphenole and its salts, phthalide, tolylfluanid, N-(4-chloro-2-nitro-phenyl)-N-ethyl-4-methyl-benzenesulfonamide;
      F.VIII-4) Guanidines and other: guanidine, dodine, dodine free base, guazatine, guazatine-acetate, iminoctadine, iminoctadine-triacetate, iminoctadine-tris(albesilate), 2,6-dimethyl-1 H,5H-[1,4]dithiino[2,3-c:5,6-c']dipyrrole-1,3,5,7(2H,6H)-tetraone;
      F.VIII-5) Ahtraquinones: dithianon;
F.IX) Cell wall synthesis inhibitors
   F.IX-1) Inhibitors of glucan synthesis: validamycin, polyoxin B;
   F.IX-2) Melanin synthesis inhibitors: pyroquilon, tricyclazole, carpropamide, dicyclomet, fenoxanil;
F.X) Plant defence inducers
   F.X-1) Salicylic acid pathway: acibenzolar-*S*-methyl;
   F.X-2) Others: probenazole, isotianil, tiadinil, prohexadione-calcium;
      phosphonates: fosetyl, fosetyl-aluminum, phosphorous acid and its salts;
F.XI) Unknown mode of action:bronopol, chinomethionat, cyflufenamid, cymoxanil, dazomet, debacarb, diclomezine, difenzoquat, difenzoquat-methylsulfate, diphenylamin, fenpyrazamine, flumetover, flusulfamide, flutianil, methasulfocarb, nitrapyrin, nitrothal-isopropyl, oxathiapiprolin, oxin-copper, proquinazid, tebufloquin, tecloftalam, triazoxide, 2-butoxy-6-iodo-3-propylchromen-4-one, N-(cyclopropylmethoxyimino-(6-difluoro-methoxy-2,3-difluoro-phenyl)-methyl)-2-phenyl acetamide, N'-(4-(4-chloro-3-trifluoromethyl-phenoxy)-2,5-dimethyl-phenyl)-N-ethyl-N methyl formamidine, N' (4-(4-fluoro-3-trifluoromethyl-phenoxy)-2,5-dimethyl-phenyl)-N-ethyl-N-methyl formamidine, N'-(2-methyl-5-trifluoromethyl-4-(3-trimethylsilanyl-propoxy)-phenyl)-N-ethyl-N-methyl formamidine, N'-(5-difluoromethyl-2 methyl-4-(3-trimethylsilanyl-propoxy)-phenyl)-N-ethyl-N-methyl formamidine, 2-{1-[2-(5-methyl-3-trifluoromethyl-pyrazole-1-yl)-acetyl]-piperidin-4-yl}-thiazole-4-carboxylic acid methyl-(1,2,3,4-tetrahydro-naphthalen-1-yl)-amide, 2-{1-[2-(5-methyl-3-trifluoromethyl-pyrazole-1-yl)-acetyl]-piperidin-4-yl}-thiazole-4-carboxylic acid methyl-(R)-1,2,3,4-tetrahydro-naphthalen-1-yl-amide, methoxy-acetic acid 6-tert-butyl-8-fluoro-2,3-dimethyl-quinolin-4-yl ester and N-Methyl-2-{1-[(5-methyl-3-trifluoromethyl-1H-pyrazol-1-yl)-acetyl]-piperidin-4-yl}-N-[(1*R*)-1,2,3,4-tetrahydronaphthalen-1-yl]-4-thiazolecarboxamide, 3-[5-(4-chloro-phenyl)-2,3-dimethyl-isoxazolidin-3 yl]-pyridine, pyrisoxazole, 5-amino-2-isopropyl-3-oxo-4-ortho-tolyl-2,3-dihydro-pyrazole-1 carbothioic acid S-allyl ester, N-(6-methoxy-pyridin-3-yl) cyclopropanecarboxylic acid amide, 5-chloro-1 (4,6-dimethoxy-pyrimidin-2-yl)-2-methyl-1 H-benzoimidazole, 2-(4-chloro-phenyl)-N-[4-(3,4-dimethoxy-phenyl)-isoxazol-5-yl]-2-prop-2-ynyloxy-acetamide;
F.XI) Growth regulators: abscisic acid, amidochlor, ancymidol, 6-benzylaminopurine, brassino-lide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid, maleic hydrazide, mefluidide, mepiquat (mepiquat chloride), naphthaleneacetic acid, N 6-benzyladenine, paclobutrazol, prohexadione (prohexadione-calcium), prohydrojasmon, thidiazuron, triapenthenol, tributyl phos-phorotrithioate, 2,3,5 tri iodobenzoic acid, trinexapac-ethyl and uniconazole;
F.XII) Biological control agents
   *Ampelomyces quisqualis* (e.g. AQ 10^{®} from Intrachem Bio GmbH & Co. KG, Germany), *Asper-gillus flavus* (e.g. AFLAGUARD^{®} from Syngenta, CH), *Aureobasidium pullulans* (e.g. BOTEC-TOR^{®} from bio-ferm GmbH, Germany), *Bacillus pumilus* (e.g. NRRL Accession No. B-30087 in SONATA^{®} and BALLAD^{®} Plus from AgraQuest Inc., USA), *Bacillus subtilis* (e.g. isolate NRRL-Nr. B-21661 in RHAPSODY^{®}, SERENADE^{®} MAX and SERENADE^{®} ASO from AgraQuest Inc., USA), *Bacillus subtilis* var. *amyloliquefaciens* FZB24 (e.g. TAEGRO^{®} from Novozyme Biologi-cals, Inc., USA), *Candida oleophila* I-82 (e.g. ASPIRE^{®} from Ecogen Inc., USA), *Candida sai-toana* (e.g. BIOCURE^{®} (in mixture with lysozyme) and BIOCOAT^{®} from Micro Flo Company, USA (BASF SE) and Arysta), Chitosan (e.g. ARMOUR-ZEN from BotriZen Ltd., NZ), *Clonosta-chys rosea f. catenulata,* also named *Gliocladium catenulatum* (e.g. isolate J1446: PRESTOP^{®} from Verdera, Finland), *Coniothyrium minitans* (e.g. CONTANS^{®} from Prophyta, Germany), *Cryphonectria parasitica* (e.g. *Endothia parasitica* from CNICM, France), *Cryptococcus albidus* (e.g. YIELD PLUS^{®} from Anchor Bio-Technologies, South Africa), *Fusarium oxysporum* (e.g. BIOFOX^{®} from S.I.A.P.A., Italy, FUSACLEAN^{®} from Natural Plant Protection, France), *Metschnikowia fructicola* (e.g. SHEMER^{®} from Agrogreen, Israel), *Microdochium dimerum* (e.g. ANTIBOT^{®} from Agrauxine, France), *Phlebiopsis gigantea* (e.g. ROTSOP^{®} from Verdera, Finland), *Pseudozyma flocculosa* (e.g. SPORODEX^{®} from Plant Products Co. Ltd., Canada), *Pythium oligandrum* DV74 (e.g. POLYVERSUM^{®} from Remeslo SSRO, Biopreparaty, Czech Rep.), *Reynoutria sachlinensis* (e.g. REGALIA^{®} from Marrone Biolnnovations, USA), *Talaromy-ces flavus* V117b (e.g. PROTUS^{®} from Prophyta, Germany), *Trichoderma asperellum* SKT-1 (e.g. ECO-HOPE^{®} from Kumiai Chemical Industry Co., Ltd., Japan), *T. atroviride* LC52 (e.g. SENTINEL^{®} from Agrimm Technologies Ltd, NZ), *T. harzianum* T-22 (e.g. PLANTSHIELD^{®} der Firma BioWorks Inc., USA), *T. harzianum* TH 35 (e.g. ROOT PRO^{®} from Mycontrol Ltd., Israel), *T. harzianum* T-39 (e.g. TRICHODEX^{®} and TRICHODERMA 2000^{®} from Mycontrol Ltd., Israel and Makhteshim Ltd., Israel), *T. harzianum* and *T. viride* (e.g. TRICHOPEL from Agrimm Technologies Ltd, NZ), *T. harzianum* ICC012 and *T. viride* ICC080 (e.g. REMEDIER^{®} WP from Isa-gro Ricerca, Italy), *T. polysporum* and *T. harzianum* (e.g. BINAB^{®} from BINAB Bio-Innovation AB, Sweden), *T. stromaticum* (e.g. TRICOVAB^{®} from C.E.P.L.A.C., Brazil), *T. virens* GL-21 (e.g. SOILGARD^{®} from Certis LLC, USA), *T. viride* (e.g. TRIECO^{®} from Ecosense Labs. (India) Pvt. Ltd., Indien, BIO-CURE^{®} F from T. Stanes & Co. Ltd., Indien), *T. viride* TV1 (e.g. T. viride TV1 from Agribiotec srl, Italy), *Ulocladium oudemansii* HRU3 (e.g. BOTRY-ZEN^{®} from Botry-Zen Ltd, NZ).

The commercially available compounds II of the group F listed above may be found in The Pesticide Manual, 15th Edition, C. D. S. Tomlin, British Crop Protection Council (2011) among other publications. Their preparation and their activity against harmful fungi is known (cf.: http://www.alanwood.net/pesticides/); these substances are commercially available. The compounds described by IUPAC nomenclature, their preparation and their fungicidal activity are also known (cf. Can. J. Plant Sci. 48(6), 587-94, 1968; EP A 141 317; EP-A 152 031; EP-A226 917; EP A 243 970; EP A 256 503; EP-A 428 941; EP-A 532 022; EP-A 1 028 125; EP-A 1 035 122; EP A 1 201 648; EP A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657).

### Applications

The animal pest, i.e. the insects, arachnids and nematodes, the plant, soil or water in which the plant is growing can be contacted with the present compounds of formula I or composition(s) containing them by any application method known in the art. As such, "contacting" includes both direct contact (applying the compounds/compositions directly on the animal pest or plant - typically to the foliage, stem or roots of the plant) and indirect contact (applying the compounds/compositions to the locus of the animal pest or plant).

The compounds of formula I or the pesticidal compositions comprising them may be used to protect growing plants and crops from attack or infestation by animal pests, especially insects, acaridae or arachnids by contacting the plant/crop with a pesticidally effective amount of compounds of formula I. The term "crop" refers both to growing and harvested crops.

The compounds of the present invention and the compositions comprising them are particularly important in the control of a multitude of insects on various cultivated plants, such as cereal, root crops, oil crops, vegetables, spices, ornamentals, for example seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants such as potatoes, sugar cane, tobacco, grapes, petunias, geranium/pelargoniums, pansies and impatiens.

The compounds of the present invention are employed as such or in form of compositions by treating the insects or the plants, plant propagation materials, such as seeds, soil, surfaces, materials or rooms to be protected from insecticidal attack with a insecticidally effective amount of the active compounds. The application can be carried out both before and after the infection of the plants, plant propagation materials, such as seeds, soil, surfaces, materials or rooms by the insects.

The present invention also includes a method of combating animal pests which comprises contacting the animal pests, their habit, breeding ground, food supply, cultivated plants, seed, soil, area, material or environment in which the animal pests are growing or may grow, or the materials, plants, seeds, soils, surfaces or spaces to be protected from animal attack or infestation with a pesticidally effective amount of a mixture of at least one active compound I.

Moreover, animal pests may be controlled by contacting the target pest, its food supply, habitat, breeding ground or its locus with a pesticidally effective amount of compounds of formula I. As such, the application may be carried out before or after the infection of the locus, growing crops, or harvested crops by the pest.

The compounds of the invention can also be applied preventively to places at which occurrence of the pests is expected.

The compounds of formula I may be also used to protect growing plants from attack or infestation by pests by contacting the plant with a pesticidally effective amount of compounds of formula I. As such, "contacting" includes both direct contact (applying the compounds/compositions directly on the pest and/or plant - typically to the foliage, stem or roots of the plant) and indirect contact (applying the compounds/compositions to the locus of the pest and/or plant).

"Locus" means a habitat, breeding ground, plant, seed, soil, area, material or environment in which a pest or parasite is growing or may grow.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants. Seedlings and young plants, which are to be transplanted after germination or after emergence from soil, may also be included. These plant propagation materials may be treated prophylactically with a plant protection compound either at or before planting or transplanting.

The term "cultivated plants" is to be understood as including plants which have been modified by breeding, mutagenesis or genetic engineering. Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-transtional modification of protein(s) (oligo- or polypeptides) poly for example by glycosylation or polymer additions such as prenylat-ed, acetylated or farnesylated moieties or PEG moieties(e.g. as disclosed in Biotechnol Prog. 2001 Jul-Aug;17(4):720-8., Protein Eng Des Sel. 2004 Jan;17(1):57-66, Nat Protoc. 2007;2(5):1225-35., Curr Opin Chem Biol. 2006 Oct;10(5):487-91. Epub 2006 Aug 28., Bio-materials. 2001 Mar;22(5):405-17, Bioconjug Chem. 2005 Jan-Feb;16(1):113-21).

The term "cultivated plants" is to be understood also including plants that have been rendered tolerant to applications of specific classes of herbicides, such as hydroxy-phenylpyruvate dioxy-genase (HPPD) inhibitors; acetolactate synthase (ALS) inhibitors, such as sulfonyl ureas (see e. g. US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073) or imidazolinones (see e. g. US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073); enolpyruvylshikimate-3-phosphate synthase (EPSPS) inhibitors, such as glyphosate (see e. g. WO 92/00377); glutamine synthetase (GS) inhibitors, such as glufosinate (see e. g. EP-A-0242236, EP-A-242246) or oxynil herbicides (see e. g. US 5,559,024) as a result of conventional methods of breeding or genetic engineering. Several cultivated plants have been rendered tolerant to herbicides by conventional methods of breeding (mutagenesis), for example Clearfield® summer rape (Canola) being tolerant to imidazolinones, e. g. imazamox. Genetic engineering methods have been used to render cultivated plants, such as soybean, cotton, corn, beets and rape, tolerant to herbicides, such as glyphosate and glufosinate, some of which are commercially available under the trade names RoundupReady® (glyphosate) and LibertyLink® (glufosinate).

The term "cultivated plants" is to be understood also including plants that are by the use of recombinant DNA techniques capable to synthesize one or more insecticidal proteins, especially those known from the bacterial genus Bacillus, particularly from Bacillus thuringiensis, such as delta-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, for example Photorhabdus spp. or Xenorhabdus spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, for example WO 02/015701). Further examples of such toxins or genetically-modified plants capable of synthesizing such toxins are dis-closed, for example, in EP-A 374 753, WO 93/007278, WO 95/34656, EP-A 427 529, EP-A 451 878, WO 03/018810 und WO 03/052073. The methods for producing such genetically modified plants are generally known to the person skilled in the art and are described, for example, in the publications mentioned above. These insecticidal proteins contained in the genetically modified plants impart to the plants producing these proteins protection from harmful pests from certain taxonomic groups of arthropods, particularly to beetles (Coleoptera), flies (Diptera), and butterflies and moths (Lepidoptera) and to plant parasitic nematodes (Nematoda).

The term "cultivated plants" is to be understood also including plants that are by the use of recombinant DNA techniques capable to synthesize one or more proteins to increase the resistance or tolerance of those plants to bacterial, viral or fungal pathogens. Examples of such proteins are the so-called " pathogenesis-related proteins" (PR proteins, see, for example EP-A 0 392 225), plant disease resistance genes (for example potato cultivars, which express resistance genes acting against Phytophthora infestans derived from the mexican wild potato Solanum bulbocastanum) or T4-lyso-zym (e. g. potato cultivars capable of synthesizing these proteins with increased resistance against bacteria such as Erwinia amylvora). The methods for producing such genetically modified plants are generally known to the person skilled in the art and are described, for example, in the publications mentioned above.

The term "cultivated plants" is to be understood also including plants that are by the use of recombinant DNA techniques capable to synthesize one or more proteins to increase the productivity (e. g. bio mass production, grain yield, starch content, oil content or protein content), tolerance to drought, salinity or other growth-limiting environ-mental factors or tolerance to pests and fungal, bacterial or viral pathogens of those plants.

The term "cultivated plants" is to be understood also including plants that contain by the use of recombinant DNA techniques a modified amount of substances of content or new substances of content, specifically to improve human or animal nutrition, for ex-ample oil crops that produce health-promoting long-chain omega-3 fatty acids or unsaturated omega-9 fatty acids (e. g. Nexera® rape).

The term "cultivated plants" is to be understood also including plants that contain by the use of recombinant DNA techniques a modified amount of substances of content or new substances of content, specifically to improve raw material production, for example potatoes that produce increased amounts of amylopectin (e. g. Amflora® potato).

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

In the case of soil treatment or of application to the pests dwelling place or nest, the quantity of active ingredient ranges from 0.0001 to 500 g per 100 m², preferably from 0.001 to 20 g per 100 m².

Customary application rates in the protection of materials are, for example, from 0.01 g to 1000 g of active compound per m² treated material, desirably from 0.1 g to 50 g per m².

Insecticidal compositions for use in the impregnation of materials typically contain from 0.001 to 95 weight %, preferably from 0.1 to 45 weight %, and more preferably from 1 to 25 weight % of at least one repellent and/or insecticide.

For use in treating crop plants, the rate of application of the active ingredients of this invention may be in the range of 0.1 g to 4000 g per hectare, desirably from 25 g to 600 g per hectare, more desirably from 50 g to 500 g per hectare.

The compounds of formula I are effective through both contact (via soil, glass, wall, bed net, carpet, plant parts or animal parts), and ingestion (bait, or plant part).

The compounds of the invention may also be applied against non-crop insect pests, such as ants, termites, wasps, flies, mosquitos, crickets, or cockroaches. For use against said non-crop pests, compounds of formula I are preferably used in a bait composition.

The bait can be a liquid, a solid or a semisolid preparation (e.g. a gel). Solid baits can be formed into various shapes and forms suitable to the respective application e.g. granules, blocks, sticks, disks. Liquid baits can be filled into various devices to ensure proper application, e.g. open containers, spray devices, droplet sources, or evaporation sources. Gels can be based on aqueous or oily matrices and can be formulated to particular necessities in terms of stickyness, moisture retention or aging characteristics.

The bait employed in the composition is a product, which is sufficiently attractive to incite insects such as ants, termites, wasps, flies, mosquitos, crickets etc. or cockroaches to eat it. The attractiveness can be manipulated by using feeding stimulants or sex pheromones. Food stimulants are chosen, for example, but not exclusively, from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are described in the literature and are known to those skilled in the art.

For use in bait compositions, the typical content of active ingredient is from 0.001 weight % to 15 weight %, desirably from 0.001 weight % to 5% weight % of active compound.

Formulations of compounds of formula I as aerosols (e.g in spray cans), oil sprays or pump sprays are highly suitable for the non-professional user for controlling pests such as flies, fleas, ticks, mosquitos or cockroaches. Aerosol recipes are preferably composed of the active compound, solvents such as lower alcohols (e.g. methanol, ethanol, propanol, butanol), ketones (e.g. acetone, methyl ethyl ketone), paraffin hydrocarbons (e.g. kerosenes) having boiling ranges of approximately 50 to 250 °C, dimethylformamide, N-methylpyrrolidone, dimethyl sulfoxide, aromatic hydrocarbons such as toluene, xylene, water, furthermore auxiliaries such as emulsifiers such as sorbitol monooleate, oleyl ethoxylate having 3-7 mol of ethylene oxide, fatty alcohol ethoxylate, perfume oils such as ethereal oils, esters of medium fatty acids with lower alcohols, aromatic carbonyl compounds, if appropriate stabilizers such as sodium benzoate, amphoteric surfactants, lower epoxides, triethyl orthoformate and, if required, propellants such as propane, butane, nitrogen, compressed air, dimethyl ether, carbon dioxide, nitrous oxide, or mixtures of these gases.

The oil spray formulations differ from the aerosol recipes in that no propellants are used.

For use in spray compositions, the content of active ingredient is from 0.001 to 80 weights %, preferably from 0.01 to 50 weight % and most preferably from 0.01 to 15 weight %.

The compounds of formula I and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of formula I and its respective compositions also comprise treating surfaces of huts and houses, air spraying and impregnation of curtains, tents, clothing items, bed nets, tsetse-fly trap or the like. Insecticidal compositions for application to fibers, fabric, knitgoods, nonwovens, netting material or foils and tarpaulins preferably comprise a mixture including the insecticide, optionally a repellent and at least one binder. Suitable repellents for example are N,N-Diethyl-meta-toluamide (DEET), N,N-diethylphenylacetamide (DEPA), 1-(3-cyclohexan-1-yl-carbonyl)-2-methylpiperine, (2-hydroxymethylcyclohexyl) acetic acid lactone, 2-ethyl-1,3-hexandiol, indalone, Methyl-neodecanamide (MNDA), a pyrethroid not used for insect control such as {(+/-)-3-allyl-2-methyl-4-oxocyclopent-2-(+)-enyl-(+)-trans-chrysantemate (Esbiothrin), a repellent derived from or identical with plant extracts like limonene, eugenol, (+)-Eucamalol (1), (-)-1-epi-eucamalol or crude plant extracts from plants like Eucalyptus maculata, Vitex rotundifolia, Cymbopogan martinii, Cymbopogan citratus (lemon grass), Cymopogan nartdus (citronella). Suitable binders are selected for example from polymers and copolymers of vinyl esters of aliphatic acids (such as such as vinyl acetate and vinyl versatate), acrylic and methacrylic esters of alcohols, such as butyl acrylate, 2-ethylhexylacrylate, and methyl acrylate, mono- and di-ethylenically unsaturated hydrocarbons, such as styrene, and aliphatic diens, such as butadiene.

The impregnation of curtains and bednets is done in general by dipping the textile material into emulsions or dispersions of the insecticide or spraying them onto the nets.

The compounds of formula I and its compositions can be used for protecting wooden materials such as trees, board fences, sleepers, etc. and buildings such as houses, outhouses, factories, but also construction materials, furniture, leathers, fibers, vinyl articles, electric wires and cables etc. from ants and/or termites, and for controlling ants and termites from doing harm to crops or human being (e.g. when the pests invade into houses and public facilities). The compounds of formula I are applied not only to the surrounding soil surface or into the under-floor soil in order to protect wooden materials but it can also be applied to lumbered articles such as surfaces of the under-floor concrete, alcove posts, beams, plywoods, furniture, etc., wooden articles such as particle boards, half boards, etc. and vinyl articles such as coated electric wires, vinyl sheets, heat insulating material such as styrene foams, etc. In case of application against ants doing harm to crops or human beings, the ant controller of the present invention is applied to the crops or the surrounding soil, or is directly applied to the nest of ants or the like.

### Seed treatment

The compounds of formula I are also suitable for the treatment of seeds in order to protect the seed from insect pest, in particular from soil-living insect pests and the resulting plant' s roots and shoots against soil pests and foliar insects.

The compounds of formula I are particularly useful for the protection of the seed from soil pests and the resulting plant's roots and shoots against soil pests and foliar insects. The protection of the resulting plant's roots and shoots is preferred. More preferred is the protection of resulting plant's shoots from piercing and sucking insects, wherein the protection from aphids is most preferred.

The present invention therefore comprises a method for the protection of seeds from insects, in particular from soil insects and of the seedling's roots and shoots from insects, in particular from soil and foliar insects, said method comprising contacting the seeds before sowing and/or after pregermination with a compound of the general formula I or a salt thereof. Particularly preferred is a method, wherein the plant's roots and shoots are protected, more preferably a method, wherein the plants shoots are protected form piercing and sucking insects, most preferably aa method, wherein the plants shoots are protected from aphids.

The term seed embraces seeds and plant propagules of all kinds including but not limited to true seeds, seed pieces, suckers, corms, bulbs, fruit, tubers, grains, cuttings, cut shoots and the like and means in a preferred embodiment true seeds.

The term seed treatment comprises all suitable seed treatment techniques known in the art, such as seed dressing, seed coating, seed dusting, seed soaking and seed pelleting.
The present invention also comprises seeds coated with or containing the active compound.

The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.
Suitable seed is seed of cereals, root crops, oil crops, vegetables, spices, ornamentals, for example seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants such as potatoes, sugar cane, tobacco, grapes, petunias, geranium/pelargoniums, pansies and impatiens.

In addition, the active compound may also be used for the treatment seeds from plants, which tolerate the action of herbicides or fungicides or insecticides owing to breeding, including genetic engineering methods.

For example, the active compound can be employed in treatment of seeds from plants, which are resistant to herbicides from the group consisting of the sulfonylureas, imidazolinones, glufosinate-ammonium or glyphosate-isopropylammonium and analogous active substances (see for example, EP-A-0242236, EP-A-242246) (WO 92/00377) (EP-A-0257993, U.S. Pat. No. 5,013,659) or in transgenic crop plants, for example cotton, with the capability of producing Bacillus thuringiensis toxins (Bt toxins) which make the plants resistant to certain pests (EP-A-0142924, EP-A-0193259),

Furthermore, the active compound can be used also for the treatment of seeds from plants, which have modified characteristics in comparison with existing plants consist, which can be generated for example by traditional breeding methods and/or the generation of mutants, or by recombinant procedures). For example, a number of cases have been described of recombinant modifications of crop plants for the purpose of modifying the starch synthesized in the plants (e.g. WO 92/11376, WO 92/14827, WO 91/19806) or of transgenic crop plants having a modified fatty acid composition (WO 91/13972).

The seed treatment application of the active compound is carried out by spraying or by dusting the seeds before sowing of the plants and before emergence of the plants.

Compositions which are especially useful for seed treatment are e.g.:
A Soluble concentrates (SL, LS)
D Emulsions (EW, EO, ES)
E Suspensions (SC, OD, FS)
F Water-dispersible granules and water-soluble granules (WG, SG)
G Water-dispersible powders and water-soluble powders (WP, SP, WS)
H Gel-Formulations (GF)
I Dustable powders (DP, DS)

Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter

In a preferred embodiment a FS formulation is used for seed treatment. Typcially, a FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.

Especially preferred FS formulations of compounds of formula I for seed treatment usually comprise from 0.1 to 80% by weight (1 to 800 g/l) of the active ingredient, from 0.1 to 20 % by weight (1 to 200 g/l) of at least one surfactant, e.g. 0.05 to 5 % by weight of a wetter and from 0.5 to 15 % by weight of a dispersing agent, up to 20 % by weight, e.g. from 5 to 20 % of an anti-freeze agent, from 0 to 15 % by weight, e.g. 1 to 15 % by weight of a pigment and/or a dye, from 0 to 40 % by weight, e.g. 1 to 40 % by weight of a binder (sticker /adhesion agent), optionally up to 5 % by weight, e.g. from 0.1 to 5 % by weight of a thickener, optionally from 0.1 to 2 % of an anti-foam agent, and optionally a preservative such as a biocide, antioxidant or the like, e.g. in an amount from 0.01 to 1 % by weight and a filler/vehicle up to 100 % by weight.

Seed Treatment formulations may additionally also comprise binders and optionally colorants.

Binders can be added to improve the adhesion of the active materials on the seeds after treatment. Suitable binders are homo- and copolymers from alkylene oxides like ethylene oxide or propylene oxide, polyvinylacetate, polyvinylalcohols, polyvinylpyrrolidones, and copolymers thereof, ethylene-vinyl acetate copolymers, acrylic homo- and copolymers, polyethyleneamines, polyethyleneamides and polyethyleneimines, polysaccharides like celluloses, tylose and starch, polyolefin homo- and copolymers like olefin/maleic anhydride copolymers, polyurethanes, polyesters, polystyrene homo and copolymers

Optionally, also colorants can be included in the formulation. Suitable colorants or dyes for seed treatment formulations are Rhodamin B, C.I. Pigment Red 112, C.I. Solvent Red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

### Examples of a gelling agent is carrageen (Satiagel^{®})

In the treatment of seed, the application rates of the compounds I are generally from 0.1 g to 10 kg per 100 kg of seed, preferably from 1 g to 5 kg per 100 kg of seed, more preferably from 1 g to 1000 g per 100 kg of seed and in particular from 1 g to 200 g per 100 kg of seed.

The invention therefore also relates to seed comprising a compound of the formula I, or an agriculturally useful salt of I, as defined herein. The amount of the compound I or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops such as lettuce the rate can be higher.

### Animal health

The compounds of formula I or the enantiomers or veterinarily acceptable salts thereof are in particular also suitable for being used for combating parasites in and on animals.

An object of the present invention is therfore also to provide new methods to control parasites in and on animals. Another object of the invention is to provide safer pesticides for animals. Another object of the invention is further to provide pesticides for animals that may be used in lower doses than existing pesticides. And another object of the invention is to provide pesticides for animals, which provide a long residual control of the parasites.

The invention also relates to compositions containing a parasiticidally effective amount of compounds of formula I or the enantiomers or veterinarily acceptable salts thereof and an acceptable carrier, for combating parasites in and on animals.

The present invention also provides a method for treating, controlling, preventing and protecting animals against infestation and infection by parasites, which comprises orally, topically or parenterally administering or applying to the animals a parasiticidally effective amount of a compound of formula I or the enantiomers or veterinarily acceptable salts thereof or a composition comprising it.

The invention also provides a process for the preparation of a composition for treating, controlling, preventing or protecting animals against infestation or infection by parasites which comprises a parasiticidally effective amount of a compound of formula I or the enantiomers or veterinarily acceptable salts thereof or a composition comprising it.

Activity of compounds against agricultural pests does not suggest their suitability for control of endo- and ectoparasites in and on animals which requires, for example, low, non-emetic dosages in the case of oral application, metabolic compatibility with the animal, low toxicity, and a safe handling.

Surprisingly it has now been found that compounds of formula I are suitable for combating endo- and ectoparasites in and on animals.

Compounds of formula I or the enantiomers or veterinarily acceptable salts thereof and compositions comprising them are preferably used for controlling and preventing infestations and infections animals including warm-blooded animals (including humans) and fish. They are for example suitable for controlling and preventing infestations and infections in mammals such as cattle, sheep, swine, camels, deer, horses, pigs, poultry, rabbits, goats, dogs and cats, water buffalo, donkeys, fallow deer and reindeer, and also in fur-bearing animals such as mink, chinchilla and raccoon, birds such as hens, geese, turkeys and ducks and fish such as fresh- and salt-water fish such as trout, carp and eels.

Compounds of formula I or the enantiomers or veterinarily acceptable salts thereof and compositions comprising them are preferably used for controlling and preventing infestations and infections in domestic animals, such as dogs or cats.

Infestations in warm-blooded animals and fish include, but are not limited to, lice, biting lice, ticks, nasal bots, keds, biting flies, muscoid flies, flies, myiasitic fly larvae, chiggers, gnats, mosquitoes and fleas.

The compounds of formula I or the enantiomers or veterinarily acceptable salts thereof and compositions comprising them are suitable for systemic and/or non-systemic control of ecto- and/or endoparasites. They are active against all or some stages of development.

The compounds of formula I are especially useful for combating ectoparasites.

The compounds of formula I are especially useful for combating parasites of the following orders and species, respectively:
fleas (Siphonaptera), e.g. *Ctenocephalides felis, Ctenocephalides canis, Xenopsylla cheopis, Pulex irritans, Tunga penetrans,* and *Nosopsyllus fasciatus,*
cockroaches (Blattaria - Blattodea), e.g. *Blattella germanica, Blattella asahinae, Periplaneta americana, Periplaneta japonica, Periplaneta brunnea, Periplaneta fuligginosa, Periplaneta aus-tralasiae,* and *Blatta orientalis,*
flies, mosquitoes (Diptera), e.g. *Aedes aegypti, Aedes albopictus, Aedes vexans, Anastrepha ludens, Anopheles maculipennis, Anopheles crucians, Anopheles albimanus, Anopheles gambiae, Anopheles freeborni, Anopheles leucosphyrus, Anopheles minimus, Anopheles quadrimaculatus, Calliphora vicina, Chrysomya bezziana, Chrysomya hominivorax, Chrysomya macellaria, Chrysops discalis, Chrysops silacea, Chrysops atlanticus, Cochliomyia hominivorax, Cordylobia anthropophaga, Culicoides furens, Culex pipiens, Culex nigripalpus, Culex quinquefasciatus, Culex tarsalis, Culiseta inornata, Culiseta melanura, Dermatobia hominis, Fannia canicularis, Gasterophilus intestinalis, Glossina morsitans, Glossina palpalis, Glossina fuscipes, Glossina tachinoides, Haematobia irritans, Haplodiplosis equestris, Hippelates spp., Hypoderma lineata, Leptoconops torrens, Lucilia caprina, Lucilia cuprina, Lucilia sericata, Lycoria pectoralis, Mansonia spp., Musca domestica, Muscina stabulans, Oestrus ovis, Phlebotomus argentipes, Psorophora columbiae, Psorophora discolor, Prosimulium mixtum, Sarcophaga haemorrhoidalis, Sarcophaga sp., Simulium vittatum, Stomoxys calcitrans, Tabanus bovinus, Tabanus atratus, Tabanus lineola,* and *Tabanus similis,*
lice (Phthiraptera), e.g. *Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus* and *Solenopotes capillatus.*
ticks and parasitic mites (Parasitiformes): ticks (Ixodida), e.g. *Ixodes scapularis, Ixodes holocyclus, Ixodes pacificus, Rhiphicephalus sanguineus, Dermacentor andersoni, Dermacentor variabilis, Amblyomma americanum, Ambryomma maculatum, Ornithodorus hermsi, Ornithodorus turicata* and parasitic mites (Mesostigmata), e.g. *Ornithonyssus bacoti* and *Dermanyssus gallinae,*
Actinedida (Prostigmata) und Acaridida (Astigmata) e.g. *Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp.,Knemidocoptes spp., Cytodites spp.,* and *Laminosioptes spp,*
Bugs (Heteropterida): *Cimex lectularius, Cimex hemipterus, Reduvius senilis, Triatoma spp., Rhodnius ssp., Panstrongylus ssp.* and *Arilus critatus,*
Anoplurida, e.g. *Haematopinus spp., Linognathus spp., Pediculus spp., Phtirus spp.,* and *Solenopotes spp,*
Mallophagida (suborders Arnblycerina and Ischnocerina), e.g. *Trimenopon spp., Menopon spp., Trinoton spp., Bovicola spp., Werneckiella spp., Lepikentron spp., Trichodectes spp.,* and *Felicola spp,*
Roundworms Nematoda:
   Wipeworms and Trichinosis (Trichosyringida), e.g. Trichinellidae *(Trichinella spp.),* (Trichuridae) *Trichuris spp., Capillaria spp,*
   Rhabditida, e.g. *Rhabditis spp, Strongyloides spp., Helicephalobus spp,*
   Strongylida, e.g. *Strongylus spp., Ancylostoma spp., Necator americanus, Bunostomum spp. (Hookworm), Trichostrongylus spp., Haemonchus contortus., Ostertagia spp. , Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp. Aleurostrongylus abstrusus,* and *Dioctophyma renale,*
   Intestinal roundworms (Ascaridida), e.g. *Ascaris lumbricoides, Ascaris suum, Ascaridia galli, Parascaris equorum, Enterobius vermicularis (Threadworm), Toxocara canis, Toxascaris leonine, Skrjabinema spp.,* and *Oxyuris equi,*
   Camallanida, e.g. *Dracunculus medinensis* (guinea worm)
   Spirurida, e.g. *Thelazia spp. Wuchereria spp., Brugia spp., Onchocerca spp., Dirofilari spp.a, Dipetalonema spp., Setaria spp., Elaeophora spp., Spirocerca lupi,* and *Habronema spp.,*
   Thorny headed worms (Acanthocephala), e.g. *Acanthocephalus spp., Macracanthorhynchus hirudinaceus* and *Oncicola spp,*
   Planarians (Plathelminthes):
      Flukes (Trematoda), e.g. *Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp.,* and *Nanocyetes spp,*
      Cercomeromorpha, in particular Cestoda (Tapeworms), e.g. *Diphyllobothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp.,* and *Hymenolepis spp.*

The compounds of formula I and compositions containing them are particularly useful for the control of pests from the orders Diptera, Siphonaptera and Ixodida.

Moreover, the use of the compounds of formula I and compositions containing them for combating mosquitoes is especially preferred.

The use of the compounds of formula I and compositions containing them for combating flies is a further preferred embodiment of the present invention.

Furthermore, the use of the compounds of formula I and compositions containing them for combating fleas is especially preferred.

The use of the compounds of formula I and compositions containing them for combating ticks is a further preferred embodiment of the present invention.

The compounds of formula I also are especially useful for combating endoparasites (roundworms nematoda, thorny headed worms and planarians).

Administration can be carried out both prophylactically and therapeutically.

Administration of the active compounds is carried out directly or in the form of suitable preparations, orally, topically/dermally or parenterally.

For oral administration to warm-blooded animals, the formula I compounds may be formulated as animal feeds, animal feed premixes, animal feed concentrates, pills, solutions, pastes, suspensions, drenches, gels, tablets, boluses and capsules. In addition, the formula I compounds may be administered to the animals in their drinking water. For oral administration, the dosage form chosen should provide the animal with 0.01 mg/kg to 100 mg/kg of animal body weight per day of the formula I compound, preferably with 0.5 mg/kg to 100 mg/kg of animal body weight per day.

Alternatively, the formula I compounds may be administered to animals parenterally, for example, by intraruminal, intramuscular, intravenous or subcutaneous injection. The formula I compounds may be dispersed or dissolved in a physiologically acceptable carrier for subcutaneous injection. Alternatively, the formula I compounds may be formulated into an implant for subcutaneous administration. In addition the formula I compound may be transdermally administered to animals. For parenteral administration, the dosage form chosen should provide the animal with 0.01 mg/kg to 100 mg/kg of animal body weight per day of the formula I compound.

The formula I compounds may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the formula I compound. In addition, the formula I compounds may be formulated as ear tags for animals, particularly quadrupeds such as cattle and sheep.

Suitable preparations are:
- Solutions such as oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations such as powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further ingredients such as acids, bases, buffer salts, preservatives, and solubilizers. The solutions are filtered and filled sterile.

Suitable solvents are physiologically tolerable solvents such as water, alkanols such as ethanol, butanol, benzyl alcohol, glycerol, propylene glycol, polyethylene glycols, N-methyl-pyrrolidone, 2-pyrrolidone, and mixtures thereof.

The active compounds can optionally be dissolved in physiologically tolerable vegetable or synthetic oils which are suitable for injection.

Suitable solubilizers are solvents which promote the dissolution of the active compound in the main solvent or prevent its precipitation. Examples are polyvinylpyrrolidone, polyvinyl alcohol, polyoxyethylated castor oil, and polyoxyethylated sorbitan ester.

Suitable preservatives are benzyl alcohol, trichlorobutanol, p-hydroxybenzoic acid esters, and n-butanol.
Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on.

Solutions for use on the skin are prepared according to the state of the art and according to what is described above for injection solutions, sterile procedures not being necessary.

Further suitable solvents are polypropylene glycol, phenyl ethanol, phenoxy ethanol, ester such as ethyl or butyl acetate, benzyl benzoate, ethers such as alkyleneglycol alkylether, e.g. dipropylenglycol monomethylether, ketons such as acetone, methylethylketone, aromatic hydrocarbons, vegetable and synthetic oils, dimethylformamide, dimethylacetamide, transcutol, solketal, propylencarbonate, and mixtures thereof.

It may be advantageous to add thickeners during preparation. Suitable thickeners are inorganic thickeners such as bentonites, colloidal silicic acid, aluminium monostearate, organic thickeners such as cellulose derivatives, polyvinyl alcohols and their copolymers, acrylates and methacrylates.

Gels are applied to or spread on the skin or introduced into body cavities. Gels are prepared by treating solutions which have been prepared as described in the case of the injection solutions with sufficient thickener that a clear material having an ointment-like consistency results. The thickeners employed are the thickeners given above.

Pour-on formulations are poured or sprayed onto limited areas of the skin, the active compound penetrating the skin and acting systemically.

Pour-on formulations are prepared by dissolving, suspending or emulsifying the active compound in suitable skin-compatible solvents or solvent mixtures. If appropriate, other auxiliaries such as colorants, bioabsorption-promoting substances, antioxidants, light stabilizers, adhesives are added.

Suitable solvents which are: water, alkanols, glycols, polyethylene glycols, polypropylene glycols, glycerol, aromatic alcohols such as benzyl alcohol, phenylethanol, phenoxyethanol, esters such as ethyl acetate, butyl acetate, benzyl benzoate, ethers such as alkylene glycol alkyl ethers such as dipropylene glycol monomethyl ether, diethylene glycol mono-butyl ether, ketones such as acetone, methyl ethyl ketone, cyclic carbonates such as propylene carbonate, ethylene carbonate, aromatic and/or aliphatic hydrocarbons, vegetable or synthetic oils, DMF, dimethylacetamide, n-alkylpyrrolidones such as methylpyrrolidone, n-butylpyrrolidone or n-octylpyrrolidone, N-methylpyrrolidone, 2-pyrrolidone, 2,2-dimethyl-4-oxy-methylene-1,3-dioxolane and glycerol formal.

Suitable colorants are all colorants permitted for use on animals and which can be dissolved or suspended.

Suitable absorption-promoting substances are, for example, DMSO, spreading oils such as isopropyl myristate, dipropylene glycol pelargonate, silicone oils and copolymers thereof with polyethers, fatty acid esters, triglycerides, fatty alcohols.

Suitable antioxidants are sulfites or metabisulfites such as potassium metabisulfite, ascorbic acid, butylhydroxytoluene, butylhydroxyanisole, tocopherol.

Suitable light stabilizers are, for example, novantisolic acid.

Suitable adhesives are, for example, cellulose derivatives, starch derivatives, polyacrylates, natural polymers such as alginates, gelatin.

Emulsions can be administered orally, dermally or as injections.

Emulsions are either of the water-in-oil type or of the oil-in-water type.

They are prepared by dissolving the active compound either in the hydrophobic or in the hydrophilic phase and homogenizing this with the solvent of the other phase with the aid of suitable emulsifiers and, if appropriate, other auxiliaries such as colorants, absorption-promoting substances, preservatives, antioxidants, light stabilizers, viscosity-enhancing substances.

Suitable hydrophobic phases (oils) are:
liquid paraffins, silicone oils, natural vegetable oils such as sesame oil, almond oil, castor oil, synthetic triglycerides such as caprylic/capric biglyceride, triglyceride mixture with vegetable fatty acids of the chain length C₈-C₁₂ or other specially selected natural fatty acids, partial glyceride mixtures of saturated or unsaturated fatty acids possibly also containing hydroxyl groups, mono- and diglycerides of the C₈-C₁₀ fatty acids,
fatty acid esters such as ethyl stearate, di-n-butyryl adipate, hexyl laurate, dipropylene glycol perlargonate, esters of a branched fatty acid of medium chain length with saturated fatty alcohols of chain length C₁₆-C₁₈, isopropyl myristate, isopropyl palmitate, caprylic/capric acid esters of saturated fatty alcohols of chain length C₁₂-C₁₈, isopropyl stearate, oleyl oleate, decyl oleate, ethyl oleate, ethyl lactate, waxy fatty acid esters such as synthetic duck coccygeal gland fat, dibutyl phthalate, diisopropyl adipate, and ester mixtures related to the latter, fatty alcohols such as isotridecyl alcohol, 2-octyldodecanol, cetylstearyl alcohol, oleyl alcohol, and fatty acids such as oleic acid and mixtures thereof.

Suitable hydrophilic phases are: water, alcohols such as propylene glycol, glycerol, sorbitol and mixtures thereof.

Suitable emulsifiers are:
non-ionic surfactants, e.g. polyethoxylated castor oil, polyethoxylated sorbitan monooleate, sorbitan monostearate, glycerol monostearate, polyoxyethyl stearate, alkylphenol polyglycol ether; ampholytic surfactants such as di-sodium N-lauryl-p-iminodipropionate or lecithin;
anionic surfactants, such as sodium lauryl sulfate, fatty alcohol ether sulfates, mono/dialkyl polyglycol ether orthophosphoric acid ester monoethanolamine salt; cation-active surfactants, such as cetyltrimethylammonium chloride.

Suitable further auxiliaries are: substances which enhance the viscosity and stabilize the emulsion, such as carboxymethylcellulose, methylcellulose and other cellulose and starch derivatives, polyacrylates, alginates, gelatin, gum arabic, polyvinylpyrrolidone, polyvinyl alcohol, copolymers of methyl vinyl ether and maleic anhydride, polyethylene glycols, waxes, colloidal silicic acid or mixtures of the substances mentioned.

Suspensions can be administered orally or topically/dermally. They are prepared by suspending the active compound in a suspending agent, if appropriate with addition of other auxiliaries such as wetting agents, colorants, bioabsorption-promoting substances, preservatives, antioxidants, light stabilizers.

Liquid suspending agents are all homogeneous solvents and solvent mixtures.

Suitable wetting agents (dispersants) are the emulsifiers given above.

Other auxiliaries which may be mentioned are those given above.
Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form.

Suitable excipients are all physiologically tolerable solid inert substances. Those used are inorganic and organic substances. Inorganic substances are, for example, sodium chloride, carbonates such as calcium carbonate, hydrogencarbonates, aluminium oxides, titanium oxide, silicic acids, argillaceous earths, precipitated or colloidal silica, or phosphates. Organic substances are, for example, sugar, cellulose, foodstuffs and feeds such as milk powder, animal meal, grain meals and shreds, starches.

Suitable auxiliaries are preservatives, antioxidants, and/or colorants which have been mentioned above.
Other suitable auxiliaries are lubricants and glidants such as magnesium stearate, stearic acid, talc, bentonites, disintegration-promoting substances such as starch or crosslinked polyvinylpyrrolidone, binders such as starch, gelatin or linear polyvinylpyrrolidone, and dry binders such as microcrystalline cellulose.

In general, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions such as desired parasiticidal effect and duration, target species, mode of application, and the like.

The compositions which can be used in the invention can comprise generally from about 0.001 to 95% of the compound of formula I.

Generally it is favorable to apply the compounds of formula I in total amounts of 0.5 mg/kg to 100 mg/kg per day, preferably 1 mg/kg to 50 mg/kg per day.

Ready-to-use preparations contain the compounds acting against parasites, preferably ectoparasites, in concentrations of 10 ppm to 80 per cent by weight, preferably from 0.1 to 65 per cent by weight, more preferably from 1 to 50 per cent by weight, most preferably from 5 to 40 per cent by weight.

Preparations which are diluted before use contain the compounds acting against ectoparasites in concentrations of 0.5 to 90 per cent by weight, preferably of 1 to 50 per cent by weight.

Furthermore, the preparations comprise the compounds of formula I against endoparasites in concentrations of 10 ppm to 2 per cent by weight, preferably of 0.05 to 0.9 per cent by weight, very particularly preferably of 0.005 to 0.25 per cent by weight.

In a preferred embodiment of the present invention, the compositions comprising the compounds of formula I them are applied dermally / topically.

In a further preferred embodiment, the topical application is conducted in the form of compound-containing shaped articles such as collars, medallions, ear tags, bands for fixing at body parts, and adhesive strips and foils.
Generally it is favorable to apply solid formulations which release compounds of formula I in total amounts of 10 mg/kg to 300 mg/kg, preferably 20 mg/kg to 200 mg/kg, most preferably 25 mg/kg to 160 mg/kg body weight of the treated animal in the course of three weeks.

For the preparation of the shaped articles, thermoplastic and flexible plastics as well as elastomers and thermoplastic elastomers are used. Suitable plastics and elastomers are polyvinyl resins, polyurethane, polyacrylate, epoxy resins, cellulose, cellulose derivatives, polyamides and polyester which are sufficiently compatible with the compounds of formula I. A detailed list of plastics and elastomers as well as preparation procedures for the shaped articles is given e.g. in WO 03/086075.

### Examples

The present invention is now illustrated in further details by the following examples, without imposing any limitation thereto.

### S. Synthesis Examples

### Synthesis Example S.1: N-[1-[(6-chloro-3-pyridyl)methyl]-2-pyridylidene]-3-methoxy-propanamide (example compound E1.3)

### Step 1: Preparation of 1-[(6-chloro-3-pyridyl)methyl]pyridin-1-ium-2-amine chloride (intermediate compound IE.1)]

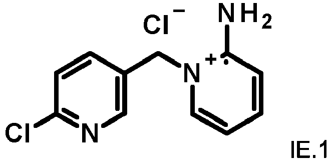

A solution 2-chloro-5-chloromethyl-pyridine (16.20g, 100 mmol) and 2-amino-pyridine (9.60g, 102 mmol) in ethanol (100 mL) was refluxed for 24 hours. The reaction was then cooled to room temperature, and concentrated *in vacuo.* Then 100 mL of phenylmethyl was added to the residue, and the mixture was concentrated *in vacuo.* The 75 mL of CH₂Cl₂ was added to the residue and the mixture was stirred rapidly for 15 minutes, during which time a precipitate forms. The precipitate was then filtered, and washed with CH₂Cl₂ (50 mL), diethyl ether(50 mL), and dried under vacuum to afford the product 1-[(6-chloro-3-pyridyl)methyl]pyridin-1-ium-2-amine chloride IE.1 as a pale yellow solid which (14.0g, 55% yield).
LC-MS: mass calc' d. for C₁₁H₁₁ClN₃ [M]⁺ 220.1, found 220.1; t_{R}= 0.529 min.

### Step 2: Preparation of N-[1-[(6-chloro-3-pyridyl)methyl]-2-pyridylidene]-3-methoxypropanamide (example compound E1.3)

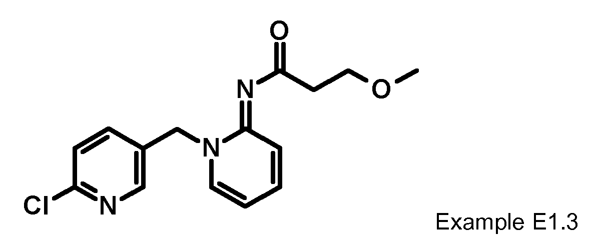

To a solution of 1-[(6-chloro-3-pyridyl)methyl]pyridin-1-ium-2-amine chloride amine salt IE.1(0.250g, 0.97 mmol), 3-methoxypropanoic acid (0.132g, 1.26 mmol), and triethylamine (0.267g, 2.63 mmol) in CH₂Cl₂ (8 mL) at 0 C was added a solution of Propyl phosphoric anhydride (50% wt) (0.496g, 1.55 mmol, 1 mL of a 50% wt solution in EtOAc). The reaction was then allowed to warm to room temperature, and stir for 60 hours. The reaction was dilluted with EtOAc (200 mL), washed with saturated aqueous NaHCO₃, layers separated, and the organic layer dried over Na₂SO₄ and concentrated *in vacuo* to afford the desired product E1.3 as a beige solid (0.120g, 40% yield).
LC-MS: mass calc' d. for C₁₅H₁₇ClN₃O₂ [M+H]+ 306.1, found 306.1; t_{R}= 0.630 min.

### Synthesis Example S.2: N-[1-[(6-chloro-3-pyridyl)methyl]-2-pyridylidene]-2-methylsulfanylacetamide (example compound E1.9)

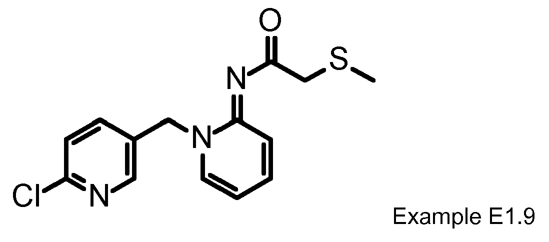

Example E1.9

To a solution of intermediate amine salt IE.1(1.30g, 5.08 mmol), methylthioacetic acid (0.70g, 6.60 mmol), and triethylamine (1.39g, 13.70 mmol) in CH₂Cl₂ (15 mL) at 0 C was added a solution of PPA (50% wt) (2.58g, 1.60 mmol, 5 mL of a 50% wt solution in EtOAc). The reaction was then allowed to warm to room temperature, and stir for 60 hours. The reaction was dilluted with EtOAc (200 mL), washed with saturated aqueous NaHCO₃, layers separated, and the organic layer dried over Na₂SO₄ and concentrated *in vacuo* to afford a residue. The residue was purified using silica gel chromatography eluting with 100% EtOAc, then 4% MeOH/CH₂Cl₂ to give the desired product E1.9 as a beige solid (0.750g, 46% yield). HRMS (ESI, Na) *m*/*z* calculated for C₁₁H₁₈N₂O₃Na (M + Na)⁺ 249.1210,
LC-MS: mass calc' d. for C₁₄H₁₅ClN₃OS [M+H]+ 308.1, found 308.1; t_{R}= 0.611 min.

### Synthesis Example S.3: N-[1-[(6-chloro-3-pyridyl)methyl]-2-pyridylidene]-2-methylsulfonyl-acetamide (example compound E1.10)

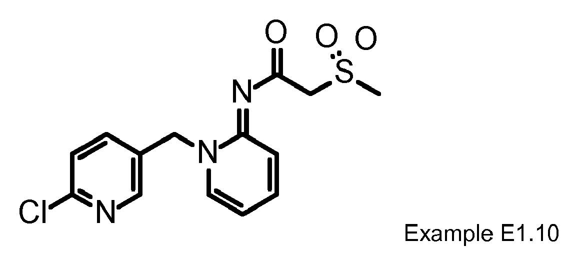

Example E1.10

To a solution of the amide E1.9 (0.400g, 1.300 mmol), in CH₂Cl₂ (7 mL) at 0 C was added mCPBA (0.750g, 3.250 mmol) in one portion. The reaction stirred 20 min and then became extremely thick as a precipitate formed, then 7 mL of CH₂Cl₂ was added. The reaction was then stirred 6 hours at room temperature then dilluted with saturated aqueous NaHCO₃ (100 mL), extracted with EtOAc (200 mL), the organic layer was dried over Na₂SO₄, filtered and concentrated *in vacuo.* The resulting residue was purified using silica gel chromatography eluting with 100% EtOAc, then 4% MeOH/CH₂Cl₂ to give the desired product E1.10 as a beige solid (0.300g, 84% yield).
LC-MS: mass calc' d. for C₁₄H₁₅ClN₃O₃S [M+H]+ 340.0 , found 340.0; t_{R}= 0.621 min.

### C. Compound examples

Some compound examples according to the present invention are shown with their physical data in tables below and their biological activity is demonstrated thereafter.

The Compound examples can be characterized by their physic-chemical data^{*)}, e.g. by coupled High Performance Liquid Chromatography, by mass spectrometry (HPLC/MS) or by their melting point.
^{*)}Analytical UPLC column: Phenomenex Kinetex 1.7 µm XB-C18 100A; 50 x 2.1 mm; mobile phase: A: water + 0.1% trifluoroacetic acid (TFA); B: acetonitrile + 0.1% TFA; gradient: 5-100% B in 1.50 minutes; 100% B 0.20 min; flow: 0.8-1.0mL/min in 1.50 minutes at 60°C.
MS-method: ESI positive.

Examples and data of compounds of formula E1 according to the present invention are given in table E1 below.

Compound examples of 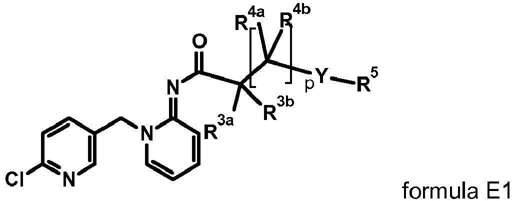 with their physico chemical data are given in table E1 herein below.

**Table E1:**

| **No.** | **R^{3a}** | **R^{3b}** | **p** | **R^{4a}** | **R^{4b}** | **Y** | **R^{5 a)}** | **Physicochemical data^{*)}** |
|---|---|---|---|---|---|---|---|---|
| E1-1 | H | H | 0 | - | - | O | CH₃ | r.t.= 0.614 |
| | | | | | | | | m/z = 292.1 |
| E1-2 | H | H | 0 | - | - | O | CH₂CH₂OCH₃ | r.t.= 0.669 |
| | | | | | | | | m/z = 336.1 |
| E1-3 | H | H | 1 | H | H | O | CH₃ | r.t.= 0.630 |
| | | | | | | | | m/z = 306.1 |
| E1-4 | H | H | 1 | H | CH₂CH₅ | O | CH₃ | r.t.= 0.755 |
| | | | | | | | | m/z = 334.1 |
| E1-5 | CH₃ | CH₃ | 1 | H | H | O | CH₃ | r.t.= 0.777 |
| | | | | | | | | m/z = 334.1 |
| E1-6 | H | H | 0 | - | - | S | CF₃ | r.t.= 0.775 |
| | | | | | | | | m/z = 362.1 |
| E1-7 | H | H | 0 | - | - | S | n-C₃F₇ | r.t.= 0.969 |
| | | | | | | | | m/z = 462.2 |
| E1-8 | H | H | 0 | - | - | SO | CF₃ | r.t.= 0.823 |
| | | | | | | | | m/z = 378.0 |
| E1-9 | H | H | 0 | - | - | S | CH₃ | r.t.= 0.611 |
| | | | | | | | | m/z = 308.1 |
| E1-10 | H | H | 0 | - | - | SO₂ | CH₃ | r.t.= 0.621 |
| | | | | | | | | m/z = 340.0 |
| E1-11 | F | F | 0 | - | - | S | 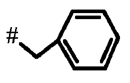 | r.t.= 1.121 |
| | | | | | | | | m/z = 420.1 |
| E1-12 | F | F | 0 | - | - | SO₂ | 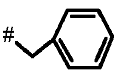 | r.t.= 1.032 |
| | | | | | | | | m/z = 452.2 |

| | | | | | | | | |
|---|---|---|---|---|---|---|---|---|
| ^{a)} # denotes the attachment point to the remainder of the molecule; ^{*)} analytical UPLC column (see below) | | | | | | | | |

In the following examples and tables E, Het is denoted according to the tables below:

| | | | |
|---|---|---|---|
| Het1A | Het1B | Het1C | Het4.0 |
| 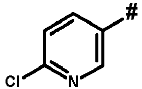 | 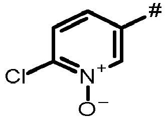 | 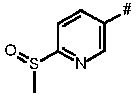 | 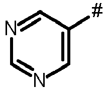 |

| | | | |
|---|---|---|---|
| Het11A | Het23A | Het24.0 | Het27A |
| 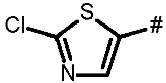 | 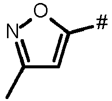 | 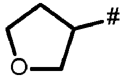 | 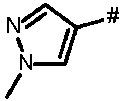 |

E2 Compound examples of formula 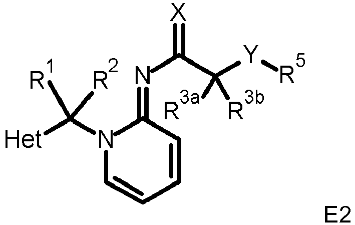 with their physico chemical data are given in table E2 herein below.

**Table E2:**

| **No.** | **Het** | **R¹** | **R²** | **R^{3a}** | **R^{3b}** | **R⁵** | **X** | **Y** | **RT [min]** | **m/z [MH]⁺** |
|---|---|---|---|---|---|---|---|---|---|---|
| E2-1 | Het1B | H | H | F | F | CH₂(C₆H₅) | O | SO₂ | 0.857 | 468.1 |
| E2-2 | Het1A | H | H | CH₃ | H | CF₃ | O | S | 0.882 | 376.1 |
| E2-3 | Het1A | H | H | H | H | CF₂CHF₂ | O | S | 0.837 | 394.1 |
| E2-4 | Het1A | H | H | H | H | CN | O | S | NA | NA |
| E2-5 | Het1A | H | H | F | F | CH₃ | O | SO₂ | 0.844 | 376.3 |
| E2-6 | Het1A | H | H | F | F | CH₃ | O | S | 0.946 | 344.3 |
| E2-7 | Het1A | H | H | H | H | CH₃ | O | SO | 0.501 | 324.1 |
| E2-8 | Het1A | H | H | F | F | CH₃ | S | S | 0.974 | 360.0 |
| E2-9 | Het1A | H | H | F | F | CH₃ | O | SO | 0.722 | 360.5 |
| E2-10 | Het1A | H | H | H | H | CH₂(C₆H₅) | O | S | 0.763 | 338.1 |
| E2-11 | Het1A | H | H | H | H | CF₂CF₂CF₃ | O | SO | 1.033 | 478.0 |
| E2-12 | Het1A | H | H | H | H | SCH₃ | O | S | 0.724 | 340.0 |
| E2-13 | Het1A | H | H | H | H | CH₃ | S | S | 0.792 | 324.0 |
| E2-14 | Het1A | H | H | SCH₃ | H | CH₃ | O | O | NA | NA |
| E2-15 | Het1A | H | H | H | H | CF₂CHF₂ | O | SO | 0.826 | 432.1 |
| E2-16 | Het1A | H | H | CH₃ | H | CF₃ | O | SO | 0.873 | 392.5 |
| E2-17 | Het1A | H | H | H | H | SCH₂CH₃ | O | S | 0.794 | 353.8 |
| E2-18 | Het11A | H | H | H | H | CH₃ | O | S | 0.662 | 314.2 |
| E2-19 | Het1A | CH₃ | H | F | F | CH₃ | O | S | 1.017 | 358.3 |
| E2-20 | Het1A | CH₃ | H | H | H | CH₃ | O | SO₂ | 0,591 | 354.3 |
| E2-21 | Het1A | H | H | H | H | CF₂CF₃ | O | S | 0.918 | 412.5 |
| E2-22 | Het1A | H | H | F | F | (2-fluorophenyl)methyl | O | S | 1.160 | 438.6 |
| E2-23 | Het1A | H | H | F | F | (2,5-dichlorophenyl)methyl | O | S | 1.277 | 490.4 |
| E2-24 | Het1A | H | H | F | F | (2,4-dichlorophenyl)methyl | O | S | 1.296 | 490.4 |
| E2-25 | Het1A | H | H | F | F | (CH₂)₃OC₆H₅ | O | S | 1.219 | 465.0 |
| E2-26 | Het1A | H | H | F | F | (4-fluorophenyl)methyl | O | S | 1.162 | 440.6 |
| E2-27 | Het1A | H | H | F | F | CH₂CH(CH₃)₂ | O | S | 1.160 | 386.5 |
| E2-28 | Het1A | H | H | F | F | CH₂CH₂CO₂CH₃ | O | S | 0.976 | 416.6 |
| E2-29 | Het1A | H | H | F | F | cC₆H₁₁ | O | S | 1.233 | 412.9 |
| E2-30 | Het1A | H | H | F | F | (4-chlorophenyl)methyl | O | S | 1.227 | 456.3 |
| E2-31 | Het1A | H | H | F | F | (CH₂)₂C₆H₅ | O | S | 1.199 | 434.6 |
| E2-32 | Het1A | H | H | F | F | CH₂CH₂CH(CH₃)₃ | O | S | 1.229 | 400.6 |
| E2-33 | Het1A | H | H | F | F | CH(CH₃)(CH₂CH₃) | O | S | 1.154 | 386.5 |
| E2-34 | Het1A | H | H | F | F | CH₂CO₂CH(CH₃)₂ | O | S | 1.071 | 430.6 |
| E2-35 | Het1A | H | H | F | F | 2-furylmethyl | O | S | 1.079 | 410.7 |
| E2-36 | Het1A | H | H | F | F | CH₂CH(CH₃)CH₂CH₃ | O | S | 1.223 | 400.5 |
| E2-37 | Het1A | H | H | F | F | CH₂CO₂(CH₂)₃CH₃ | O | S | 1.144 | 444.9 |
| E2-38 | Het1A | H | H | F | F | CHCH₃CO₂CH₂CH₃ | O | S | 1.067 | 430.9 |
| E2-39 | Het1A | H | H | F | F | (3-chlorophenyl)methyl | O | S | 1.221 | 456.2 |
| E2-40 | Het1A | H | H | F | F | o-tolylmethyl | O | S | 1.205 | 434.6 |
| E2-41 | Het1A | H | H | F | F | (2-chlorophenyl)methyl | O | S | 1.209 | 454.6 |
| E2-42 | Het1A | H | H | F | F | (3,4-dichlorophenyl)methyl | O | S | 1.283 | 490.3 |
| E2-43 | Het1A | H | H | F | F | m-tolylmethyl | O | S | 1.201 | 433.8 |
| E2-44 | Het1A | H | H | F | F | 3-(trifluoromethyl)phenyl | O | S | 1.221 | 473.8 |
| E2-45 | Het1A | H | H | F | F | 4-(4-methoxyphenoxy)phenyl | O | S | 1.270 | 527.9 |
| E2-46 | Het1A | H | H | F | F | 4,6-dimethoxypyrimidin-2-yl | O | S | 1.108 | 467.8 |
| E2-47 | Het1A | H | H | F | F | CH₂CO₂(CH₂)₅CH(CH₃)₂ | O | S | 1.343 | 499.9 |
| E2-48 | Het1A | H | H | F | F | 3-chloro-4-fluoro-phenyl | O | S | 1.217 | 457.7 |
| E2-49 | Het1A | H | H | F | F | 3,5-bis(trifluoromethyl)phenyl | O | S | 1.324 | 541.8 |
| E2-50 | Het1A | H | H | F | F | CH₂CH₂OCH₂CH₃ | O | S | 1.002 | 401.8 |
| E2-51 | Het1A | H | H | F | F | CH₂CF₃ | O | S | 1.065 | 411.7 |
| E2-52 | Het1A | H | H | F | F | p-tolylmethyl | O | S | 1.202 | 433.8 |
| E2-53 | Het1A | H | H | F | F | CH₂CH₂OCH₃ | O | S | 0.952 | 387.9 |
| E2-54 | Het1A | H | H | F | F | (CH₂)₄OC₆H₅ | O | S | 1.242 | 477.8 |
| E2-55 | Het1A | H | H | F | F | CH₂CH₂CH₂CH₃ | O | S | 1.156 | 385.8 |
| E2-56 | Het1A | H | H | F | F | CH₂CHCH₂ | O | S | 1.047 | 369.7 |
| E2-57 | Het1A | CH₃ | H | H | H | CH₃ | O | S | 0.660 | 322.3 |
| E2-58 | Het1A | CH₃ | H | F | F | CH₃ | O | SO₂ | 0.782 | 374.3 |
| E2-59 | Het1A | CH₃ | H | F | F | CH₃ | O | SO | NA | NA |
| E2-60 | Het1A | H | H | F | F | (4-methoxyphenyl)methyl | O | SO₂ | 1.057 | 481.8 |
| E2-61 | Het1A | H | H | F | F | (2-fluorophenyl)methyl | O | SO₂ | 1.064 | 469.7 |
| E2-62 | Het1A | H | H | F | F | (2,5-dichlorophenyl)methyl | O | SO₂ | 1.168 | 521.7 |
| E2-63 | Het1A | H | H | F | F | (2,4-dichlorophenyl)methyl | O | SO₂ | 1.185 | 521.6 |
| E2-64 | Het1A | H | H | F | F | (CH₂)₃OC₆H₅ | O | SO₂ | 1.139 | 495.8 |
| E2-65 | Het1A | H | H | F | F | (4-fluorophenyl)methyl | O | SO₂ | 1.073 | 469.8 |
| E2-66 | Het1A | H | H | F | F | CH₂CH(CH₃)₂ | O | SO₂ | 1.049 | 417.8 |
| E2-67 | Het1A | H | H | F | F | CH₂CH₂CO₂CH₃ | O | SO₂ | 0.931 | 447.8 |
| E2-68 | Het1A | H | H | F | F | (4-chlorophenyl)methyl | O | SO₂ | 1.129 | 487.5 |
| E2-69 | Het1A | H | H | F | F | (CH₂)₂C₆H₅ | O | SO₂ | 1.119 | 465.8 |
| E2-70 | Het1A | H | H | F | F | CH₂CH₂CH(CH₃)₃ | O | SO₂ | 1.111 | 431.7 |
| E2-71 | Het1A | H | H | F | F | CH₂CO₂CH(CH₃)₂ | O | SO₂ | 1.031 | 461.7 |
| E2-72 | Het1A | H | H | F | F | 2-furylmethyl | O | SO₂ | 0.993 | 441.8 |
| E2-73 | Het1A | H | H | F | F | CH₂CH(CH₃)CH₂CH₃ | O | SO₂ | 1.107 | 431.8 |
| E2-74 | Het1A | H | H | F | F | CH₂CH₂CO₂CH₂CH₃ | O | SO₂ | 0.994 | 461.7 |
| E2-75 | Het1A | H | H | F | F | CH₂CO₂(CH₂)₃CH₃ | O | SO₂ | 1.099 | 461.7 |
| E2-76 | Het1A | H | H | F | F | CHCH₃CO₂CH₂CH₃ | O | SO₂ | 1.018 | 461.8 |
| E2-77 | Het1A | H | H | F | F | (3-chlorophenyl)methyl | O | SO₂ | 1.124 | 487.5 |
| E2-78 | Het1A | H | H | F | F | o-tolylmethyl | O | SO₂ | 1.109 | 465.8 |
| E2-79 | Het1A | H | H | F | F | (2-chlorophenyl)methyl | O | SO₂ | 1.106 | 485.7 |
| E2-80 | Het1A | H | H | F | F | (3,4-dichlorophenyl)methyl | O | SO₂ | 1.183 | 519.7 |
| E2-81 | Het1A | H | H | F | F | 4-Cl-C₆H₄ | O | S | 1.200 | 441.5 |
| E2-82 | Het1A | H | H | F | F | 5-methyl-1,3,4-thiadiazol-2-yl | O | S | 0.934 | 427.7 |
| E2-83 | Het1A | H | H | F | F | 5-(trifluoromethyl)-2-pyridyl | O | S | 1.139 | 474.7 |
| E2-84 | Het1A | H | H | F | F | 2,5-dichlorophenyl | O | S | 1.245 | 475.6 |
| E2-85 | Het1A | H | H | F | F | 3-cyclopropyl-1H-1,2,4-triazol-5-yl | O | S | 0.840 | 436.8 |
| E2-86 | Het1A | H | H | F | F | 4-(4-chlorophenyl)thiazol-2-yl | O | S | 1.283 | 522.8 |
| E2-87 | Het1A | H | H | F | F | 3-Cl-C₆H₄ | O | S | 1.197 | 441.5 |
| E2-88 | Het1A | H | H | F | F | 4-F-C₆H₄ | O | S | 1.135 | 423.7 |
| E2-89 | Het1A | H | H | F | F | 2,6-dichlorophenyl | O | S | 1.191 | 475.6 |
| E2-90 | Het1A | H | H | F | F | 2-Cl-C₆H₄ | O | S | 1.160 | 439.7 |
| E2-91 | Het1A | H | H | F | F | 2-F-C₆H₄ | O | S | 1.115 | 423.7 |
| E2-92 | Het1A | H | H | F | F | 1 H-imidazol-2-yl | O | S | 0.683 | 395.7 |
| E2-93 | Het1A | H | H | F | F | 1,3-benzothiazol-2-yl | O | S | 1.148 | 462.7 |
| E2-94 | Het1A | H | H | F | F | m-tolylmethyl | O | SO₂ | 1.117 | 466.6 |
| E2-95 | Het1A | H | H | F | F | 3-(trifluoromethyl)phenyl | O | SO₂ | 1.159 | 506.7 |
| E2-96 | Het1A | H | H | F | F | 4-methoxyphenyl | O | SO₂ | 1.050 | 468.6 |
| E2-97 | Het1A | H | H | F | F | 4,6-dimethoxypyrimidin-2-yl | O | SO₂ | 1.037 | 500.6 |
| E2-98 | Het1A | H | H | F | F | CH₂CO₂(CH₂)₅CH(CH₃)₂ | O | SO₂ | 1.314 | 532.7 |
| E2-99 | Het1A | H | H | F | F | 3-chloro-4-fluoro-phenyl | O | SO₂ | 1.155 | 492.3 |
| E2-100 | Het1A | H | H | F | F | 3,5-bis(trifluoromethyl)phenyl | O | SO₂ | 1.268 | 574.8 |
| E2-101 | Het1A | H | H | F | F | 3-isopropyl-1H-1,2,4-triazol-5-yl | O | SO₂ | 1.201 | 481.0 |
| E2-102 | Het1A | H | H | F | F | p-tolylmethyl | O | SO₂ | 1.119 | 466.6 |
| E2-103 | Het1A | H | H | F | F | (CH₂)₄OC₆H₅ | O | SO₂ | 1.174 | 511.0 |
| E2-104 | Het1A | H | H | F | F | CH₂CH₂CH₂CH₃ | O | SO₂ | 1.057 | 420.5 |
| E2-105 | Het1A | H | H | F | F | 4-Cl-C₆H₄ | O | SO₂ | 1.126 | 473.5 |
| E2-106 | Het1A | H | H | F | F | 5-methyl-1,3,4-thiadiazol-2-yl | O | SO₂ | 0.959 | 459.7 |
| E2-107 | Het1A | H | H | F | F | 5-(trifluoromethyl)-2-pyridyl | O | SO₂ | 1.080 | 506.7 |
| E2-108 | Het1A | H | H | F | F | 3-cyclopropyl-1H-1,2,4-triazol-5-yl | O | SO₂ | 0.886 | 468.8 |
| E2-109 | Het1A | H | H | F | F | 4-(4-chlorophenyl)thiazol-2-yl | O | SO₂ | 1.228 | 555.8 |
| E2-110 | Het1A | H | H | F | F | 1-methylimidazol-2-yl | O | SO₂ | 0.870 | 441.8 |
| E2-111 | Het1A | H | H | F | F | 3-Cl-C₆H₄ | O | SO₂ | 1.124 | 473.5 |
| E2-112 | Het1A | H | H | F | F | 2-pyridyl | O | SO₂ | 0.912 | 438.7 |
| E2-113 | Het1A | H | H | F | F | 4-F-C₆H₄ | O | SO₂ | 1.063 | 455.8 |
| E2-114 | Het1A | H | H | F | F | CH₂CH₂CH₃ | O | SO₂ | 0.998 | 403.3 |
| E2-115 | Het24.0 | H | H | H | H | CH₃ | O | S | 0.508 | 267.4 |
| E2-116 | Het24.0 | H | H | F | F | CH₃ | O | S | 0.818 | 302.7 |
| E2-117 | Het24.0 | H | H | F | F | CH₃ | O | SO | 0.633 | 319.5 |
| E2-118 | Het24.0 | H | H | F | F | CH₃ | O | SO₂ | 0.732 | 334.7 |
| E2-119 | Het24.0 | H | H | H | H | CH₃ | O | SO₂ | 0.428 | 298.7 |
| E2-120 | Het1A | H | H | OCH₂CH₃ | H | CH₂CH₃ | O | O | 0.729 | 350.6 |
| E2-121 | Het1A | H | H | F | F | CH₂CH₂CH₃ | O | S | 1.055 | 371.7 |
| E2-122 | Het24.0 | H | H | F | F | CH₂CH₃ | O | S | 0.881 | 317.5 |
| E2-123 | Het24.0 | H | H | F | F | CH₂CH₂CH₃ | O | S | 0.967 | 309.6 |
| E2-124 | Het1A | H | H | F | F | CH₂CH₃ | O | S | 1,01 | 357,7 |
| E2-125 | Het1A | H | H | F | F | CH(CH₃)₂ | O | S | 1,068 | 371,7 |
| E2-126 | Het1A | H | H | F | F | CH₂CH₃ | O | SO | NA | NA |
| E2-127 | Het1A | H | H | F | F | CH₂CH₂CH₃ | O | SO | 0.855 | 388.6 |
| E2-128 | Het1A | H | H | SCH₂CH₃ | H | CH₂CH₃ | O | S | 0.878 | 382.6 |
| E2-129 | Het1A | H | H | SCH₃ | H | CH₃ | O | S | 0.767 | 354.5 |
| E2-130 | Het1A | H | H | O(4-Cl-C₆H₄) | H | 4-Cl-C₆H₄ | O | O | 1.194 | 516.6 |
| E2-131 | Het1A | H | H | F | F | CH₂CH₃ | O | SO₂ | 0.965 | 403.7 |
| E2-132 | Het1A | H | H | F | F | CH(CH₃)₂ | O | SO₂ | 0.965 | 404.3 |
| E2-133 | Het4.0 | H | H | H | H | CH₃ | O | S | 0.447 | 275.5 |
| E2-134 | Het27A | H | H | F | F | CH₃ | O | S | 0.763 | 313.3 |
| E2-135 | Het27A | H | H | F | F | CH₃ | O | SO₂ | 0.682 | 345.3 |
| E2-136 | Het24.0 | H | H | F | F | CH₂CH₃ | O | SO | 0.702 | 332.8 |
| E2-137 | Het24.0 | H | H | F | F | CH₂CH₂CH₃ | O | SO | 0.768 | 346.8 |
| E2-138 | Het4.0 | H | H | F | F | CH₃ | O | S | 0.729 | 311.5 |
| E2-139 | Het1A | H | H | H | H | CF2CF3 | O | SO | 0.935 | 427.7 |
| E2-140 | Het1A | H | H | SCH(CH₃)₂ | H | CH(CH₃)₂ | O | S | 1.146 | 432.7 |
| E2-141 | Het1C | H | H | F | F | CH₃ | O | S | 0.754 | 371.7 |
| E2-142 | Het4.0 | H | H | F | F | CH₃ | O | SO | 0.568 | 327.5 |
| E2-143 | Het23A | H | H | F | F | CH₃ | O | S | 0.882 | 314.3 |
| E2-144 | Het23A | H | H | F | F | CH₃ | O | SO₂ | 0.762 | 346.3 |
| E2-145 | Het1A | H | H | F | F | CH(CH₃)₂ | O | SO₂ | 0.941 | 403.7 |
| E2-146 | Het1A | H | H | F | F | CH(CH₃)₂ | O | SO | 1.053 | 371.8 |
| E2-147 | Het1A | H | H | F | F | 2-pyridyl | O | S | 0.905 | 406.7 |
| E2-148 | Het1A | H | H | F | F | (CH₂)₇CH₃ | O | S | 1.376 | 441.8 |
| E2-149 | Het1A | H | H | F | F | (CH₂)₆CH₃ | O | S | 1.316 | 427.8 |
| E2-150 | Het1A | H | H | F | F | C₆H₅ | O | S | 1.084 | 427.7 |
| E2-151 | Het1A | H | H | F | F | (CH₂)₉CH₃ | O | S | 1.488 | 469.9 |
| E2-152 | Het1A | H | H | F | F | 4-pyridyl | O | S | 0.721 | 406.7 |
| E2-153 | Het24.0 | H | H | F | F | CH₂CH₃ | O | SO₂ | NA | NA |
| E2-154 | Het1A | H | H | SCH₃ | H | CH₂CH₃ | O | O | 0.746 | 308.3 |
| E2-155 | Het1A | H | H | SCH₃ | H | CH(CH₃)₂ | O | O | 0.804 | 366.3 |
| E2-156 | Het1A | H | H | SCH₃ | H | C(CH₃)₃ | O | O | 0.842 | 380.4 |
| E2-157 | Het1A | H | H | F | F | CH₂CH₂OCH₂CH₃ | O | SO₂ | 0.942 | 433.8 |
| E2-158 | Het1A | H | H | F | F | CH₂CH₂OCH₃ | O | SO₂ | 0.875 | 419.7 |
| E2-159 | Het1A | H | H | F | F | C₆H₅ | O | SO₂ | 1.048 | 437.3 |
| E2-160 | Het1A | H | H | F | F | (CH₂)₇CH₃ | O | SO₂ | 1.324 | 473.5 |
| E2-161 | Het1A | H | H | F | F | (CH₂)₆CH₃ | O | SO₂ | 1.267 | 459.5 |
| E2-162 | Het1A | H | H | F | F | (CH₂)₉CH₃ | O | SO₂ | 1.439 | 501.5 |
| E2-163 | Het1A | H | H | SCH₂CH₃ | H | CH₃ | O | S | 0.835 | 368.3 |
| E2-164 | Het1A | H | H | SCH(CH₃)₂ | H | CH₃ | O | S | 0.898 | 382.3 |
| E2-165 | Het1A | H | H | SCH(CH₃)CH₂CH₃ | H | CH(CH₃)(CH₂CH₃) | O | S | 1.141 | 437.4 |
| E2-166 | Het1A | H | H | SCH₂CF₃ | H | CH₂CF₃ | O | S | 1.125 | 489.8 |
| E2-167 | Het1A | H | H | F | F | (4-methoxyphenyl)methyl | O | SO | 0.988 | 465.4 |
| E2-168 | Het1A | H | H | F | F | (2-fluorophenyl)methyl | O | SO | 1.003 | 453.4 |
| E2-169 | Het1A | H | H | F | F | (2,5-dichlorophenyl)methyl | O | SO | 1.155 | 505.1 |
| E2-170 | Het1A | H | H | F | F | (2,4-dichlorophenyl)methyl | O | SO | 1.195 | 505.1 |
| E2-171 | Het1A | H | H | F | F | (4-fluorophenyl)methyl | O | SO | 1.072 | 453.3 |
| E2-172 | Het1A | H | H | F | F | (CH₂)₃OC₆H₅ | O | SO | 1.055 | 479.4 |
| E2-173 | Het1A | H | H | F | F | CH₂CH(CH₃)₂ | O | SO | 1.016 | 401.4 |
| E2-174 | Het1A | H | H | F | F | CH₂CH₂CO₂CH₃ | O | SO | 0.908 | 431.3 |
| E2-175 | Het1A | H | H | F | F | cC₆H₁₁ | O | SO | 1.080 | 427.4 |
| E2-176 | Het1A | H | H | F | F | (4-chlorophenyl)methyl | O | SO | 1.126 | 469.3 |
| E2-177 | Het1A | H | H | F | F | (CH₂)₂C₆H₅ | O | SO | 1.075 | 449.3 |
| E2-178 | Het1A | H | H | F | F | CH₂CH₂CH(CH₃)₃ | O | SO | 1.074 | 415.4 |
| E2-179 | Het1A | H | H | F | F | CH₂CO₂CH(CH₃)₂ | O | SO | 1.027 | 445.4 |
| E2-180 | Het1A | H | H | F | F | 2-furylmethyl | O | SO | 0.978 | 425.3 |
| E2-181 | Het1A | H | H | F | F | CH₂CH(CH₃)CH₂CH₃ | O | SO | 1.018 | 415.4 |
| E2-182 | Het1A | H | H | F | F | CH₂CH₂CO₂CH₂CH₃ | O | SO | 0.917 | 445.3 |
| E2-183 | Het1A | H | H | F | F | CH₂CO₂(CH₂)₃CH₃ | O | SO | 1.049 | 459.4 |
| E2-184 | Het1A | H | H | F | F | CHCH₃CO₂CH₂CH₃ | O | SO | 0.960 | 445.4 |
| E2-185 | Het1A | H | H | F | F | (3-chlorophenyl)methyl | O | SO | 1.065 | 469.3 |
| E2-186 | Het1A | H | H | F | F | o-tolylmethyl | O | SO | 1.043 | 449.4 |
| E2-187 | Het1A | H | H | F | F | (2-chlorophenyl)methyl | O | SO | 1.044 | 469.3 |
| E2-188 | Het1A | H | H | F | F | (3,4-dichlorophenyl)methyl | O | SO | 1.127 | 505.1 |
| E2-189 | Het1A | H | H | F | F | 3-(trifluoromethyl)phenyl | O | SO | 1.157 | 489.4 |
| E2-190 | Het1A | H | H | F | F | 4-methoxyphenyl | O | SO | 1.038 | 451.3 |
| E2-191 | Het1A | H | H | F | F | CH₂(C₆H₅) | O | SO | 1.048 | 435.4 |
| E2-192 | Het1A | H | H | F | F | 3-chloro-4-fluoro-phenyl | O | SO | 1.158 | 473.3 |
| E2-193 | Het1A | H | H | F | F | 3,5-bis(trifluoromethyl)phenyl | O | SO | 1.303 | 557.4 |
| E2-194 | Het1A | H | H | F | F | CH₂CH₂OCH₂CH₃ | O | SO | 0.939 | 417.4 |
| E2-195 | Het1A | H | H | F | F | CH₂CF₃ | O | SO | 1.020 | 427.3 |
| E2-196 | Het1A | H | H | F | F | p-tolylmethyl | O | SO | 1.103 | 449.4 |
| E2-197 | Het1A | H | H | F | F | CH₂CH₂OCH₃ | O | SO | 0.869 | 403.3 |
| E2-198 | Het1A | H | H | F | F | (CH₂)₄OC₆H₅ | O | SO | 1.150 | 493.4 |
| E2-199 | Het1A | H | H | F | F | CH₂CH₂CH₂CH₃ | O | SO | 1.019 | 401.3 |
| E2-200 | Het1A | H | H | F | F | m-tolylmethyl | O | SO | 1.117 | 449.4 |
| E2-201 | Het1A | H | H | F | F | 4-(4-chlorophenyl)thiazol-2-yl | O | SO | 1.164 | 538.8 |
| E2-202 | Het1A | H | H | F | F | 2,5-dichlorophenyl | O | SO | 1.105 | 489.9 |
| E2-203 | Het1A | H | H | F | F | 3-Cl-C₆H₄ | O | SO | 1.028 | 457.6 |
| E2-204 | Het1A | H | H | F | F | 4-F-C₆H₄ | O | SO | 0.967 | 439.8 |
| E2-205 | Het1A | H | H | F | F | 2,6-dichlorophenyl | O | SO | 1.038 | 491.8 |
| E2-206 | Het1A | H | H | F | F | 2-Cl-C₆H₄ | O | SO | 1.017 | 457.6 |
| E2-207 | Het1A | H | H | F | F | 4-Cl-C₆H₄ | O | SO | 1.028 | 457.6 |
| E2-208 | Het1A | H | H | F | F | 5-(trifluoromethyl)-2-pyridyl | O | SO | 1.020 | 490.7 |
| E2-209 | Het1A | H | H | F | F | 4-methylthiazol-2-yl | O | SO | 0.925 | 442.7 |
| E2-210 | Het1A | H | H | F | F | C₆H₅ | O | SO | 0.939 | 421.8 |
| E2-211 | Het1A | H | H | F | F | (CH₂)₇CH₃ | O | SO | 1.191 | 457.8 |
| E2-212 | Het1A | H | H | F | F | (CH₂)₆CH₃ | O | SO | 1.129 | 443.9 |
| E2-213 | Het1A | H | H | F | F | (CH₂)₉CH₃ | O | SO | 1.313 | 485.9 |
| E2-214 | Het1A | H | H | CH₃ | H | 3,4-dichlorophenyl | O | O | 0.971 | 435.7 |
| E2-215 | Het1A | H | H | -CH₂-CH₂- | | CH₃ | O | S | 0.747 | 333.7 |
| E2-216 | Het1A | H | H | -CH₂-CH₂- | | CH₃ | O | SO₂ | 0.737 | 366.3 |
| E2-217 | Het1A | H | H | -CH₂-CH₂- | | H | O | O | 0.609 | 304.4 |

E3. Compound examples of formula 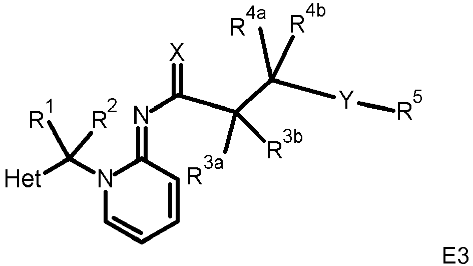 with their physico chemical data are given in table E3 herein below.

**Table E3:**

| **No.** | **Het** | **R¹** | **R²** | **R^{3a}** | **R^{3b}** | **R^{4a}** | **R^{4b}** | **R⁵** | **X** | **Y** | **RT [min]** | **m/z [MH]⁺** |
|---|---|---|---|---|---|---|---|---|---|---|---|---|
| E3-1 | Het1A | H | H | H | H | H | H | CH₃ | O | S | 0.730 | 322.1 |
| E3-2 | Het1A | H | H | H | H | H | H | CH₃ | O | SO₂ | 0.532 | 354.1 |
| E3-3 | Het1A | H | H | H | H | =O | | CH₂CH₃ | S | O | 0.839 | 372.1 |
| E3-4 | Het4.0 | H | H | H | H | H | H | CH₃ | O | S | 0.502 | 289.5 |
| E3-5 | Het1A | H | H | H | H | OCH₃ | H | CH₃ | O | O | 0.635 | 335.8 |
| E3-6 | Het1A | H | H | H | H | H | H | CH₃ | O | SO | 0.440 | 338.1 |
| E3-7 | Het1A | H | H | H | H | =O | | CH₂CH₃ | O | O | 0.689 | 334.1 |
| E3-8 | Het1A | CH₃ | H | H | H | =O | | CH₂CH₃ | O | O | NA | NA |
| E3-9 | Het1A | CH₃ | H | F | F | =O | | CH₃ | O | O | NA | NA |
| E3-10 | Het1A | H | H | H | H | =S | | CH₂CH₃ | O | O | 0.671 | 370.1 |

E4. Compound examples of formula 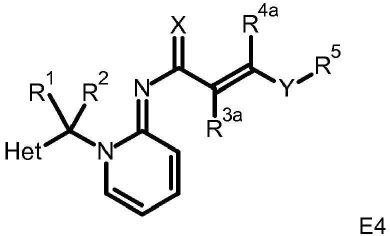 with their physico chemical data are given in table E4 herein below.

**Table E4:**

| **No.** | **Het** | **R¹** | **R²** | **R^{3a}** | **R^{4a}** | **R⁵** | **X** | **Y** | **RT [min]** | **m/z [MH]⁺** |
|---|---|---|---|---|---|---|---|---|---|---|
| E4-1 | Het1A | H | H | H | H | CH₂CH₃ | O | S | NA | NA |

E5. Compound examples of formula 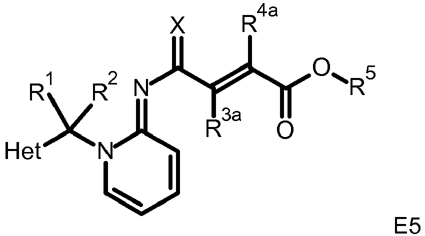 with their physico chemical data are given in table E5 herein below.

**Table E5:**

| **No.** | **Het** | **R¹** | **R²** | **R^{3a}** | **R^{4a}** | **R⁵** | **X** | **RT [min]** | **m/z [MH]⁺** |
|---|---|---|---|---|---|---|---|---|---|
| E5-1 | Het1A | H | H | H | H | CH₂CH₃ | O | 0.750 | 346.5 |

### B. Biological examples

The biological activity of the compounds of formula I of the present invention may be evaluated in biological tests as described in the following.

General conditions: If not otherwise specified, most test solutions are to be prepared as follows:The active compound is to be dissolved at the desired concentration in a mixture of 1:1 (vol:vol) distilled water:acteon. Further, the test solutions are to be prepared at the day of use (and, if not otherwised specified, in general at concentrations wt/vol).

### B.1 Green Peach Aphid (Myzus persicae)

The active compounds were formulated by a Tecan liquid handler in 100% cyclohexanone as a 10,000 ppm solution supplied in tubes. The 10,000 ppm solution was serially diluted in 100% cyclohexanone to make interim solutions. These served as stock solutions for which final dilutions were made by the Tecan in 50% acetone:50% water (v/v) into 5 or 10ml glass vials. A nonionic surfactant (Kinetic®) was included in the solution at a volume of 0.01 % (v/v). The vials were then inserted into an automated electrostatic sprayer equipped with an atomizing nozzle for application to plants/insects.

Bell pepper plants at the first true-leaf stage were infested prior to treatment by placing heavily infested leaves from the main colony on top of the treatment plants. Aphids were allowed to transfer overnight to accomplish an infestation of 30-50 aphids per plant and the host leaves were removed. The infested plants were then sprayed by an automated electrostatic plant sprayer equipped with an atomizing spray nozzle. The plants were dried in the sprayer fume hood, removed, and then maintained in a growth room under fluorescent lighting in a 24-hr photoperiod at about 25°C and about 20-40% relative humidity. Aphid mortality on the treated plants, relative to mortality on untreated control plants, was determined after 5 days.

In this test, compound E1-6, E1-7, E1-8, E1-9, E1-10, E2- 1, E2- 2, E2- 3, E2- 4, E2- 5, E2- 6, E2- 7, E2- 8, E2- 9, E2- 10, E2- 11, E2- 12, E2- 13, E2- 14, E2- 15, E2- 16, E2- 17, E2- 18, E2- 19, E2- 20, E2- 21, E2- 22, E2- 26, E2- 27, E2- 28, E2- 30, E2- 31, E2- 32, E2- 33, E2- 34, E2- 35, E2- 36, E2- 37, E2- 38, E2- 42, E2- 44, E2- 45, E2- 46, E2- 50, E2- 51, E2- 53, E2- 54, E2- 55, E2- 57, E2- 58, E2- 59, E2- 60, E2- 62, E2- 63, E2- 64, E2- 66, E2- 67, E2- 69, E2- 70, E2- 71, E2- 72, E2- 74, E2- 75, E2- 76, E2- 77, E2- 78, E2- 79, E2- 80, E2- 82, E2- 83, E2- 84, E2- 87, E2- 88, E2- 89, E2- 90, E2- 91, E2- 92, E2- 95, E2- 97, E2- 98, E2- 99, E2- 100, E2- 101, E2- 102, E2- 103, E2- 104, E2- 105, E2- 108, E2- 109, E2- 110, E2- 111, E2- 112, E2- 113, E2- 114, E2- 120, E2- 121, E2- 122, E2- 123, E2- 124, E2- 125, E2- 126, E2- 127, E2- 128, E2- 129, E2- 131, E2- 132, E2- 133, E2- 134, E2- 135, E2- 138, E2- 139, E2- 140, E2- 141, E2- 142, E2- 144, E2- 145, E2- 146, E2- 147, E2- 150, E2- 152, E2- 154, E2- 155, E2- 156, E2- 157, E2- 158, E2- 159, E2- 161, E2- 163, E2- 164, E2- 165, E2- 166, E2- 167, E2- 168, E2- 169, E2- 170, E2- 171, E2- 172, E2- 173, E2- 174, E2- 175, E2- 176, E2- 177, E2- 178, E2- 179, E2- 180, E2- 181, E2- 182, E2- 183, E2- 184, E2- 185, E2- 187, E2- 188, E2- 189, E2- 191, E2- 192, E2- 193, E2- 194, E2- 195, E2- 196, E2- 200, E2- 201, E2- 203, E2- 205, E2- 207, E2- 208, E2- 209, E2- 211, E3- 1, E3- 2, E3- 5, E3- 6, E3- 7, E3- 8, E3- 9, E4- 1, and E5- 1 at 2500 ppm showed at least 75 % mortality in comparison with untreated controls.

### B.2 Vetch aphid (Megoura viciae)

For evaluating control of vetch aphid (*Megoura viciae*) through contact or systemic means the test unit consisted of 24-well-microtiter plates containing broad bean leaf disks.
The compounds were formulated using a solution containing 75% v/v water and 25% v/v DMSO. Different concentrations of formulated compounds were sprayed onto the leaf disks at 2.5 µl, using a custom built micro atomizer, at two replications.

After application, the leaf disks were air-dried and 5 - 8 adult aphids were placed on the leaf disks inside the microtiter plate wells. The aphids were then allowed to suck on the treated leaf disks and were incubated at about 23 ± 1 °C and about 50 ± 5 % relative humidity for 5 days. Aphid mortality and fecundity was then visually assessed.

In this test, compound E1-6, E1-8, E1-9, E1-10, E2- 1, E2- 2, E2- 3, E2- 4, E2- 5, E2- 6, E2- 7, E2- 8, E2- 9, E2- 10, E2- 11, E2- 12, E2- 13, E2- 14, E2- 15, E2- 16, E2- 17, E2- 19, E2- 20, E2- 21, E2- 24, E2- 27, E2- 28, E2- 31, E2- 33, E2- 34, E2- 36, E2- 44, E2- 45, E2- 50, E2- 51, E2- 53, E2- 55, E2- 57, E2- 58, E2- 59, E2- 63, E2- 66, E2- 69, E2- 71, E2- 72, E2- 74, E2- 75, E2- 76, E2- 82, E2- 87, E2- 95, E2- 98, E2- 99, E2- 104, E2- 105, E2- 109, E2- 110, E2- 111, E2- 112, E2- 114, E2- 120, E2- 121, E2- 124, E2- 125, E2- 126, E2- 127, E2- 128, E2- 129, E2- 131, E2- 132, E2- 135, E2- 138, E2- 139, E2- 140, E2- 141, E2- 142, E2- 145, E2- 146, E2- 147, E2- 150, E2- 152, E2- 154, E2- 155, E2- 156, E2- 157, E2- 158, E2- 159, E2- 163, E2- 164, E2- 165, E2- 166, E2- 167, E2- 168, E2- 169, E2- 170, E2- 171, E2- 172, E2- 173, E2- 174, E2- 176, E2- 177, E2- 178, E2- 179, E2- 180, E2- 181, E2- 182, E2- 183, E2- 184, E2- 185, E2- 187, E2- 188, E2- 189, E2- 191, E2- 192, E2- 194, E2- 195, E2- 200, E2- 203, E2- 205, E2- 207, E2- 208, E2- 209, E2- 211, E3- 1, E3- 2, E3- 5, E3- 6, E3- 8, E3- 9, E4- 1, and E5- 1 at 2500 ppm showed at least 75 % mortality in comparison with untreated controls.

### B.3 Cotton aphid (Aphis gossypii)

The active compounds were formulated by a Tecan liquid handler in 100% cyclohexanone as a 10,000 ppm solution supplied in tubes. The 10,000 ppm solution was serially diluted in 100% cyclohexanone to make interim solutions. These served as stock solutions for which final dilutions were made by the Tecan in 50% acetone:50% water (v/v) into 5 or 10ml glass vials. A nonionic surfactant (Kinetic®) was included in the solution at a volume of 0.01 % (v/v). The vials were then inserted into an automated electrostatic sprayer equipped with an atomizing nozzle for application to plants/insects.

Cotton plants at the cotyledon stage were infested with aphids prior to treatment by placing a heavily infested leaf from the main aphid colony on top of each cotyledon. Aphids were allowed to transfer overnight to accomplish an infestation of 80-100 aphids per plant and the host leaf was removed. The infested plants were then sprayed by an automated electrostatic plant sprayer equipped with an atomizing spray nozzle. The plants were dried in the sprayer fume hood, removed from the sprayer, and then maintained in a growth room under fluorescent lighting in a 24-hr photoperiod at 25°C and 20-40% relative humidity. Aphid mortality on the treated plants, relative to mortality on untreated control plants, was determined after 5 days.

In this test, compound E1-8, E2- 4, E2- 14, E2- 139, E2- 154, E2- 155, E2- 159, and E2- 184 at 500 ppm showed at least 75 % mortality in comparison with untreated controls.

### B.4 Cowpea aphid (Aphis craccivora)

The active compound was dissolved at the desired concentration in a mixture of 1:1 (vol:vol) distilled water: acetone. Surfactant (Alkamuls® EL 620) was added at a rate of 0.1% (vol/vol). The test solution was prepared at the day of use.

Potted cowpea plants were colonized with approximately 50 - 100 aphids of various stages by manually transferring a leaf tissue cut from infested plant 24 hours before application. Plants were sprayed after the pest population has been recorded. Treated plants we are maintained on light carts at about 28°C. Percent mortality was assessed after 72 hours.

In this test, compounds E1-6, E1-8, E1-9, E1-10, E2- 2, E2- 3, E2- 4, E2- 5, E2- 6, E2- 7, E2- 8, E2- 9, E2- 12, E2- 13, E2- 14, E2- 15, E2- 16, E2- 17, E2- 19, E2- 20, E2- 21, E2- 26, E2- 28, E2- 34, E2- 51, E2- 57, E2- 58, E2- 59, E2- 61, E2- 66, E2- 71, E2- 74, E2- 75, E2- 76, E2- 82, E2- 95, E2- 98, E2- 99, E2- 101, E2- 105, E2- 107, E2- 111, E2- 112, E2- 113, E2- 114, E2- 115, E2- 120, E2- 124, E2- 125, E2- 126, E2- 127, E2- 128, E2- 129, E2- 131, E2- 133, E2- 138, E2- 139, E2- 141, E2- 142, E3- 1, E3- 2, E3- 5, E3- 6, E3- 7, E3- 8, E3- 9, E4- 1, and E5- 1 at 500 ppm showed at least 75 % mortality in comparison with untreated controls.

### B.5 Silverleaf whitefly (bemisia argentifolii)

The active compounds were formulated by a Tecan liquid handler in 100% cyclohexanone as a 10,000 ppm solution supplied in tubes. The 10,000 ppm solution was serially diluted in 100% cyclohexanone to make interim solutions. These served as stock solutions for which final dilutions were made by the Tecan in 50% acetone:50% water (v/v) into 5 or 10ml glass vials. A nonionic surfactant (Kinetic®) was included in the solution at a volume of 0.01 % (v/v). The vials were then inserted into an automated electrostatic sprayer equipped with an atomizing nozzle for application to plants/insects.

Cotton plants at the cotyledon stage (one plant per pot) were sprayed by an automated electrostatic plant sprayer equipped with an atomizing spray nozzle. The plants were dried in the sprayer fume hood and then removed from the sprayer. Each pot was placed into a plastic cup and about 10 to 12 whitefly adults (approximately 3-5 days old) were introduced. The insects were collected using an aspirator and a nontoxic Tygon® tubing connected to a barrier pipette tip. The tip, containing the collected insects, was then gently inserted into the soil containing the treated plant, allowing insects to crawl out of the tip to reach the foliage for feeding. Cups were covered with a reusable screened lid. Test plants were maintained in a growth room at about 25°C and about 20-40% relative humidity for 3 days, avoiding direct exposure to fluorescent light (24 hour photoperiod) to prevent trapping of heat inside the cup. Mortality was assessed 3 days after treatment, compared to untreated control plants.

In this test, compound E1-8, E2- 4, E2- 5, E2- 9, E2- 14, E2- 139, E2- 143, E2- 147, E2- 155, and E2- 189 at 500 ppm showed at least 75 % mortality in comparison with untreated controls.

### B.6 Orchid thrips (dichromothrips corbetti)

*Dichromothrips corbetti* adults used for bioassay were obtained from a colony maintained continuously under laboratory conditions. For testing purposes, the test compound is diluted in a 1:1 mixture of acetone:water (vol:vol), plus 0.01 % vol/vol Alkamuls^{®} EL 620 surfactant.

Thrips potency of each compound was evaluated by using a floral-immersion technique. Plastic petri dishes were used as test arenas. All petals of individual, intact orchid flowers were dipped into treatment solution and allowed to dry. Treated flowers were placed into individual petri dishes along with about 20 adult thrips. The petri dishes were then covered with lids. All test arenas were held under continuous light and a temperature of about 28°C for duration of the assay. After 3 days, the numbers of live thrips were counted on each flower, and along inner walls of each petri dish. The percentmortality was recorded 72 hours after treatment.

In this test, compounds E1-8, E1-10, E2- 2, E2- 4, E2- 5, E2- 6, E2- 7, E2- 9, E2- 11, E2- 12, E2- 14, E2- 15, E2- 16, E2- 17, E2- 19, E2- 20, E2- 21, E2- 25, E2- 28, E2- 32, E2- 35, E2- 45, E2- 46, E2- 47, E2- 48, E2- 50, E2- 51, E2- 56, E2- 58, E2- 59, E2- 61, E2- 64, E2- 66, E2- 71, E2- 72, E2- 74, E2- 75, E2- 76, E2- 77, E2- 79, E2- 80, E2- 82, E2- 86, E2- 91, E2- 95, E2- 98, E2- 99, E2- 107, E2- 113, E2- 114, E2- 117, E2- 120, E2- 124, E2- 126, E2- 128, E2- 129, E2- 131, E2- 133, E2- 138, E2- 139, E2- 140, E3- 5, E3- 6, E3- 9, E4- 1, and E5- 1 at 500 ppm showed at least 75 % mortality in comparison with untreated controls.

### B.7 Rice green leafhopper (Nephotettix virescens)

Rice seedlings were cleaned and washed 24 hours before spraying. The active compounds were formulated in 50:50 acetone:water (vol:vol), and 0.1 % vol/vol surfactant (EL 620) was added. Potted rice seedlings were sprayed with 5 ml test solution, air dried, placed in cages and inoculated with 10 adults. Treated rice plants were kept at about 28-29°C and relative humidity of about 50-60%. Percent mortality was recorded after 72 hours.

In this test, compounds E1-6, E1-8, E1-9, E1-10, E2- 3, E2- 4, E2- 5, E2- 6, E2- 7, E2- 8, E2- 9, E2- 10, E2- 11, E2- 12, E2- 14, E2- 15, E2- 16, E2- 17, E2- 19, E2- 20, E2- 21, E2- 28, E2- 34, E2- 35, E2- 45, E2- 50, E2- 51, E2- 53, E2- 56, E2- 57, E2- 58, E2- 59, E2- 71, E2- 74, E2- 75, E2- 76, E2- 82, E2- 98, E2- 112, E2- 114, E2- 116, E2- 117, E2- 118, E2- 120, E2- 121, E2- 124, E2- 125, E2- 126, E2- 127, E2- 128, E2- 129, E2- 131, E2- 132, E2- 134, E2- 135, E2- 136, E2- 138, E2- 139, E2- 141, E2- 142, E2- 144, E3- 2, E3- 5, E3- 6, E3- 9, and E4- 1 at 500 ppm showed at least 75 % mortality in comparison with untreated controls.

### B.8 Rice brown plant hopper (Nilaparvata lugens)

Rice seedlings were cleaned and washed 24 hours before spraying. The active compounds was formulated in 50:50 acetone:water (vol:vol) and 0.1 % vol/vol surfactant (EL 620) was added. Potted rice seedlings were sprayed with 5 ml test solution, air dried, placed in cages and inoculated with 10 adults. Treated rice plants were kept at about 28-29°C and relative humidity of about 50-60%. Percent mortality was recorded after 72 hours.

In this test, compounds E1-6, E1-9, E1-10, E2- 3, E2- 4, E2- 5, E2- 6, E2- 7, E2- 8, E2- 9, E2- 12, E2- 13, E2- 14, E2- 15, E2- 17, E2- 19, E2- 20, E2- 21, E2- 28, E2- 34, E2- 35, E2- 45, E2- 50, E2- 51, E2- 56, E2- 57, E2- 58, E2- 59, E2- 71, E2- 74, E2- 75, E2- 76, E2- 98, E2- 114, E2- 120, E2- 126, E2- 129, E2- 131, E2- 133, E2- 135, E2- 139, E2- 142, E3- 5, E3- 6, and E4- 1 at 500 ppm showed at least 75 % mortality in comparison with untreated controls.

### B.9 Boll weevil (Anthonomus grandis)

For evaluating control of boll weevil (*Anthonomus grandis*) the test unit consisted of 96-well-microtiter plates containing an insect diet and 5-10 *A. grandis* eggs.

The compounds were formulated using a solution containing 75% v/v water and 25% v/v DMSO. Different concentrations of formulated compounds were sprayed onto the insect diet at 5 µl, using a custom built micro atomizer, at two replications.

After application, microtiter plates were incubated at about 25 ± 1°C and about 75 ± 5 % relative humidity for 5 days. Egg and larval mortality was then visually assessed.

In this test, compounds E2- 2, E2- 4, E2- 6, E2- 7, E2- 14, E2- 15, E2- 17, E2- 33, E2- 39, E2- 49, E2- 53, E2- 57, E2- 66, E2- 74, E2- 82, E2- 84, E2- 99, E2- 100, E2- 110, E2- 112, E2- 113, E2- 116, E2- 122, E2- 123, E2- 126, E2- 132, E2- 154, E2- 159, E2- 180, E2- 189, and E2- 195 at 500 ppm showed at least 75 % mortality in comparison with untreated controls.

### B.10 Mediterranean fruitfly (Ceratitis capitata)

For evaluating control of Mediterranean fruitfly (*Ceratitis capitata*) the test unit consisted of microtiter plates containing an insect diet and 50-80 *C. capitata* eggs.

The compounds were formulated using a solution containing 75% v/v water and 25% v/v DMSO. Different concentrations of formulated compounds were sprayed onto the insect diet at 5 µl, using a custom built micro atomizer, at two replications.

After application, microtiter plates were incubated at about 28 ± 1 °C and about 80 ± 5 % relative humidity for 5 days. Egg and larval mortality was then visually assessed.

In this test, compounds E2- 3, E2- 4, E2- 7, E2- 12, E2- 15, E2- 17, E2- 35, E2- 39, E2- 46, E2- 63, E2- 66, E2- 69, E2- 72, E2- 74, E2- 76, E2- 78, E2- 92, E2- 95, E2- 97, E2- 99, E2- 100, E2- 105, E2- 107, E2- 108, E2- 109, E2- 110, E2- 121, E2- 140, E2- 159, and E3- 6 at 2500 ppm showed at least 75 % mortality in comparison with untreated controls.

### B.11 Tobacco budworm (Heliothis virescens)

For evaluating control of tobacco budworm (Heliothis virescens) the test unit consisted of 96-well-microtiter plates containing an insect diet and 15-25 H. virescens eggs.

The compounds were formulated using a solution containing 75% v/v water and 25% v/v DMSO. Different concentrations of formulated compounds were sprayed onto the insect diet at 10 µl, using a custom built micro atomizer, at two replications.

After application, microtiter plates were incubated at about 28 + 1°C and about 80 + 5 % relative humidity for 5 days. Egg and larval mortality was then visually assessed.

In this test, compounds E2- 2, E2- 4, E2- 13, E2- 14, E2- 15, E2- 26, E2- 44, E2- 49, E2- 51, E2- 66, E2- 105, E2- 109, E2- 110, E2- 113, E2- 116, E2- 132, E2- 153, E2- 155, E2- 156, E2- 159, E2- 193, E3- 1, and E3- 2, at 2500 ppm showed at least 75 % mortality in comparison with untreated controls.

### B.12 Diamond back moth (Plutella xylostella)

The active compound is dissolved at the desired concentration in a mixture of 1:1 (vol:vol) distilled water: aceteone. Surfactant (Alkamuls® EL 620) is added at a rate of 0.1 % (vol/vol).The test solution is prepared at the day of use.

Leaves of cabbage were dipped in test solution and air-dried. Treated leaves were placed in petri dish eslined with moist filter paper and inoculated with ten 3rd instar lar-vae. Mortality was recorded 72 hours after treatment. Feeding damages were also rec-orded using a scale of 0-100%.

In this test, compounds E2- 5, E2- 6, E2- 9, E2- 19, E2- 44, E2-49, E2- 51, E2- 58, E2- 59, E2- 113, E2- 124, and E2- 145 at 500 ppm showed at least 75 % mortality in comparison with untreated controls.

## Claims

1. N-substituted pyridylidene carbonyl compounds of formula (I): 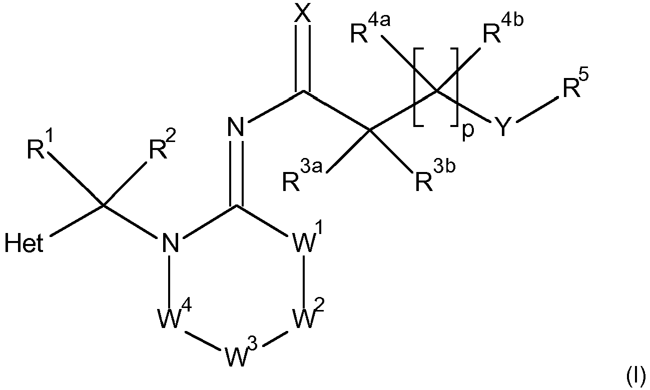 wherein
p is an integer selected from 0, 1, 2, 3, 4, 5 or 6;
X is O or S;
Y is O or S(O)ₘ, wherein m is 0, 1, 2;
Het is a 5 or 6 membered carbon-bound or optionally nitrogen-bound heterocyclic or heteroaromatic ring system, each ring members selected from carbon atoms and at least one, up to three heteroatoms independently selected from sulfur, oxygen or nitrogen, wherein the carbon, sulfur and nitrogen ring members can independently be partly or fully oxidized, and wherein each ring is optionally substituted by k substituents selected from R^{6a}, wherein k is an integer selected from 1, 2, 3, 4, or 5, and two or more substituents R^{6a} are selected independently from one another;
W¹, W², W³ and W⁴ represent a carbon chain group connected to N and C=N, and thus forming a saturated, unsaturated, or partially unsaturated 5 or 6 membered nitrogen containing heterocycle,
wherein W¹, W², W³ and W⁴ each individually represent CR^{w}, wherein each R^{w} is selected independently from one another from hydrogen, halogen, cyano, azido, nitro, SCN, SF₅, C₁-C₁₀-alkyl, C₃-C₈-cycloalkyl, C₂-C₁₀-alkenyl, C₂-C₁₀-alkinyl, and wherein the carbon atoms of the aforementioned aliphatic and cyclo-aliphatic radicals may be unsubstituted or may be partly or fully halogenated or may optionally be further substituted independently from one another with one or more R⁷;
OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=S)OR⁸, C(=S)SR⁸, C(=NR^{9a})R⁷, C(=NR^{9a})NR^{9a}R^{9b}, Si(R¹¹)₂R¹²;
phenyl, optionally substituted with with 1, 2, 3, 4 or 5 substituents selected independently from R¹⁰;
a 3-, 4-, 5-, 6- or 7- membered saturated, partly saturated or unsaturated aromatic heterocyclic ring comprising 1, 2, 3 or 4 heteroatoms selected from oxygen, nitrogen and/or sulfur, optionally substituted with 1, 2, 3, 4 or 5 substituents selected independently from R¹⁰, and wherein the nitrogen and/or the sulfur atom(s) of the heterocyclic ring may optionally be oxidized;
or
two of R^{w} present on one ring carbon atom may together form =O, =CR¹³R¹⁴, =S =NR^{17a}, =NOR¹⁶;=NNR^{17a};
or
two R^{w} of adjacent carbon atoms, may form both together and together with the existing bond a double bond between the adjacent carbon atoms;
and wherein
one of W² or W³ may optionally represent a single or a double bond between the adjacent carbon atoms;
R¹, R² are independently from each other selected from the group consisting of hydrogen, halogen, CN, SCN, nitro C₁-C₆-alkyl, C₃-C₆-cycloalkyl, wherein each of the aforementioned radicals are unsubstituted, partly or completely halogenated or may carry any combination of one or more radicals R⁷; Si(R¹¹)₂R¹², OR¹⁶, OSO₂R¹⁶, S(O)ₙR¹⁶, S(O)ₙNR^{17a}R^{17b}, NR^{17a}R^{17b}, C(=O)NR^{17a}R^{17b}, C(=S)NR^{17a}R^{17b}, C(=O)OR¹⁶, C(=O)R¹⁵, C(=S)R¹⁵; phenyl, optionally substituted with one or more, e.g. 1, 2, 3, 4 or 5 substituents R¹⁰, which are independently selected from one another, a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with 1, 2, 3 or 4, substituents R¹⁰, selected independently from one another, and wherein the nitrogen and/or the sulfur atom(s) of the heterocyclic ring may optionally be oxidized,
or
R¹ and R² form, together with the carbon atom, which they attached to, a 3- to 6-membered saturated or partly unsaturated carbocyclic or heterocyclic ring, wherein each of the carbon atoms of said cycle are unsubstituted or may carry any combination of 1 or 2 radicals R⁷,
or
R¹ and R² may together be =O, =CR¹³R¹⁴; =S;;, =NR^{17a}, =NOR¹⁶;=NNR^{17a};
R^{3a}, R^{3b} are selected each independently from one another from the group consisting of hydrogen, halogen, CN, C₁-C₆-alkyl, C₁-C₆-alkoxy, C₁-C₆-alkylthio, C₁-C₆-alkylsulfinyl, C₁-C₆-alkylsulfonyl and C₃-C₈-cycloalkyl, wherein each of the six last mentioned radicals are unsubstituted, partly or completely halogenated, OR⁸, OSO₂R⁸, S(O),R⁸, S(O)ₙNR^{9a}R^{9b}, NR^{9a}R^{9b}, C(=O)NR^{9a}R^{9b}, C(=S)NR^{9a}R^{9b}, C(=O)R⁷, C(=S)R⁷,
phenyl, optionally substituted with one or more, e.g. 1, 2, 3, 4 or 5 substituents R¹⁰, which are independently selected from one another,
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with 1, 2, 3 or 4, substituents R¹⁰, selected independently from one another, and wherein the nitrogen and/or the sulfur atom(s) of the heterocyclic ring may optionally be oxidized,
or
wherein R^{3a} and R^{3b} may form together with the carbon atom they are bound to, a 3, 4 or 5 membered aliphatic ring, wherein each of the carbon atoms of the ring may be unsubstituted or may be partly or fully halogenated,
or
wherein R^{3a} and R^{3b} may together form =O, =CR¹³R¹⁴; =S, =NR^{17a}, =NOR¹⁶;=NNR^{17a},
and, if p is 1 or more, then one of R^{3a} or R^{3b} may form a double bond with the R^{4a} or R^{4b} of the adjacent carbon atom
R^{4a}, R^{4b} are selected each independently from one another and independently from the integer of p from the group consisting of hydrogen, halogen, CN, C₁-C₆-alkyl, C₁-C₆-alkoxy, C₁-C₆-alkylthio, C₁-C₆-alkylsulfinyl, C₁-C₆-alkylsulfonyl and C₃-C₈-cycloalkyl, wherein each of the six last mentioned radicals are unsubstituted, partly or completely halogenated,
or
wherein R^{4a} and R^{4b} may form together with the carbon atom they are bound to, a 3, 4 or 5 membered aliphatic carbocyclic ring, wherein each of the carbon atoms of the ring may be unsubstituted or may be partly or fully halogenated,
or
wherein R^{4a} and R^{4b} may together form =O, =CR¹³R¹⁴; =S, =NR^{17a}, =NOR¹⁶;=NNR^{17a},
or, if p is 1 or more,
one of R^{4a} or R^{4b} may for a double bond with R^{3a} or R^{3b} or with another R^{4a}, or R^{4b} of the adjacent carbon atom(s).
R⁵ is selected from the group consisting of hydrogen, cyano, C₁-C₆-alkyl, C₇-C₁₂-alkyl, C₁-C₆-alkylthio, C₁-C₆-alkylsulfinyl, C₁-C₆-alkylsulfonyl, C₃-C₈-cycloalkyl, C₃-C₈-cycloalkylthio, C₃-C₈-cycloalkylsufinyl, C₃-C₈-cycloalkylsulfonyl, C₂-C₆-alkenyl, C₂-C₆-alkenylthio, C₂-C₆-alkenylsulfinyl, C₂-C₆-alkenylsulfonyl, C₂-C₆-alkinyl, C₂-C₆-alkinylthio, C₂-C₆-alkinylsulfinyl and C₂-C₆-alkinylsulfonyl wherein the carbon chain atoms of the aforementioned aliphatic and cycloaliphatic last radicals are unsubstituted, partly or completely halogenated or may carry any combination of one or more radicals R⁷;
C(=O)NR^{9a}R^{9b} C(=S)NR^{9a}R^{9b}, C(=O)R⁷, C(=S)R⁷;
phenyl or CH₂-phenyl, optionally substituted with one or more, e.g. 1, 2, 3, 4 or 5 substituents R¹⁰, which are independently selected from one another;
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with q substituents R^{y}, selected independently from q and independently from one another, and wherein the nitrogen and/or the sulfur atom(s) of the heterocyclic ring may optionally be oxidized, wherein the heterocyclic ring may be bonded directly or linked via a CH₂ group to the remainder of the molecule, and wherein
q is an integer selected from 1, 2, 3 or 4, and
R^{y} is selected from the group conisisting of hydrogen, halogen, cyano, , nitro, C₁-C₁₀-alkyl, C₂-C₁₀-alkenyl, wherein the carbon atoms of the aforementioned aliphatic radicals may optionally be substituted with one or more R¹⁵, which are selected independently from one another,
OR¹⁶, -S(O)ₙR¹⁶, S(O)ₙNR^{17a}R^{17b}, NR^{17a}R^{17b} C(=O)R¹⁵, C(=O)OR¹⁶,-C(=NR^{17a})R¹⁵, C(=O)NR^{17a}R^{17b}, C(=S)NR^{17a}R^{17b},
or
two R^{y} present together on one atom of a partly saturated heterocyclic may be =O, =CR¹³R¹⁴, =NR^{17a}, =NOR¹⁶ or =NNR^{17a};
or
two R^{y} on adjacent carbon atoms may be a bridge selected from CH₂CH₂CH₂CH₂, CH=CH-CH=CH, N=CH-CH=CH, CH=N-CH=CH, N=CH-N=CH, CH₂CH₂CH₂, CH=CHCH₂, CH₂CH₂NR^{17a}, CH₂CH=N, CH=CH-NR^{17a}, and form together with the carbon atoms to which the two R^{y} are bonded to a 5-membered or 6-membered fused partly saturated or unsaturated, aromatic carbocyclic or heteocyclic ring, wherein the ring may optionally be substituted with one or two substituents selected from =O, OH, CH₃, OCH₃, halogen, cyano, halomethyl or halomethoxy;
R^{6a} is selected from the group consisting of hydrogen, halogen, cyano, azido, nitro, SCN, SF₅, C₁-C₁₀-alkyl, C₃-C₈-cycloalkyl, C₂-C₁₀-alkenyl, C₂-C₁₀-alkinyl, and wherein the carbon atoms of the aforementioned aliphatic and cyclo-aliphatic radicals may optionally be further substituted independently from one another with one or more R⁷,
OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=S)OR⁸, C(=S)SR⁸, C(=NR^{9a})R⁷, C(=NR^{9a})NR^{9a}R^{9b}, Si(R¹¹)₂R¹²;
phenyl, optionally substituted with with 1, 2, 3, 4 or 5 substituents selected independently from R¹⁰;
a 3-, 4-, 5-, 6- or 7- membered saturated, partly saturated or unsaturated aromatic heterocyclic ring comprising 1, 2, 3 or 4 heteroatoms selected from oxygen, nitrogen and/or sulfur, optionally substituted with 1, 2, 3, 4 or 5 substituents selected independently from R¹⁰, and wherein the nitrogen and/or the sulfur atom(s) of the heterocyclic ring may optionally be oxidized;
or two of R^{6a} present on one ring carbon or sulfur atom may together form =O, =CR¹³R¹⁴; =S;;, =NR^{17a}, =NOR¹⁶;=NNR^{17a};
or two R^{6a} together form a C₂-C₇ alkylene chain, thus forming, together with the ring atom(s) to which they are bound, a 3-, 4-, 5-, 6-, 7- or 8-membered ring, where the alkylene chain may be interrupted by 1 or 2 O, S and/or NR^{17a} and/or 1 or 2 of the CH₂ groups of the alkylene chain may be replaced by a group C=O, C=S and/or C=NR^{17a}; and/or the alkylene chain may be substituted by one or more radicals selected from the group consisting of halogen, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, phenyl which may be substituted by one or more, e.g. 1, 2, 3, 4 or 5 radicals R¹⁰, and a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, where the heterocyclic ring may be substituted by one or more radicals R¹⁰;
R⁷ is each independently from one another selected from the group consisting of hydrogen, halogen, cyano, azido, nitro, -SCN, SF₅, C₁-C₆-alkyl, C₁-C₆-haloalkyl, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkinyl, C₂-C₆ haloalkinyl, Si(R¹¹)₂R¹², OR¹⁶, OSO₂R¹⁶, S(O)ₙR¹⁶, S(O)ₙNR^{17a}R^{17b}, NR^{17a}R^{17b}, C(=O)NR^{17a}R^{17b}, C(=S)NR^{17a}R^{17b}, C(=O)OR¹⁶, C(=O)R¹⁵, C(₌S)R¹⁵, C(=NR^{17a})R¹⁵,
phenyl, optionally substituted with 1, 2, 3, 4 or 5 substituents R¹⁰, which are independently selected from one another,
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with 1, 2, 3 or 4 substituents R¹⁰, selected independently from one another, and wherein the nitrogen and/or the sulfur atom(s) of the heterocyclic ring may optionally be oxidized,
or
two R⁷ present on one carbon atom may together form =O, =CR¹³R¹⁴; =S;;, =NR^{17a}, =NOR¹⁶;=NNR^{17a};
or
two R⁷ may form a 3-, 4-, 5-, 6-, 7- or 8-membered saturated or partly unsaturated carbocyclic or heterocyclic ring together with the carbon atoms to which the two R⁷ are bonded to;
R⁸ is each independently from one another selected from the group consisting of hydrogen, cyano, C₁-C₆-alkyl, C₁-C₆-haloalkyl, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, C₃-C₈-cycloalkyl, C₄-C₈-alkylcycloalkyl, C₃-C₈-halocycloalkyl, C₂-C₆-alkenyl, C₂-C₆-haloalkenyl, C₂-C₆-alkinyl, C₂-C₆ haloalkinyl, -Si(R¹¹)₂R¹², S(O)ₙR¹⁶, S(O)ₙNR^{17a}R^{17b}, NR^{17a}R^{17b}, -N=CR¹³R¹⁴, -C(=O)R¹⁵, C(=O)NR^{17a}R^{17b}, C(=S)NR^{17a}R^{17b}, C(=O)OR¹⁶, phenyl, optionally substituted with one or more substituents R¹⁰; which are selected independently from one another,
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with 1, 2, 3 or 4 substituents R¹⁰, selected independently from one another, and wherein the nitrogen and/or the sulfur atom(s) of the heterocyclic ring may optionally be oxidized;
R^{9a}, R^{9b} are each independently from one another selected from the group consisting of hydrogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkinyl, C₂-C₆ haloalkinyl, S(O)ₙR¹⁶, -S(O)ₙNR^{17a}R^{17b}, C(=O)R¹⁵, C(=O)OR¹⁶, C(=O)NR^{17a}R^{17b}, C(=S)R¹⁵, C(=S)SR¹⁶, C(=S)NR^{17a}R^{17b}, C(=NR^{17a})R¹⁵; phenyl, optionally substituted with one or more, e.g. 1, 2, 3 or 4, substituents R¹⁰, which are selected independently from one another; a 3-, 4-, 5-, 6- or 7- membered saturated, partly saturated or unsaturated aromatic heterocyclic ring comprising 1, 2, 3 or 4 heteroatoms selected from oxygen, nitrogen and/or sulfur, optionally substituted with 1, 2, 3 or 4 substituents R¹⁰, selected independently from one another, and wherein the nitrogen and/or the sulfur atom(s) of the heterocyclic ring may optionally be oxidized;
or,
R^{9a} and R^{9b} are together a C₂-C₇ alkylene chain and form a 3-, 4-, 5-, 6-, 7- or 8-membered saturated, partly saturated or unsaturated aromatic ring together with the nitrogen atom they are bonded to, wherein the alkylene chain may contain one or two heteratoms selected from oxygen, sulfur or nitrogen, and may optionally be substituted with halogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkinyl, C₂-C₆ haloalkinyl,
phenyl, optionally substituted with one or more substituents R¹⁰; which are selected independently from one another, a 3-, 4-, 5-, 6,- or 7-membered saturated, partly saturated or unsaturated aromatic heterocyclic ring comprising 1, 2 or 3 heteroatoms selected from oxygen, nitrogen and/or sulfur, optionally substituted with one or more substituents R¹⁰, selected independently from one another, and wherein the nitrogen and/or the sulfur atom(s) of the heterocyclic ring may optionally be oxidized;
or
R^{9a} and R^{9b} together may form a =CR¹³R¹⁴, =NR¹⁷ or =NOR¹⁶ radical;
R¹⁰ is each independently from one another selected from the group consisting of hydrogen, halogen, cyano, azido, nitro, SCN, SF₅, C₁-C₁₀-alkyl, C₃-C₈-cycloalkyl, C₂-C₁₀-alkenyl, C₂-C₁₀-alkinyl, wherein the carbon atoms of the aforementioned aliphatic and cyclo-aliphatic radicals may optionally be substituted with one or more R¹⁵, which are selected independently from one another, Si(R¹¹)₂R¹², OR¹⁶, OS(O)ₙR¹⁶, -S(O)ₙR¹⁶, S(O)ₙNR^{17a}R^{17b}, NR^{17a}R^{17b}, C(=O)R¹⁵, C(=S)R¹⁵, C(=O)OR¹⁶, -C(=NR^{17a})R¹⁵, C(=O)NR^{17a}R^{17b}, C(=S)NR^{17a}R^{17b}, phenyl, optionally substituted with halogen, cyano, nitro, C₁-C₆-alkyl, C₁-C₆-haloalkyl, C₁-C₆-alkoxy or C₁-C₆-haloalkoxy,
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with one or more substituents selected independently from one another from halogen, cyano, NO₂, C₁-C₆-alkyl, C₁-C₆-haloalkyl, C₁-C₆-alkoxy or C₁-C₆-haloalkoxy, and wherein the nitrogen and/or the sulfur atom(s) of the heterocyclic ring may optionally be oxidized;
or
two R¹⁰ present together on one atom of a partly saturated heterocyclic may be =O, =CR¹³R¹⁴ =NR^{17a}, =NOR¹⁶ or =NNR^{17a};
or,
two R¹⁰ on adjacent carbon atoms may be a bridge selected from CH₂CH₂CH₂CH₂, CH=CH-CH=CH, N=CH-CH=CH, CH=N-CH=CH, N=CH-N=CH, OCH₂CH₂CH₂, OCH=CHCH₂, CH₂OCH₂CH₂, OCH₂CH₂O, OCH₂OCH₂, CH₂CH₂CH₂, CH=CHCH₂, CH₂CH₂O, CH=CHO, CH₂OCH₂, CH₂C(=O)O, C(=O)OCH₂, O(CH₂)O, SCH₂CH₂CH₂, SCH=CHCH₂, CH₂SCH₂CH₂, SCH₂CH₂S, SCH₂SCH₂, CH₂CH₂S, CH=CHS, CH₂SCH₂, CH₂C(=S)S, C(=S)SCH₂, S(CH₂)S, CH₂CH₂NR^{17a}, CH₂CH=N, CH=CH-NR^{17a}, OCH=N, SCH=N and form together with the carbon atoms to which the two R¹⁰ are bonded to a 5-membered or 6-membered partly saturated or unsaturated, aromatic carbocyclic or heteocyclic ring, wherein the ring may optionally be substituted with one or two substituents selected from =O, OH, CH₃, OCH₃, halogen, cyano, halomethyl or halomethoxy;
R¹¹, R¹² (are each independently from one another selected from the group consisting of hydrogen, halogen, C₁-C₆ alkyl, C₁-C₆ haloalkyl, C₁-C₆ alkoxy, C₁-C₆ alkoxy-alkyl, C₂-C₆ alkenyl, C₂-C₆ haloalkenyl, C₂-C₆ alkinyl, C₂-C₆ haloalkinyl, C₃-C₈ cycloalkyl, C₃-C₈ halocycloalkyl, C₁-C₆ alkoxyalkyl, C₁-C₆ haloalkoxyalkyl and phenyl, optionally substituted with one or more substituents R¹⁰; which are selected independently from one another;
R¹³, R¹⁴ are each independently from one another selected from the group consisting of hydrogen, C₁-C₄ alkyl, C₁-C₆ cycloalkyl, C₁-C₄ alkoxyalkyl, phenyl and benzyl;
R¹⁵ is each independently from one another selected from the group consisting of hydrogen, halogen, cyano, nitro, OH, SH, SCN, SF₅, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl,
C₁-C₆-alkyl, C₂-C₆-alkenyl, C₂-C₆-alkinyl, C₃-C₈-cycloalkyl, wherein the four last mentioned aliphatic and cyclo-aliphatic radicals may be unsubstituted, partially or fully halogenated and/or oxgenated and/or may carry 1 or 2 radicals selected from C₁-C₄ alkoxy;
phenyl, benzyl, pyridyl, phenoxy, wherein the last four radicals may be unsubstituted, partially or fully halogenated and/or to carry 1, 2 or 3 substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino or di-(C₁-C₆-alkyl)amino,
or
two R¹⁵ present on the same carbon atom may together be =O, =CH(C₁-C₄), =C(C₁-C₄-alkyl)C₁-C₄-alkyl, =N(C₁-C₆-alkyl) or =NO(C₁-C₆-alkyl);
R¹⁶ is each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkinyl, C₃-C₈-cycloalkyl, wherein the four last mentioned radicals may be unsubstituted, partially or fully halogenated and/or oxygenated and/or may carry 1 or 2 radicals selected from C₁-C₄ alkoxy, phenyl, benzyl, pyridyl, phenoxy, wherein the last four radicals may be unsubstituted, partially or fully halogenated and/or carry 1, 2 or 3 substituents selected from C₁-C₆-alkyl, C₁-C₆-haloalkyl, C₁-C₆-alkoxy, C₁-C₆ haloalkoxy or (C₁-C₆-alkoxy)carbonyl;
R^{17a}, R^{17b} are each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl,
C₁-C₆-alkyl, C₂-C₆-alkenyl, C₂-C₆-alkinyl, C₃-C₈-cycloalkyl, wherein the four last mentioned aliphatic and cyclo-aliphatic radicals may be unsubstituted, partially or fully halogenated and/or oxygenated and/or may carry 1 or 2 radicals selected from C₁-C₄-alkoxy,
phenyl, benzyl, pyridyl, phenoxy, wherein the four last mentioned radicals may be unsubstituted, partially or fully halogenated and/or carry 1, 2 or 3 substituents selected from C₁-C₆-alkyl, C₁-C₆-haloalkyl, C₁-C₆-alkoxy, C₁-C₆ haloalkoxy or (C₁-C₆-alkoxy)carbonyl,
or,
R^{17a} and R^{17b} may together be a C₂-C₆ alkylene chain forming a 3- to 7-membered saturated, partly saturated or unsaturated ring together with the nitrogen atom R^{17a} and R^{17b} are bonded to, wherein the alkylene chain may contain 1 or 2 heteroatoms selected from oxygen, sulfur or nitrogen, and may optionally be substituted with halogen, C₁-C₄-haloalkyl, C₁-C₄-alkoxy or C₁-C₄-haloalkoxy, and wherein the nitrogen and/or the sulfur atom(s) of the heterocyclic ring may optionally be oxidized;
n is an integer selected independently from one another from 0, 1 or 2;
and/or an enantiomer, diastereomer, E/Z-isomer or agriculturally or veterinarily acceptable salts thereof for controlling and/or combating animal pests.

2. The compounds of formula (I) according to claim 1, wherein
Het is selected from the group consisting of radicals of the following formula Het-1 to Het-28: 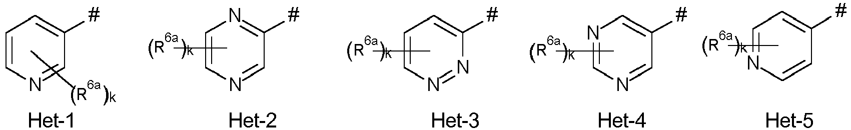 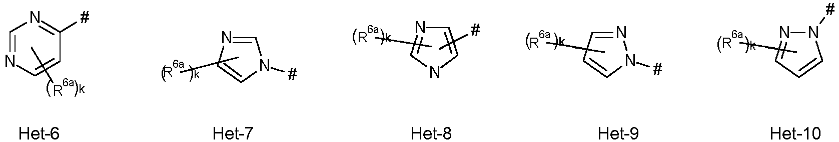 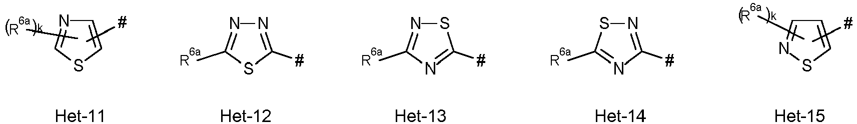 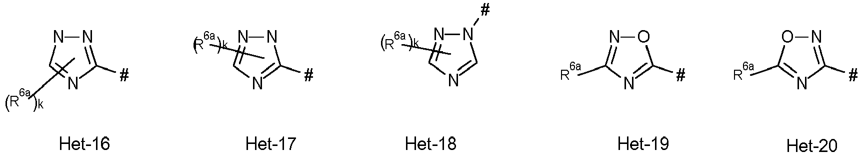 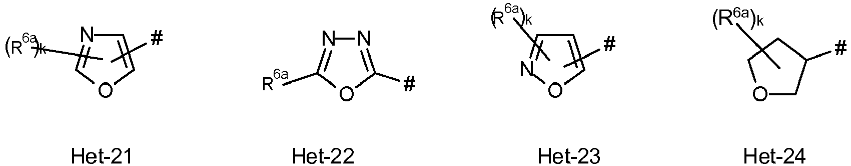 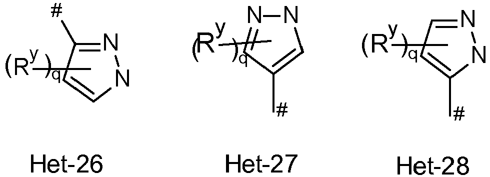 wherein # denotes the bond in formula (I), and wherein
k is 0, 1 or 2; and
R^{6a} is each independently from one another selected from the group consisting of hydrogen, halogen, cyano, C₁-C₆-alkyl, C₃-C₈-cycloalkyl, C₂-C₆-alkenyl, C₂-C₆-alkinyl and wherein the carbon atoms of the aforementioned aliphatic and cyclo-aliphatic radicals may optionally be further substituted independently from one another with one or more R⁷, OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=NR^{9a})R⁷, C(=NR^{9a})NR^{9a}R^{9b}.

3. The compounds of formula (I) according to claim 2, wherein
Het is selected from the group consisting of radicals of formulae Het-1, Het-11a and Het-24: 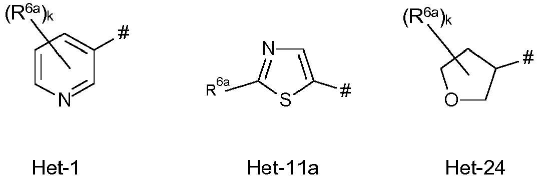
wherein # denotes the bond in formula (I), and wherein
R^{6a} is selected from hydrogen, halogen, C₁-C₄-alkoxy or C₁-C₄-alkyl, wherein the carbon atoms of the latter two radicals may be partially of fully halogenated;
k is 0, 1 or 2.

4. The compounds of formula (I) according to any of the preceding claims, wherein
R¹, R² are independently from each other selected from the group consisting of hydrogen, halogen, CN, C₁-C₆-alkyl, C₃-C₆-cycloalkyl, C₁-C₆-haloalkyl, C₂-C₆-halocycloalkyl;
or
R¹ and R² may together be =O, =CR¹³R¹⁴ or =S;
or
R¹ and R² form, together with the carbon atom, which they attached to, a 3- to 5-membered saturated carbocyclic ring, in particular
R¹ and R² are both hydrogen,
or
R¹ is hydrogen and R² is CH₃.

5. The compounds of formula (I) according to any of the preceding claims, wherein
R^{3a}, R^{3b} are selected each independently from one another from the group consisting of hydrogen, halogen, CN, C₁-C₆-alkyl, C₁-C₆-haloalkyl, C₁-C₆-alkoxy, C₁-C₆-haloalkoxy, C₁-C₆-haloalkylthio, C₁-C₆-alkylthio and OR⁸, wherein R⁸ is C(=O)R¹⁵, and R¹⁵ is C₁-C₆-alkyl, wherein the aliphatic radical may be unsubstituted, partially or fully halogenated, in particular
R^{3a}, R^{3b} are selected each independently from one another from the group consisting of hydrogen, fluorine, CN, C₁-C₆-alkyl and C₁-C₆-haloalkyl, or
R^{3a}, R^{3b} form together with the carbon atom they are bound to, a cyclopropane ring, wherein each of the carbon atoms of the ring may be unsubstituted or may be partly or fully halogenated.

6. The compounds of formula (I) according to any of claims 1 to 5, wherein
p is 0.

7. The compounds of formula (I) according to any of the preceding claims, wherein
Y is selected from S, S=O or S(O)₂.

8. The compounds of formula (I) according to any of the preceding claims, wherein
R⁵ is selected from the group consisting of hydrogen, cyano, C₁-C₆-alkyl, C₇-C₁₀-alkyl, C₃-C₁₀-cycloalkyl, C₂-C₆-alkenyl and C₂-C₆-alkinyl, wherein the carbon chain atoms of the four aforementioned aliphatic and cycloaliphatic radicals are unsubstituted, partly or completely halogenated or may carry any combination of one or more radicals R⁷, wherein R⁷ is selected independently from one another from halogen, C₁-C₆-alkyl, C₁-C₆-haloalkyl, C₁-C₆-alkoxy, C₁-C₆-haloalkoxy or C(=O)O-R¹⁶, wherein R¹⁶ is - independently from one another - C₁-C₆-alkyl, wherein the alkyl radical may be unsubstituted, partially or fully halogenated and/or may carry 1 or 2 radicals selected from C₁-C₄ alkoxy;
phenyl or CH₂-phenyl, optionally substituted with 1, 2 or 3 substituents, which are independently selected from one another from hydrogen, halogen, C₁-C₄-alkyl, C₁-C₄-haloalkyl, C₁-C₄-alkoxy, C₁-C₄-haloalkoxy or phenoxy;
a 5- or 6- membered saturated, partly saturated or unsaturated aromatic heterocyclic ring selected from pyridine, furan, oxazole, oxadiazole, isoxazole, thiazole, thiadiazole or isothiazole, wherein the heterocyclic ring may be bonded directly or linked via a CH₂ group to the remainder of the molecule, wherein the heterocyclic ring is unsubstituted or optionally substituted with one, two or three substituents R^{y} selected independently from one another, and wherein the
R^{y} is selected from the group conisisting of halogen, cyano and C₁-C₄-alkyl, wherein the carbon atoms of the alkyl radical may be unsubstituted or partly or fully halogenated and/or may carry 1,2 or 3 radicals selected from C₁-C₄ alkoxy,
in particular
R⁵ is selected from the group consisting of hydrogen, cyano, C₁-C₆-alkyl, C₃-C-cycloalkyl, C₂-C₆-alkenyl and C₂-C₆-alkinyl, wherein each of the four last mentioned aliphatic radicals are unsubstituted, partly or completely halogenated
or
R⁵ is from the group consisting of S-CN, C₁-C₄ alkylthio, C₃-C₆ cycloalkylthio, C₂-C₆ alkenylthio and C₂-C₆ alkinylthio, wherein each of the four last mentioned aliphatic radicals are unsubstituted, partly or completely halogenated
or
R⁵ is phenyl or CH₂-phenyl, wherein the aromatic ring is optionally substituted with 1 or 2 substituents, which are independently selected from one another from the group consisting of hydrogen, halogen, CN, C₁-C₄ alkyl, C₁-C₄ alkoxy, wherein both of the two last mentioned aliphatic radicals may be unsubstituted or partly or completely halogenated.

9. The compounds of formula (I) according to any of the preceding claims, wherein
R⁵ is a 5- or 6- membered aromatic heterocyclic ring selected from the group consisting of 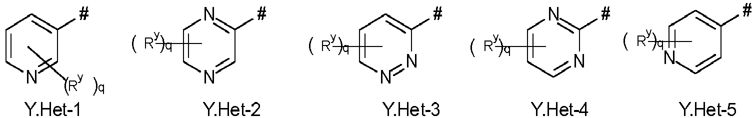 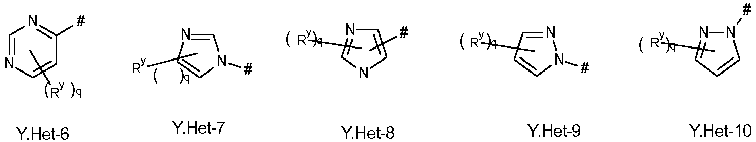 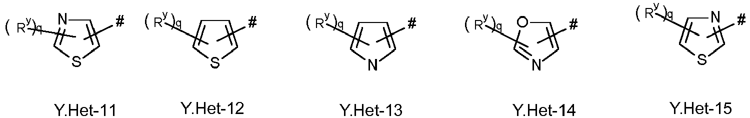 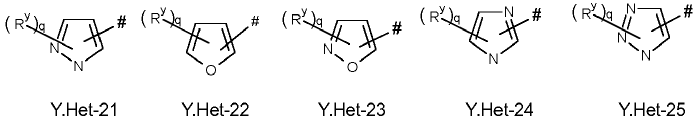 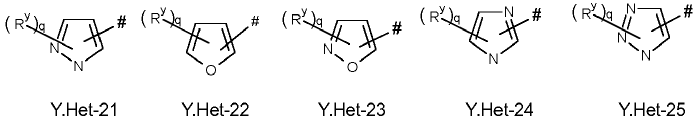 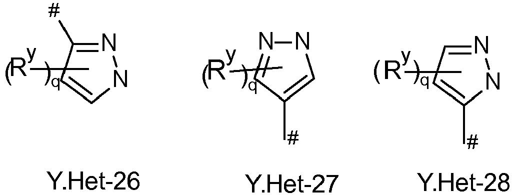
wherein # denotes the bond the remainder of the molecule, and
wherein the bond may be a single bond or linked via a CH₂ group to the remainder of the molecule;
wherein
R^{y} is selected independently from the value of q and independently from one another, from the group consisting of hydrogen, halogen, CN, C₁-C₄ alkyl and C₁-C₄ alkoxy, wherein both of the last two mentioned aliphatic radicals may be unsubstituted, partly or completely halogenated;
phenyl, optionally substituted with halogen, cyano, C₁-C₄ alkyl and C₁-C₄ alkoxy, wherein both of the last two mentioned aliphatic radicals may be unsubstituted, partly or completely halogenated;
or
two of R^{y} on adjacent carbon atoms may be a bridge selected from CH=CH-CH=CH or CH=CHCH₂, and thus form together with the carbon atoms to which the two R^{y} are bonded to a fused 5-membered or 6-membered aromatic carbocyclic ring, wherein this ring may optionally be substituted with one or two substituents selected from halogen, C₁-C₄-alkoxy, C₁-C₄-haloalkoxy, C₁-C₄-alkyl or C₁-C₄-haloalkyl.

10. The compounds of formula (I) according to any of the preceding claims, wherein
W¹, W², W³ and W⁴ represent a carbon chain group connected to N and C=N, and thus forming a saturated, unsaturated or partially unsaturated 6-membered nitrogen containing heterocycle selected from the following group consisting of W.Het-1, W.Het-2, W.Het-3, W.Het-4, W.Het-5, W.Het-6, W.Het-7, W.Het-8, W.Het-9, W.Het-10, W.Het-11 and W.Het-12: 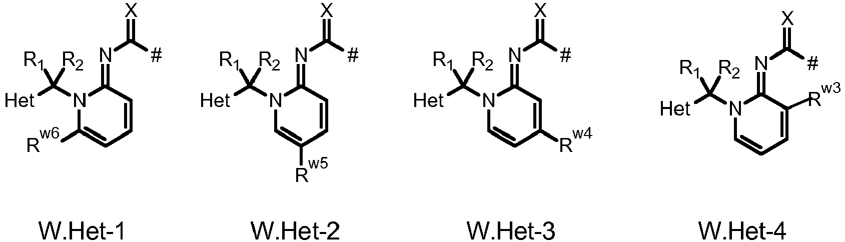 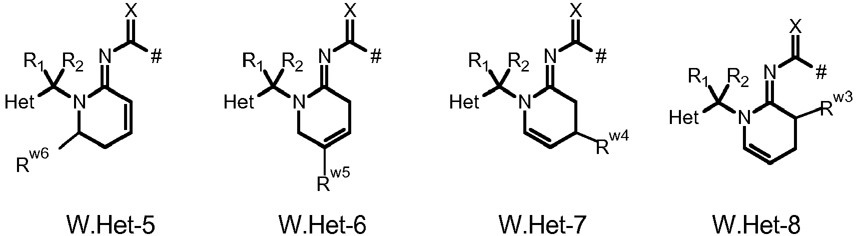 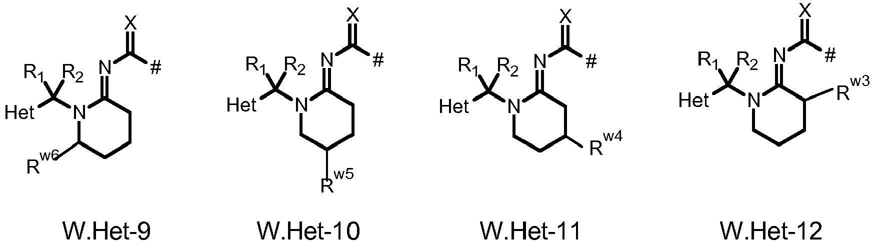
wherein # denotes the bond to the remainder of the molecule; R¹, R² and Het are as defined in claim 1, and wherein
R^{w3}, R^{w4}, R^{w5} and R^{w6} are selected from hydrogen, halogen, C₁-C₄-alkoxy and C₁-C₄-alkyl, wherein both of the last two mentioned aliphatic radicals may be unsubstituted, partly or completely halogenated.

11. The compounds of formula (I) according to claim 1 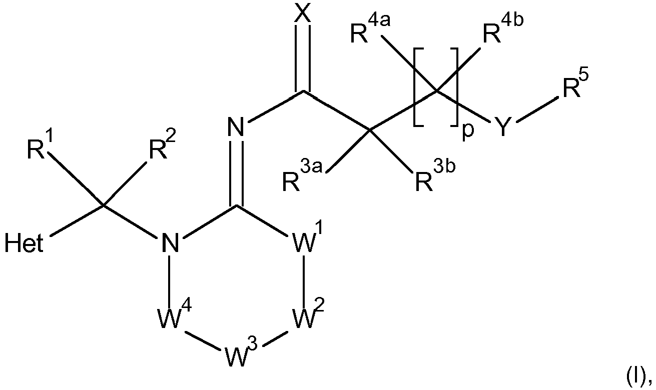 wherein
W¹, W², W³ and W⁴ represent a carbon chain group connected to N and C=N, and thus forming a saturated, unsaturated or partly unsaturated 6-membered nitrogen containing heterocycle selected from W.Het-1, W.Het-5 and W.Het-9 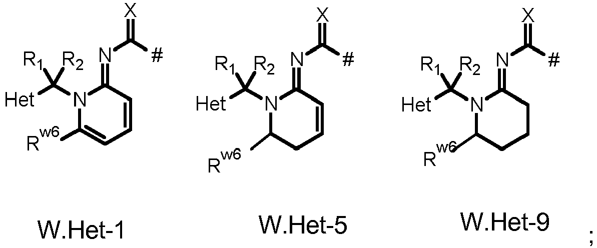
wherein # denotes the bond to the remainder of the molecule, R^{w6} is selected from hydrogen, halogen, C₁-C₄-alkyl; or C₁-C₄-haloalkyl;
and wherein
Het is selected from the group consisting of radicals of formulae Het-1, Het-11a and Het-24 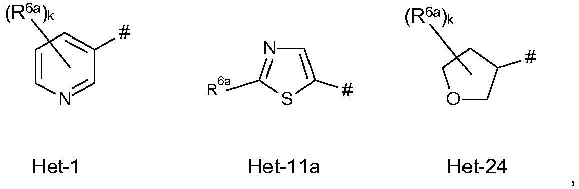
wherein # denotes the bond in formula (I), and wherein
R^{6a} is selected from hydrogen, halogen, C₁-C₄-alkoxy or C₁-C₄-alkyl, wherein the carbon atoms of the latter two radicals may be partially of fully halogenated;
k is 0, 1 or 2;
R¹, R² are independently from each other selected from the group consisting of hydrogen, halogen, cyano, C₁-C₃-alkyl, or C₁-C₃-haloalkyl;
X is selected from O or S;
and wherein
R^{3a}, R^{3b} are selected independently from one another from hydrogen, halogen, CN, C₁-C₆-alkyl, C₁-C₆-haloalkyl,
or wherein
R^{3a}, R^{3b} form together with the carbon atom they are bound to, a cyclocpropane ring, wherein each of the carbon atoms of the ring may be unsubstituted or may be partly or fully halogenated;
p is 0;
Y is S(O)ₘ, wherein m is 0, 1 or 2;
and
R⁵ is selected from the group consisting of hydrogen, halogen, CN, S-CN, C₁-C₆-alkyl, C₁-C₄ thioalkyl, C₃-C₆-cycloalkyl, C₃-C₆ cycloalkylthio, C₂-C₆-alkenyl, C₂-C₆ alkenylthio, C₂-C₆-alkinyl or C₂-C₆ alkinylthio, wherein each of the eight last mentioned aliphatic and cycloaliphatic radicals are unsubstituted, partly or completely halogenated.

12. The compounds of formula (I) according to claim 11, wherein wherein
W¹, W², W³ and W⁴ represent a carbon chain group connected to N and C=N, and thus forming an unsaturated 6-membered nitrogen containing heterocycle W.Het-1 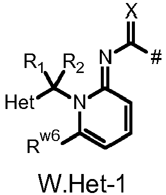
wherein # denotes the bond to the remainder of the molecule, R^{w6} is selected from hydrogen, halogen, C₁-C₄-alkyl or C₁-C₄-haloalkyl;
wherein
Het is
Het-1 or Het-11a 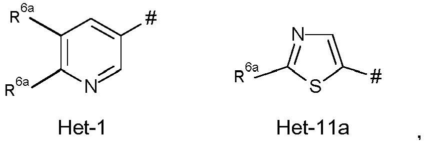 wherein # denotes the bond in formula (I), and wherein R^{6a} is selected from hydrogen, halogen or C₁-C₄-haloalkyl;
R¹, R² are both hydrogen;
X is selected from O or S and wherein
R^{3a}, R^{3b} are selected independently from one another from hydrogen or fluorine;
p is 0;
Y is S(O)ₘ, wherein m is 0, 1 or 2;
and
R⁵ is C₁-C₃-alkyl, which is unsubstituted or partly or completely halogenated.

13. An agricultural or veterinary composition for combating animal pests comprising at least one compound as defined in any of claims 1 to 12 and at least one inert liquid and/or solid acceptable carrier and optionally, if desired, at least one surfactant.

14. A non-therapeutic method for combating or controlling invertebrate pests of the group of insects, arachnids or nematodes, which method comprises contacting said pest or its food supply, habitat or breeding grounds with a pesticidally effective amount of at least one compound as defined in any one of claims 1 to 12.

15. A method for protecting growing plants from attack or infestation by invertebrate pests of the group of insects, arachnids or nematodes, which method comprises contacting a plant, or soil or water in which the plant is growing, with a pesticidally effective amount of at least one compound as defined in any of claims 1 to 12.

16. A method for the protection of plant proparagation material, especially seeds, from soil insects and of the seedlings roots and shoots from soil and foliar insects comprising contacting the plant proparagtion material before sowing and/or after pregermination with at least one compound as defined in any one of claims 1 to 12.

## Patentansprüche

1. N-substituierte Pyridylidencarbonylverbindungen der Formel (I) : 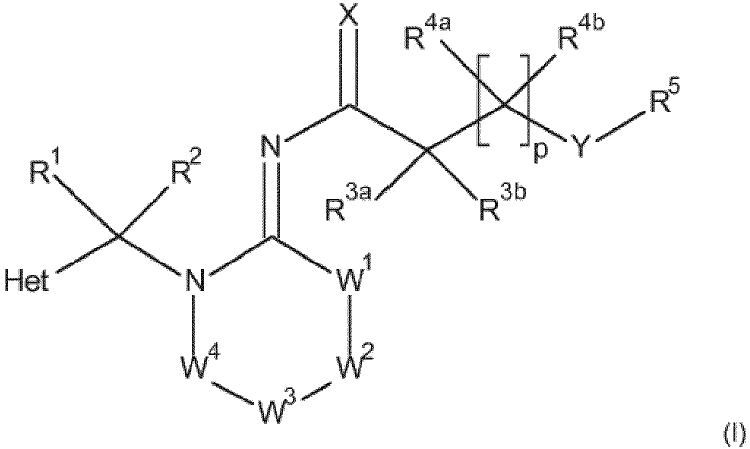 mit den folgenden Bedeutungen:
p ist eine ganze Zahl, ausgewählt aus 0, 1, 2, 3, 4, 5 oder 6;
X bedeutet 0 oder S;
Y bedeutet 0 oder S(O)ₘ, wobei m 0, 1, 2 ist;
Het bedeutet ein 5- oder 6-gliedriges über Kohlenstoff gebundenes oder gegebenenfalls über Stickstoff gebundenes heterocyclisches oder heteroaromatisches Ringsystem, wobei jedes Ringglied aus Kohlenstoffatomen und mindestens einem bis zu drei Heteroatomen, unabhängig ausgewählt aus Schwefel, Sauerstoff oder Stickstoff, ausgewählt ist, wobei die Kohlenstoff-, Schwefel- und Stickstoffringglieder unabhängig teilweise oder vollständig oxidiert sein können und wobei jeder Ring gegebenenfalls durch k Substituenten, ausgewählt aus R^{6a}, substituiert ist, wobei k eine ganze Zahl ausgewählt aus 1, 2, 3, 4 oder 5 ist, und zwei oder mehr Substituenten R^{6a} unabhängig voneinander ausgewählt sind;
W¹, W², W³ und W⁴ bedeuten eine Kohlenstoffkettengruppe, die mit N und C=N verknüpft ist, wodurch ein gesättigter, ungesättigter oder teilweise ungesättigter 5- oder 6-gliedriger stickstoffhaltiger Heterocyclus gebildet wird,
wobei W¹, W², W³ und W⁴ jeweils unabhängig CR^{w} bedeuten, wobei jedes
R^{w} unabhängig voneinander ausgewählt ist aus Wasserstoff, Halogen, Cyano, Azido, Nitro, SCN, SF₅, C₁-C₁ₒ-Alkyl, C₃-C₈-Cycloalkyl, C₂-C₁₀-Alkenyl, C₂-C₁₀-Alkinyl, und wobei die Kohlenstoffatome der obengenannten aliphatischen und cycloaliphatischen Reste unsubstituiert sein können oder teilweise oder vollständig halogeniert sein können oder gegebenenfalls unabhängig voneinander weiter durch ein oder mehrere R⁷ substituiert sein können;
OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=S)OR⁸, C(=S)SR⁸, C(=NR^{9a})R⁷, C(=NR^{9a})NR^{9a}R^{9b}, Si (R¹¹)₂R¹²;
Phenyl, gegebenenfalls substituiert durch 1, 2, 3, 4, oder 5 Substituenten, unabhängig ausgewählt aus R¹⁰;
einem 3-, 4-, 5-, 6- oder 7-gliedrigen gesättigten, teilweise gesättigten oder ungesättigten aromatischen heterocyclischen Ring umfassend 1, 2, 3 oder 4 Heteroatome, ausgewählt aus Sauerstoff, Stickstoff und/oder Schwefel, gegebenenfalls substituiert durch 1, 2, 3, 4 oder 5 Substituenten, unabhängig ausgewählt aus R¹⁰, und wobei das/die Stickstoffatom(e) und/oder Schwefelatom(e) des heterocyclischen Rings gegebenenfalls oxidiert sein kann/können;
oder
zwei von R^{w}, die an einem Ringkohlenstoffatom vorliegen, gemeinsam =O, =CR¹³R¹⁴, =S, =NR^{17a}, =NOR¹⁶, =NNR^{17a} bilden können;
oder
zwei R^{w} von benachbarten Kohlenstoffatomen sowohl gemeinsam als auch gemeinsam mit der vorhandenen Bindung eine Doppelbindung zwischen den benachbarten Kohlenstoffatomen bilden können;
und wobei
eines von W² oder W³ gegebenenfalls eine Einfach- oder Doppelbindung zwischen den benachbarten Kohlenstoffatomen bedeuten kann;
R¹, R² sind unabhängig voneinander ausgewählt aus der Gruppe bestehend aus Wasserstoff, Halogen, CN, SCN, Nitro-C₁-C₆-alkyl, C₃-C₆-Cycloalkyl, wobei jeder der obengenannten Reste unsubstituiert, teilweise oder vollständig halogeniert sein kann oder eine beliebige Kombination von einem oder mehreren Resten R⁷ tragen kann;
Si(R¹¹)₂R¹², OR¹⁶, OSO₂R¹⁶, S(O)ₙR¹⁶, S(O)ₙNR^{17a}R^{17b}, NR^{17a}R^{17b}, C(=O)NR^{17a}R^{17b}, C(=S)NR^{17a}R^{17b}, C(=O)OR¹⁶, C(=O)OR¹⁵, C(=S)OR¹⁵; Phenyl, gegebenenfalls substituiert durch einen oder mehrere, z.B. 1, 2, 3, 4 oder 5, Substituenten R¹⁰, die unabhängig voneinander ausgewählt sind,
einem 3-, 4-, 5-, 6- oder 7-gliedrigen gesättigten, teilweise gesättigten oder ungesättigten aromatischen heterocyclischen Ring umfassend 1, 2 oder 3 Heteroatome, ausgewählt aus Sauerstoff, Stickstoff und/oder Schwefel, gegebenenfalls substituiert durch 1, 2, 3 oder 4 Substituenten R¹⁰, unabhängig voneinander ausgewählt, und wobei das/die Stickstoffatom(e) und/oder Schwefelatom(e) des heterocyclischen Rings gegebenenfalls oxidiert sein kann/können,
oder
R¹ und R² bilden gemeinsam mit dem Kohlenstoffatom, an das sie gebunden sind, einen 3- bis 6-gliedrigen gesättigten oder teilweise ungesättigten carbocyclischen oder heterocyclischen Ring, wobei jedes der Kohlenstoffatome des Cyclus unsubstituiert sein kann oder eine beliebige Kombination von 1 oder 2 Resten R⁷ tragen kann,
oder
R¹ und R² können gemeinsam =O, =CR¹³R¹⁴, =S, =NR^{17a}, =NOR¹⁶, =NNR^{17a} bedeuten;
R^{3a}, R^{3b} sind unabhängig voneinander ausgewählt aus der Gruppe bestehend aus Wasserstoff, Halogen, CN, C₁-C₆-Alkyl, C₁-C₆-Alkoxy, C₁-C₆-Alkylthio, C₁-C₆-Alkylsulfinyl, C₁-C₆-Alkylsulfonyl und C₃-C₈-Cycloalkyl, wobei jeder der sechs letztgenannten Reste unsubstituiert, teilweise oder vollständig halogeniert sein kann,
OR⁸, OSO₂R⁸, S(O)ₙR⁸, S(O)ₙNR^{9a}R^{9b}, NR^{9a}R^{9b}, C(=O)NR^{9a}R^{9b}, C(=S)NR^{9a}R^{9b}, C(=O)R⁷, C(=S)R⁷, Phenyl, gegebenenfalls substituiert durch einen oder mehrere, z.B. 1, 2, 3, 4, oder 5, Substituenten R¹⁰, die unabhängig voneinander ausgewählt sind,
einem 3-, 4-, 5-, 6- oder 7-gliedrigen gesättigten, teilweise gesättigten oder ungesättigten aromatischen heterocyclischen Ring umfassend 1, 2 oder 3 Heteroatome, ausgewählt aus Sauerstoff, Stickstoff und/oder Schwefel, gegebenenfalls substituiert durch 1, 2, 3 oder 4 Substituenten R¹⁰, unabhängig voneinander ausgewählt, und wobei das/die Stickstoffatom(e) und/oder Schwefelatom(e) des heterocyclischen Rings gegebenenfalls oxidiert sein kann/können,
oder wobei R^{3a} und R^{3b} gemeinsam mit dem Kohlenstoffatom, an das sie gebunden sind, einen 3-, 4- oder 5-gliedrigen aliphatischen Ring bilden können, wobei jedes der Kohlenstoffatome des Rings unsubstituiert sein kann oder teilweise oder vollständig halogeniert sein kann,
oder wobei R^{3a} und R^{3b} gemeinsam =O, =CR¹³R¹⁴, =S, =NR^{17a}, =NOR¹⁶, =NNR^{17a} bilden können, und, wenn p 1 oder mehr bedeutet, dann kann eines von R^{3a} oder R^{3b} mit dem R^{4a} oder R^{4b} des benachbarten Kohlenstoffatoms eine Doppelbindung bilden,
R^{4a}, R^{4b} sind unabhängig voneinander und unabhängig von der ganzen Zahl von p ausgewählt aus der Gruppe bestehend aus Wasserstoff, Halogen, CN, C₁-C₆-Alkyl, C₁-C₆-Alkoxy, C₁-C₆-Alkylthio, C₁-C₆-Alkylsulfinyl, C₁-C₆-Alkylsulfonyl und C₃-C₈-Cycloalkyl, wobei jeder der sechs letztgenannten Reste unsubstituiert, teilweise oder vollständig halogeniert ist,
oder wobei R^{4a}, R^{4b} mit dem Kohlenstoffatom, an das sie gebunden sind, einen 3-, 4- oder 5-gliedrigen aliphatischen carbocyclischen Ring bilden können, wobei jedes der Kohlenstoffatome des Rings unsubstituiert sein kann oder teilweise oder vollständig halogeniert sein kann, oder
wobei R^{4a} und R^{4b} gemeinsam =O, =CR¹³R¹⁴, =S, =NR^{17a}, =NOR¹⁶, =NNR^{17a} bilden können, oder, wenn p 1 oder mehr bedeutet,
eines von R^{4a} oder R^{4b} mit R^{3a} oder R^{3b} oder mit einem anderen R^{4a} oder R^{4b} des/der benachbarten Kohlenstoffatom(e) eine Doppelbindung bilden kann;
R⁵ ist ausgewählt aus der Gruppe bestehend aus Wasserstoff, Cyano, C₁-C₆-Alkyl, C₇-C₁₂-Alkyl, C₁-C₆-Alkylthio, C₁-C₆-Alkylsulfinyl, C₁-C₆-Alkylsulfonyl, C₃-C₈-Cycloalkyl, C₃-C₈-Cycloalkylthio, C₃-C₈-Cycloalkylsulfinyl, C₃-C₈-Cycloalkylsulfonyl, C₂-C₆-Alkenyl, C₂-C₆-Alkenylthio, C₂-C₆-Alkenylsulfinyl, C₂-C₆-Alkenylsulfonyl, C₂-C₆-Alkinyl, C₂-C₆-Alkinylthio, C₂-C₆-Alkinylsulfinyl und C₂-C₆-Alkinylsulfinyl, wobei die Kohlenstoffkettenatome der obengenannten aliphatischen und cycloaliphatischen letzten Reste unsubstituiert, teilweise oder vollständig halogeniert sind oder eine beliebige Kombination von einem oder mehreren Resten R⁷ tragen können;
C(=O)NR^{9a}R^{9b}, C(=S)NR^{9a}R^{9b}, C(=O)R⁷, C(=S)R⁷ ; Phenyl oder CH₂-Phenyl, gegebenenfalls substituiert durch einen oder mehrere, z.B. 1, 2, 3, 4 oder 5, Substituenten R¹⁰, die unabhängig voneinander ausgewählt sind;
einem 3-, 4-, 5-, 6- oder 7-gliedrigen gesättigten, teilweise gesättigten oder ungesättigten aromatischen heterocyclischen Ring umfassend 1, 2 oder 3 Heteroatome, ausgewählt aus Sauerstoff, Stickstoff und/oder Schwefel, gegebenenfalls substituiert durch 9 Substituenten R^{y}, unabhängig ausgewählt aus 9 und unabhängig voneinander, und wobei das/die Stickstoffatom(e) und/oder Schwefelatom(e) des heterocyclischen Rings gegebenenfalls oxidiert sein kann/können, wobei der heterocyclische Ring direkt oder über eine CH₂-Gruppe verknüpft an den Rest des Moleküls gebunden sein kann, und wobei
q eine ganze Zahl, ausgewählt aus 1, 2, 3 oder 4 ist, und
R^{y} ausgewählt ist aus der Gruppe bestehend aus Wasserstoff, Halogen, Cyano, Nitro, C₁-C₁₀-Alkyl, C₂-C₁₀-Alkenyl, wobei die Kohlenstoffatome der obengenannten aliphatischen Reste gegebenenfalls durch ein oder mehrere R¹⁵, die unabhängig voneinander ausgewählt sind, substituiert sein können, OR¹⁶, S(O)ₙR¹⁶, S(O)ₙNR^{17a}R^{17b}, NR^{17a}R^{17b}, C(=O)R¹⁵, C(=O)OR¹⁶, C(=NR^{17a})R¹⁵, C(=O)NR^{17a}R^{17b}, C(=S)NR^{17a}R^{17b},
oder zwei R^{y}, die gemeinsam an einem Atom eines teilweise gesättigten Heterocyclus vorliegen, können =O, =CR¹³R¹⁴, =NR^{17a}, =NOR¹⁶ oder NNR^{17a} sein;
oder zwei R^{y} an benachbarten Kohlenstoffatomen können eine Brücke, ausgewählt aus CH₂CH₂CH₂CH₂, CH=CH-CH=CH, N=CH-CH=CH, CH=N-CH=CH, N=CH-N=CH, CH₂CH₂CH₂, CH=CHCH₂, CH₂CH₂NR^{17a}, CH₂CH=N, CH=CH-NR^{17a}, sein; und bilden gemeinsam mit den Kohlenstoffatomen, an die die zwei R^{y} an einen 5-gliedrigen oder 6-gliedrigen anellierten, teilweise gesättigten oder ungesättigten, aromatischen carbocyclischen oder heterocyclischen Ring gebunden sind, wobei der Ring gegebenenfalls durch einen oder zwei Substituenten, ausgewählt aus =0, OH, CH₃, OCH₃, Halogen, Cyano, Halogenmethyl oder Halogenmethoxy, substituiert sein kann;
R^{6a} ist ausgewählt aus der Gruppe bestehend aus Wasserstoff, Halogen, Cyano, Azido, Nitro, SCN, SF₅, C₁-C₁₀-Alkyl, C₃-C₈-Cycloalkyl, C₂-C₁₀-Alkenyl, C₂-C₁₀-Alkinyl, und wobei die Kohlenstoffatome der obengenannten aliphatischen und cycloaliphatischen Reste gegebenenfalls unabhängig voneinander weiter durch ein oder mehrere R⁷ substituiert sein können,
OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=S)OR⁸, C(=S)SR⁸, C(=NR^{9a})R⁷, C(=NR^{9a})NR^{9a}R^{9b}, Si (R¹¹)₂R¹²;
Phenyl, gegebenenfalls substituiert durch 1, 2, 3, 4, oder 5 Substituenten, unabhängig ausgewählt aus R¹⁰;
einem 3-, 4-, 5-, 6- oder 7-gliedrigen gesättigten, teilweise gesättigten oder ungesättigten aromatischen heterocyclischen Ring umfassend 1, 2, 3 oder 4 Heteroatome, ausgewählt aus Sauerstoff, Stickstoff und/oder Schwefel, gegebenenfalls substituiert durch 1, 2, 3, 4 oder 5 Substituenten, unabhängig ausgewählt aus R¹⁰, und wobei das/die Stickstoffatom(e) und/oder Schwefelatom(e) des heterocyclischen Rings gegebenenfalls oxidiert sein kann/können;
oder zwei von R^{6a}, die an einem Ringkohlenstoff- oder -schwefelatom vorliegen, können gemeinsam =O, =CR¹³R¹⁴, =S, =NR^{17a}, =NOR¹⁶, =NNR^{17a} bilden; oder zwei R^{6a} bilden gemeinsam eine C₂-C₇-Alkylenkette und bilden so gemeinsam mit dem/den Ringatom(en), an die sie gebunden sind, einen 3-, 4-, 5-, 6-, 7- oder 8-gliedrigen Ring, wobei die Alkylenkette durch 1 oder 2 0, S und/oder NR^{17a} unterbrochen sein kann und/oder 1 oder 2 der CH₂-Gruppen der Alkylenkette durch eine Gruppe C=0, C=S und/oder C=NR^{17a} ersetzt sein kann/können; und/oder die Alkylenkette substituiert sein kann durch einen oder mehrere Reste, ausgewählt aus der Gruppe bestehend aus Halogen, C₁-C₆-Halogenalkyl, C₁-C₆-Alkoxy, C₁-C₆-Halogenalkoxy, C₁-C₆-Alkylthio, C₁-C₆-Halogenalkylthio, C₃-C₈-Cycloalkyl, C₃-C₈-Halogencycloalkyl, C₂-C₆-Alkenyl, C₂-C₆-Halogenalkenyl, C₂-C₆-Alkinyl, C₂-C₆-Halogenalkinyl, Phenyl, das durch einen oder mehrere, z.B. 1, 2, 3, 4 oder 5, Reste R¹⁰ substituiert sein kann,
und einen 3-, 4-, 5-, 6- oder 7-gliedrigen gesättigten, teilweise ungesättigten oder aromatischen heterocyclischen Ring mit 1, 2 oder 3 Heteroatomen oder Heteroatomgruppen, ausgewählt aus N, 0, S, NO, SO und SO₂ als Ringglieder, wobei der heterocyclische Ring durch einen oder mehrere Reste R¹⁰ substituiert sein kann;
R⁷ ist jeweils unabhängig voneinander ausgewählt aus der Gruppe bestehend aus Wasserstoff, Halogen, Cyano, Azido, Nitro, -SCN, SF₅, C₁-C₆-Alkyl, C₁-C₆-Halogenalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, C₃-C₈-Cycloalkyl, C₃-C₈-Halogencycloalkyl, C₂-C₆-Alkenyl, C₂-C₆-Halogenalkenyl, C₂-C₆-Alkinyl, C₂-C₆-Halogenalkinyl, Si (R¹¹)₂R¹², OR¹⁶, OSO₂R¹⁶, S(O)ₙR¹⁶, S(O)ₙNR^{17a}R^{17b}, NR^{17a}R^{17b}, C(=O)NR^{17a}R^{17b}, C(=S)NR^{17a}R^{17b}, C(=O)OR¹⁶, C(=O)R¹⁵, C(=S)R¹⁵, C(=NR^{17a})R¹⁵,
Phenyl, gegebenenfalls substituiert durch 1, 2, 3, 4 oder 5, Substituenten R¹⁰, die unabhängig voneinander ausgewählt sind, einem 3-, 4-, 5-, 6- oder 7-gliedrigen gesättigten, teilweise gesättigten oder ungesättigten aromatischen heterocyclischen Ring umfassend 1, 2 oder 3 Heteroatome, ausgewählt aus Sauerstoff, Stickstoff und/oder Schwefel, gegebenenfalls substituiert durch 1, 2, 3 oder 4 Substituenten R¹⁰, unabhängig voneinander ausgewählt, und wobei das/die Stickstoffatom(e) und/oder Schwefelatom(e) des heterocyclischen Rings gegebenenfalls oxidiert sein kann/können,
oder zwei R⁷, die an einem Kohlenstoffatom vorliegen, können gemeinsam =O, =CR¹³R¹⁴, =S, =NR^{17a}, =NOR¹⁶, =NNR^{17a} bilden;
oder zwei R⁷ können gemeinsam mit den Kohlenstoffatomen, an die die zwei R⁷ gebunden sind, einen 3-, 4-, 5-, 6-, 7- oder 8-gliedrigen gesättigten oder teilweise ungesättigten carbocyclischen oder heterocyclischen Ring bilden;
R⁸ ist jeweils unabhängig voneinander ausgewählt aus der Gruppe bestehend aus Wasserstoff, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, C₃-C₈-Cycloalkyl, C₃-C₈-Alkylcycloalkyl, C₃-C₈-Halogencycloalkyl, C₂-C₆-Alkenyl, C₂-C₆-Halogenalkenyl, C₂-C₆-Alkinyl, C₂-C₆-Halogenalkinyl, Si(R¹¹)₂R¹², S(O)ₙR¹⁶, S(O)ₙNR^{17a}R^{17b}, NR^{17a}R^{17b}, N=CR¹³R¹⁴, -C(=O)R¹⁵, C(=O)NR^{17a}R^{17b}, C(=S)NR^{17a}R^{17b}, C(=O)OR¹⁶,
Phenyl, gegebenenfalls substituiert durch einen oder mehrere, Substituenten R¹⁰, die unabhängig voneinander ausgewählt sind,
einem 3-, 4-, 5-, 6- oder 7-gliedrigen gesättigten, teilweise gesättigten oder ungesättigten aromatischen heterocyclischen Ring umfassend 1, 2 oder 3 Heteroatome, ausgewählt aus Sauerstoff, Stickstoff und/oder Schwefel, gegebenenfalls substituiert durch 1, 2, 3 oder 4 Substituenten R¹⁰, unabhängig voneinander ausgewählt, und wobei das/die Stickstoffatom(e) und/oder Schwefelatom(e) des heterocyclischen Rings gegebenenfalls oxidiert sein kann/können;
R^{9a}, R^{9b} sind unabhängig voneinander ausgewählt aus der Gruppe bestehend aus Wasserstoff, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₃-C₈-Cycloalkyl, C₃-C₈-Halogencycloalkyl, C₂-C₆-Alkenyl, C₂-C₆-Halogenalkenyl, C₂-C₆-Alkinyl, C₂-C₆-Halogenalkinyl, S(O)ₙR¹⁶, -S(O)ₙNR^{17a}R^{17b},-C(=O)R¹⁵, C(=O)OR¹⁶, C(=O)NR^{17a}R^{17b}, C(=S)R¹⁵, C(=S)SR¹⁶, C(=S)NR^{17a}R^{17b}, C(=NR^{17a}) R¹⁵; Phenyl, gegebenenfalls substituiert durch einen oder mehrere, z.B. 1, 2, 3 oder 4, Substituenten R¹⁰, die unabhängig voneinander ausgewählt sind,
oder einem 3-, 4-, 5-, 6- oder 7-gliedrigen gesättigten, teilweise gesättigten oder ungesättigten aromatischen heterocyclischen Ring umfassend 1, 2, 3 oder 4 Heteroatome, ausgewählt aus Sauerstoff, Stickstoff und/oder Schwefel, gegebenenfalls substituiert durch 1, 2, 3 oder 4 Substituenten R¹⁰, unabhängig voneinander ausgewählt, und wobei das/die Stickstoffatom(e) und/oder Schwefelatom(e) des heterocyclischen Rings gegebenenfalls oxidiert sein kann/können;
R^{9a}, R^{9b} sind gemeinsam eine C₂-C₇-Alkylenkette und bilden gemeinsam mit dem Stickstoffatom, an das sie gebunden sind, einen 3-, 4-, 5-, 6-7- oder 8-gliedrigen gesättigten, teilweise gesättigten oder ungesättigten aromatischen Ring, wobei die Alkylenkette ein oder zwei Heteroatome, ausgewählt aus Sauerstoff, Schwefel oder Stickstoff, enthalten kann und gegebenenfalls substituiert sein kann durch Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₃-C₈-Cycloalkyl, C₃-C₈-Halogencycloalkyl, C₂-C₆-Alkenyl, C₂-C₆-Halogenalkenyl, C₂-C₆-Alkinyl, C₂-C₆-Halogenalkinyl, Phenyl, gegebenenfalls substituiert durch einen oder mehrere, Substituenten R¹⁰, die unabhängig voneinander ausgewählt sind, einem 3-, 4-, 5-, 6- oder 7-gliedrigen gesättigten, teilweise gesättigten oder ungesättigten aromatischen heterocyclischen Ring umfassend 1, 2 oder 3 Heteroatome, ausgewählt aus Sauerstoff, Stickstoff und/oder Schwefel, gegebenenfalls substituiert durch einen oder mehrere Substituenten R¹⁰, unabhängig voneinander ausgewählt, und wobei das/die Stickstoffund/oder Schwefelstoffatom(e) des heterocyclischen Rings gegebenenfalls oxodiert sein kann/können;
oder
R^{9a}, R^{9b} können gemeinsam einen Rest =CR¹³R¹⁴, =NR¹⁷ oder =NOR¹⁶ bilden;
R¹⁰ ist jeweils unabhängig voneinander ausgewählt aus der Gruppe bestehend aus Wasserstoff, Halogen, Cyano, Azido, Nitro, SCN, SF₅, C₁-C₁₀-Alkyl, C₃-C₈-Cycloalkyl, C₂-C₁₀-Alkenyl, C₂-C₁₀-Alkinyl, wobei die Kohlenstoffatome der obengenannten aliphatischen und cycloaliphatischen Reste gegebenenfalls durch ein oder mehrere R¹⁵, die unabhängig voneinander ausgewählt sind, substituiert sein können,
Si(R¹¹)₂R¹², OR¹⁶, OS(O)ₙR¹⁶, -S(O)ₙR¹⁶, S(O)ₙNR^{17a}R^{17b}, NR^{17a}R^{17b}, C(=O)R¹⁵, C(=S)R¹⁵, C(=O)OR¹⁶, -C(=NR^{17a})R¹⁵, C(=O)NR^{17a}R^{17b}, C(=S)NR^{17a}R^{17b},
Phenyl, gegebenenfalls substituiert durch Halogen, Cyano, Nitro, C₁-C₆-Alkyl, C₁-C₆-Halogenalkyl, C₁-C₆-Alkoxy oder C₁-C₆-Halogenalkoxy,
einem 3-, 4-, 5-, 6- oder 7-gliedrigen gesättigten, teilweise gesättigten oder ungesättigten aromatischen heterocyclischen Ring umfassend 1, 2 oder 3 Heteroatome, ausgewählt aus Sauerstoff, Stickstoff und/oder Schwefel, gegebenenfalls substituiert durch einen oder mehrere Substituenten, unabhängig voneinander ausgewählt aus Halogen, Cyano, NO₂, C₁-C₆-Alkyl, C₁-C₆-Halogenalkyl, C₁-C₆-Alkoxy oder C₁-C₆-Halogenalkoxy, und wobei das/die Stickstoff- und/oder Schwefelatom(e) des heterocyclischen Rings gegebenenfalls oxidiert sein kann/können;
oder zwei R¹⁰, die gemeinsam an einem Atom eines teilweise gesättigten Heterocyclus vorliegen, können =O, =CR¹³R¹⁴, =NR^{17a} oder =NOR¹⁶ oder =NNR^{17a} sein;
oder zwei R¹⁰ an benachbarten Kohlenstoffatomen können eine Brücke, ausgewählt aus
CH₂CH₂CH₂CH₂, CH=CH-CH=CH, N=CH-CH=CH, CH=N-CH=CH, N=CH-N=CH, OCH₂CH₂CH₂, OCH=CHCH₂, CH₂OCH₂CH₂, OCH₂CH₂O, OCH₂OCH₂, CH₂CH₂CH₂, CH=CHCH₂, CH₂CH₂O, CH=CHO, CH₂OCH₂, CH₂C(=O)O, C(=O)OCH₂, O(CH₂)O, SCH₂CH₂CH₂, SCH=CHCH₂, CH₂SCH₂CH₂, SCH₂CH₂S, SCH₂SCH₂, CH₂CH₂S, CH=CHS, CH₂SCH₂, CH₂C(=S)S, C(=S)SCH₂, S(CH₂)S, CH₂CH₂NR^{17a}, CH₂CH=N, CH=CH-NR^{17a}, OCH=N, SCH=N, sein und bilden gemeinsam mit den Kohlenstoffatomen, an die die zwei R¹⁰ gebunden sind, an einen 5-gliedrigen oder 6-gliedrigen teilweise gesättigten oder ungesättigten, aromatischen carbocyclischen oder heterocyclischen Ring, wobei der Ring gegebenenfalls durch einen oder zwei Substituenten, ausgewählt aus =O, OH, CH₃, OCH₃, Halogen, Cyano, Halogenmethyl oder Halogenmethoxy, substituiert sein kann;
R¹¹, R¹² sind jeweils unabhängig voneinander ausgewählt aus der Gruppe bestehend aus Wasserstoff, Halogen, C₁-C₆-Alkyl, C₁-C₆-Halogenalkyl, C₁-C₆-Alkoxy, C₁-C₆-Alkoxyalkyl, C₂-C₆-Alkenyl, C₂-C₆-Halogenalkenyl, C₂-C₆-Alkinyl, C₂-C₆-Halogenalkinyl, C₃-C₈-Cycloalkyl, C₃-C₈-Halogencycloalkyl, C₁-C₆-Alkoxyalkyl, C₁-C₆-Halogenalkoxyalkyl und Phenyl, gegebenenfalls substituiert durch einen oder mehrere Substituenten R¹⁰, die unabhängig voneinander ausgewählt sind;
R¹³, R¹⁴ sind jeweils unabhängig voneinander ausgewählt aus der Gruppe bestehend aus Wasserstoff, C₁-C₄-Alkyl, C₁-C₆-Cycloalkyl, C₁-C₄-Alkoxyalkyl, Phenyl und Benzyl;
R¹⁵ ist jeweils unabhängig voneinander ausgewählt aus der Gruppe bestehend aus Wasserstoff, Halogen, Cyano, Nitro, OH, SH, SCN, SF₅, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, Trimethylsilyl, Triethylsilyl, *tert*.-Butyldimethylsilyl,
C₁-C₆-Alkyl, C₂-C₆-Alkenyl, C₂-C₆-Alkinyl, C₃-C₈-Cycloalkyl, wobei die vier letztgenannten aliphatischen und cycloaliphatischen Reste unsubstituiert sein können, teilweise oder vollständig halogeniert sein können und/oder sauerstoffbeladen sein können und/oder 1 oder 2 Reste, ausgewählt aus C₁-C₄-Alkoxy, tragen können;
Phenyl, Benzyl, Pyridyl, Phenoxy, wobei die letztgenannten vier Reste unsubstituiert sein können, teilweise oder vollständig halogeniert sein können und/oder 1, 2 oder 3 Substituenten, ausgewählt aus C₁-C₆-Alkyl, C₁-C₆-Halogenalkyl, C₁-C₆-Alkoxy, C₁-C₆-Halogenalkoxy, (C₁-C₆-Alkoxy) carbonyl, (C₁-C₆-Alkyl)amino oder Di(C₁-C₆-Alkyl)amino, tragen können,
oder zwei R¹⁵, die an demselben Kohlenstoffatom vorliegen, können gemeinsam =O, =CH(C₁-C₄), =C(C₁-C₄-Alkyl)C₁-C₄-alkyl, =N(C₁-C₆-Alkyl) oder =NO(C₁-C₆-Alkyl) sein;
R¹⁶ ist jeweils unabhängig voneinander ausgewählt aus der Gruppe bestehend aus Wasserstoff, Cyano, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, Trimethylsilyl, Triethylsilyl, *tert.-*Butyldimethylsilyl,
C₁-C₆-Alkyl, C₂-C₆-Alkenyl, C₂-C₆-Alkinyl, C₃-C₈-Cycloalkyl, wobei die vier letztgenannten Reste unsubstituiert sein können, teilweise oder vollständig halogeniert sein können und/oder sauerstoffbeladen sein können und/oder ein oder zwei Reste, ausgewählt aus C₁-C₄-Alkoxy tragen können, Phenyl, Benzyl, Pyridyl, Phenoxy, wobei die vier letztgenannten Reste unsubstituiert sein können, teilweise oder vollständige halogeniert sein können und/oder 1, 2 oder 3 Substituenten, ausgewählt aus C₁-C₆-Alkyl, C₁-C₆-Halogenalkyl, C₁-C₆-Alkoxy, C₁-C₆-Halogenalkoxy oder (C₁-C₆-Alkoxy)carbonyl, tragen können;
R^{17a}, R^{17b} sind jeweils unabhängig voneinander ausgewählt aus der Gruppe bestehend aus Wasserstoff, Cyano, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, Trimethylsilyl, Triethylsilyl, *tert*.-Butyldimethylsilyl, C₁-C₆-Alkyl, C₂-C₆-Alkenyl, C₂-C₆-Alkinyl, C₃-C₈-Cycloalkyl, wobei die vier letztgenannten aliphatischen und cycloaliphatischen Reste unsubstituiert sein können, teilweise oder vollständig halogeniert sein können und/oder sauerstoffbeladen sein können und/oder ein oder zwei Reste, ausgewählt aus C₁-C₄-Alkoxy tragen können,
Phenyl, Benzyl, Pyridyl, Phenoxy, wobei die vier letztgenannten Reste unsubstituiert sein können, teilweise oder vollständig halogeniert sein können und/oder 1, 2 oder 3 Substituenten, ausgewählt aus C₁-C₆-Alkyl, C₁-C₆-Halogenalkyl, C₁-C₆-Alkoxy, C₁-C₆-Halogenalkoxy oder (C₁-C₆-Alkoxy)carbonyl, tragen können,
oder
R^{17a} und R^{17b} können gemeinsam eine C₂-C₆-Alkylenkette sein, die gemeinsam mit dem Stickstoffatom, an das R^{17a} und R^{17b} gebunden sind, einen 3- bis 7-gliedrigen gesättigten, teilweise gesättigten oder ungesättigten Ring bildet, wobei die Alyklenkette ein oder zwei Heteroatome, ausgewählt aus Sauerstoff, Schwefel oder Stickstoff, enthalten kann und gegebenenfalls durch Halogen, C₁-C₄-Halogenalkyl, C₁-C₄-Alkoxy oder C₁-C₄-Halogenalkoxy substituiert sein kann, und wobei das/die Stickstoff- und/oder Schwefelatom(e) des heterocyclischen Rings gegebenenfalls oxidiert sein kann/können;
n ist eine ganze Zahl, unabhängig voneinander ausgewählt aus 0, 1 oder 2;
und/oder ein Enantiomer, Diastereomer, E/Z-Isomer, oder landwirtschaftlich oder veterinärmedizinisch unbedenkliche Salze davon, zum Kontrollieren und/oder Bekämpfen von tierischen Schädlingen.

2. Verbindungen der Formel (I) nach Anspruch 1, wobei Het aus der Gruppe bestehend aus Resten der folgenden Formeln Het-1 bis Het-28 ausgewählt ist: 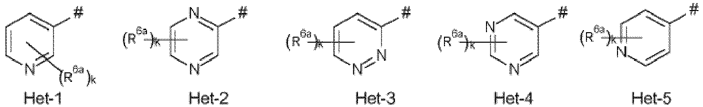 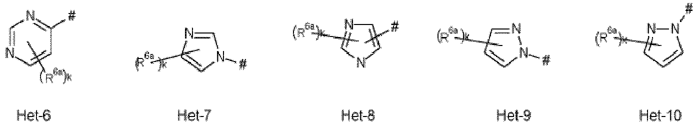 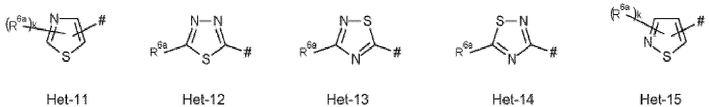 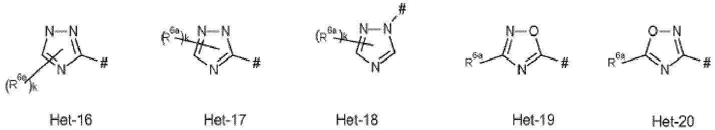 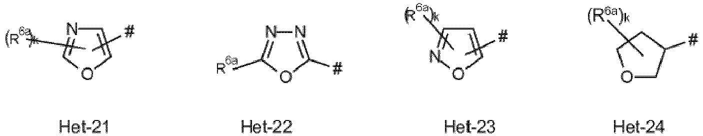 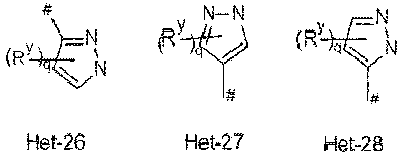 wobei # die Bindung in Formel (I) angibt und wobei
k 0, 1 oder 2 ist;
und
R^{6a} unabhängig voneinander ausgewählt ist aus der Gruppe bestehend aus Wasserstoff, Halogen, Cyano, C₁-C₆-Alkyl, C₃-C₈-Cycloalkyl, C₂-C₆-Alkenyl, C₂-C₆-Alkinyl und wobei die Kohlenstoffatome der obengenannten aliphatischen und cycloaliphatischen Reste gegebenenfalls unabhängig voneinander weiter durch ein oder mehrere R⁷ substituiert sein können,
OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=NR^{9a})R⁷, C(=NR^{9a})NR^{9a}R^{9b}.

3. Verbindungen der Formel (I) nach Anspruch 2, wobei
Het aus der Gruppe bestehend aus Resten der Formeln Het-1, Het-11a und Het-24 ausgewählt ist: 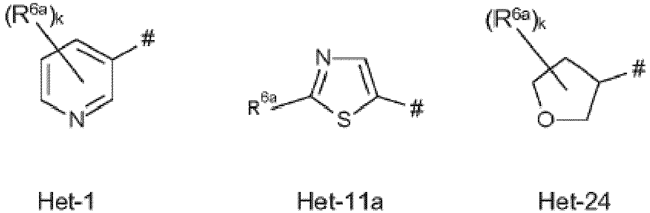
wobei # die Bindung in Formel (I) angibt und wobei
R^{6a} ausgewählt ist aus der Wasserstoff, Halogen, C₁-C₄-Alkoxy oder C₁-C₄-Alkyl, wobei die Kohlenstoffatome der letztgenannten zwei Reste teilweise oder vollständig halogeniert sein können;
k 0, 1 oder 2 ist.

4. Verbindungen der Formel (I) nach einem der vorhergehenden Ansprüche, wobei
R¹, R² unabhängig voneinander ausgewählt sind aus der Gruppe bestehend aus Wasserstoff, Halogen, CN, C₁-C₆-Alkyl, C₃-C₆-Cycloalkyl, C₁-C₆-Halogenalkyl, C₂-C₆-Halogencycloalkyl;
oder
R¹ und R² gemeinsam =O, =CR¹³R¹⁴ oder =S sein können;
oder
R¹ und R² gemeinsam mit dem Kohlenstoffatom, an das sie gebunden sind, einen 3- bis 5-gliedrigen gesättigten carbocyclischen Ring bilden, insbesondere
R¹ und R² beide Wasserstoff bedeuten,
oder
R¹ Wasserstoff bedeutet und R² CH₃ bedeutet.

5. Verbindungen der Formel (I) nach einem der vorhergehenden Ansprüche, wobei
R^{3a}, R^{3b} jeweils unabhängig voneinander ausgewählt sind aus der Gruppe bestehend aus Wasserstoff, Halogen, CN, C₁-C₆-Alkyl, C₁-C₆-Halogenalkyl, C₁-C₆-Alkoxy, C₁-C₆-Halogenalkoxy, C₁-C₆-Halogenalkylthio, C₁-C₆-Alkylthio und OR⁸, wobei R⁸ C(=O)R¹⁵ bedeutet und R¹⁵ C₁-C₆-Alkyl bedeutet, wobei der aliphatische Rest unsubstituiert, teilweise oder vollständig halogeniert sein kann, insbesondere
R^{3a}, R^{3b} jeweils unabhängig voneinander ausgewählt sind aus der Gruppe bestehend aus Wasserstoff, Fluor, CN, C₁-C₆-Alkyl und C₁-C₆-Halogenalkyl, oder
R^{3a}, R^{3b} gemeinsam mit dem Kohlenstoffatom, an das sie gebunden sind, einen Cyclopropanring bilden, wobei jedes der Kohlenstoffatome des Rings unsubstituiert sein kann oder teilweise oder vollständig halogeniert sein kann.

6. Verbindungen der Formel (I) nach einem der Ansprüche 1 bis 5, wobei
p 0 bedeutet.

7. Verbindungen der Formel (I) nach einem der vorhergehenden Ansprüche, wobei
Y aus S, S=O oder S(O)₂ ausgewählt ist.

8. Verbindungen der Formel (I) nach einem der vorhergehenden Ansprüche, wobei
R⁵ ausgewählt ist aus der Gruppe bestehend aus Wasserstoff, Cyano, C₁-C₆-Alkyl, C₇-C₁₀-Alkyl, C₃-C₁₀-Cycloalkyl, C₂-C₆-Alkenyl, C₂-C₆-Alkinyl, wobei die Kohlenstoffkettenatome der vier obengenannten aliphatischen und cycloaliphatischen Reste unsubstituiert sind, teilweise oder vollständig halogeniert sind oder eine beliebige Kombination von einem oder mehreren Resten R⁷ tragen können, wobei R⁷ unabhängig voneinander ausgewählt ist aus Halogen, C₁-C₆-Alkyl, C₁-C₆-Halogenalkyl, C₁-C₆-Alkoxy, C₁-C₆-Halogenalkoxy oder C(=O)O-R¹⁶, wobei R¹⁶ - unabhängig voneinander - C₁-C₆-Alkyl bedeutet, wobei der Alkylrest unsubstituiert sein kann, teilweise oder vollständig halogeniert sein kann und/oder ein oder zwei Reste, ausgewählt aus C₁-C₄-Alkoxy, tragen kann;
Phenyl oder CH₂-Phenyl, gegebenenfalls substituiert durch 1, 2 oder 3 Substituenten, die unabhängig voneinander ausgewählt sind aus Wasserstoff, Halogen, C₁-C₄-Alkyl, C₁-C₄-Halogenalkyl, C₁-C₄-Alkoxy, C₁-C₄-Halogenalkoxy oder Phenoxy; einem 5- oder 6-gliedrigen gesättigten, teilweise gesättigten oder ungesättigten aromatischen heterocyclischen Ring, ausgewählt aus Pyridin, Furan, Oxazol, Oxadiazol, Isoxazol, Thiazol, Thiadiazol oder Isothiazol, wobei der heterocyclische Ring an den Rest des Moleküls direkt gebunden oder über eine CH₂-Gruppe verknüpft sein kann, wobei der heterocyclische Ring unsubstituiert oder gegebenenfalls durch einen, zwei oder drei Substituenten R^{y}, unabhängig voneinander ausgewählt, substituiert sein kann, und wobei das
R^{y} ausgewählt ist aus der Gruppe bestehend aus Halogen, Cyano und C₁-C₄-Alkyl, wobei die Kohlenstoffatome des Alkylrests unsubstituiert sein können oder teilweise oder vollständig halogeniert sein können und/oder 1, 2 oder 3 Reste, ausgewählt aus C₁-C₄-Alkoxy, tragen können, insbesondere ist
R⁵ aus der Gruppe bestehend aus Wasserstoff, Cyano, C₁-C₆-Alkyl, C₃-C-Cycloalkyl, C₂-C₆-Alkenyl und C₂-C₆-Alkinyl ausgewählt, wobei jeder der vier letztgenannten aliphatischen Reste unsubstituiert oder teilweise oder vollständig halogeniert ist
oder
R⁵ ist aus der Gruppe bestehend aus S-CN, C₁-C₄-Alkylthio, C₃-C₆-Cycloalkylthio, C₂-C₆-Alkenylthio und C₂-C₆-Alkinylthio, wobei jeder der vier letztgenannten aliphatischen Reste unsubstituiert oder teilweise oder vollständig halogeniert ist
oder
R⁵ ist Phenyl oder CH₂-Phenyl, wobei der aromatische Ring gegebenenfalls durch 1 oder 2 Substituenten substituiert ist, die unabhängig voneinander aus der Gruppe bestehend aus Wasserstoff, Halogen, CN, C₁-C₄-Alkyl, C₁-C₄-Alkoxy ausgewählt ist, wobei beide der zwei letztgenannten aliphatischen Reste unsubstituiert oder teilweise oder vollständig halogeniert sein können.

9. Verbindungen der Formel (I) nach einem der vorhergehenden Ansprüche, wobei
R⁵ ein 5- oder 6-gliedriger aromatischer heterocyclischer Ring ist, ausgewählt aus der Gruppe bestehend aus 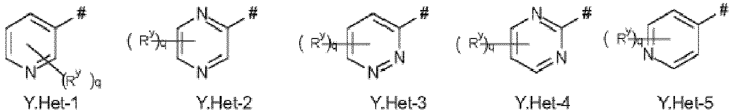 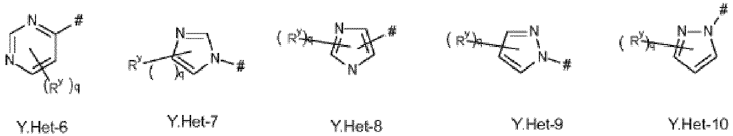 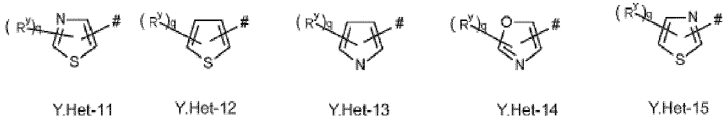 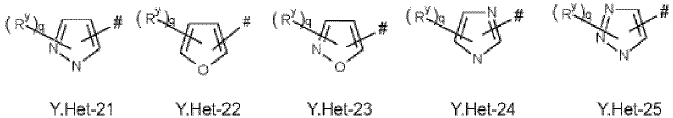 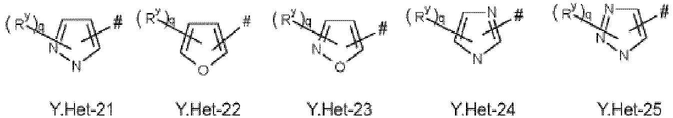 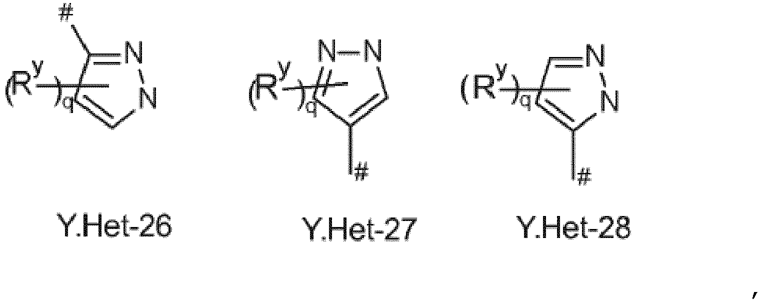 wobei # die Bindung der Rest des Moleküls bedeutet, und
wobei die Bindung eine Einzelbindung oder über eine CH₂-Gruppe an den Rest des Moleküls verknüpft sein kann; wobei
R^{y} unabhängig vom Wert von q und unabhängig voneinander ausgewählt ist aus der Gruppe bestehend aus Wasserstoff, Halogen, CN, C₁-C₄-Alkyl und C₁-C₄-Alkoxy, wobei beide der letzten zwei genannten aliphatischen Reste unsubstituiert oder teilweise oder vollständig halogeniert sein können; Phenyl, gegebenenfalls substituiert durch Halogen, Cyano, C₁-C₄-Alkyl und C₁-C₄-Alkoxy, wobei beide der letzten zwei genannten aliphatischen Reste unsubstituiert oder teilweise oder vollständig halogeniert sein können;
oder
zwei von R^{y} an benachbarten Kohlenstoffatomen eine Brücke, ausgewählt aus CH=CH-CH=CH oder CH=CHCH₂ sein können und so gemeinsam mit den Kohlenstoffatomen, an die die zwei R^{y} gebunden sind, einen anellierten 5-gliedrigen oder 6-gliedrigen aromatischen carbocyclischen Ring binden, wobei dieser Ring gegebenenfalls durch einen oder zwei Substituenten, ausgewählt aus Halogen, C₁-C₄-Alkoxy, C₁-C₄-Halogenalkoxy, C₁-C₄-Alkyl oder C₁-C₄-Halogenalkyl, substituiert sein kann.

10. Verbindungen der Formel (I) nach einem der vorhergehenden Ansprüche, wobei
W¹, W², W³ und W⁴ eine Kohlenstoffkettengruppe, die mit N und C=N verbunden ist, bedeuten, wodurch ein gesättigter, ungesättigter oder teilweise ungesättigter 6-gliedriger stickstoffhaltiger Heterocyclus, ausgewählt aus der folgenden Gruppe bestehend aus W.Het-1, W.Het-2, W.Het-3, W.Het-4, W.Het-5, W.Het-6, W.Het-7, W.Het-8, W.Het-9, W.Het-10, W.Het-11 und W.Het-12, gebildet wird: 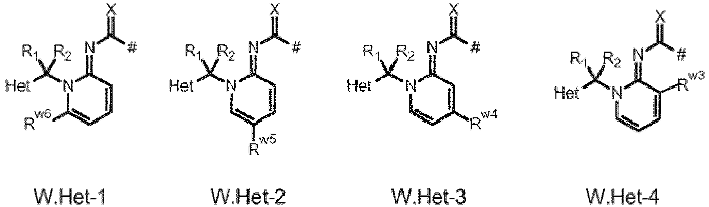 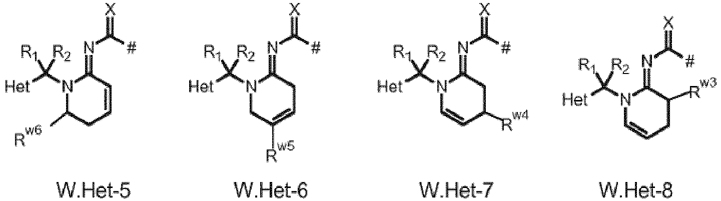 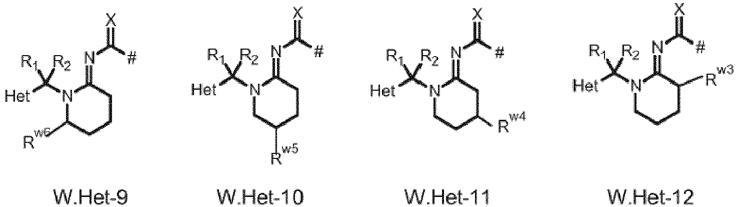
wobei # die Bindung an den Rest des Moleküls angibt; R¹, R² und Het wie in Anspruch 1 definiert sind, und wobei
R^{w3}, R^{w4}, R^{w5} und R^{w6} aus Wasserstoff, Halogen, C₁-C₄-Alkoxy und C₁-C₄-Alkyl ausgewählt sind, wobei beide der letzten zwei genannten aliphatischen Reste unsubstituiert oder teilweise oder vollständig halogeniert sein können.

11. Verbindungen der Formel (I) nach Anspruch 1 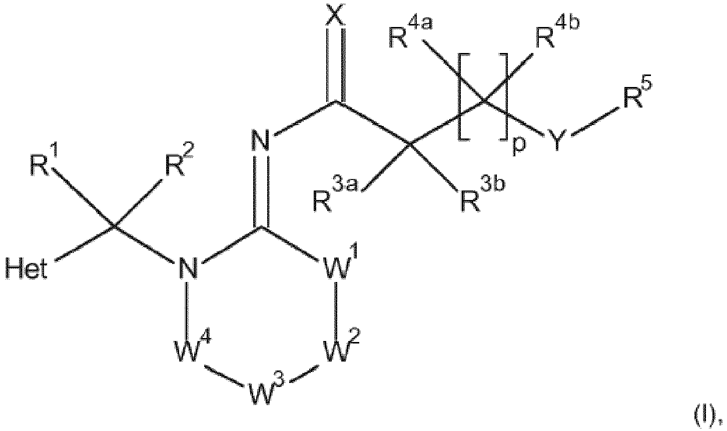 worin
W¹, W², W³ und W⁴ eine Kohlenstoffkettengruppe, die mit N und C=N verbunden ist, bedeuten, wodurch ein gesättigter, ungesättigter oder teilweise ungesättigter 6-gliedriger stickstoffhaltiger Heterocyclus, ausgewählt aus W.Het-1, W.Het-5 und W.Het-9, gebildet wird 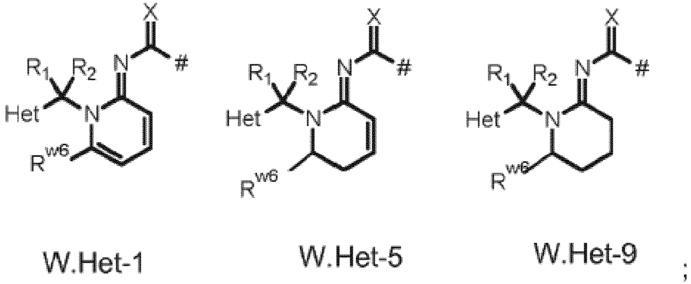
wobei # die Bindung an den Rest des Moleküls angibt,
R^{w6} aus Wasserstoff, Halogen, C₁-C₄-Alkyl oder C₁-C₄-Halogenalkyl ausgewählt ist;
und wobei
Het aus der Gruppe bestehend aus Resten der Formeln Het-1, Het-11a und Het-24 ausgewählt ist 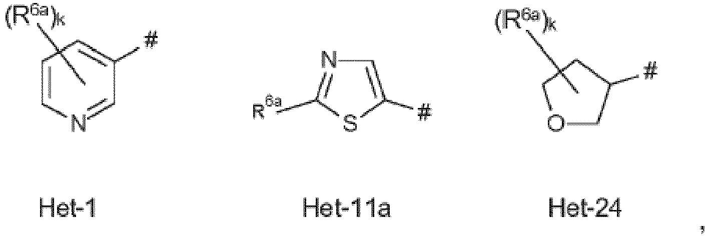
wobei # die Bindung in Formel (I) angibt und wobei
R^{6a} aus Wasserstoff, Halogen, C₁-C₄-Alkoxy oder C₁-C₄-Alkyl ausgewählt ist, wobei die Kohlenstoffatome der letztgenannten zwei Reste teilweise oder vollständig halogeniert sein können;
k 0, 1 oder 2 bedeutet;
R¹, R² unabhängig voneinander ausgewählt sind aus der Gruppe bestehend aus Wasserstoff, Halogen, Cyano, C₁-C₃-Alkyl oder C₁-C₃-Halogenalkyl;
X aus 0 oder S ausgewählt ist; und wobei
R^{3a}, R^{3b} unabhängig voneinander aus Wasserstoff, Halogen, CN, C₁-C₆-Alkyl, C₁-C₆-Halogenalkyl ausgewählt sind, oder wobei
R^{3a}, R^{3b} gemeinsam mit dem Kohlenstoffatom, an das sie gebunden sind, einen Cyclopropanring bilden, wobei jedes der Kohlenstoffatome des Rings unsubstituiert sein kann oder teilweise oder vollständig halogeniert sein kann;
p 0 bedeutet;
Y S(O)ₘ bedeutet, wobei m 0, 1 oder 2 bedeutet;
und
R⁵ ausgewählt ist aus der Gruppe bestehend aus Wasserstoff, Halogen, CN, S-CN, C₁-C₆-Alkyl, C₁-C₄-Thioalkyl, C₃-C₆-Cycloalkyl, C₃-C₆-Cycloalkylthio, C₂-C₆-Alkenyl, C₂-C₆-Alkenylthio, C₂-C₆-Alkinyl oder C₂-C₆-Alkinylthio, wobei jeder der acht letztgenannten aliphatischen und cycloaliphatischen Reste unsubstituiert oder teilweise oder vollständig halogeniert ist.

12. Verbindungen der Formel (I) nach Anspruch 11, wobei
W¹, W², W³ und W⁴ eine Kohlenstoffkettengruppe, die mit N und C=N verknüpft ist, bedeuten, wodurch ein ungesättigter 6-gliedriger stickstoffhaltiger Heterocyclus W.Het-1 gebildet wird, 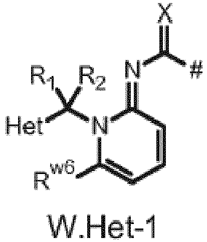
wobei # die Bindung an den Rest des Moleküls angibt,
R^{w6} aus Wasserstoff, Halogen, C₁-C₄-Alkyl oder C₁-C₄-Halogenalkyl ausgewählt ist; wobei
Het Het-1 oder Het-11a bedeutet 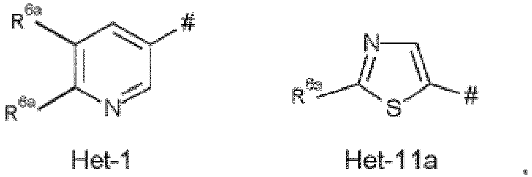 wobei # die Bindung in Formel (I) angibt,
und wobei
R^{6a} aus Wasserstoff, Halogen oder C₁-C₄-Halogenalkyl ausgewählt ist;
R¹, R² beide Wasserstoff bedeuten;
X aus 0 oder S ausgewählt ist und wobei
R^{3a}, R^{3b} unabhängig voneinander aus Wasserstoff oder Fluor ausgewählt sind;
p 0 bedeutet;
Y S(O)ₘ bedeutet, wobei m 0, 1 oder 2 bedeutet;
und
R⁵ C₁-C₃-Alkyl bedeutet, das unsubstituiert ist oder teilweise oder vollständig halogeniert ist.

13. Landwirtschaftliche oder veterinärmedizinische Zusammensetzung zum Bekämpfen von tierischen Schädlingen, die mindestens eine Verbindung wie in einem der Ansprüche 1 bis 12 definiert und mindestens einen inerten flüssigen und/oder festen unbedenklichen Träger sowie gegebenenfalls, falls erwünscht, mindestens ein Tensid umfasst.

14. Nichttherapeutisches Verfahren zum Bekämpfen oder Kontrollieren von wirbellosen Schädlingen der Gruppe Insekten, Arachnida oder Nematoden, wobei das Verfahren umfasst, dass man den Schädling oder seine Nahrungsquelle, seinen Lebensraum oder seine Brutstätten mit einer pestizidwirksamen Menge an mindestens einer Verbindung wie in einem der Ansprüche 1 bis 12 definiert in Kontakt bringt.

15. Verfahren zum Schützen von wachsenden Pflanzen gegen Befall oder Verseuchung durch wirbellose Schädlinge der Gruppe der Insekten, Arachnida oder Nematoden, wobei das Verfahren das Inkontaktbringen einer Pflanze, oder vom Boden oder Wasser, worin die Pflanze wächst, mit einer pestizidwirksamen Menge an mindestens einer Verbindung wie in einem der Ansprüche 1 bis 12 definiert umfasst.

16. Verfahren zum Schutz von pflanzlichem Vermehrungsgut, insbesondere Samen, gegen Bodeninsekten und für den Schutz von Keimlingen, Wurzeln und Sprossen gegen Boden- und Blattinsekten, umfassend das Inkontaktbringen des pflanzlichen Vermehrungsguts vor dem Aussäen und/oder nach dem Vorkeimen mit mindestens einer Verbindung wie in einem der Ansprüche 1 bis 12 definiert.

## Revendications

1. Composés de pyridylidène carbonyle N-substitués de formule (I) 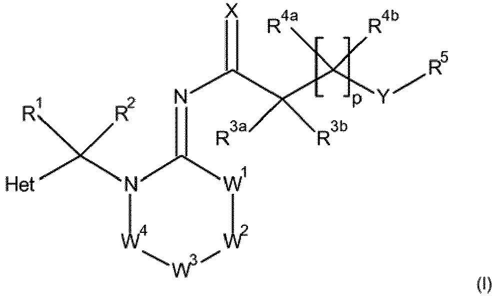 dans lesquels
p est un nombre entier choisi parmi 0, 1, 2, 3, 4, 5 ou 6 ;
X est O ou S ;
Y est O ou S(O)ₘ, où m vaut 0, 1, 2 ;
Het est un noyau hétérocyclique ou hétéroaromatique de 5 ou 6 chaînons lié au carbone ou éventuellement lié à l'azote, chaque chaînon de cycle étant choisi parmi des atomes de carbone et au moins un, jusqu'à trois, hétéroatomes choisis indépendamment parmi le soufre, l'oxygène ou l'azote, où les chaînons de cycle de carbone, de soufre et d'azote peuvent être indépendamment partiellement ou totalement oxydés, et où chaque cycle est éventuellement substitué par k substituants choisis parmi R^{6a}, où k est un nombre entier choisi parmi 1, 2, 3, 4, ou 5, et deux, ou plus, substituants R^{6a} sont choisis indépendamment les uns des autres ;
W¹, W², W³ et W⁴ représentent un groupement de chaîne carbonée relié à N et C=N, et formant ainsi un hétérocycle azoté saturé, insaturé ou partiellement insaturé de 5 ou 6 chaînons,
où W¹, W², W³ et W⁴ représentent chacun individuellement CR^{w}, où chaque R^{w} est choisi indépendamment les uns des autres parmi hydrogène, halogène, cyano, azido, nitro, SCN, SF₅, C₁-C₁₀-alkyle, C₃-C₈-cycloalkyle, C₂-C₁₀alcényle, C₂-C₁₀-alcynyle, et où les atomes de carbone des radicaux aliphatiques et cycloaliphatiques susmentionnés peuvent être non substitués ou peuvent être partiellement ou totalement halogénés ou peuvent être éventuellement substitués davantage indépendamment les uns des autres par un ou plusieurs R⁷ ;
OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=S)OR⁸, C(=S)SR⁸, C(=NR^{9a})R⁷, C(=NR^{9a})NR^{9a}R^{9b}, Si(R¹¹)₂R¹² ;
phényle, éventuellement substitué par 1, 2, 3, 4 ou 5 substituants choisis indépendamment parmi R¹⁰ ;
un cycle hétérocyclique aromatique saturé, partiellement saturé ou insaturé de 3, 4, 5, 6 ou 7 chaînons comprenant 1, 2, 3 ou 4 hétéroatomes choisis parmi l'oxygène, l'azote et/ou le soufre, éventuellement substitué par 1, 2, 3, 4 ou 5 substituants choisis indépendamment parmi R¹⁰, et où le ou les atomes d'azote et/ou de soufre du cycle hétérocyclique peuvent éventuellement être oxydés ;
ou deux parmi R^{w} présents sur un seul atome de carbone de cycle peuvent former ensemble =O, =CR¹³R¹⁴, =S, =NR^{17a}, =NOR¹⁶ ; =NNR^{17a} ;
ou deux R^{w} d'atomes de carbone adjacents peuvent former, ensemble ainsi que conjointement avec la liaison existante, une double liaison entre les atomes de carbone adjacents ;
et où l'un parmi W² ou W³ peut éventuellement représenter une liaison simple ou double entre les atomes de carbone adjacents ;
R¹, R² sont choisis indépendamment l'un de l'autre dans le groupe constitué par hydrogène, halogène, CN, SCN, nitro, C₁-C₆-alkyle, C₃-C₆-cycloalkyle, où chacun parmi les radicaux susmentionnés est non substitué, partiellement ou complètement halogéné ou peut porter une combinaison quelconque d'un ou plusieurs radicaux R⁷ ;
Si(R¹¹)₂R¹², OR¹⁶, OSO₂R¹⁶, S(O)ₙR¹⁶, S(O)ₙNR^{17a}R^{17b}, NR^{17a}R^{17b}, C(=O)NR^{17a}R^{17b}, C(=S)NR^{17a}R^{17b}, C(=O)OR¹⁶, C(=O)R¹⁵, C(=S)R¹⁵ ;
phényle, éventuellement substitué par un ou plusieurs, par exemple 1, 2, 3, 4 ou 5 substituants R¹⁰, qui sont choisis indépendamment les uns des autres,
un cycle hétérocyclique aromatique saturé, partiellement saturé ou insaturé de 3, 4, 5, 6 ou 7 chaînons comprenant 1, 2 ou 3 hétéroatomes choisis parmi l'oxygène, l'azote et/ou le soufre, éventuellement substitué par 1, 2, 3 ou 4, substituants R¹⁰, choisis indépendamment les uns des autres, et où le ou les atomes d'azote et/ou de soufre du cycle hétérocyclique peuvent éventuellement être oxydés, ou R¹ et R² forment, conjointement avec l'atome de carbone auquel ils sont fixés, un cycle carbocyclique ou hétérocyclique de 3 à 6 chaînons saturé ou partiellement insaturé, où chacun parmi les atomes de carbone dudit cycle est non substitué ou peut porter une combinaison quelconque de 1 ou 2 radicaux R⁷, ou R¹ et R² peuvent être ensemble =O, =CR¹³R¹⁴ ; =S, =NR^{17a}, =NOR¹⁶ ; =NNR^{17a} ;
R^{3a}, R^{3b} sont choisis chacun indépendamment l'un de l'autre dans le groupe constitué par hydrogène, halogène, CN, C₁-C₆-alkyle, C₁-C₆-alcoxy, C₁-C₆-alkylthio, C₁-C₆-alkylsulfinyle, C₁-C₆-alkylsulfonyle et C₃-C₈-cycloalkyle, où chacun parmi les six radicaux venant d'être mentionnés est non substitué, partiellement ou complètement halogéné,
OR⁸, OSO₂R⁸, S(O)ₙR⁸, S(O)ₙNR^{9a}R^{9b}, NR^{9a}R^{9b}, C(=O)NR^{9a}R^{9b}, C(=S)NR^{9a}R^{9b}, C(=O)R⁷, C(=S)R⁷,
phényle, éventuellement substitué par un ou plusieurs, par exemple 1, 2, 3, 4 ou 5 substituants R¹⁰, qui sont choisis indépendamment les uns des autres,
un cycle hétérocyclique aromatique saturé, partiellement saturé ou insaturé de 3, 4, 5, 6 ou 7 chaînons comprenant 1, 2 ou 3 hétéroatomes choisis parmi l'oxygène, l'azote et/ou le soufre, éventuellement substitué par 1, 2, 3 ou 4, substituants R¹⁰, choisis indépendamment les uns des autres, et où le ou les atomes d'azote et/ou de soufre du cycle hétérocyclique peuvent éventuellement être oxydés, ou où R^{3a} et R^{3b} peuvent former, conjointement avec l'atome de carbone auquel ils sont fixés, un cycle aliphatique de 3, 4 ou 5 chaînons, où chacun parmi les atomes de carbone du cycle peut être non substitué ou peut être partiellement ou totalement halogéné, ou
où R^{3a} et R^{3b} peuvent former ensemble =O, =CR¹³R¹⁴ ; =S, =NR^{17a}, =NOR¹⁶ ; =NNR^{17a},
et, si p vaut 1 ou plus, alors l'un parmi R^{3a} ou R^{3b} peut former une double liaison avec R^{4a} ou R^{4b} de l'atome de carbone adjacent,
R^{4a}, R^{4b} sont choisis chacun indépendamment l'un de l'autre et indépendamment du nombre entier p dans le groupe constitué par hydrogène, halogène, CN, C₁-C₆-alkyle, C₁-C₆-alcoxy, C₁-C₆-alkylthio, C₁-C₆-alkyl-sulfinyle, C₁-C₆-alkylsulfonyle et C₃-C₈-cycloalkyle, où chacun parmi les six radicaux venant d'être mentionnés est non substitué, partiellement ou complètement halogéné, ou
où R^{4a} et R^{4b} peuvent former, conjointement avec l'atome de carbone auquel ils sont fixés, un cycle carbocyclique aliphatique de 3, 4 ou 5 chaînons, où chacun parmi les atomes de carbone du cycle peut être non substitué ou peut être partiellement ou totalement halogéné, ou
où R^{4a} et R^{4b} peuvent former ensemble =O, =CR¹³R¹⁴ ; =S, =NR^{17a}, =NOR¹⁶ ; =NNR^{17a},
ou, si p vaut 1 ou plus, l'un parmi R^{4a} ou R^{4b} peut former une double liaison avec R^{3a} ou R^{3b} ou avec un autre R^{4a}, ou R^{4b} du ou des atomes de carbone adjacents,
R⁵ est choisi dans le groupe constitué par hydrogène, cyano, C₁-C₆-alkyle, C₇-C₁₂-alkyle, C₁-C₆-alkylthio, C₁-C₆-alkylsulfinyle, C₁-C₆-alkylsulfonyle, C₃-C₈-cycloalkyle, C₃-C₈-cycloalkylthio, C₃-C₈-cycloalkylsulfinyle, C₃-C₈-cycloalkylsulfonyle, C₂-C₆-alcényle, C₂-C₆-alcénylthio, C₂-C₆-alcénylsulfinyle, C₂-C₆-alcénylsulfonyle, C₂-C₆-alcynyle, C₂-C₆-alcynylthio, C₂-C₆-alcynylsulfinyle et C₂-C₆-alcynylsulfonyle où les atomes de la chaîne carbonée des radicaux aliphatiques et cycloaliphatiques venant d'être mentionnés sont non substitués, partiellement ou complètement halogénés ou peuvent porter une combinaison quelconque d'un ou plusieurs radicaux R⁷ ;
C(=O)NR^{9a}R^{9b}, C(=S)NR^{9a}R^{9b}, C(=O)R⁷, C(=S)R⁷ ;
phényle ou CH₂-phényle, éventuellement substitué par un ou plusieurs, par exemple 1, 2, 3, 4 ou 5 substituants R¹⁰, qui sont choisis indépendamment les uns des autres ;
un cycle hétérocyclique aromatique saturé, partiellement saturé ou insaturé de 3, 4, 5, 6 ou 7 chaînons comprenant 1, 2 ou 3 hétéroatomes choisis parmi l'oxygène, l'azote et/ou le soufre, éventuellement substitué par q substituants R^{y}, choisis indépendamment de q et indépendamment les uns des autres, et où le ou les atomes d'azote et/ou de soufre du cycle hétérocyclique peuvent éventuellement être oxydés, où le cycle hétérocyclique peut être lié directement ou lié via un groupement CH₂ au reste de la molécule, et où
q est un nombre entier choisi parmi 1, 2, 3 ou 4,
et
R^{y} est choisi dans le groupe constitué par hydrogène, halogène, cyano, nitro, C₁-C₁₀-alkyle, C₂-C₁₀-alcényle, où les atomes de carbone des radicaux aliphatiques susmentionnés peuvent éventuellement être substitués par un ou plusieurs R¹⁵, qui sont choisis indépendamment les uns des autres,
OR¹⁶, -S(O)ₙR¹⁶, S(O)ₙNR^{17a}R^{17b}, NR^{17a}R^{17b}, C(=O)R¹⁵, C(=O)OR¹⁶, -C(=NR^{17a})R¹⁵, C(=O)NR^{17a}R^{17b}, C(=S)NR^{17a}R^{17b} ; ou
deux R^{y} présents ensemble sur un atome d'un hétérocycle partiellement saturé peuvent être =O, =CR¹³R¹⁴, =NR^{17a}, =NOR¹⁶ ou =NNR^{17a} ; ou
deux R^{y} sur des atomes de carbone adjacents peuvent être un pont choisi parmi CH₂CH₂CH₂CH₂, CH=CH-CH=CH, N=CH-CH=CH, CH=N-CH=CH, N=CH-N=CH, CH₂CH₂CH₂, CH=CHCH₂, CH₂CH₂NR^{17a}, CH₂CH=N, CH=CH-NR^{17a}, et forment, conjointement avec les atomes de carbone auxquels les deux R^{y} sont fixés, à un cycle carbocyclique ou hétérocyclique condensé et partiellement saturé ou insaturé, aromatique, de 5 chaînons ou 6 chaînons, où le cycle peut éventuellement être substitué par un ou deux substituants choisis parmi =O, OH, CH₃, OCH₃, halogène, cyano, halogénométhyle ou halogénométhoxy ;
R^{6a} est choisi dans le groupe constitué par hydrogène, halogène, cyano, azido, nitro, SCN, SF₅, C₁-C₁₀-alkyle, C₃-C₈-cycloalkyle, C₂-C₁₀-alcényle, C₂-C₁₀-alcynyle, et où les atomes de carbone des radicaux aliphatiques et cycloaliphatiques susmentionnés peuvent éventuellement être substitués davantage indépendamment les uns des autres par un ou plusieurs R⁷,
OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=S)OR⁸, C(=S)SR⁸, C(=NR^{9a})R⁷, C(=NR^{9a})NR^{9a}R^{9b}, Si(R¹¹)₂R¹² ;
phényle, éventuellement substitué par 1, 2, 3, 4 ou 5 substituants choisis indépendamment parmi R¹⁰ ;
un cycle hétérocyclique aromatique saturé, partiellement saturé ou insaturé de 3, 4, 5, 6 ou 7 chaînons comprenant 1, 2, 3 ou 4 hétéroatomes choisis parmi l'oxygène, l'azote et/ou le soufre, éventuellement substitué par 1, 2, 3, 4 ou 5 substituants choisis indépendamment parmi R¹⁰, et où le ou les atomes d'azote et/ou de soufre du cycle hétérocyclique peuvent éventuellement être oxydés ;
ou deux parmi R^{6a} présents sur un seul atome de soufre ou de carbone de cycle peuvent former ensemble =O, =CR¹³R¹⁴ ; =S, =NR^{17a}, =NOR¹⁶ ; =NNR^{17a} ;
ou deux R^{6a} forment ensemble une chaîne C₂-C₇-alkylène, formant ainsi, conjointement avec le ou les atomes de cycle auxquels ils sont fixés, un cycle de 3, 4, 5, 6, 7 ou 8 chaînons, où la chaîne alkylène peut être interrompue par 1 ou 2 0, S et/ou NR^{17a} et/ou 1 ou 2 parmi les groupements CH₂ de la chaîne alkylène peuvent être remplacés par un groupement C=O, C=S et/ou C=NR^{17a} ; et/ou la chaîne alkylène peut être substituée par un ou plusieurs radicaux choisis dans le groupe constitué par halogène, C₁-C₆-halogénoalkyle, C₁-C₆-alcoxy, C₁-C₆-halogénoalcoxy, C₁-C₆-alkylthio, C₁-C₆-halogénoalkylthio, C₃-C₈-cycloalkyle, C₃-C₈-halogéno-cycloalkyle, C₂-C₆-alcényle, C₂-C₆-halogénoalcényle, C₂-C₆-alcynyle, C₂-C₆-halogénoalcynyle, phényle pouvant être substitué par un ou plusieurs, par exemple 1, 2, 3, 4 ou 5 radicaux R¹⁰, et un cycle hétérocyclique de 3, 4, 5, 6 ou 7 chaînons saturé, partiellement insaturé ou aromatique contenant 1, 2 ou 3 hétéroatomes ou groupements d'hétéroatomes choisis parmi N, O, S, NO, SO et SO₂, comme chaînons de cycle, où le cycle hétérocyclique peut être substitué par un ou plusieurs radicaux R¹⁰ ;
R⁷ est choisi chacun indépendamment les uns des autres dans le groupe constitué par hydrogène, halogène, cyano, azido, nitro, -SCN, SF₅, C₁-C₆-alkyle, C₁-C₆-halogénoalkyle, C₁-C₆-alcoxy, C₁-C₆-halogénoalcoxy, C₁-C₆-alkylthio, C₁-C₆-alkylsulfinyle, C₁-C₆-alkylsulfonyle, C₁-C₆-halogénoalkylthio, C₃-C₈-cycloalkyle, C₃-C₈-halogénocycloalkyle, C₂-C₆-alcényle, C₂-C₆-halogénoalcényle, C₂-C₆-alcynyle, C₂-C₆-halogénoalcynyle, Si(R¹¹)₂R¹², OR¹⁶, OSO₂R¹⁶, S(O)ₙR¹⁶, S(O)ₙNR^{17a}R^{17b}, NR^{17a}R^{17b}, C(=O)NR^{17a}R^{17b}, C(=S)NR^{17a}R^{17b}, C(=O)OR¹⁶, C(=O)R¹⁵, C(=S)R¹⁵, C(=NR^{17a})R¹⁵,
phényle, éventuellement substitué par 1, 2, 3, 4 ou 5 substituants R¹⁰, qui sont choisis indépendamment les uns des autres,
un cycle hétérocyclique aromatique saturé, partiellement saturé ou insaturé de 3, 4, 5, 6 ou 7 chaînons comprenant 1, 2 ou 3 hétéroatomes choisis parmi l'oxygène, l'azote et/ou le soufre, éventuellement substitué par 1, 2, 3 ou 4 substituants R¹⁰, choisis indépendamment les uns des autres, et où le ou les atomes d'azote et/ou de soufre du cycle hétérocyclique peuvent éventuellement être oxydés,
ou deux R⁷ présents sur un seul atome de carbone peuvent former ensemble =O, =CR¹³R¹⁴ ; =S, =NR^{17a}, =NOR¹⁶ ; =NNR^{17a} ;
ou deux R⁷ peuvent former un cycle carbocyclique ou hétérocyclique de 3, 4, 5, 6, 7 ou 8 chaînons saturé ou partiellement insaturé conjointement avec les atomes de carbone auxquels les deux R⁷ sont fixés ;
R⁸ est choisi chacun indépendamment les uns des autres dans le groupe constitué par hydrogène, cyano, C₁-C₆-alkyle, C₁-C₆-halogénoalkyle, C₁-C₆-alcoxy, C₁-C₆-halogénoalcoxy, C₁-C₆-alkylthio, C₁-C₆-alkylsulfinyle, C₁-C₆-alkylsulfonyle, C₁-C₆-halogénoalkylthio, C₃-C₈-cycloalkyle, C₄-C₈-alkylcycloalkyle, C₃-C₈-halogénocycloalkyle, C₂-C₆-alcényle, C₂-C₆-halogénoalcényle, C₂-C₆-alcynyle, C₂-C₆-halogénoalcynyle, -Si(R¹¹)₂R¹², S(O)ₙR¹⁶, S(O)ₙNR^{17a}R^{17b}, NR^{17a}R^{17b}, -N=CR¹³R¹⁴, -C(=O)R¹⁵, C(=O)NR^{17a}R^{17b}, C(=S)NR^{17a}R^{17b}, C(=O)OR¹⁶, phényle, éventuellement substitué par un ou plusieurs substituants R¹⁰ ; qui sont choisis indépendamment les uns des autres,
un cycle hétérocyclique aromatique saturé, partiellement saturé ou insaturé de 3, 4, 5, 6 ou 7 chaînons comprenant 1, 2 ou 3 hétéroatomes choisis parmi l'oxygène, l'azote et/ou le soufre, éventuellement substitué par 1, 2, 3 ou 4 substituants R¹⁰, choisis indépendamment les uns des autres, et où le ou les atomes d'azote et/ou de soufre du cycle hétérocyclique peuvent éventuellement être oxydés ;
R^{9a}, R^{9b} sont choisis chacun indépendamment l'un de l'autre dans le groupe constitué par hydrogène, C₁-C₆-alkyle, C₁-C₆-halogénoalkyle, C₁-C₆-alcoxy, C₁-C₆-halogénoalcoxy, C₁-C₆-alkylthio, C₁-C₆-halogénoalkylthio, C₃-C₈-cycloalkyle, C₃-C₈-halogénocycloalkyle, C₂-C₆-alcényle, C₂-C₆-halogénoalcényle, C₂-C₆-alcynyle, C₂-C₆-halogénoalcynyle,
S(O)ₙR¹⁶, -S(O)ₙNR^{17a}R^{17b}, C(=O)R¹⁵, C(=O)OR¹⁶, C(=O)NR^{17a}R^{17b}, C(=S)R¹⁵, C(=S)SR¹⁶, C(=S)NR^{17a}R^{17b}, C(=NR^{17a})R¹⁵ ;
phényle, éventuellement substitué par un ou plusieurs, par exemple 1, 2, 3 ou 4, substituants R¹⁰, qui sont choisis indépendamment les uns des autres ;
un cycle hétérocyclique aromatique saturé, partiellement saturé ou insaturé de 3, 4, 5, 6 ou 7 chaînons comprenant 1, 2, 3 ou 4 hétéroatomes choisis parmi l'oxygène, l'azote et/ou le soufre, éventuellement substitué par 1, 2, 3 ou 4 substituants R¹⁰, choisis indépendamment les uns des autres, et où le ou les atomes d'azote et/ou de soufre du cycle hétérocyclique peuvent éventuellement être oxydés ; ou
R^{9a} et R^{9b} sont ensemble une chaîne C₂-C₇-alkylène et forment un cycle aromatique de 3, 4, 5, 6, 7 ou 8 chaînons saturé, partiellement saturé ou insaturé conjointement avec l'atome d'azote auquel ils sont fixés, où la chaîne alkylène peut contenir un ou deux hétéroatomes choisis parmi l'oxygène, le soufre ou l'azote, et peut éventuellement être substituée par halogène, C₁-C₆-alkyle, C₁-C₆-halogénoalkyle, C₁-C₆-alcoxy, C₁-C₆-halogénoalcoxy, C₁-C₆-alkylthio, C₁-C₆-halogénoalkylthio, C₃-C₈-cycloalkyle, C₃-C₈-halogénocycloalkyle, C₂-C₆-alcényle, C₂-C₆-halogénoalcényle, C₂-C₆-alcynyle, C₂-C₆-halogénoalcynyle,
phényle, éventuellement substitué par un ou plusieurs substituants R¹⁰ ; qui sont choisis indépendamment les uns des autres,
un cycle hétérocyclique aromatique de 3, 4, 5, 6, ou 7 chaînons saturé, partiellement saturé ou insaturé comprenant 1, 2 ou 3 hétéroatomes choisis parmi l'oxygène, l'azote et/ou le soufre, éventuellement substitué par un ou plusieurs substituants R¹⁰, choisis indépendamment les uns des autres, et où le ou les atomes d'azote et/ou de soufre du cycle hétérocyclique peuvent éventuellement être oxydés ; ou
R^{9a} et R^{9b} peuvent former ensemble un radical =CR¹³R¹⁴, =NR¹⁷ ou =NOR¹⁶ ;
R¹⁰ est choisi chacun indépendamment les uns des autres dans le groupe constitué par hydrogène, halogène, cyano, azido, nitro, SCN, SF₅, C₁-C₁₀-alkyle, C₃-C₈-cycloalkyle, C₂-C₁₀-alcényle, C₂-C₁₀-alcynyle, où les atomes de carbone des radicaux aliphatiques et cycloaliphatiques susmentionnés peuvent éventuellement être substitués par un ou plusieurs R¹⁵, qui sont choisis indépendamment les uns des autres, Si(R¹¹)₂R¹², OR¹⁶, OS(O)ₙR¹⁶, -S(O)ₙR¹⁶, S(O)ₙNR^{17a}R^{17b}, NR^{17a}R^{17b}, C(=O)R¹⁵, C(=S)R¹⁵, C(=O)OR¹⁶, -C(=NR^{17a})R¹⁵, C(=O)NR^{17a}R^{17b}, C(=S)NR^{17a}R^{17b},
phényle, éventuellement substitué par halogène, cyano, nitro, C₁-C₆-alkyle, C₁-C₆-halogénoalkyle, C₁-C₆-alcoxy ou C₁-C₆-halogénoalcoxy,
un cycle hétérocyclique aromatique saturé, partiellement saturé ou insaturé de 3, 4, 5, 6 ou 7 chaînons comprenant 1, 2 ou 3 hétéroatomes choisis parmi l'oxygène, l'azote et/ou le soufre, éventuellement substitué par un ou plusieurs substituants choisis indépendamment les uns des autres parmi halogène, cyano, NO₂, C₁-C₆-alkyle, C₁-C₆-halogénoalkyle, C₁-C₆-alcoxy ou C₁-C₆-halogénoalcoxy, et où le ou les atomes d'azote et/ou de soufre du cycle hétérocyclique peuvent éventuellement être oxydés ; ou deux R¹⁰ présents ensemble sur un seul atome d'un hétérocyclique partiellement saturé peuvent être =O, =CR¹³R¹⁴, =NR^{17a}, =NOR¹⁶ ou =NNR^{17a} ; ou
deux R¹⁰ sur des atomes de carbone adjacents peuvent être un pont choisi parmi CH₂CH₂CH₂CH₂, CH=CH-CH=CH, N=CH-CH=CH, CH=N-CH=CH, N=CH-N=CH, OCH₂CH₂CH₂, OCH=CHCH₂, CH₂OCH₂CH₂, OCH₂CH₂O, OCH₂OCH₂, CH₂CH₂CH₂, CH=CHCH₂, CH₂CH₂O, CH=CHO, CH₂OCH₂, CH₂C(=O)O, C(=O)OCH₂, O(CH₂)O, SCH₂CH₂CH₂, SCH=CHCH₂, CH₂SCH₂CH₂, SCH₂CH₂S, SCH₂SCH₂, CH₂CH₂S, CH=CHS, CH₂SCH₂, CH₂C(=S)S, C(=S)SCH₂, S(CH₂)S, CH₂CH₂NR^{17a}, CH₂CH=N, CH=CH-NR^{17a}, OCH=N, SCH=N et forment, conjointement avec les atomes de carbone auxquels les deux R¹⁰ sont fixés, à un cycle carbocyclique ou hétérocyclique aromatique de 5 chaînons ou 6 chaînons partiellement saturé ou insaturé, où le cycle peut éventuellement être substitué par un ou deux substituants choisis parmi =0, OH, CH₃, OCH₃, halogène, cyano, halogénométhyle ou halogénométhoxy ;
R¹¹, R¹² sont choisis chacun indépendamment l'un de l'autre dans le groupe constitué par hydrogène, halogène, C₁-C₆-alkyle, C₁-C₆-halogénoalkyle, C₁-C₆-alcoxy, C₁-C₆-alcoxyalkyle, C₂-C₆-alcényle, C₂-C₆-halogénoalcényle, C₂-C₆-alcynyle, C₂-C₆-halogénoalcynyle, C₃-C₈-cycloalkyle, C₃-C₈-halogénocycloalkyle, C₁-C₆-alcoxyalkyle, C₁-C₆-halogénoalcoxyalkyle et phényle, éventuellement substitué par un ou plusieurs substituants R¹⁰ ; qui sont choisis indépendamment les uns des autres ;
R¹³, R¹⁴ sont choisis chacun indépendamment l'un de l'autre dans le groupe constitué par hydrogène, C₁-C₄-alkyle, C₁-C₆-cycloalkyle, C₁-C₄-alcoxyalkyle, phényle et benzyle ;
R¹⁵ est choisi chacun indépendamment les uns des autres dans le groupe constitué par hydrogène, halogène, cyano, nitro, OH, SH, SCN, SF₅, C₁-C₆-alcoxy, C₁-C₆-halogénoalcoxy, C₁-C₆-alkylthio, C₁-C₆-alkylsulfinyle, C₁-C₆-alkylsulfonyle, C₁-C₆-halogénoalkylthio, triméthylsilyle, triéthylsilyle, tertio-butyldiméthylsilyle, C₁-C₆-alkyle, C₂-C₆-alcényle, C₂-C₆-alcynyle, C₃-C₈-cycloalkyle, où les quatre radicaux aliphatiques et cycloaliphatiques venant d'être mentionnés peuvent être non substitués, partiellement ou totalement halogénés et/ou oxygénés et/ou peuvent porter 1 ou 2 radicaux choisis parmi C₁-C₄-alcoxy ;
phényle, benzyle, pyridyle, phénoxy, où ces quatre derniers radicaux peuvent être non substitués, partiellement ou totalement halogénés et/ou peuvent porter 1, 2 ou 3 substituants choisis parmi C₁-C₆-alkyle, C₁-C₆-halogénoalkyle, C₁-C₆-alcoxy, C₁-C₆-halogénoalcoxy, (C₁-C₆-alcoxy)carbonyle, (C₁-C₆-alkyl)-amino ou di-(C₁-C₆-alkyl)amino, ou
deux R¹⁵ présents sur le même atome de carbone peuvent être ensemble =O, =CH(C₁-C₄), =C(C₁-C₄-alkyl)C₁-C₄-alkyle, =N(C₁-C₆-alkyl) ou =NO(C₁-C₆-alkyl) ;
R¹⁶ est choisi chacun indépendamment les uns des autres dans le groupe constitué par hydrogène, cyano, C₁-C₆-alcoxy, C₁-C₆-halogénoalcoxy, C₁-C₆-alkylthio, C₁-C₆-alkylsulfinyle, C₁-C₆-alkylsulfonyle, C₁-C₆-halogénoalkylthio, triméthylsilyle, triéthylsilyle, tertio-butyldiméthylsilyle, C₁-C₆-alkyle, C₂-C₆-alcényle, C₂-C₆-alcynyle, C₃-C₈-cycloalkyle, où les quatre radicaux venant d'être mentionnés peuvent être non substitués, partiellement ou totalement halogénés et/ou oxygénés et/ou peuvent porter 1 ou 2 radicaux choisis parmi C₁-C₄-alcoxy, phényle, benzyle, pyridyle, phénoxy, où ces quatre derniers radicaux peuvent être non substitués, partiellement ou totalement halogénés et/ou peuvent porter 1, 2 ou 3 substituants choisis parmi C₁-C₆-alkyle, C₁-C₆-halogénoalkyle, C₁-C₆-alcoxy, C₁-C₆-halogénoalcoxy ou (C₁-C₆-alcoxy)carbonyle ;
R^{17a}, R^{17b} sont choisis chacun indépendamment l'un de l'autre dans le groupe constitué par hydrogène, cyano, C₁-C₆-alcoxy, C₁-C₆-halogénoalcoxy, C₁-C₆-alkylthio, C₁-C₆-alkylsulfinyle, C₁-C₆-alkylsulfonyle, C₁-C₆-halogéno-alkylthio, triméthylsilyle, triéthylsilyle, tertio-butyldiméthylsilyle,
C₁-C₆-alkyle, C₂-C₆-alcényle, C₂-C₆-alcynyle, C₃-C₈-cycloalkyle, où les quatre radicaux aliphatiques et cycloaliphatiques venant d'être mentionnés peuvent être non substitués, partiellement ou totalement halogénés et/ou oxygénés et/ou peuvent porter 1 ou 2 radicaux choisis parmi C₁-C₄-alcoxy,
phényle, benzyle, pyridyle, phénoxy, où les quatre radicaux venant d'être mentionnés peuvent être non substitués, partiellement ou totalement halogénés et/ou peuvent porter 1, 2 ou 3 substituants choisis parmi C₁-C₆-alkyle, C₁-C₆-halogénoalkyle, C₁-C₆-alcoxy, C₁-C₆-halogénoalcoxy ou (C₁-C₆-alcoxy)carbonyle, ou
R^{17a} et R^{17b} peuvent être ensemble une chaîne C₂-C₆-alkylène formant un cycle de 3 à 7 chaînons saturé, partiellement saturé ou insaturé conjointement avec l'atome d'azote auquel R^{17a} et R^{17b} sont fixés, où la chaîne alkylène peut contenir 1 ou 2 hétéroatomes choisis parmi l'oxygène, le soufre ou l'azote, et peut éventuellement être substituée par halogène, C₁-C₄-halogénoalkyle, C₁-C₄-alcoxy ou C₁-C₄-halogénoalcoxy, et où le ou les atomes d'azote et/ou de soufre du cycle hétérocyclique peuvent éventuellement être oxydés ;
n est un nombre entier choisi indépendamment les uns des autres parmi 0, 1 ou 2 ;
et/ou un énantiomère, diastéréoisomère, isomère E/Z ou des sels acceptables sur le plan agricole ou vétérinaire de ceux-ci, pour le contrôle et/ou la lutte contre des animaux nuisibles.

2. Composés de formule (I) selon la revendication 1, dans lesquels
Het est choisi dans le groupe constitué par les radicaux de formule suivante Het-1 à Het-28 : 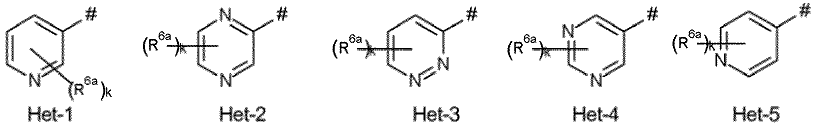 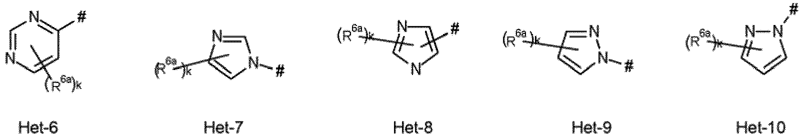 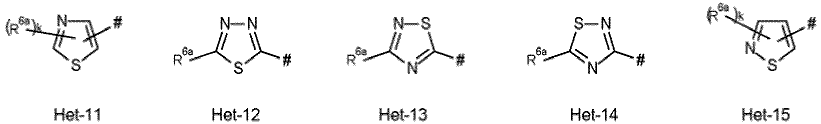 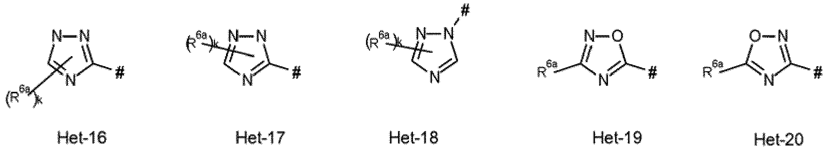 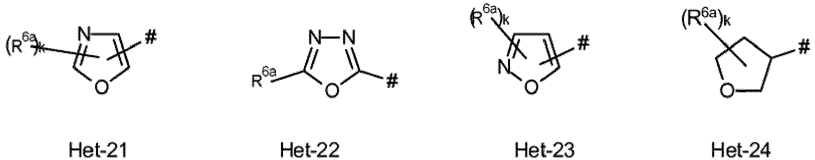 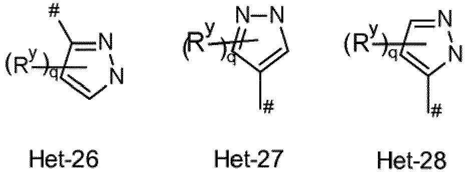 où # désigne la liaison dans la formule (I), et où
k vaut 0, 1 ou 2 ; et
R^{6a} est choisi chacun indépendamment les uns des autres dans le groupe constitué par hydrogène, halogène, cyano, C₁-C₆-alkyle, C₃-C₈-cycloalkyle, C₂-C₆-alcényle, C₂-C₆-alcynyle et où les atomes de carbone des radicaux aliphatiques et cycloaliphatiques susmentionnés peuvent éventuellement être davantage substitués indépendamment les uns des autres par un ou plusieurs R⁷, OR⁸, NR^{9a}R^{9b}, S(O)ₙR⁸, S(O)ₙNR^{9a}R^{9b}, C(=O)R⁷, C(=O)NR^{9a}R^{9b}, C(=O)OR⁸, C(=S)R⁷, C(=S)NR^{9a}R^{9b}, C(=NR^{9a})R⁷, C(=NR^{9a})NR^{9a}R^{9b}.

3. Composés de formule (I) selon la revendication 2, dans lesquels
Het est choisi dans le groupe constitué par les radicaux de formules Het-1, Het-11a et Het-24 : 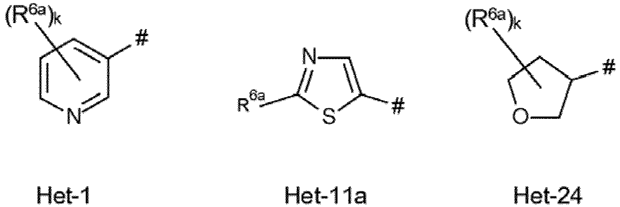 où # désigne la liaison dans la formule (I), et où R^{6a} est choisi parmi hydrogène, halogène, C₁-C₄-alcoxy ou C₁-C₄-alkyle, où les atomes de carbone de ces deux derniers radicaux peuvent être partiellement ou totalement halogénés ;
k vaut 0, 1 ou 2.

4. Composés de formule (I) selon l'une quelconque des revendications précédentes, dans lesquels R¹, R² sont choisis indépendamment l'un de l'autre dans le groupe constitué par hydrogène, halogène, CN, C₁-C₆-alkyle, C₃-C₆-cycloalkyle, C₁-C₆-halogénoalkyle, C₂-C₆-halogénocycloalkyle ; ou
R¹ et R² peuvent être ensemble =O, =CR¹³R¹⁴ ou =S ;
ou
R¹ et R² forment, conjointement avec l'atome de carbone auquel ils sont fixés, un cycle carbocyclique de 3 à 5 chaînons saturé ;
en particulier
R¹ et R² sont tous deux hydrogène, ou
R¹ est hydrogène et R² est CH₃.

5. Composés de formule (I) selon l'une quelconque des revendications précédentes, dans lesquels R^{3a}, R^{3b} sont choisis chacun indépendamment les uns des autres dans le groupe constitué par hydrogène, halogène, CN, C₁-C₆-alkyle, C₁-C₆-halogénoalkyle, C₁-C₆-alcoxy, C₁-C₆-halogénoalcoxy, C₁-C₆-halogénoalkylthio, C₁-C₆-alkylthio et OR⁸, où R⁸ est C(=O)R¹⁵, et R¹⁵ est C₁-C₆-alkyle, où le radical aliphatique peut être non substitué, partiellement ou totalement halogéné, en particulier
R^{3a}, R^{3b} sont choisis chacun indépendamment les uns des autres dans le groupe constitué par hydrogène, fluor, CN, C₁-C₆-alkyle et C₁-C₆-halogénoalkyle, ou
R^{3a}, R^{3b} forment, conjointement avec l'atome de carbone auquel ils sont fixés, un cycle cyclopropane, où chacun parmi les atomes de carbone du cycle peut être non substitué ou peut être partiellement ou totalement halogéné.

6. Composés de formule (I) selon l'une quelconque des revendications 1 à 5, dans lesquels
p vaut 0.

7. Composés de formule (I) selon l'une quelconque des revendications précédentes, dans lesquels Y est choisi parmi S, S=O ou S(O)₂.

8. Composés de formule (I) selon l'une quelconque des revendications précédentes, dans lesquels
R⁵ est choisi dans le groupe constitué par hydrogène, cyano, C₁-C₆-alkyle, C₇-C₁₀-alkyle, C₃-C₁₀-cycloalkyle, C₂-C₆-alcényle et C₂-C₆-alcynyle, où les atomes de la chaîne carbonée des quatre radicaux aliphatiques et cycloaliphatiques venant d'être mentionnés sont non substitués, partiellement ou complètement halogénés ou peuvent porter une combinaison quelconque d'un ou plusieurs radicaux R⁷, où R⁷ est choisi indépendamment les uns des autres parmi halogène, C₁-C₆-alkyle, C₁-C₆-halogénoalkyle, C₁-C₆-alcoxy, C₁-C₆-halogénoalcoxy ou C(=O)O-R¹⁶,
où R¹⁶ est indépendamment les uns des autres C₁-C₆-alkyle, où le radical alkyle peut être non substitué, partiellement ou totalement halogéné et/ou peut porter 1 ou 2 radicaux choisis parmi C₁-C₄-alcoxy ; phényle ou CH₂-phényle, éventuellement substitué par 1, 2 ou 3 substituants, qui sont choisis indépendamment les uns des autres parmi hydrogène, halogène, C₁-C₄-alkyle, C₁-C₄-halogénoalkyle, C₁-C₄-alcoxy, C₁-C₄-halogénoalcoxy ou phénoxy ;
un cycle hétérocyclique aromatique saturé, partiellement saturé ou insaturé de 5 ou 6 chaînons choisi parmi pyridine, furane, oxazole, oxadiazole, isoxazole, thiazole, thiadiazole ou isothiazole, où le cycle hétérocyclique peut être lié directement ou lié via un groupement CH₂ au reste de la molécule, où le cycle hétérocyclique est non substitué ou éventuellement substitué par un, deux ou trois substituants R^{y} choisis indépendamment les uns des autres, et où
R^{y} est choisi dans le groupe constitué par halogène, cyano et C₁-C₄-alkyle, où les atomes de carbone du radical alkyle peuvent être non substitués ou partiellement ou totalement halogénés et/ou peuvent porter 1,2 ou 3 radicaux choisis parmi C₁-C₄-alcoxy,
en particulier
R⁵ est choisi dans le groupe constitué par hydrogène, cyano, C₁-C₆-alkyle, C₃-C-cycloalkyle, C₂-C₆-alcényle et C₂-C₆-alcynyle, où chacun parmi les quatre radicaux aliphatiques venant d'être mentionnés est non substitué, partiellement ou complètement halogéné, ou
R⁵ est issu du groupe constitué par S-CN, C₁-C₄-alkylthio, C₃-C₆-cycloalkylthio, C₂-C₆-alcénylthio et C₂-C₆-alcynylthio, où chacun parmi les quatre radicaux aliphatiques venant d'être mentionnés est non substitué, partiellement ou complètement halogéné, ou R⁵ est phényle ou CH₂-phényle, où le cycle aromatique est éventuellement substitué par 1 ou 2 substituants, qui sont choisis indépendamment les uns des autres dans le groupe constitué par hydrogène, halogène, CN, C₁-C₄-alkyle, C₁-C₄-alcoxy, où les deux parmi les deux radicaux aliphatiques venant d'être mentionnés peuvent être non substitués ou partiellement ou complètement halogénés.

9. Composés de formule (I) selon l'une quelconque des revendications précédentes, dans lesquels
R⁵ est un cycle hétérocyclique aromatique de 5 ou 6 chaînons choisi dans le groupe constitué par 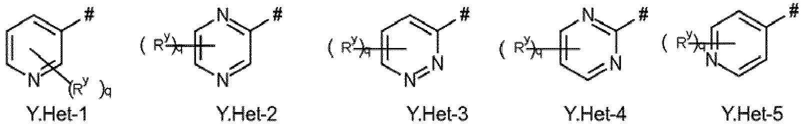 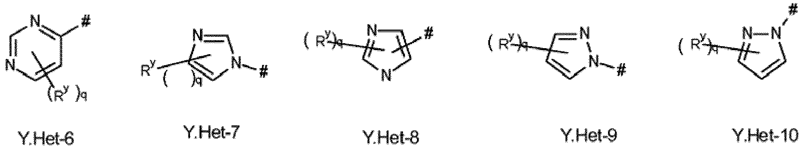 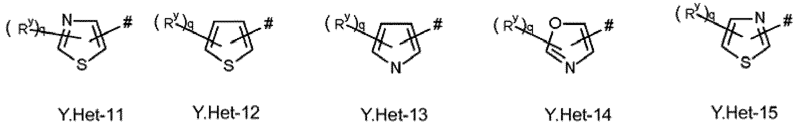 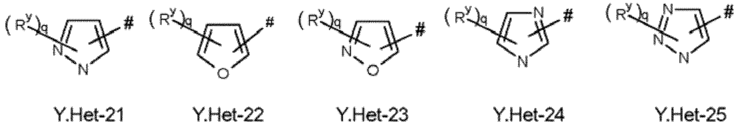 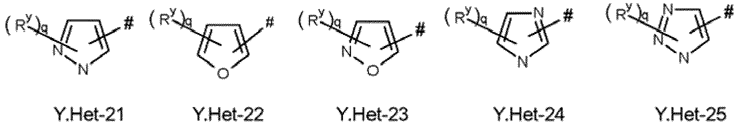 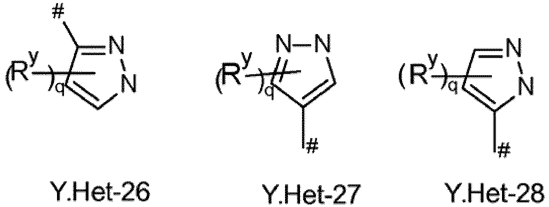 où # désigne la liaison le reste de la molécule, et
où la liaison peut être une liaison simple ou liée via un groupement CH₂ au reste de la molécule ; où
R^{y} est choisi indépendamment de la valeur de q et indépendamment les uns des autres, dans le groupe constitué par hydrogène, halogène, CN, C₁-C₄-alkyle et C₁-C₄-alcoxy, les deux parmi les deux radicaux aliphatiques venant d'être mentionnés pouvant être non substitués, partiellement ou complètement halogénés ; phényle, éventuellement substitué par halogène, cyano,
C₁-C₄-alkyle et C₁-C₄-alcoxy, les deux parmi les deux radicaux aliphatiques venant d'être mentionnés pouvant être non substitués, partiellement ou complètement halogénés ; ou
deux parmi R^{y} sur des atomes de carbone adjacents peuvent être un pont choisi parmi CH=CH-CH=CH ou CH=CHCH₂, et forment ainsi, conjointement avec les atomes de carbone auxquels les deux R^{y} sont fixés, un cycle carbocyclique aromatique condensé de 5 chaînons ou 6 chaînons, où ce cycle peut éventuellement être substitué par un ou deux substituants choisis parmi halogène, C₁-C₄-alcoxy, C₁-C₄-halogénoalcoxy, C₁-C₄-alkyle ou C₁-C₄-halogénoalkyle.

10. Composés de formule (I) selon l'une quelconque des revendications précédentes, dans lesquels
W¹, W², W³ et W⁴ représentent un groupement de chaîne carbonée relié à N et C=N, et formant ainsi un hétérocycle azoté saturé, insaturé ou partiellement insaturé de 6 chaînons, choisi parmi le groupe suivant constitué par W.Het-1, W.Het-2, W.Het-3, W.Het-4, W.Het-5, W.Het-6, W.Het-7, W.Het-8, W.Het-9, W.Het-10, W.Het-11 et W.Het-12 : 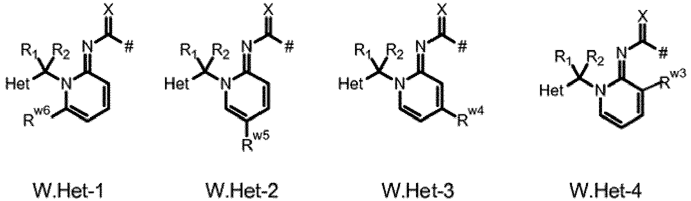 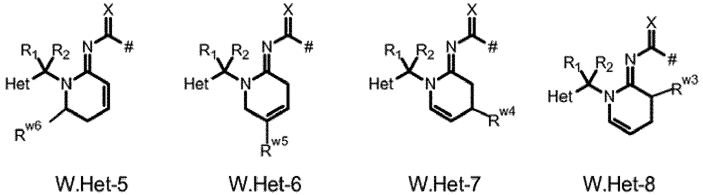 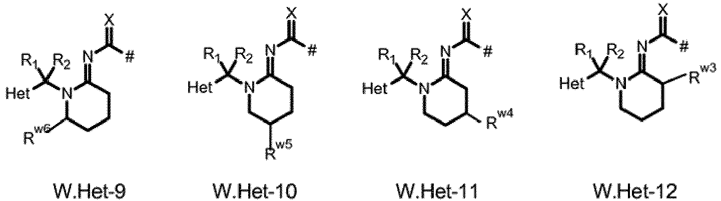 où # désigne la liaison au reste de la molécule ; R¹, R² et Het sont tels que définis selon la revendication 1, et où
R^{w3}, R^{w4}, R^{w5} et R^{w6} sont choisis parmi hydrogène, halogène, C₁-C₄-alcoxy et C₁-C₄-alkyle, les deux parmi les deux radicaux aliphatiques venant d'être mentionnés pouvant être non substitués, partiellement ou complètement halogénés.

11. Composés de formule (I) selon la revendication 1, 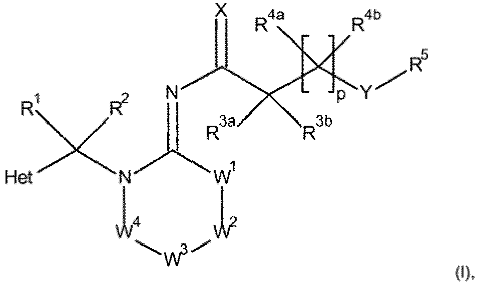 dans lesquels
W¹, W², W³ et W⁴ représentent un groupement de chaîne carbonée relié à N et C=N, et formant ainsi un hétérocycle azoté saturé, insaturé ou partiellement insaturé de 6 chaînons choisi parmi W.Het-1, W.Het-5 et W.Het-9 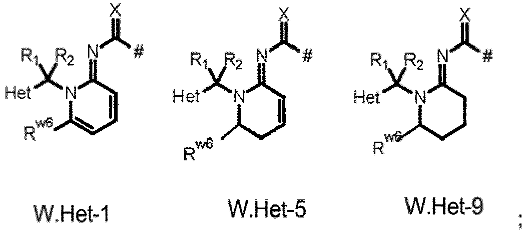 où # désigne la liaison au reste de la molécule,
R^{w6} est choisi parmi hydrogène, halogène, C₁-C₄-alkyle ; ou C₁-C₄-halogénoalkyle ; et où
Het est choisi dans le groupe constitué par les radicaux de formules Het-1, Het-11a et Het-24 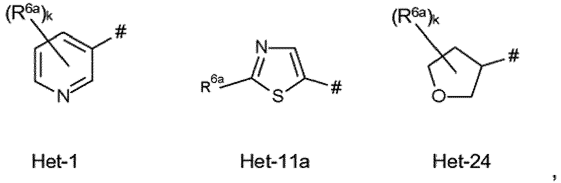 où # désigne la liaison dans la formule (I), et où
R^{6a} est choisi parmi hydrogène, halogène, C₁-C₄-alcoxy ou C₁-C₄-alkyle, où les atomes de carbone de ces deux derniers radicaux peuvent être partiellement ou totalement halogénés ; k vaut 0, 1 ou 2 ;
R¹, R² sont choisis indépendamment l'un de l'autre dans le groupe constitué par hydrogène, halogène, cyano, C₁-C₃-alkyle, ou C₁-C₃-halogénoalkyle ;
X est choisi parmi 0 ou S ;
et où
R^{3a}, R^{3b} sont choisis indépendamment l'un de l'autre parmi hydrogène, halogène, CN, C₁-C₆-alkyle, C₁-C₆-halogénoalkyle, ou où
R^{3a}, R^{3b} forment, conjointement avec l'atome de carbone auquel ils sont fixés, un cycle cyclopropane, où chacun parmi les atomes de carbone du cycle peut être non substitué ou peut être partiellement ou totalement halogéné ;
p vaut 0 ;
Y est S(O)ₘ, où m vaut 0, 1 ou 2 ; et
R⁵ est choisi dans le groupe constitué par hydrogène, halogène, CN, S-CN, C₁-C₆-alkyle, C₁-C₄-thioalkyle, C₃-C₆-cycloalkyle, C₃-C₆-cycloalkylthio, C₂-C₆-alcényle, C₂-C₆-alcénylthio, C₂-C₆-alcynyle ou C₂-C₆-alcynylthio, où chacun parmi les huit radicaux aliphatiques et cycloaliphatiques venant d'être mentionnés est non substitué, partiellement ou complètement halogéné.

12. Composés de formule (I) selon la revendication 11, dans lesquels
W¹, W², W³ et W⁴ représentent un groupement de chaîne carbonée relié à N et C=N, et formant ainsi un hétérocycle azoté insaturé de 6 chaînons W.Het-1 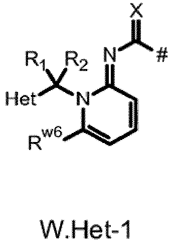 où # désigne la liaison au reste de la molécule,
R^{w6} est choisi parmi hydrogène, halogène, C₁-C₄-alkyle ou C₁-C₄-halogénoalkyle ;
où
Het est Het-1 ou Het-11a 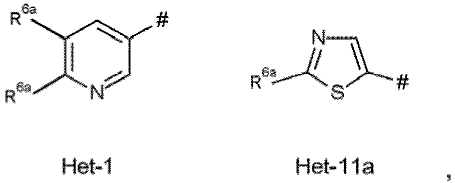 où # désigne la liaison dans la formule (I), et où
R^{6a} est choisi parmi hydrogène, halogène ou C₁-C₄-halogénoalkyle ;
R¹, R² sont tous deux hydrogène ;
X est choisi parmi 0 ou S,
et où
R^{3a}, R^{3b} sont choisis indépendamment l'un de l'autre parmi hydrogène ou fluor ;
p vaut 0 ;
Y est S(O)ₘ, où m vaut 0, 1 ou 2 ; et
R⁵ est C₁-C₃-alkyle, qui est non substitué ou partiellement ou complètement halogéné.

13. Composition agricole ou vétérinaire destinée à lutter contre des animaux nuisibles, comprenant au moins un composé tel que défini selon l'une quelconque des revendications 1 à 12, et au moins un véhicule acceptable liquide et/ou solide inerte, et éventuellement, si on le souhaite, au moins un agent tensioactif.

14. Méthode non thérapeutique de lutte ou de contrôle d'invertébrés nuisibles issus du groupe des insectes, des arachnides ou des nématodes, laquelle méthode comprend la mise en contact dudit nuisible ou de sa source d'alimentation, son habitat ou ses lieux de reproduction, avec une quantité efficace sur le plan pesticide d'au moins un composé tel que défini selon l'une quelconque des revendications 1 à 12.

15. Méthode de protection de plantes en croissance contre une attaque ou une infestation par des invertébrés nuisibles issus du groupe des insectes, des arachnides ou des nématodes, laquelle méthode comprend la mise en contact d'une plante, ou du sol ou de l'eau dans lesquels la plante se développe, avec une quantité efficace sur le plan pesticide d'au moins un composé tel que défini selon l'une quelconque des revendications 1 à 12.

16. Méthode de protection d'un matériel de propagation végétal, notamment de semences, contre des insectes du sol et des racines et pousses de plantules contre des insectes du sol et foliaires, comprenant la mise en contact du matériel de propagation végétal avant semis et/ou après prégermination, avec au moins un composé tel que défini selon l'une quelconque des revendications 1 à 12.
